# EUROPEAN PATENT APPLICATION

(11) **EP 4 306 546 A1**
(43) Date of publication of application: **17.01.2024**
(21) Application number: 22767267.2
(22) Date of filing: 11.03.2022
(51) Int. Cl.: C08B 37/00, A61K 47/61

(54) **GLYCAN, AND METHOD FOR PRODUCING MEDICINE CONTAINING GLYCAN**

(30) Priority: 12.03.2021 JP 2021040164
(71) Applicant: Daiichi Sankyo Company, Limited, Tokyo 103-8426 (JP)
(72) Inventor: UEDA, Tsuyoshi, Tokyo 103-8426 (JP); SUZUKI, Keisuke, Tokyo 103-8426 (JP); ITOH, Ryusei, Tokyo 103-8426 (JP)
(74) Representative: Marks & Clerk LLP
(86) International application number: PCT/JP2022/010851
(87) International publication number: WO 2022/191313

(57) **Abstract**

The present invention addresses the problem of providing: a novel method for pure-chemically producing a biantennary N-glycan with an α2,6-sialic acid structure at each non-reducing end, the method having excellent yield, selectivity, and efficiency; and a method for producing a novel monosaccharide or oligosaccharide, which are useful in the method, and, for instance, intermediates as well as a novel method for producing the glycoprotein or the like by using the method. Provided are: a novel method for pure-chemically producing a biantennary N-glycan with an α2,6-sialic acid structure at each non-reducing end such that for glycosidic linkage, the direct construction of which is difficult using adjacent group involvement, disaccharide blocks are individually constructed and are stereoselectively linked to another sugar block in this synthesis scheme; and a method for producing a novel monosaccharide or oligosaccharide, which are useful in the method, and, for instance, intermediates as well as a novel method for producing the glycoprotein or the like by using the method.

## Description

### Technical Field

The present invention relates to a novel method for pure-chemically producing a biantennary N-glycan with an α2,6-sialic acid structure at each non-reducing end. The present invention also relates to a method for producing a novel monosaccharide or oligosaccharide, which are useful in the method for pure-chemically producing the N-glycan, and intermediates as well as a novel method for producing the N-glycan by including the method of them. The present invention further relates to a novel method for producing, for instance, a glycoprotein (in particular, a glycan-remodeled antibody or a molecule containing an Fc region thereof, or an antibody-drug conjugate) by using the novel method for pure-chemically producing the N-glycan.

### Background Art

Protein glycosylation is known to significantly affect the function and structure of the protein. N-linked glycans, in particular, are deeply involved in physiological activities of proteins. Among them, a biantennary N-glycan with an α2,6-sialic acid structure at each non-reducing end has been reported to be an optimal structure so as to increase antibody-dependent cellular cytotoxic activity (ADCC activity) and complement-dependent cytotoxic activity (CDC activity) (Non-Patent Literature 1).

For the development and commercialization of pharmaceuticals using glycans, it is desirable to be able to stably produce large quantities of highly pure glycans at an industrially applicable price. The synthesis of α2,6-sialyl glycan reported so far can be roughly classified into two methods: 1) semi-chemical synthesis by natural extract purification and enzymatic chemical conversion and 2) pure chemical synthesis.

Examples of the semi-chemical synthesis reported include a method in which an enzymatic process and a chemical process are combined to obtain an N-linked glycan from the yolks of chicken eggs (Non-Patent Literature 2). These processes can be used to synthesize a target glycan with fewer steps than pure chemical synthesis. On the other hand, a large amount of egg yolks must be provided. In many cases, special techniques and purification equipment are required for the subsequent isolated purification from egg yolks and purification of water-soluble unprotected glycans after chemical conversion (Patent Literatures 1 to 4).

Meanwhile, examples of the α2,6-sialyl glycan pure-chemical synthesis include the following report cases:
(1) total synthesis of α2,6-sialyl moiety-containing complex glycan with 11 sugars (Non-Patent Literature 3);
(2) total synthesis of α2,6-sialyl moiety-containing immunoglobulin G peptide with 13 sugars (Non-Patent Literature 4);
(3) total synthesis of core fucose-containing α2,6-sialyl N-linked glycan with 12 sugars (Non-Patent Literature 5); and
(4) total synthesis of position 3-fluoridated α2,6-sialyl N-linked glycan with 10 sugars (Non-Patent Literature 6).

In the case that a robust production method for glycan by pure chemical synthesis is established, the flexibility of the production scale is considered to be extremely high as well as normal low molecular weight compounds by deriving it from monosaccharides. Also, in the synthesis, protecting group-modified sugars are converted and most of the purification operation is conducted for non-aqueous compounds. Therefore, the complexity of the operation and the number of operation steps should be significantly less than those for the semi-chemical synthesis.

Meanwhile, in the past reported cases described above, 1) In the construction of difficult-to-synthesize sugar parts such as β-mannoside and α-sialyl moieties and their linkage process, several steps with low selectivity and low yield are present; and 2) in any of the sugar-part conversion or the linkage process, silica gel column chromatography purification, which is not suitable for scale-up, is frequently used, so that a precision chromatographic preparation and a purification operation are essential in many steps to remove isomers and impurities generated as byproducts in the reaction, the above two points are listed as big issues on the synthesis.

As described above, the pure chemical synthesis of glycan has potential advantages over the semi-chemical synthesis at the perspective of the mass synthesis. However, it is difficult to be deemed that the technology is sufficiently advanced in terms of yield, selectivity, efficiency, and cost. There are very few cases where the pure chemical synthesis has been adopted as an actual mass synthesis method.

### Citation List

### Patent Literature

Patent Literature 1: WO 2011/027868
Patent Literature 2: WO 96/02255
Patent Literature 3: WO 2014/208742
Patent Literature 4: WO 2017/110984

### Non-Patent Literature

Non-Patent Literature 1: Proc. Natl. Acad. Sci. U.S.A.2015, 112, 10611
Non-Patent Literature 2: Beilstein J. Org. Chem. 2018, 14, 416
Non-Patent Literature 3: TetrahedronLett. 1986, 27, 5739
Non-Patent Literature 4: J. Am. Chem. Soc. 2009, 131, 16669
Non-Patent Literature 5: J. Am. Chem. Soc. 2019, 141, 6484

### Summary of Invention

### Technical Problem

One of the problems of the present invention is to provide a novel method for pure-chemically producing a biantennary N-glycan with an a2,6-sialic acid structure at each non-reducing end, the method having excellent yield, selectivity, and efficiency. Another problem of the present invention is to provide a method for producing a novel monosaccharide or oligosaccharide, which are useful in the method for pure-chemically producing the N-glycan, and intermediates as well as a novel method for producing the N-glycan by including the method of them. Still another problem of the present invention is to provide a novel method for producing, for instance, a glycoprotein (in particular, a glycan-remodeled antibody or a molecule containing an Fc region thereof, or an antibody-drug conjugate) by using the novel method for pure-chemically producing the N-glycan.

### Solution to Problem

The present inventors have conducted intensive research to solve the above problems, and, as a result, have found that for glycosidic linkage which is difficult to directly construct by using adjacent group involvement, disaccharide blocks are individually constructed and are stereoselectively linked to another sugar block; and this synthesis scheme is used for a novel method for pure-chemically producing a biantennary N-glycan with an a2,6-sialic acid structure at each non-reducing end. The present inventors have further found a method for producing a novel monosaccharide or oligosaccharide, which are useful in the method for pure-chemically producing the N-glycan, and intermediates as well as a novel method for producing the N-glycan by including the method of them, and a novel method for producing, for instance, a glycoprotein (in particular, a glycan-remodeled antibody or a molecule containing an Fc region thereof, or an antibody-drug conjugate) by using the novel method for pure-chemically producing the N-glycan. In this way, the present invention has been completed.

Specifically, the present invention pertains to the following items, but is not limited to them.
[1] A method for producing an oligosaccharide represented by the following formula XI: 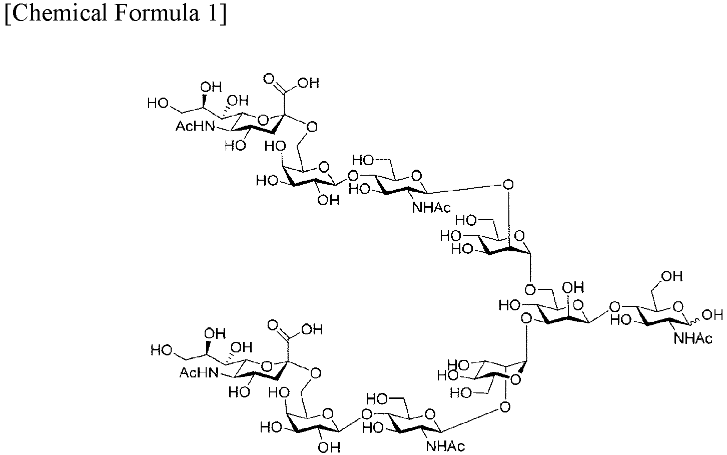 the method comprising steps of:
   (step 1) producing a compound represented by the following formula III-11: 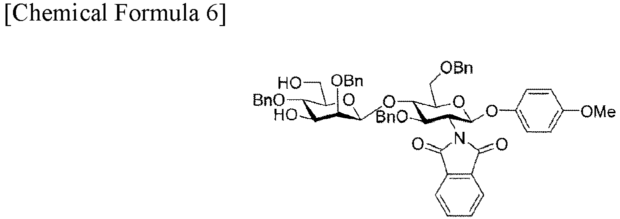
   or a compound represented by the following formula III-13: 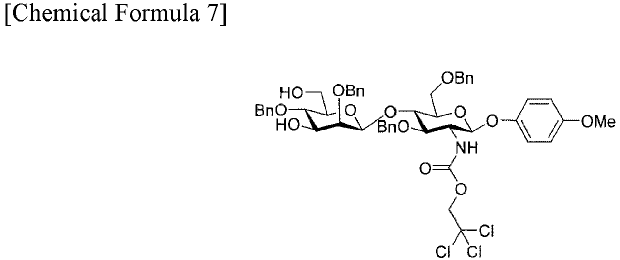
      comprising a step of
      reacting a compound represented by the following formula III-3: 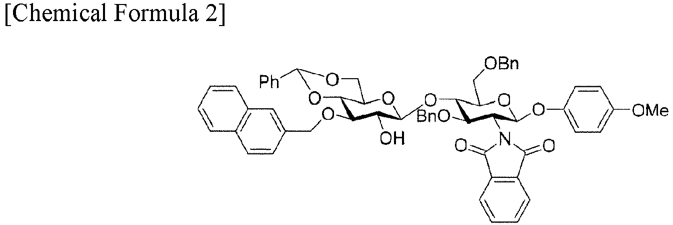
      with a compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group to give a compound represented by the following formula III-4: 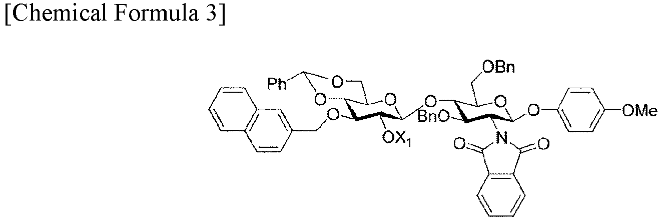
      wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group, and
      then reacting the compound represented by formula III-4 with cesium acetate or tetrabutylammonium acetate to give a compound represented by the following formula III-5: 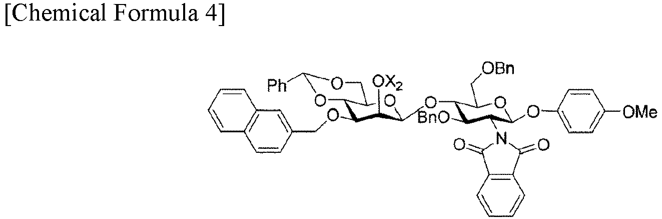
      wherein X₂ is an acetyl group,
      or reacting the compound represented by formula III-4 with tetrabutylammonium benzoate to give a compound represented by the following formula III-5: 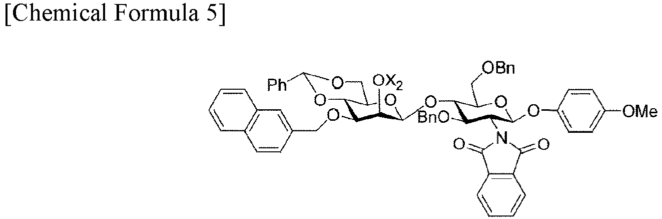
      wherein X₂ is a benzoyl group;
   (step 2) producing a compound represented by the following formula V-3: 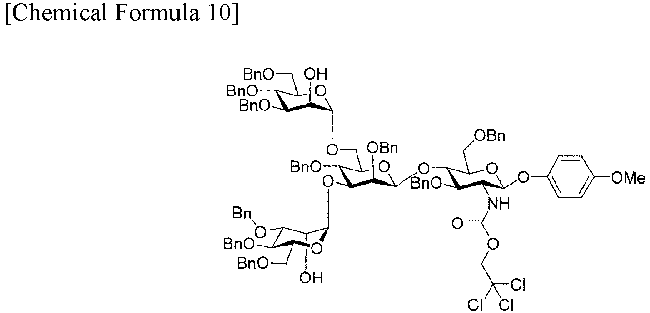
      comprising a step of
      reacting the compound represented by formula III-13 with a compound represented by the following formula IV-3: 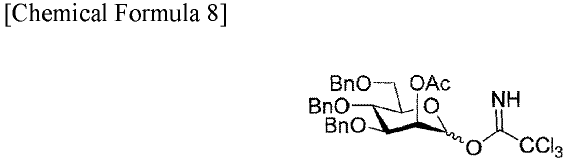
      to give a compound represented by the following formula V-1: 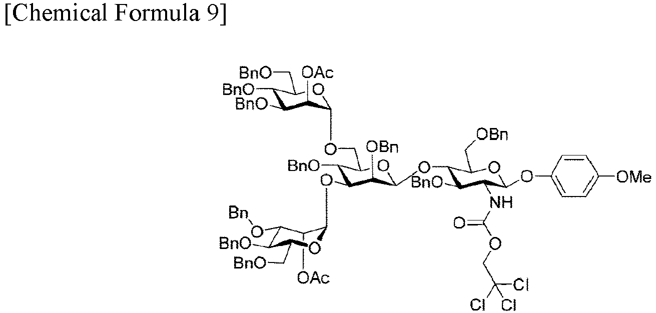
   or producing a compound represented by the following formula V-5: 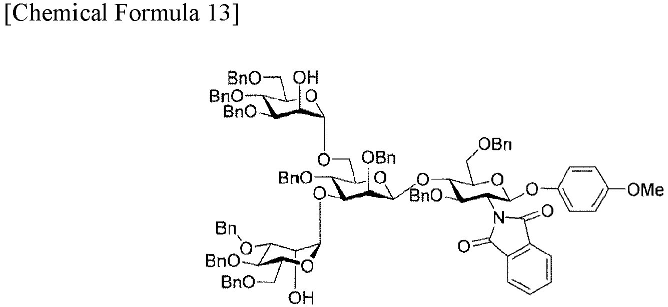
      comprising a step of
      reacting the compound represented by formula III-11 with a compound represented by the following formula IV-3: 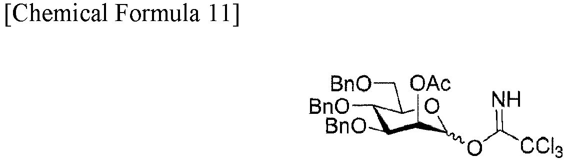
      to give a compound represented by the following formula V-4: 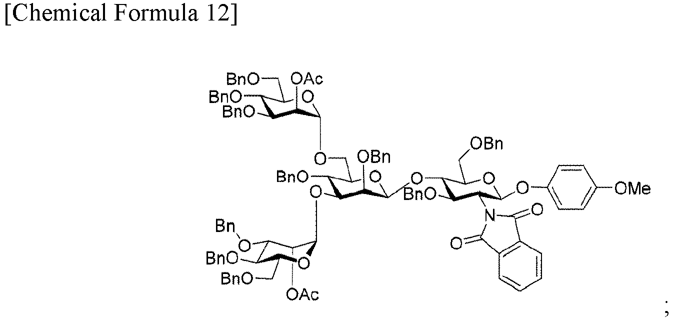
   (step 3) producing a compound represented by the following formula VI-3: 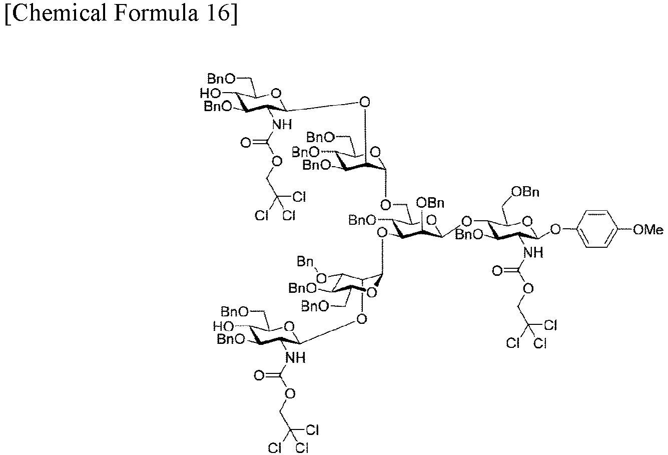
      comprising a step of
      reacting the compound represented by formula V-3 with a compound represented by the following formula II-6: 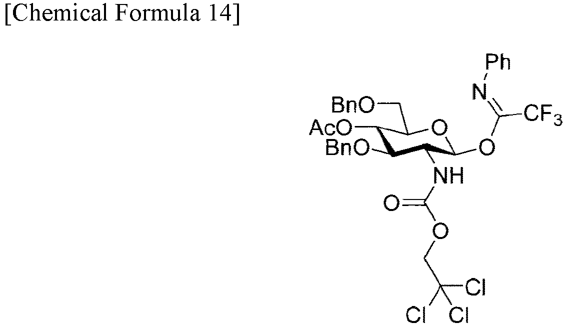
      to give a compound represented by the following formula VI-1: 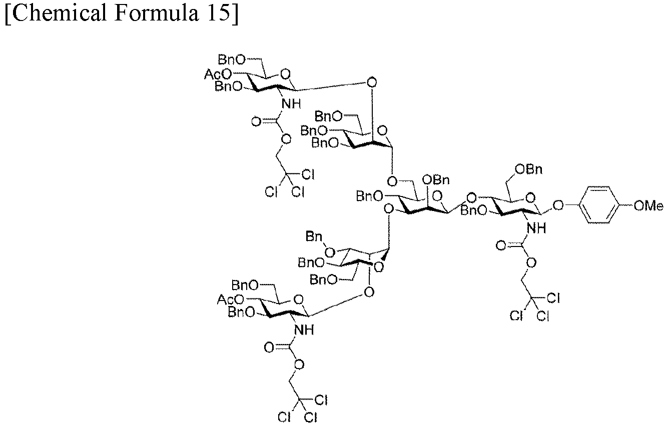
   or producing a compound represented by the following formula VI-3: 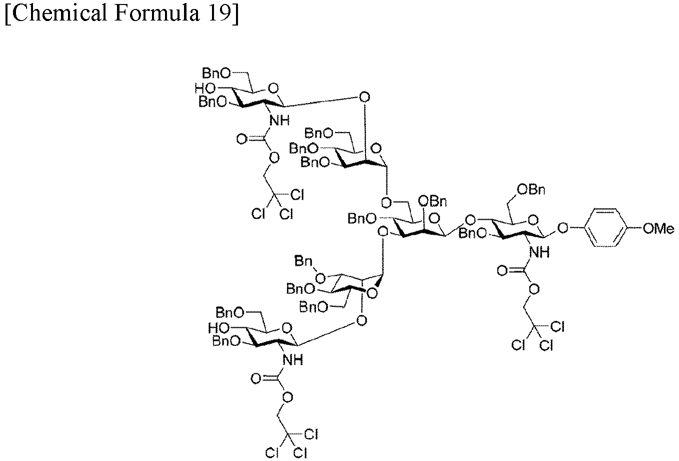
   or a compound represented by the following formula VI-6: 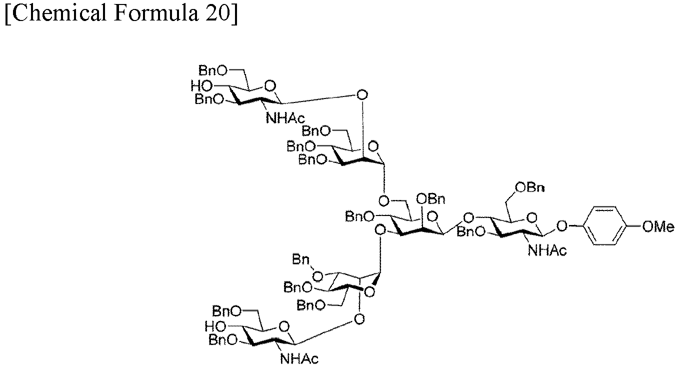
      comprising a step of
      reacting the compound represented by formula V-5 with a compound represented by the following formula II-9: 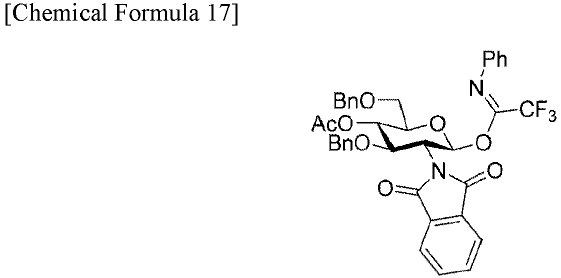
      to give a compound represented by the following formula VI-4: 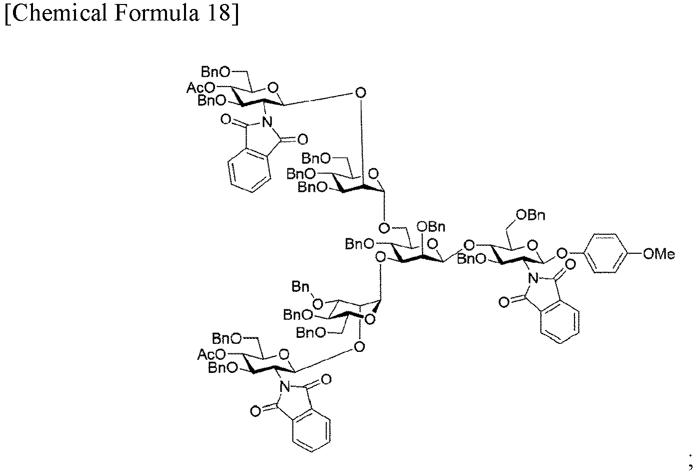
   (step 4) producing a compound represented by the following formula IX-5: 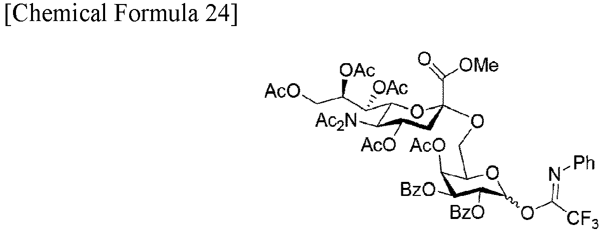
      comprising a step of
      subjecting a compound represented by the following formula VIII-5: 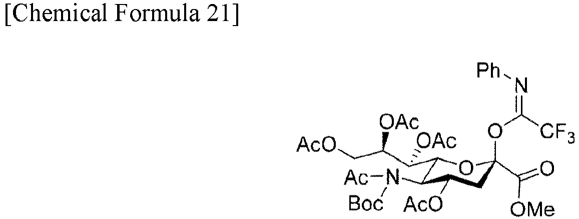
      and a compound represented by the following formula VII-3: 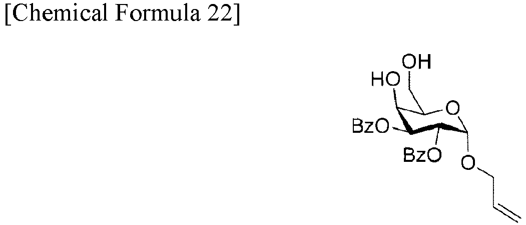
      to α-2,6-glycosidic linkage to give a compound represented by the following formula IX-1: 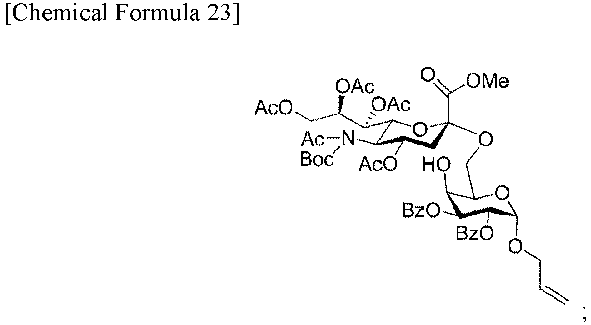 and
   (step 5) producing the oligosaccharide represented by formula XI
      comprising a step of
      reacting the compound represented by formula VI-3 with the compound represented by formula IX-5 to give a compound represented by the following formula X-1: 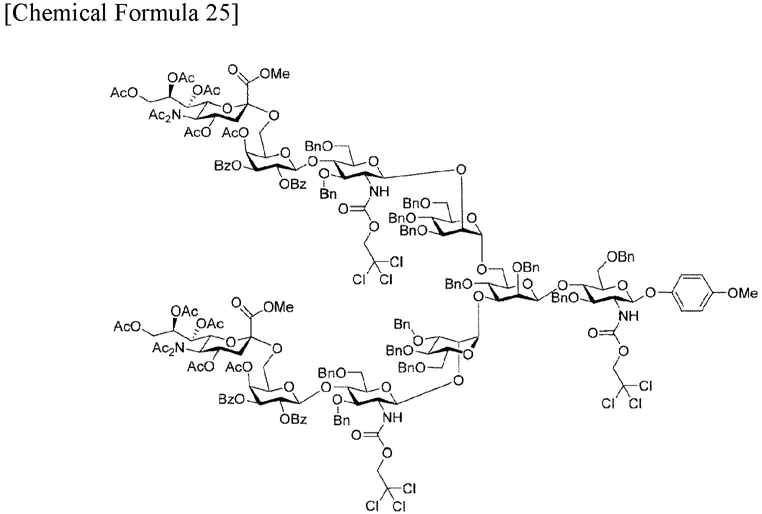
      or reacting the compound represented by formula VI-6 with the compound represented by formula IX-5 to give a compound represented by the following formula X-3: 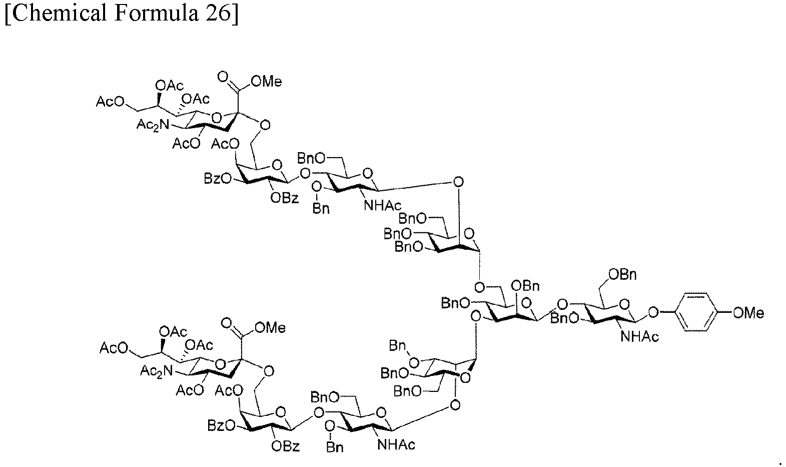
[2] The method according to [1], wherein step 1 comprises a step of
   reacting a compound represented by the following formula III-1: 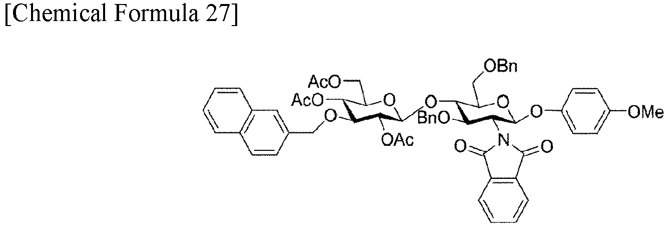
   with an alkoxide-based strong base in the presence of methyl trifluoroacetate to give a compound represented by the following formula III-2: 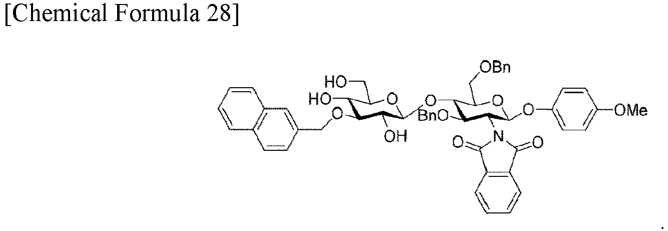
[3] The method according to [2], wherein the alkoxide-based strong base is selected from the group consisting of a sodium salt, lithium salt, and potassium salt of C1-C5 alkoxide, and a combination thereof.
[4] A method for producing an oligosaccharide represented by the following formula XI: 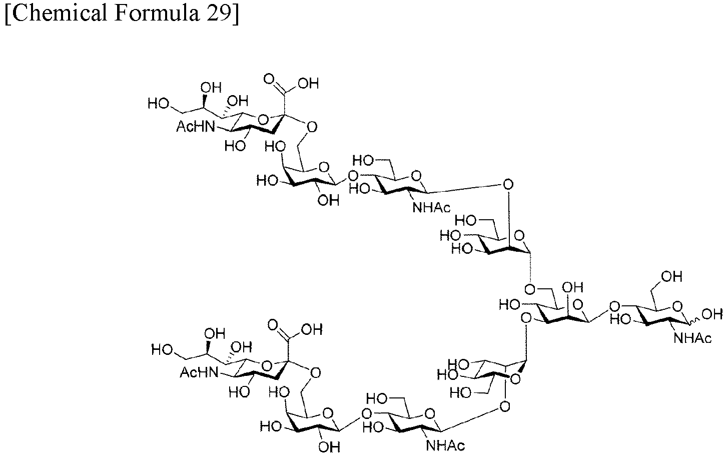 the method comprising steps of:
   (step 1) producing a compound represented by the following formula III-11: 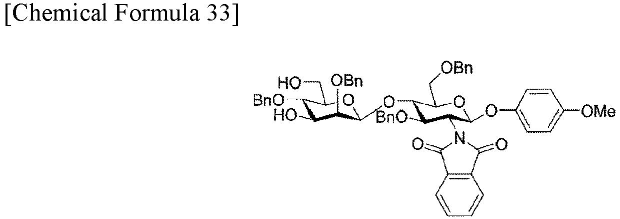
   or a compound represented by the following formula III-13: 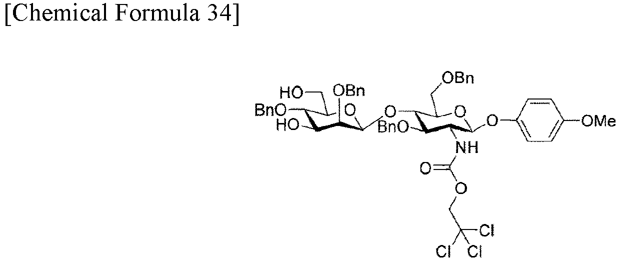
      comprising a step of
      oxidizing position 2 of D-glucopyranoside in a compound represented by the following formula III-3: 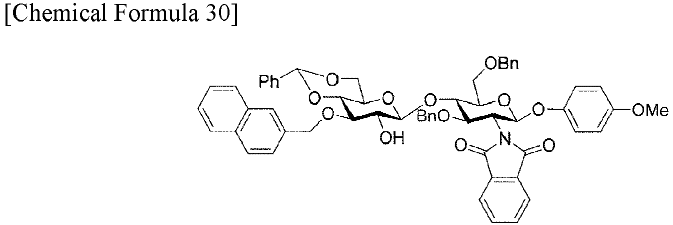
      to give a compound represented by the following formula III-7: 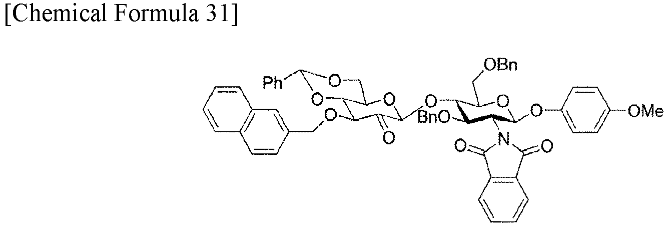
      then reducing the oxo group attached to the carbon at position 2 of 2-keto-D-glucopyranoside in the compound represented by formula III-7 to give a compound represented by the following formula III-8: 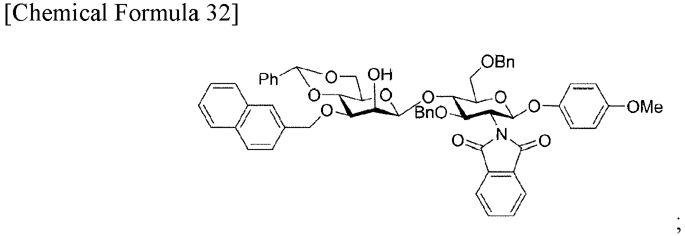
   (step 2) producing a compound represented by the following formula V-3: 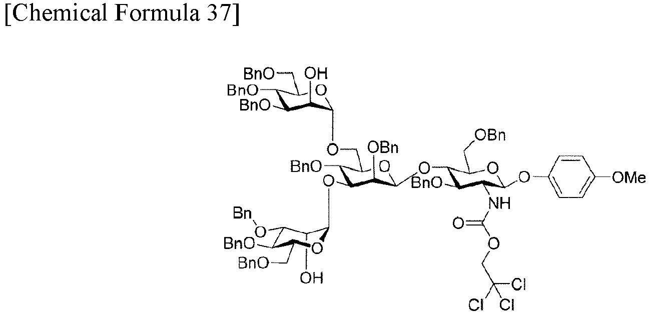
      comprising a step of
      reacting the compound represented by formula III-13 with a compound represented by the following formula IV-3: 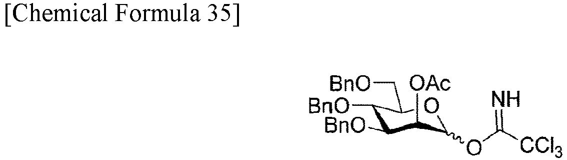
      to give a compound represented by the following formula V-1: 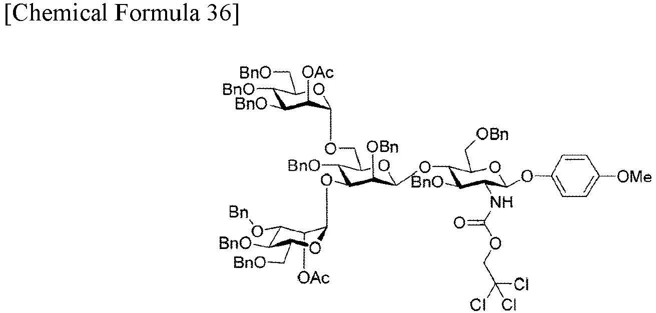
   or producing a compound represented by the following formula V-5: 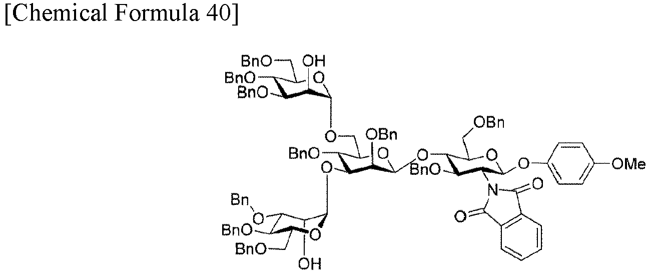
      comprising a step of
      reacting the compound represented by formula III-11 with a compound represented by the following formula IV-3: 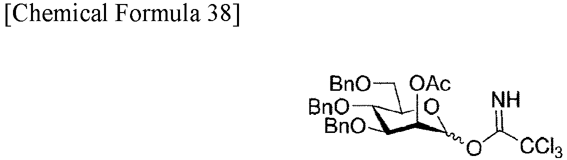
      to give a compound represented by the following formula V-4: 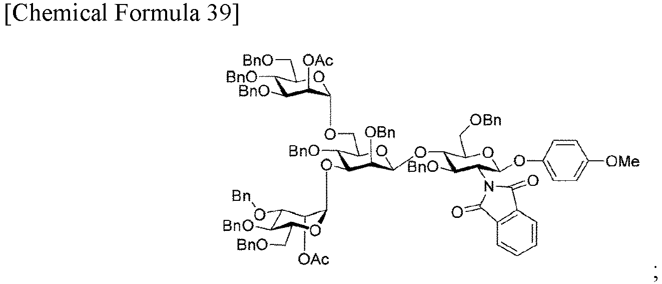
   (step 3) producing a compound represented by the following formula VI-3: 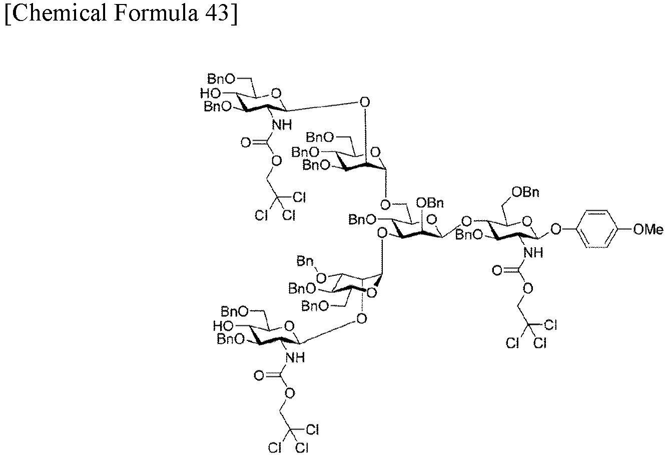
      comprising a step of
      reacting the compound represented by formula V-3 with a compound represented by the following formula II-6: 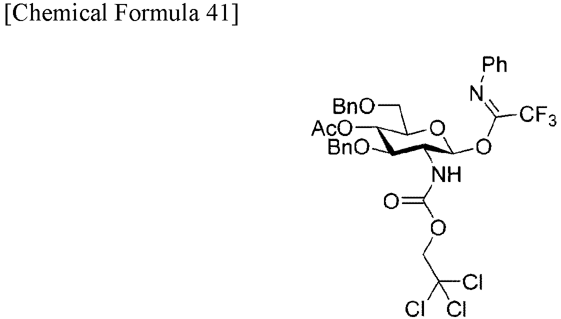
      to give a compound represented by the following formula VI-1: 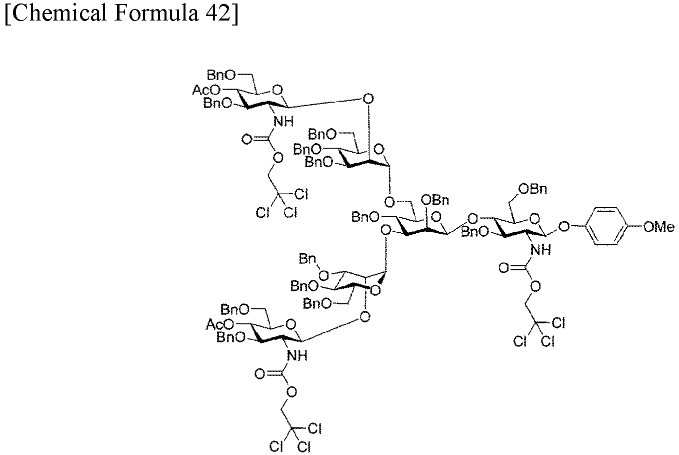
   or producing a compound represented by the following formula VI-3: 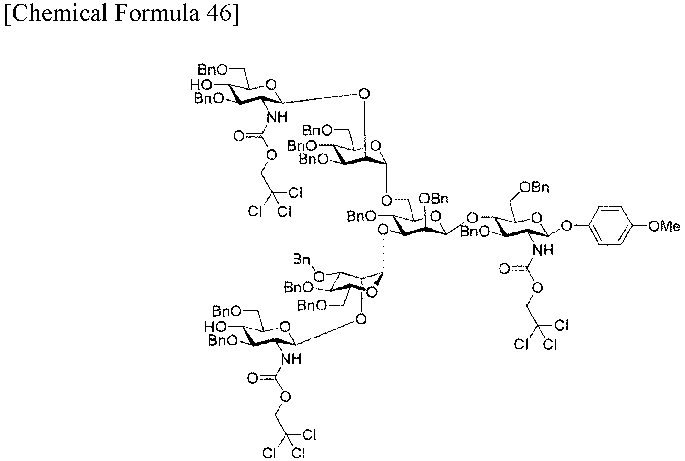
   or a compound represented by the following formula VI-6: 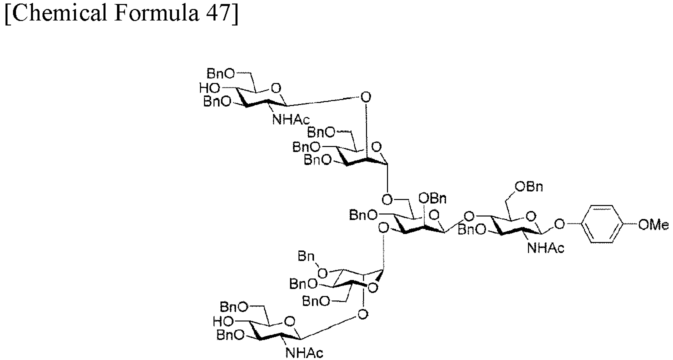
      comprising a step of
      reacting the compound represented by formula V-5 with a compound represented by the following formula II-9: 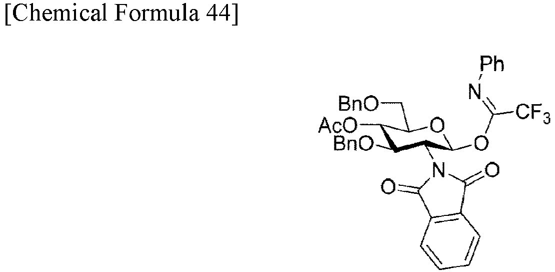
      to give a compound represented by the following formula VI-4: 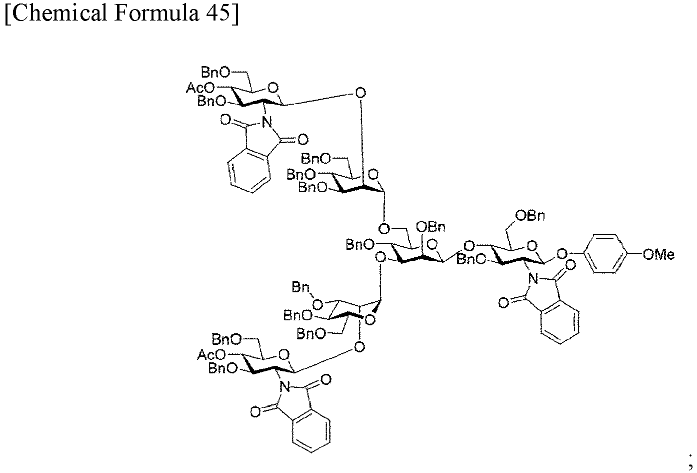
   (step 4) producing a compound represented by the following formula IX-5: 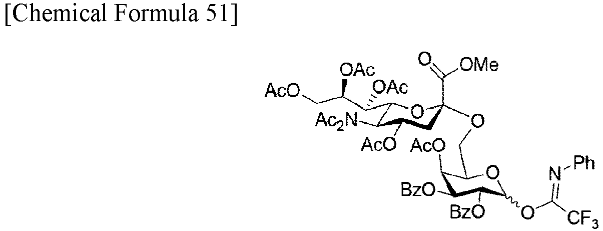
      comprising a step of
      subjecting a compound represented by the following formula VIII-5: 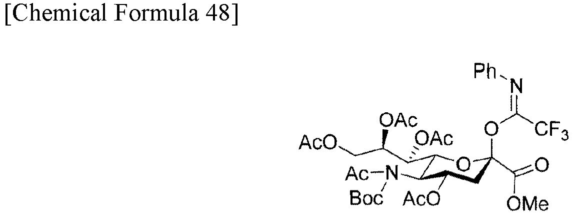
      and a compound represented by the following formula VII-3: 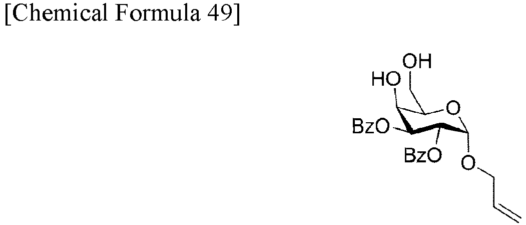
      to α-2,6-glycosidic linkage to give a compound represented by the following formula IX-1: 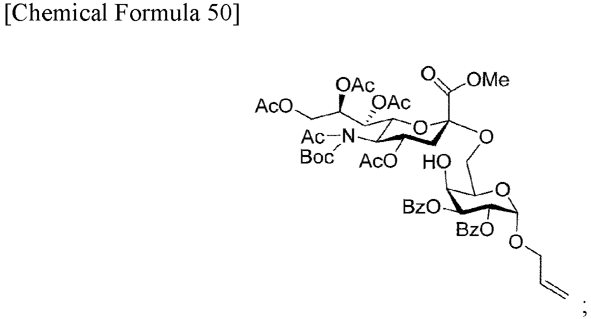 and
   (step 5) producing the oligosaccharide represented by the formula XI
      comprising a step of
      reacting the compound represented by formula VI-3 with the compound represented by formula IX-5 to give a compound represented by the following formula X-1: 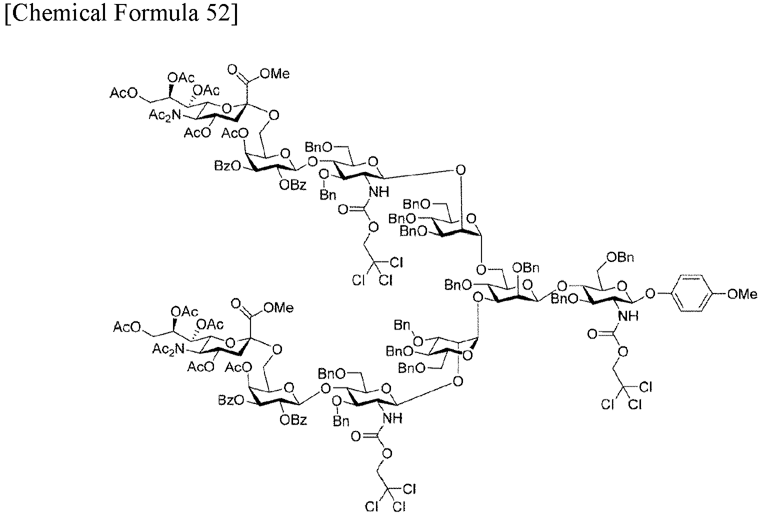
      or reacting the compound represented by formula VI-6 with the compound represented by formula IX-5 to give a compound represented by the following formula X-3: 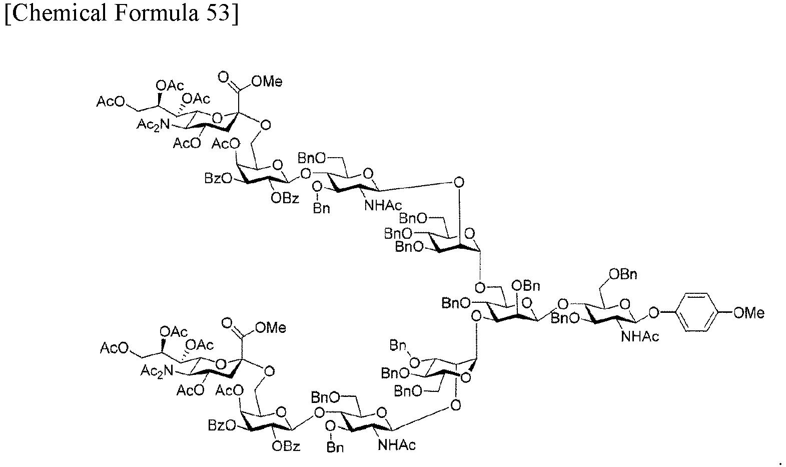
[5] The method according to [4], wherein step 1 comprises a step of
   reacting a compound represented by the following formula III-1: 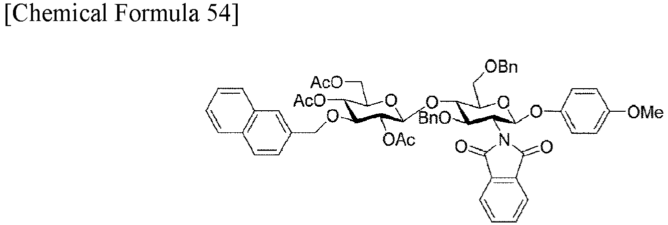
   with an alkoxide-based strong base in the presence of methyl trifluoroacetate to give a compound represented by the following formula III-2: 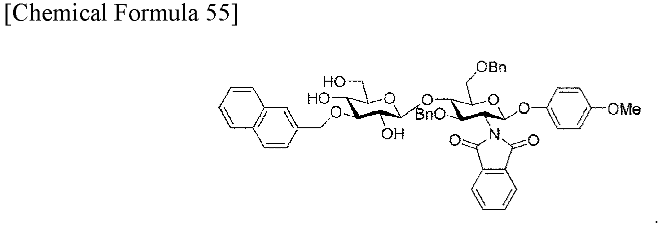
[6] The method according to [5], wherein the alkoxide-based strong base is selected from the group consisting of a sodium salt, lithium salt, and potassium salt of C1-C5 alkoxide, and a combination thereof.
[7] The method according to any one of [4] to [6], wherein in step 1, the compound represented by formula III-7 is reduced in the presence of a reducing agent selected from the group consisting of L-selectride; LS-selectride; lithium diisobutyl-tert-butoxyaluminum hydride (LDBBA); a compound represented by the following formula A: 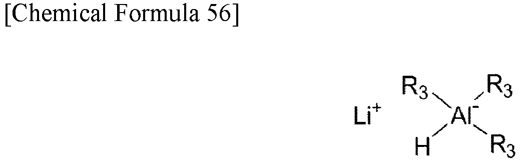
   wherein R₃ is a di-tert-butylmethylphenoxide as shown in the following formula: 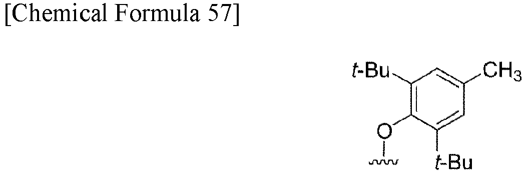
   or hydride, provided that at least two R₃ are each di-tert-butylmethylphenoxide; and a combination thereof.
[8] The method according to any one of [4] to [7], wherein in step 1, the compound represented by formula III-7 is reduced in the presence of a reducing agent that is a compound represented by the following formula A: 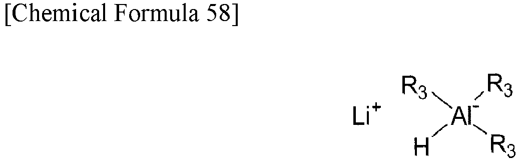
   wherein R₃ is a di-tert-butylmethylphenoxide as shown in the following formula: 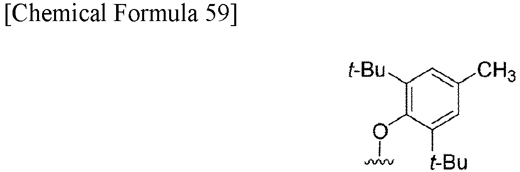
   or hydride, provided that at least two R₃ are each di-tert-butylmethylphenoxide.
[9] The method according to any one of [1] to [8], wherein step 1 further comprises a step of
   producing a compound represented by the following formula III-9: 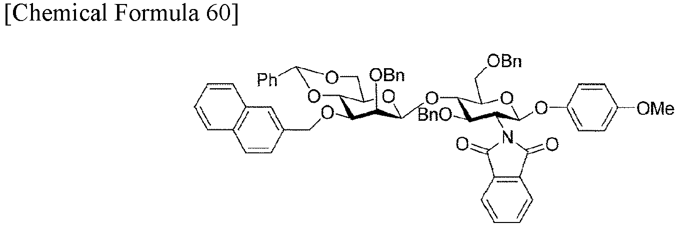
   and the step of producing the compound represented by formula III-9 comprises a step of
      protecting, in the presence of lithium tert-butoxide or lithium tert-amoxide, a hydroxyl group attached to the carbon at position 2 of D-mannopyranoside in a compound represented by the following formula III-8: 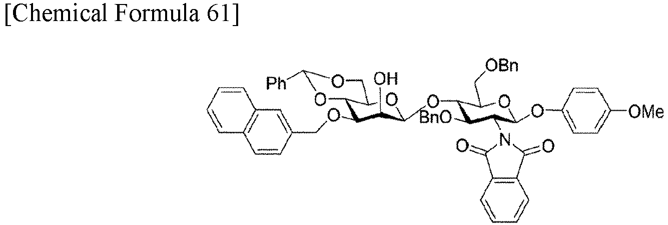
      by a benzyl group to give the compound represented by formula III-9.
[10] The method according to any one of [1] to [9], wherein step 2 further comprises a step of
   producing a compound represented by the following formula V-5: 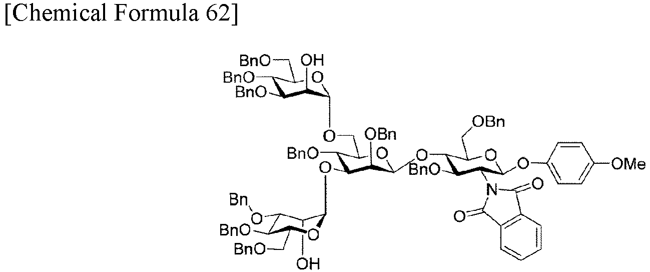
   and the step of producing the compound represented by formula V-5 comprises
   a step of reacting, in the presence of methyl trifluoroacetate, a compound represented by the following formula V-4: 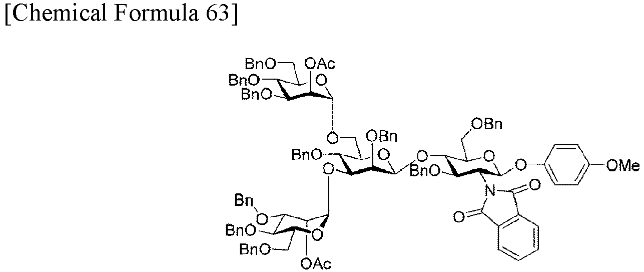
   with an alkoxide-based strong base to give the compound represented by formula V-5.
[11] The method according to [10], wherein the alkoxide-based strong base is selected from the group consisting of a sodium salt, lithium salt, and potassium salt of C1-C5 alkoxide, and a combination thereof.
[12] The method according to any one of [1] to [11], wherein step 3 further comprises a step of
   producing the compound represented by formula II-6 or the compound represented by formula II-9,
   and the step of producing the compound represented by formula II-6 comprises a step of
   reacting, in fluorous alcohol and water, a compound represented by the following formula II-4: 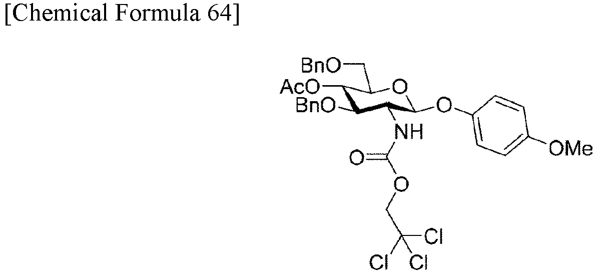
   with λ3-iοdane to give a compound represented by the following formula II-5 : 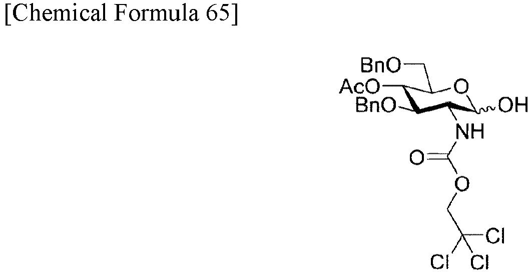
   and the step of producing the compound represented by formula II-9 comprises a step of
   reacting, in fluorous alcohol and water, a compound represented by the following formula II-7: 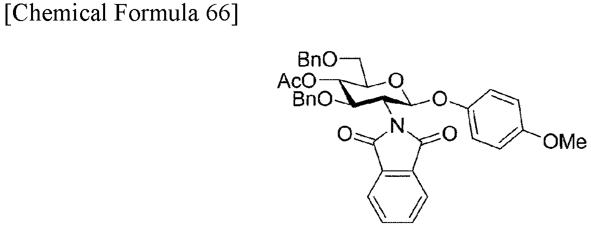
   with λ3-iοdane to give a compound represented by the following formula II-8: 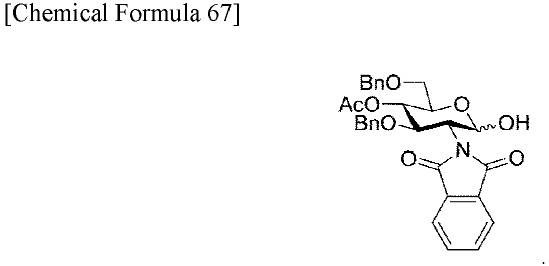
[13] The method according to [12], wherein the λ3-iodane is selected from the group consisting of [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, [hydroxy(methanesulfonyloxy)iodo]benzene, and a combination thereof.
[14] The method according to [12] or [13], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.
[15] The method according any one of [1] to [14], wherein step 3 further comprises a step of
   producing the compound represented by formula II-6 or the compound represented by formula II-9,
   and the step of producing the compound represented by formula II-6 comprises a step of
   reacting, in the presence of N-methylimidazole, a compound represented by the following formula II-5: 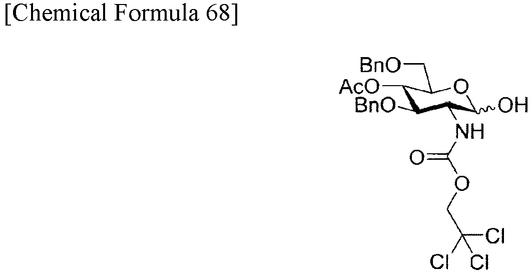
   with 2,2,2-trifluoro-N-phenylacetimidoyl chloride (TFPC) represented by the following formula: 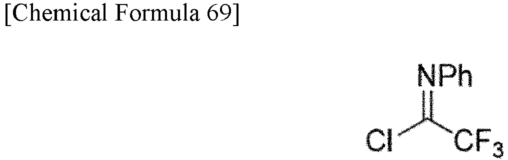
   to give the compound represented by formula II-6,
   and the step of producing the compound represented by formula II-9 comprises a step of
   reacting, in the presence of N-methylimidazole, a compound represented by the following formula II-8: 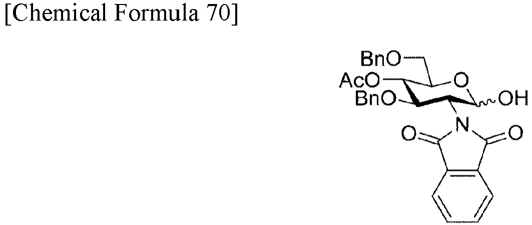
   with 2,2,2-trifluoro-N-phenylacetimidoyl chloride (TFPC) represented by the following formula: 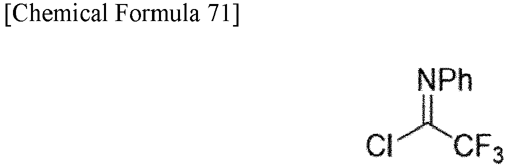
   to give the compound represented by formula II-9.
[16] The method according to any one of [1] to [15], wherein step 3 further comprises a step of
   producing a compound represented by the following formula VI-5: 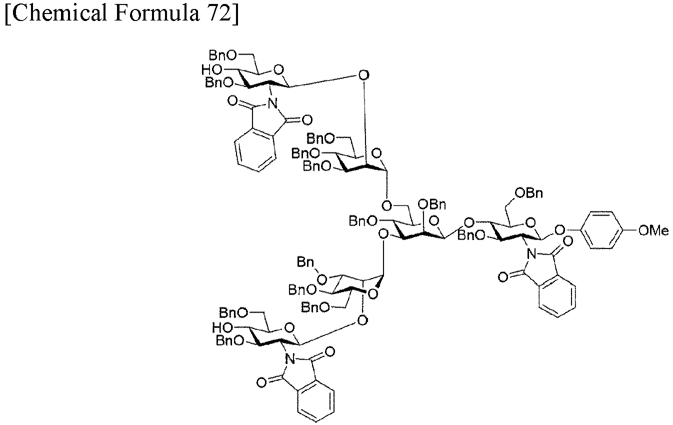
   and the step of producing the compound represented by formula VI-5 comprises a step of
   reacting, in the presence of methyl trifluoroacetate, a compound represented by the following formula VI-4: 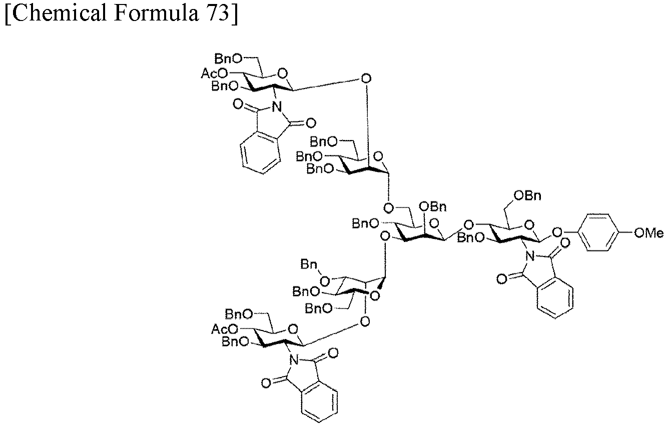
   with an alkoxide-based strong base to give the compound represented by formula VI-5.
[17] The method according to [16], wherein the alkoxide-based strong base is selected from the group consisting of a sodium salt, lithium salt, and potassium salt of C1-C5 alkoxide, and a combination thereof.
[18] The method according to any one of [1] to [17], wherein step 4 further comprises a step of
   producing the compound represented by formula VII-3,
   and the step of producing the compound represented by formula VII-3 comprises a step of
   bringing a solvent dissolving the compound represented by formula VII-3 into contact with silica gel to perform solid-phase extraction of the compound represented by formula VII-3.
[19] The method according to [18], wherein the solvent dissolving the compound represented by formula VII-3 is selected from the group consisting of toluene, dichloromethane, chloroform, and a combination thereof.
[20] The method according to any one of [1] to [19], wherein step 4 comprises a step of
   reacting, in the presence of N-methylimidazole, a compound represented by the following formula VIII-3: 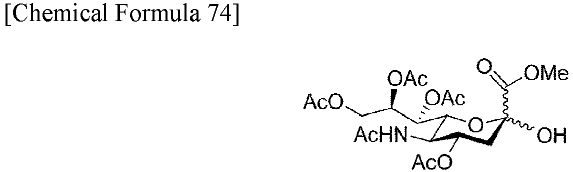
   with 2,2,2-trifluoro-N-phenylacetimidoyl chloride (TFPC) represented by the following formula: 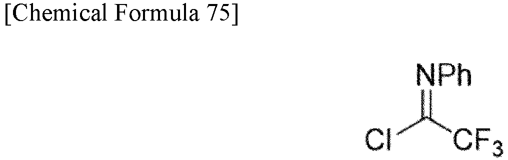
   to give a compound represented by the following formula VIII-4: 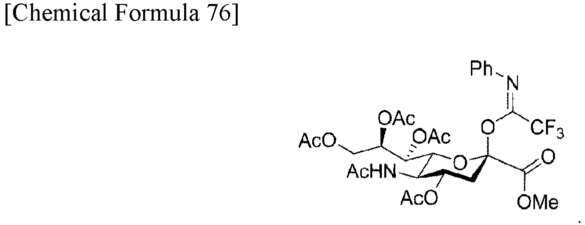
[21] The method according to any one of [1] to [20], wherein step 4 further comprises a step of
   producing a compound represented by the following formula IX-3: 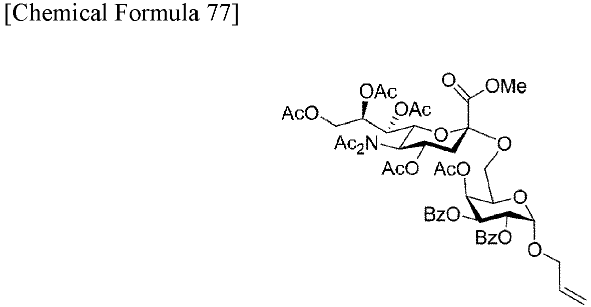
   and the step of producing the compound represented by formula IX-3 comprises a step of
   bringing a solvent dissolving the compound represented by formula IX-3 into contact with silica gel to perform solid-phase extraction of the compound represented by formula IX-3.
[22] The method according to [21], wherein the solvent dissolving the compound represented by formula IX-3 is selected from the group consisting of toluene, dichloromethane, chloroform, and a combination thereof.
[23] A method for producing a compound represented by the following formula III-2: 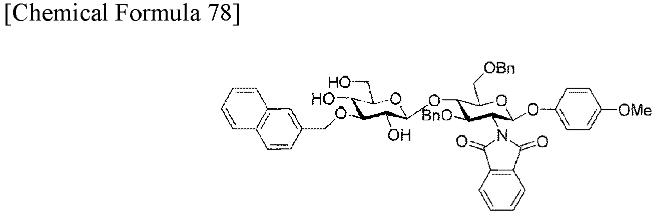 the method comprising a step of
   reacting, in the presence of methyl trifluoroacetate, a compound represented by the following formula III-1: 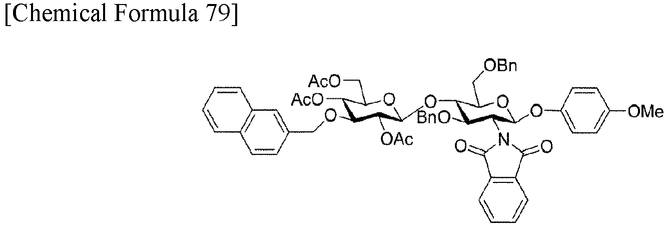
   with an alkoxide-based strong base to give the compound represented by formula III-2.
[24] The method according to [23], wherein the alkoxide-based strong base is selected from the group consisting of a sodium salt, lithium salt, and potassium salt of C1-C5 alkoxide, and a combination thereof.
[25] A method for producing a compound represented by formula III-5: 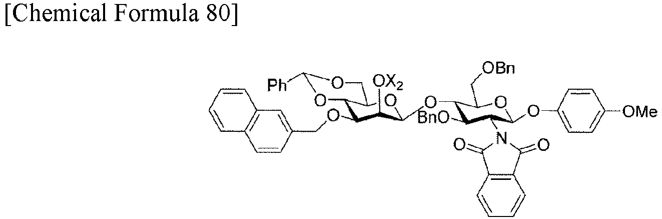
   wherein X₂ is an acetyl group
      the method comprising a step of
   reacting a compound represented by the following formula III-4: 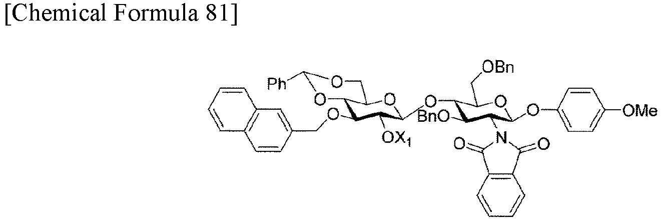
   wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group,
   with cesium acetate or tetrabutylammonium acetate to give the compound represented by formula III-5.
[26] A method for producing a compound represented by formula III-5: 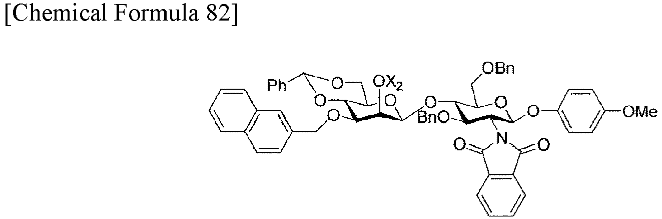
   wherein X₂ is a benzoyl group
      the method comprising a step of
   reacting a compound represented by the following formula III-4: 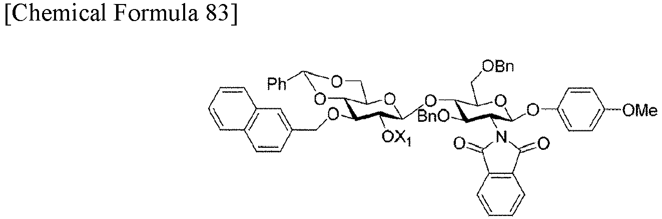
   wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group,
   with tetrabutylammonium benzoate to give the compound represented by formula III-5.
[27] A method for producing a compound represented by the following formula III-8: 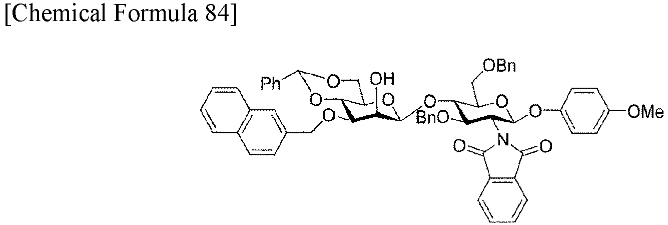 the method comprising a step of
   reducing an oxo group attached to the carbon at position 2 of 2-keto-D-glucopyranoside in a compound represented by the following formula III-7: 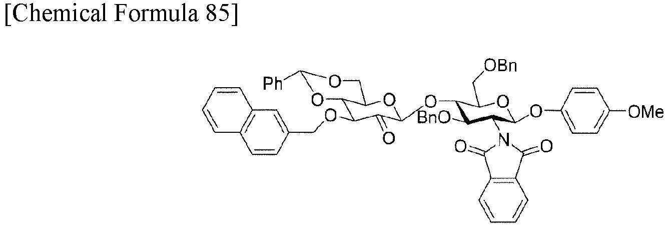
   to give the compound represented by formula III-8.
[28] The method according to [27], wherein the oxo group attached to the carbon at position 2 of 2-keto-D-glucopyranoside in the compound represented by formula III-7 is reduced in the presence of a reducing agent selected from the group consisting of L-selectride; LS-selectride; lithium diisobutyl-tert-butoxyaluminum hydride (LDBBA); a compound represented by the following formula A: 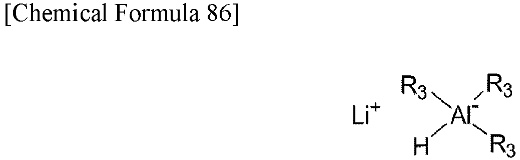
   wherein R₃ is a di-tert-butylmethylphenoxide as shown in the following formula: 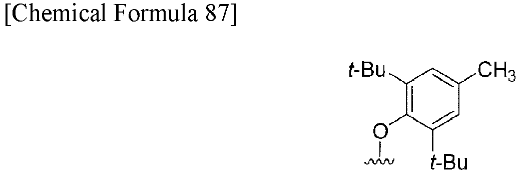
   ,or hydride provided that at least two R₃ are each di-tert-butylmethylphenoxide; and a combination thereof.
[29] The method according to [27] or [28], wherein the oxo group attached to the carbon at position 2 of 2-keto-D-glucopyranoside in the compound represented by formula III-7 is reduced in the presence of a reducing agent that is a compound represented by the following formula A: 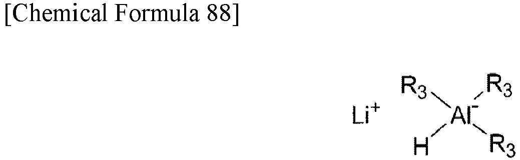
   wherein R₃ is a di-tert-butylmethylphenoxide as shown in the following formula: 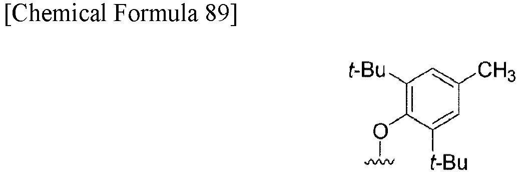
   or hydride, provided that at least two R₃ are each di-tert-butylmethylphenoxide.
[30] A method for producing a compound represented by the following formula III-9: 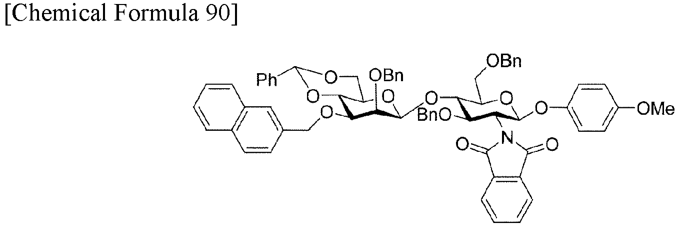 the method comprising a step of
   protecting, in the presence of lithium tert-butoxide or lithium tert-amoxide, a hydroxyl group attached to the carbon at position 2 of D-mannopyranoside in a compound represented by the following formula III-8: 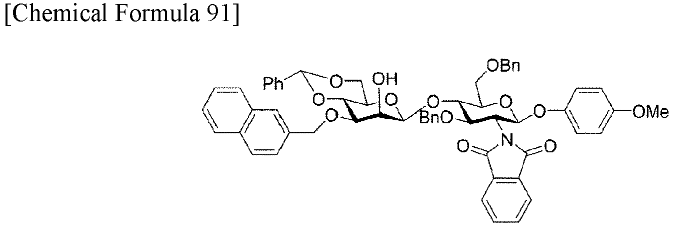
   by a benzyl group to give the compound represented by formula III-9.
[31] A method for producing a compound represented by the following formula V-5: 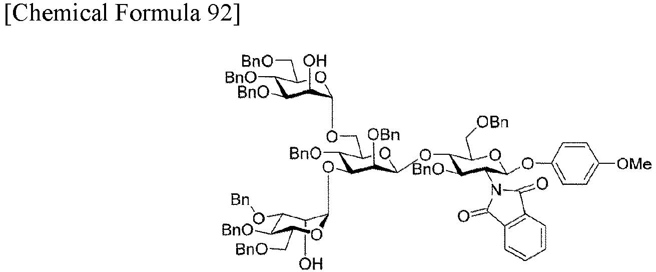 the method comprising a step of
   reacting, in the presence of methyl trifluoroacetate, a compound represented by the following formula V-4: 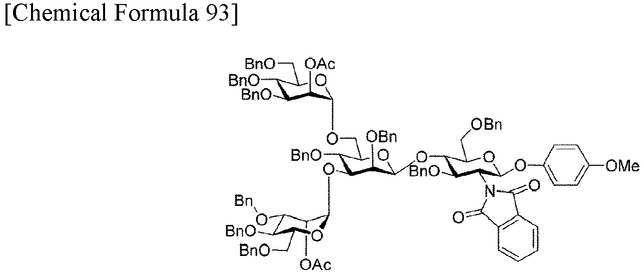
   with an alkoxide-based strong base to give the compound represented by formula V-5.
[32] The method according to [31], wherein the alkoxide-based strong base is selected from the group consisting of a sodium salt, lithium salt, and potassium salt of C1-C5 alkoxide, and a combination thereof.
[33] A method for producing a compound represented by the following formula II-5: 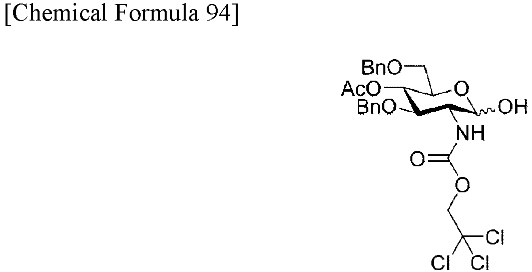
   or a compound represented by the following formula II-8: 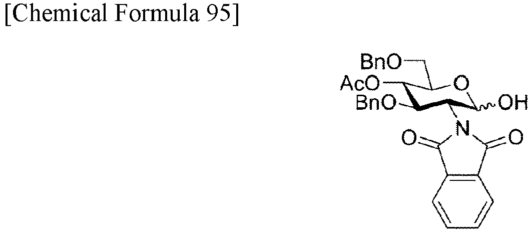
   the method comprising a step of
      reacting, in fluorous alcohol and water, a compound represented by the following formula II-4: 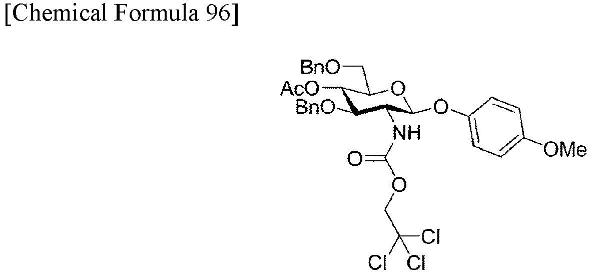
      or a compound represented by the following formula II-7: 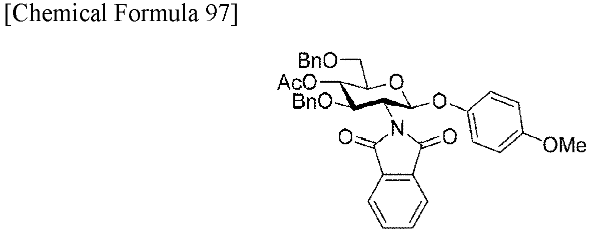
      with λ3-iοdane to give the compound represented by formula II-5 or the compound represented by formula II-8.
[34] The method according to [33], wherein the λ3-iodane is selected from the group consisting of [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, [hydroxy(methanesulfonyloxy)iodo]benzene, and a combination thereof.
[35] The method according to [33] or [34], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.
[36] A method for producing a compound represented by the following formula II-6: 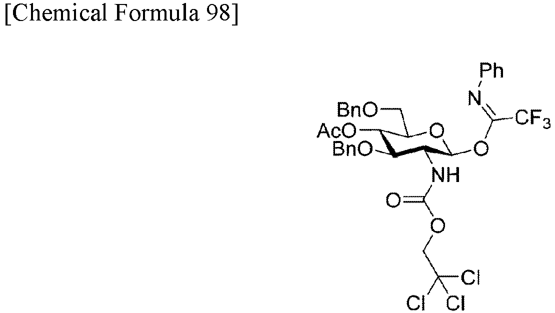
   or a compound represented by the following formula II-9: 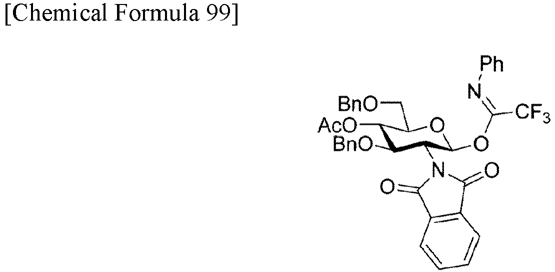
   the method comprising a step of
      reacting, in the presence of N-methylimidazole, a compound represented by the following formula II-5: 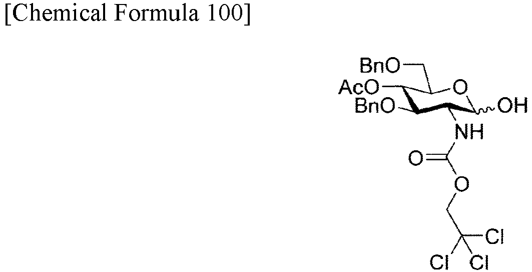
      or a compound represented by the following formula II-8: 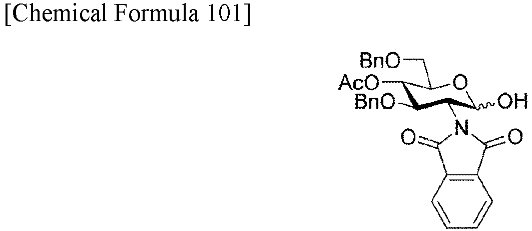
      with 2,2,2-trifluoro-N-phenylacetimidoyl chloride (TFPC) represented by the following formula: 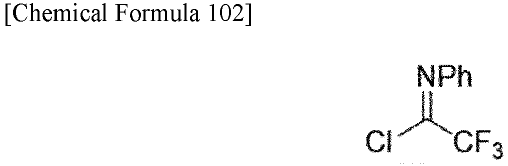
      to give the compound represented by formula II-6 or the compound represented by formula II-9.
[37] A method for producing a compound represented by the following formula VI-5: 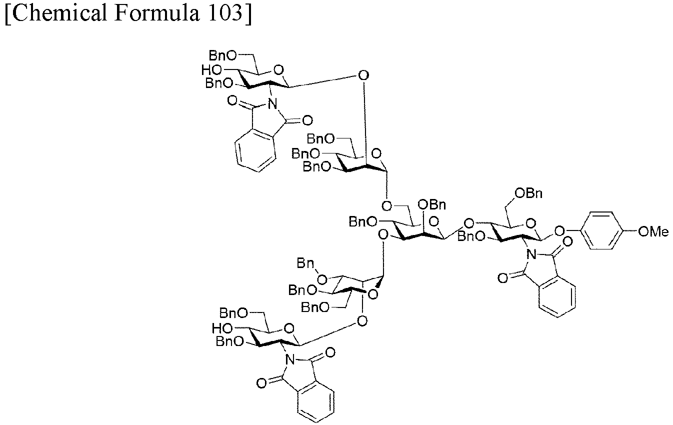 the method comprising a step of
   reacting, in the presence of methyl trifluoroacetate, a compound represented by the following formula VI-4: 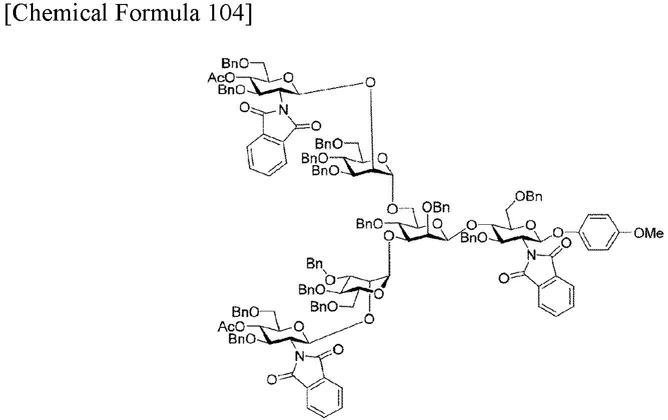
   with an alkoxide-based strong base to give the compound represented by formula VI-5.
[38] The method according to [37], wherein the alkoxide-based strong base is selected from the group consisting of a sodium salt, lithium salt, and potassium salt of C1-C5 alkoxide, and a combination thereof.
[39] A method for producing a compound represented by the following formula VII-3 : 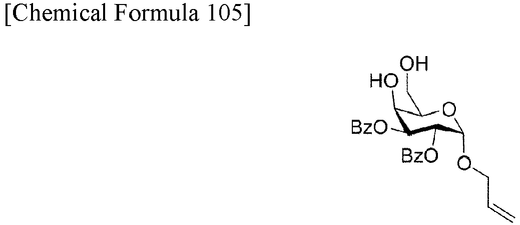 the method comprising a step of
   bringing a solvent dissolving the compound represented by formula VII-3 into contact with silica gel to perform solid-phase extraction of the compound represented by formula VII-3.
[40] A method for producing a compound represented by the following formula VIII-4: 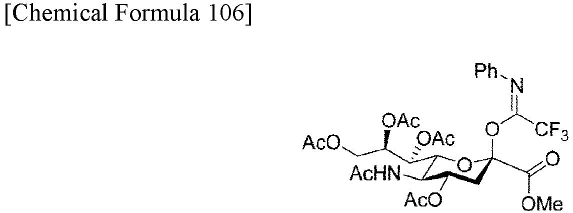 the method comprising a step of
   reacting, in the presence of N-methylimidazole, a compound represented by the following formula VIII-3: 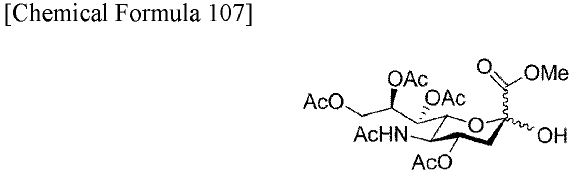
   with 2,2,2-trifluoro-N-phenylacetimidoyl chloride (TFPC) represented by the following formula: 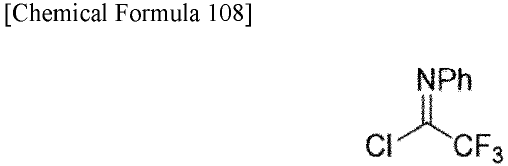
   to give the compound represented by formula VIII-4.
[41] A method for producing a compound represented by the following formula IX-1: 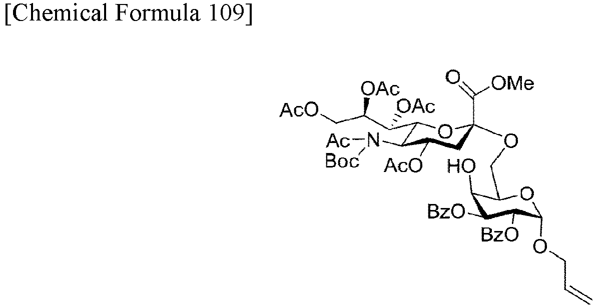 the method comprising a step of
   subjecting a compound represented by the following formula VIII-5: 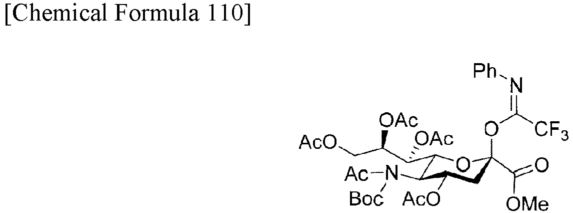
   and a compound represented by the following formula VII-3: 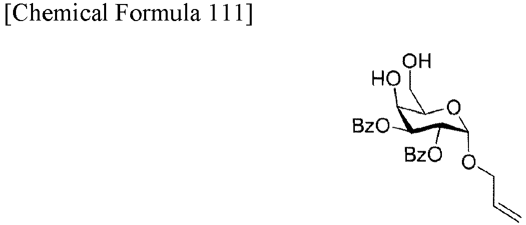
   to α-2,6-glycosidic linkage to give the compound represented by formula IX-1.
[42] A method for producing a compound represented by the following formula IX-1: 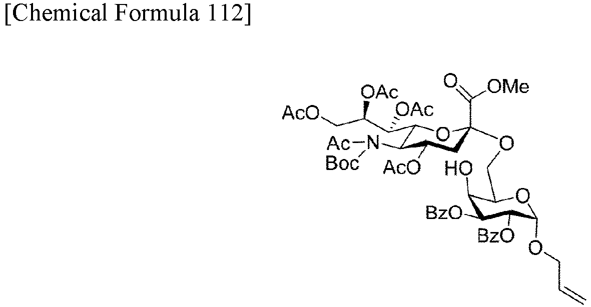 the method comprising a step of
   bringing a solvent dissolving the compound represented by formula IX-1 into contact with silica gel to perform solid-phase extraction of the compound represented by formula IX-1.
[43] The method according to [42], wherein the solvent dissolving the compound represented by formula IX-1 is selected from the group consisting of toluene, dichloromethane, chloroform, and a combination thereof.
[44] A method for producing a compound represented by the following formula IX-3: 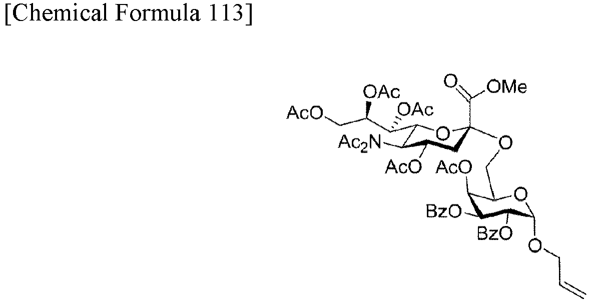 the method comprising a step of
   bringing a solvent dissolving the compound represented by formula IX-3 into contact with silica gel to perform solid-phase extraction of the compound represented by formula IX-3.
[45] The method according to [44], wherein the solvent dissolving the compound represented by formula IX-3 is selected from the group consisting of toluene, dichloromethane, chloroform, and a combination thereof.
[46] A method for producing an oligosaccharide represented by the following formula XI: 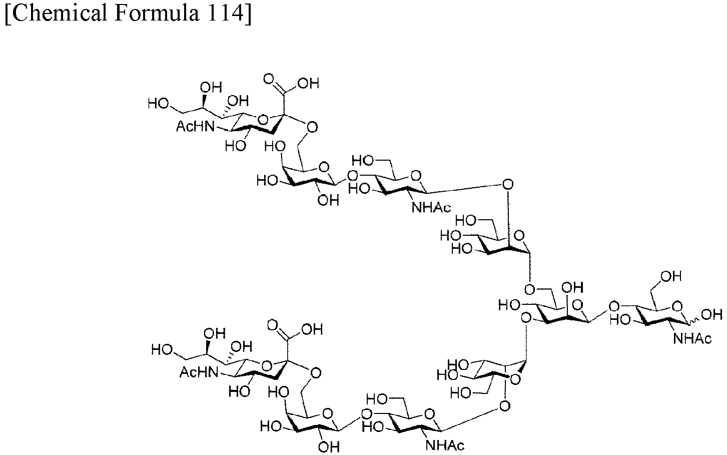 the method comprising a method according to any one of [23] to [45].
[47] A compound represented by the following formula III-6: 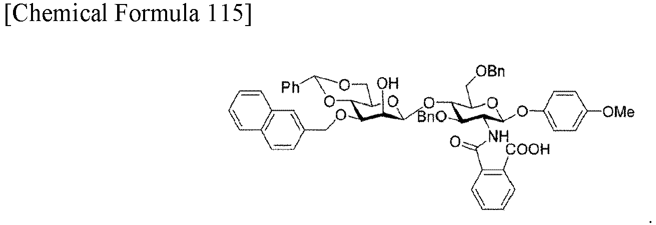
[48] A compound represented by the following formula III-10: 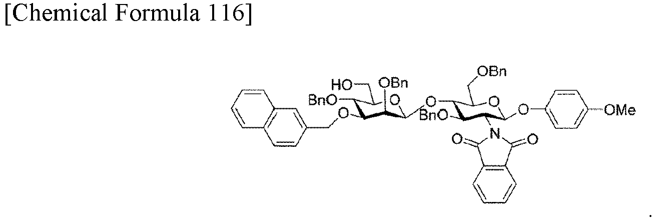
[49] A compound represented by the following formula V-1: 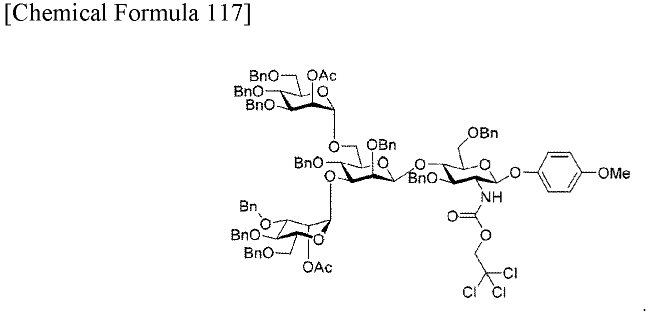
[50] A compound represented by the following formula V-2: 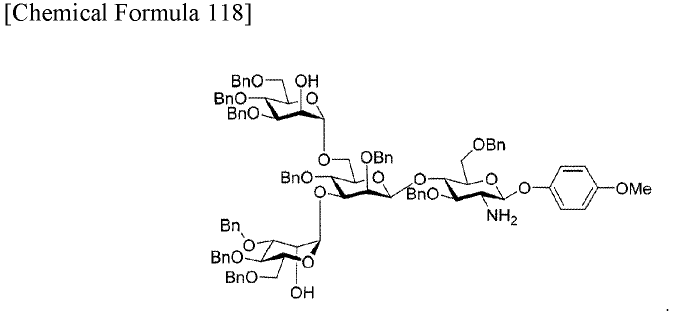
[51] A compound represented by the following formula V-3: 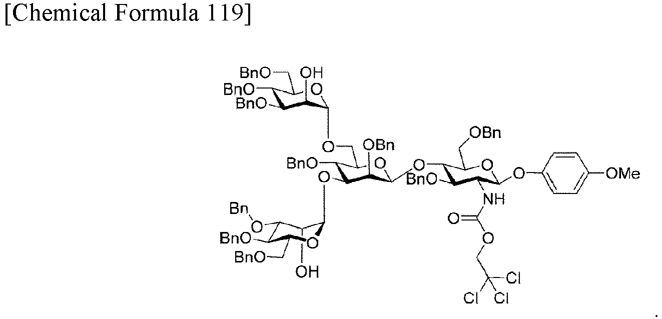
[52] A compound represented by the following formula II-6: 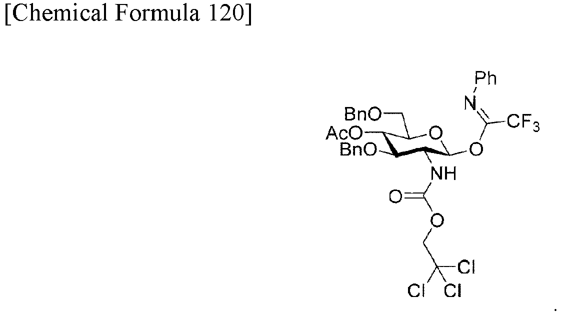
[53] A compound represented by the following formula VI-A: 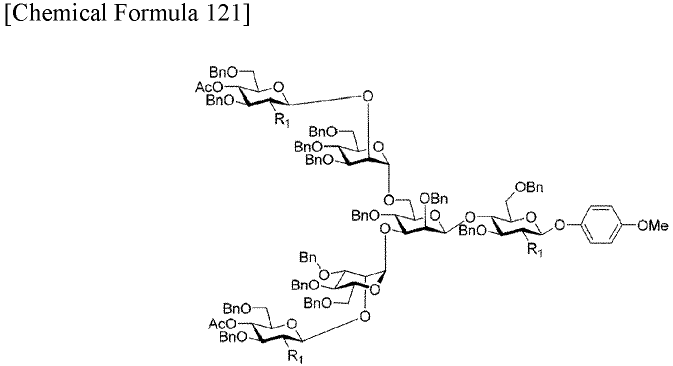 wherein R₁ represents a group selected from the group consisting of 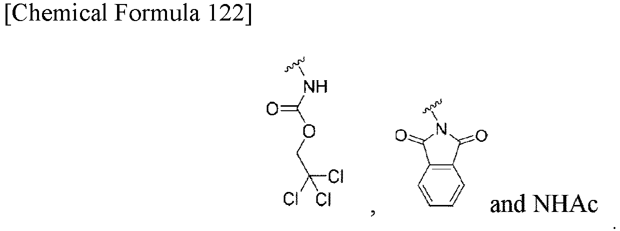
[54] A compound represented by the following formula VI-2: 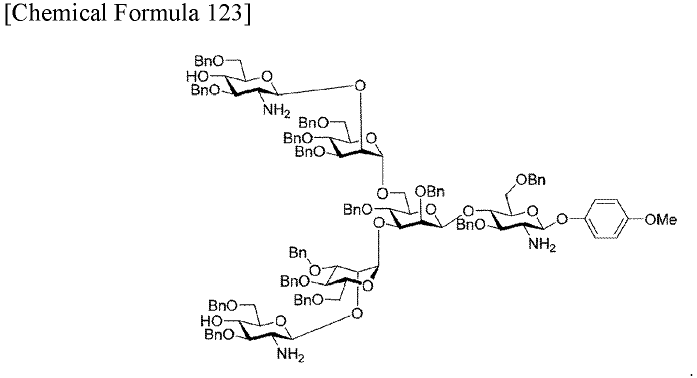
[55] A compound represented by the following formula VI-B: 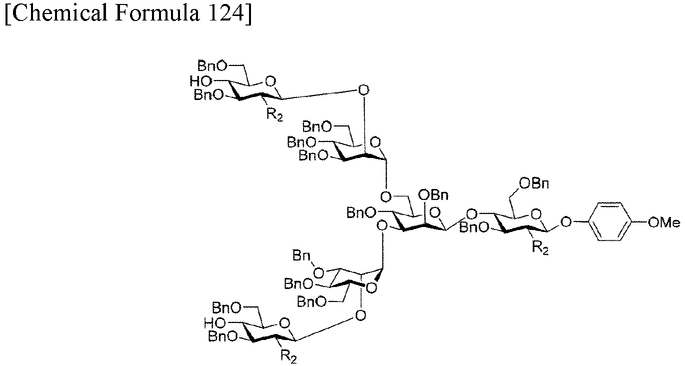 wherein R₂ represents a group selected from the group consisting of 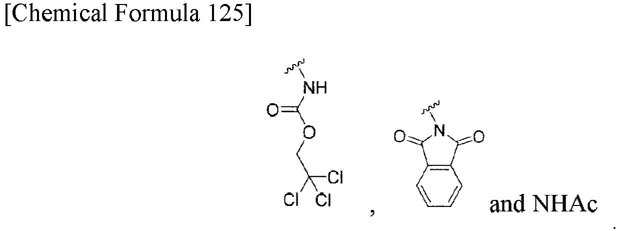
[56] A compound represented by the following formula VIII-5: 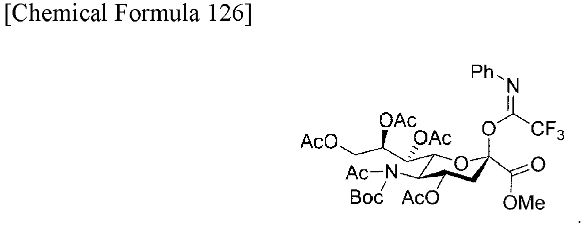
[57] A compound represented by the following formula X-1: 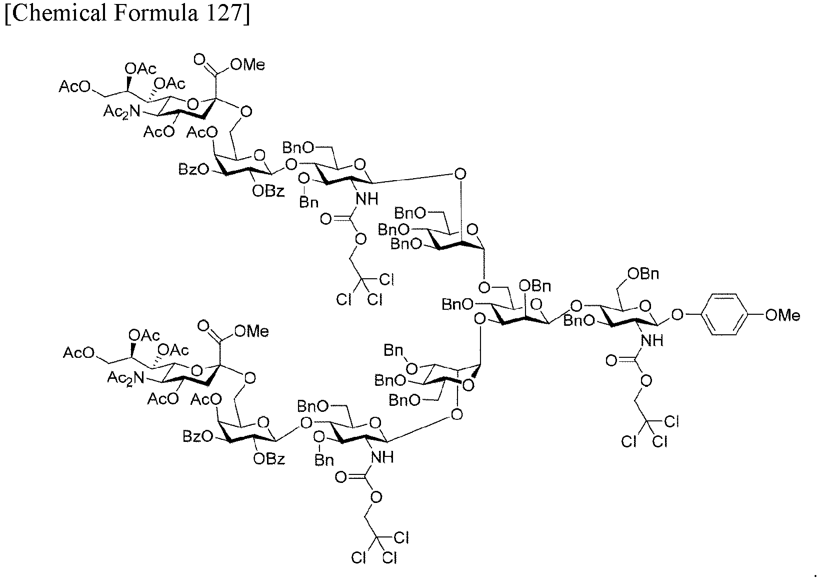
[58] A compound represented by the following formula X-2: 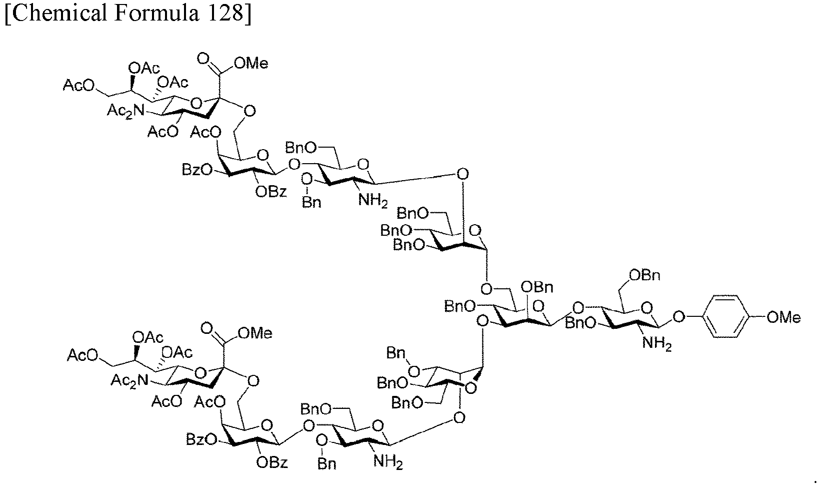
[59] A compound represented by the following formula X-3: 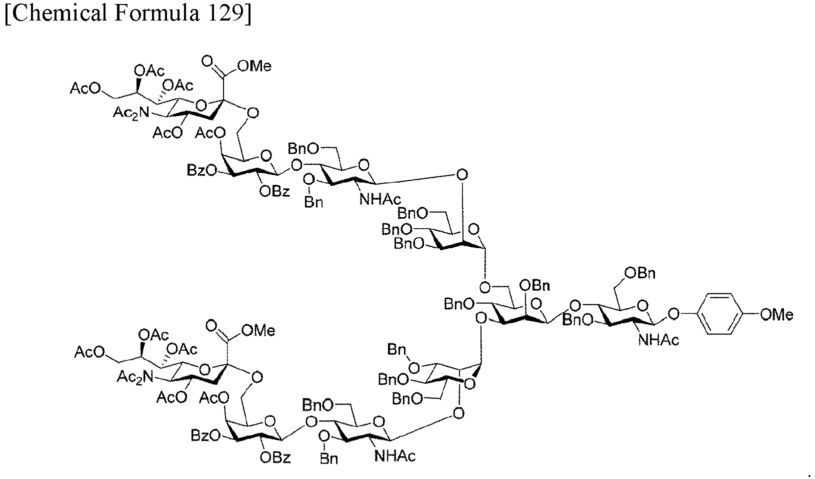
[60] A compound represented by the following formula X-4: 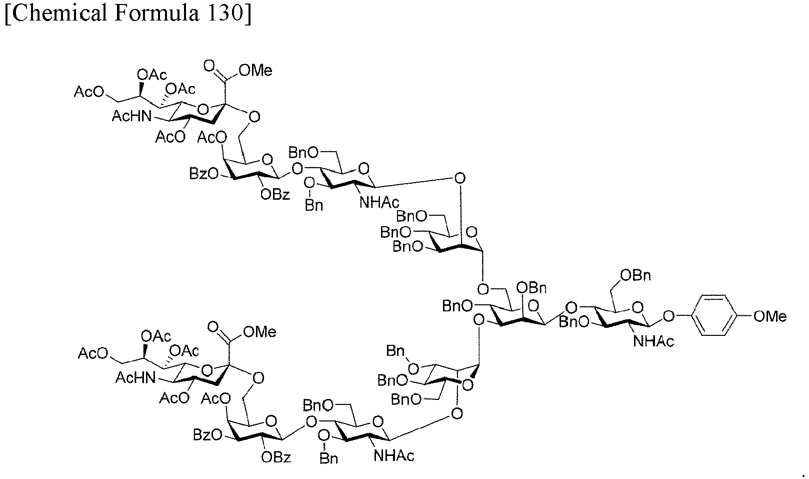
[61] A compound represented by the following formula X-5: 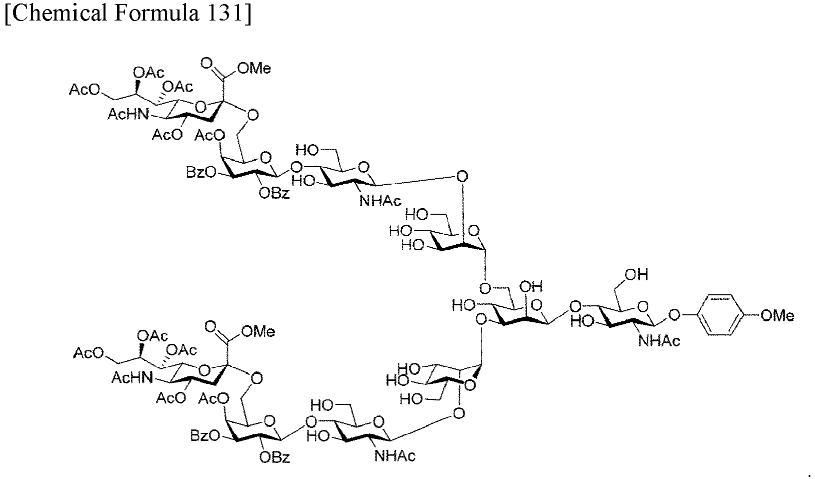
[62] A compound represented by the following formula X-6: 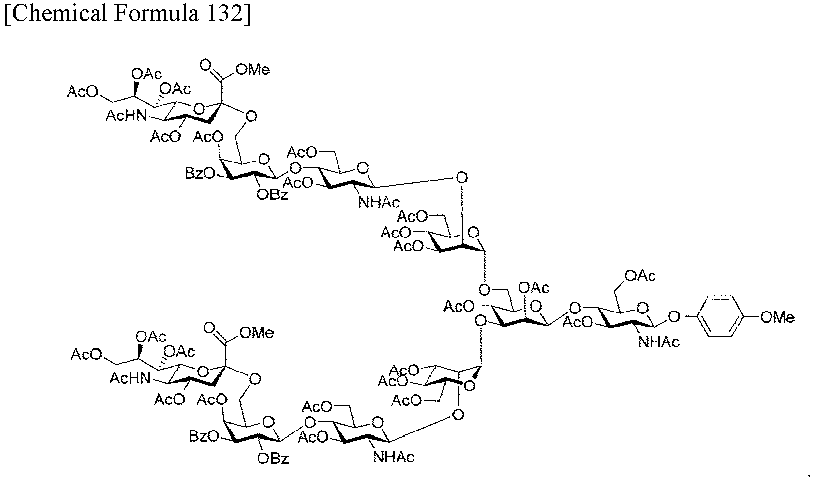
[63] A compound represented by the following formula X-7: 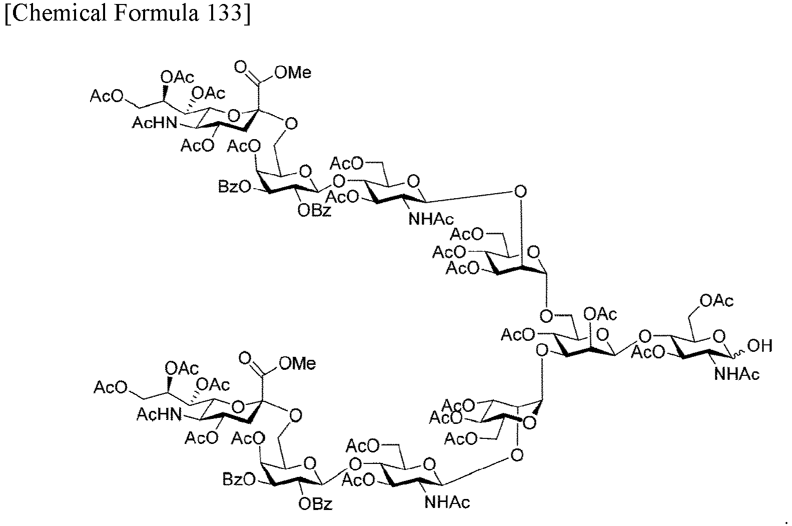
[64] A method for producing a glycan-remodeled antibody or a molecule containing an Fc region thereof,
   the method comprising a method according to any one of [1] to [22] and [46],
   the method further comprising steps of
      obtaining, from the obtained oligosaccharide represented by formula XI, a glycan donor molecule containing N-acetylglucosamine (GlcNAc) with a reducing end activated; and
      reacting the glycan donor molecule with an acceptor molecule which is an antibody with core GlcNAc optionally having a fucose as an N297-binding glycan or a molecule containing an Fc region thereof.
[65] The method according to [64], wherein the GlcNAc with a reducing end activated is oxazolinated GlcNAc.
[66] The method according to [64] or [65], wherein the glycan donor molecule is a compound (also referred to as "SG(10)-Ox") optionally having a chemically modified non-reducing end and represented by the following formula XII: 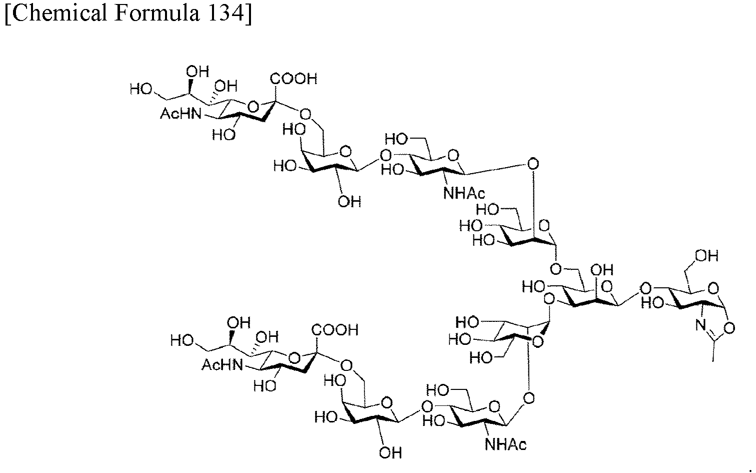
[67] The method according to any one of [64] to [66], wherein the glycan donor molecule is [N₃-PEG(3)]₂-SG(10)-Ox.
[68] The method according to [67], further comprising a step of reacting the azide group (N₃-) with a molecule having an alkyne structure.
[69] The method according to [68], wherein the molecule having an alkyne structure is selected from the group consisting of a chemotherapeutic agent, molecular targeted drug, immune activator, toxin, antimicrobial agent, antiviral agent, diagnostic agent, protein, peptide, amino acid, nucleic acid, antigen, vitamin, and hormone.
[70] A method for producing an antibody-drug conjugate, comprising the method according to any one of [64] to [69].

### Advantageous Effects of Invention

The present invention provides a novel method for pure-chemically producing a biantennary N-glycan with an α2,6-sialic acid structure at each non-reducing end, the method having excellent yield, selectivity, and efficiency. In addition, the present invention provides a method for producing a novel monosaccharide or oligosaccharide, which are useful in the method for pure-chemically producing the N-glycan, and intermediates as well as a novel method for producing the N-glycan by including the method of them. Further, the present invention provides a novel method for producing, for instance, a glycoprotein (in particular, a glycan-remodeled antibody or a molecule containing an Fc region thereof, or an antibody-drug conjugate) by using, for example, the novel method for pure-chemically producing the N-glycan.

### Brief Description of Drawing

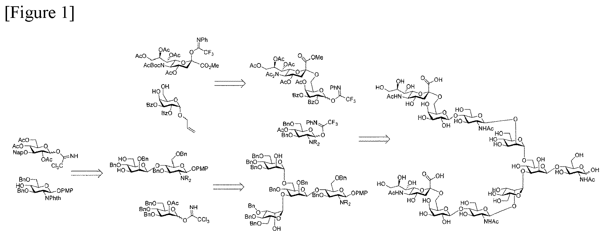

[Figure 1] Figure 1 is a simplified view of the novel method for pure-chemically producing a biantennary N-glycan with an α2,6-sialic acid structure at each non-reducing end while using a novel synthesis scheme provided in the present invention.

### Description of Embodiments

Hereinafter, preferable embodiments of the present invention will be described. Note that the below-described embodiments are representative examples of the embodiments of the present invention. The scope of the present invention should not be narrowly construed due to them.

### <1. Novel method for pure-chemically producing biantennary N-glycan with a2,6-sialic acid structure at each non-reducing end>

An embodiment of the present invention provides a novel method for pure-chemically producing a biantennary N-glycan with an α2,6-sialic acid structure at each non-reducing end while using a novel synthesis scheme. In the present invention, the biantennary N-glycan with an α2,6-sialic acid structure at each non-reducing end means an oligosaccharide represented by the following formula XI:

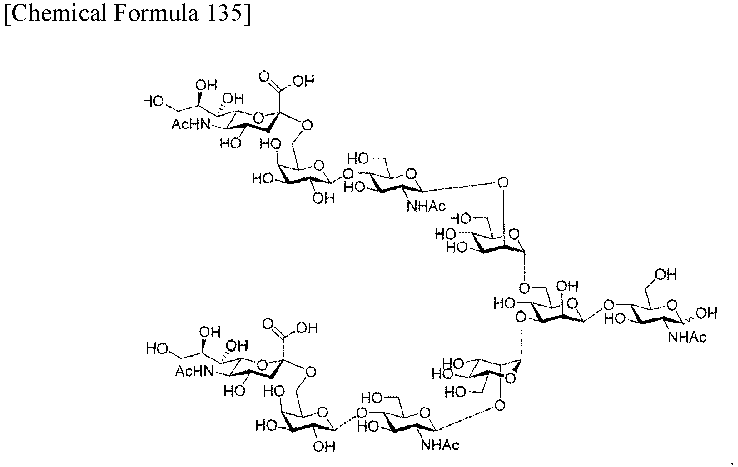

The novel synthesis scheme in the present invention includes the following steps 1 to 5.

### <Step 1>

Step 1 is a step of producing a compound represented by the following formula III-11: 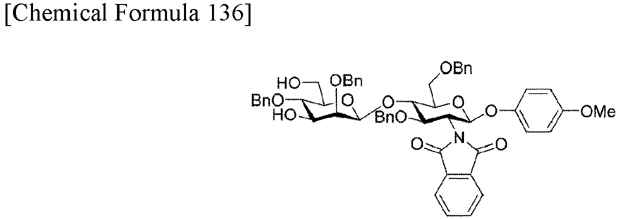 or a compound represented by the following formula III-13: 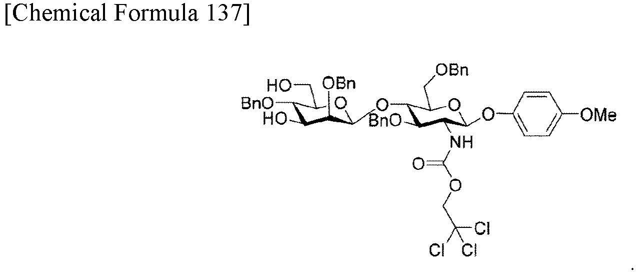 Step 1 includes, as essential substeps, substeps 1-9 and 1-10 or substeps 1-13 and 1-14 described below for stereoinversion from glucose -> mannose. The other substeps can be carried out by using or applying usual procedures for producing a monosaccharide or oligosaccharide.

In an embodiment of the present invention, step 1 includes the following sub steps:

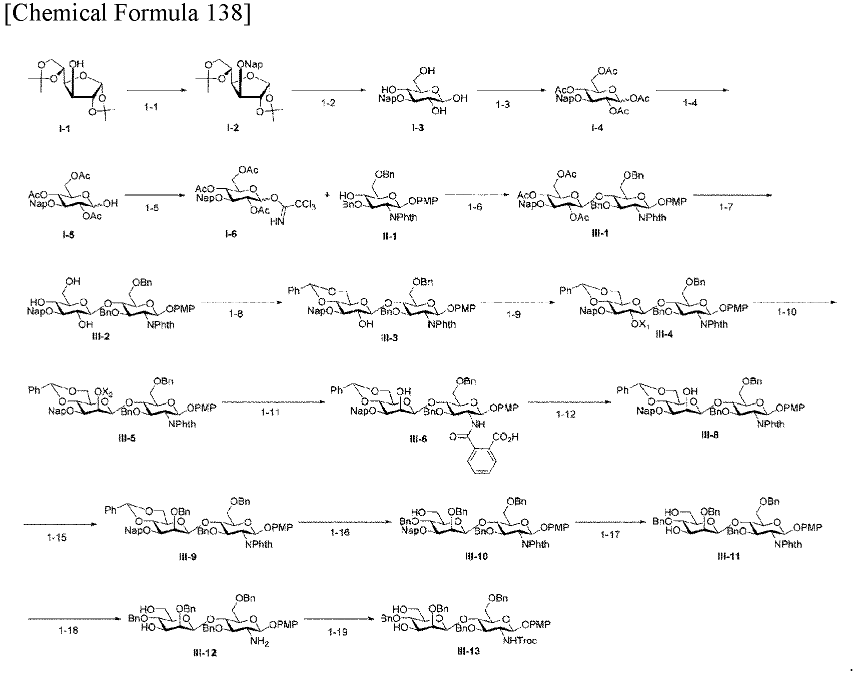

### <Substep 1-1>

Substep 1-1 is a step of producing a compound represented by the following formula I-2: 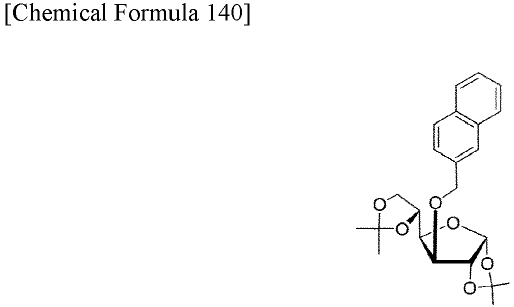 by protecting, by 2-naphthalenylmethyl (NAP) group, a hydroxyl group attached to carbon at position 3 of a compound represented by the following formula I-1: 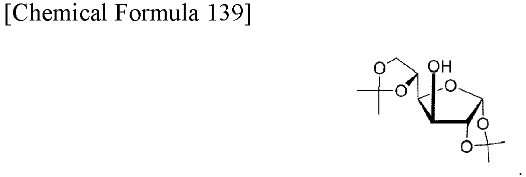 The compound represented by formula I-1, which is the starting material for this step, may be produced by a known procedure or a commercially available product may be used. Examples of the commercially available compound represented by formula 1-1 include 1,2:5,6-di-O-isopropylidene-α-D-glucofuranose manufactured by Sigma-Aldrich. This step may be performed by using or applying a known procedure. However, the procedure shown in Example 1, for example, may be preferably used.

### <Substep 1-2>

Substep 1-2 is a step of producing a compound represented by the following formula I-3: 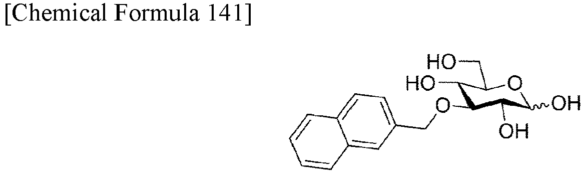 by acid hydrolysis of two isopropylidene moieties of the compound represented by formula I-2 and by pyranose ring formation. This step may be performed by using or applying a known procedure. However, the procedure shown in Example 2, for example, may be preferably used.

### <Substep 1-3>

Substep 1-3 is a step of producing a compound represented by the following formula I-4: 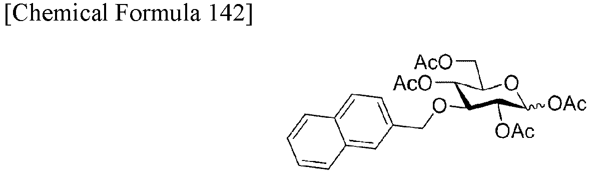 by protecting, by an acetyl group, each hydroxyl group on the compound represented by formula I-3. This step may be performed by using or applying a known procedure. However, the procedure shown in Example 3, for example, may be preferably used.

### <Substep 1-4>

Substep 1-4 is a step of producing a compound represented by the following formula I-5: 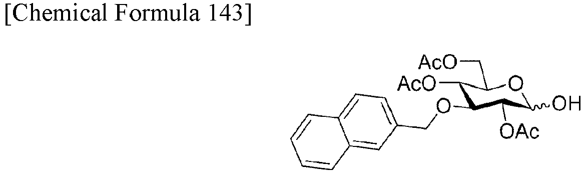 by selectively eliminating only the acetyl group of the acetyloxy group attached to carbon at position 1 of the compound represented by formula I-4. This step may be performed by using or applying a known procedure. However, the procedure shown in Example 3, for example, may be preferably used.

### <Substep 1-5>

Substep 1-5 is a step of producing a compound represented by the following formula I-6: 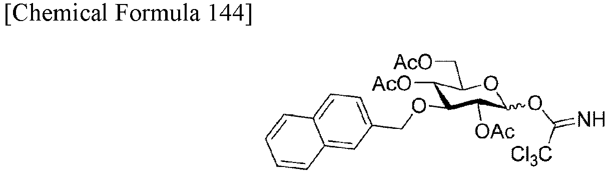 by reacting the compound represented by formula I-5 with trichloroacetonitrile. This step may be performed by using or applying a known procedure. However, the procedure shown in Example 4, for example, may be preferably used.

### <Substep 1-6>

Substep 1-6 is a step of producing a compound represented by the following formula III-1: 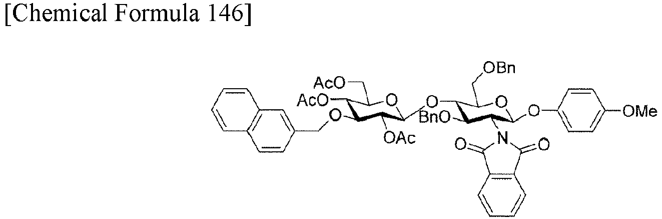 by reacting the compound represented by formula I-6 with a compound represented by the following formula II-1: 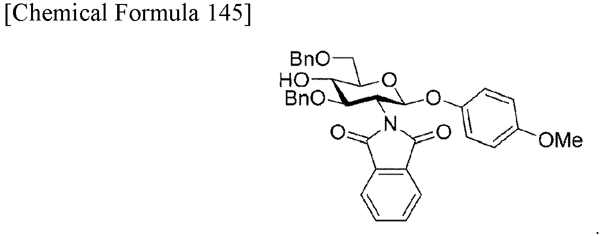 The compound represented by formula II-1 may be produced by a known procedure or a commercially available product may be used. Examples of the commercially available compound represented by formula II-1 include 4-methoxyphenyl 3,6-di-O-benzyl-2-deoxy-2-phthalimido-β-D-glucopyranoside, manufactured by TOKYO CHEMICAL INDUSTRY CO., LTD. This step may be performed by using or applying a known procedure. However, the procedure shown in Example 5, for example, may be preferably used.

### <Substep 1-7>

Substep 1-7 is a step of producing a compound represented by the following formula III-2: 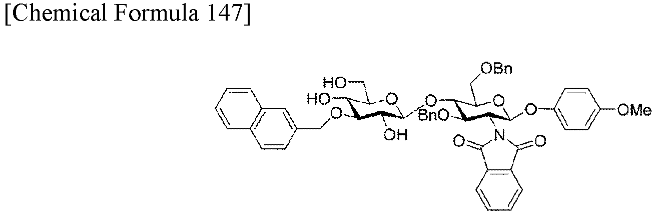 by eliminating each acetyl group from the compound represented by formula III-1. This step may be performed by using or applying a known procedure. However, the procedure shown in Example 6 or 16, for example, may be preferably used.

In an embodiment of the present invention, substep 1-7 is a step of producing the compound represented by formula III-2 by reacting the compound represented by formula III-1 with an alkoxide-based strong base in the presence of methyl trifluoroacetate. A report (Org. Biomol. Chem., 2018, 16, 4720-4727) shows that the acetyl groups were eliminated using sodium methoxide in methanol. In this case, the phthalimide ring may be opened at the same time as an undesired side reaction. By contrast, a reaction with an alkoxide-based strong base may be carried out in the presence of methyl trifluoroacetate. This technique may be used to eliminate the acetyl groups while inhibiting the ring-opening of the phthalimide group.

The alkoxide-based strong base is not limited as long as the reaction proceeds. Examples include a sodium salt, a lithium salt, or a potassium salt of C1-C5 alkoxide, or a combination thereof. Preferable examples include lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide. Particularly preferable examples include sodium methoxide, lithium tert-butoxide, or potassium tert-butoxide.

The solvent in this step is not limited as long as the reaction proceeds. Examples include an alcohol solvent, e.g., methanol, ethanol, propanol, butanol, or a mixed solvent system of an alcohol solvent and, for instance, tetrahydrofuran, acetonitrile, cyclopentyl methyl ether, toluene, or dimethylacetamide. Preferred is methanol or a mixed solvent system of methanol and tetrahydrofuran.

The reaction temperature in this step is not limited as long as the reaction proceeds, but may be, for example, from -20°C to 80°C, preferably from 0°C to 70°C, more preferably from 20°C to 65°C, and particularly preferably from 40°C to 60°C.

### <Substep 1-8>

Substep 1-8 is a step of producing a compound represented by the following formula III-3: 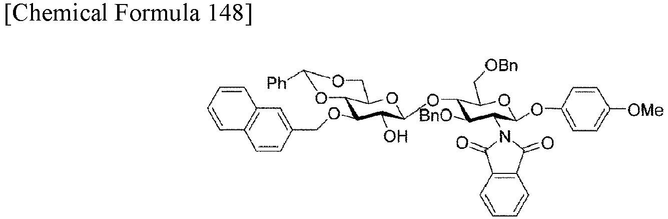 by selectively protecting, using benzaldehyde dimethyl acetal, the hydroxyl groups attached to the carbon atoms at positions 4 and 6 of D-glucopyranoside in the compound represented by formula III-2. This step may be performed by using or applying a known procedure. However, the procedure shown in Example 7 or 17, for example, may be preferably used.

### <Substep 1-9>

Substep 1-9 is a step of producing a compound represented by the following formula III-4: 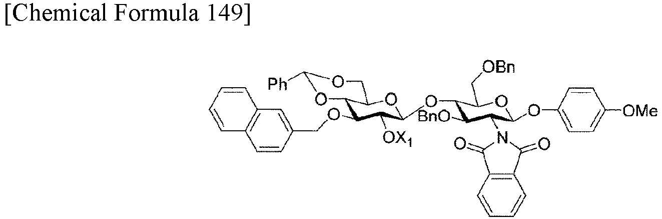 wherein X₁ represents a substituent selected from the group consisting of a trifluoromethanesulfonyl group, a nonafluorobutanesulfonyl group, a 2-nitrobenzenesulfonyl group, and a 4- nitrobenzenesulfonyl group, by reacting the compound represented by formula III-3 with a compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group. This step may be performed by using or applying a known procedure for providing a leaving group. However, the procedure shown in Example 8, for example, may be preferably used.

In this step, examples of the "a compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group" include trifluoromethanesulfonic anhydride, nonafluoro-1-butanesulfonyl fluoride, bis(nonafluoro-1-butanesulfonic acid)anhydride, 2-nitrobenzenesulfonyl chloride, or 4-nitrobenzenesulfonyl chloride. Preferred is trifluoromethanesulfonic anhydride.

The solvent in this step is not limited as long as the reaction proceeds. Examples include ethyl acetate, toluene, dichloromethane, acetonitrile, cyclopentyl methyl ether, or tert-butyl methyl ether. Preferred is ethyl acetate, toluene, or dichloromethane.

The reaction temperature in this step is not limited as long as the reaction proceeds, but may be, for example, from -40°C to 60°C, preferably from -30°C to 40°C, and more preferably from -20°C to 10°C.

This step can suitably be performed in the presence of a base. The base used in this step is not particularly limited as long as the reaction proceeds. Examples include 1-methylimidazole, pyridine, 4-dimethylaminopyridine, picoline, lutidine, or collidine. Preferred is 1-methylimidazole.

### <Substep 1-10>

Substep 1-10 is a step of producing a compound represented by the following formula III-5: 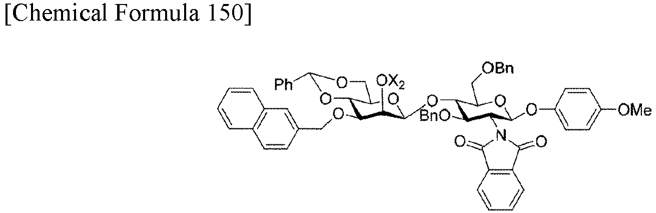
wherein X₂ is an acetyl group,
   by reacting the compound represented by formula III-4 with cesium acetate or tetrabutylammonium acetate,
   or a step of producing a compound represented by the following formula III-5: 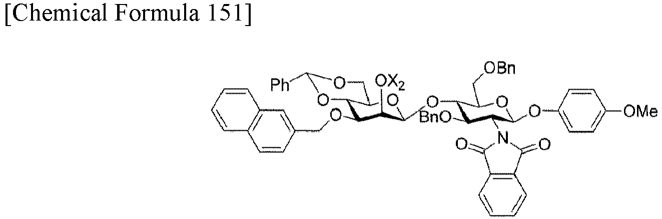
wherein X₂ is a benzoyl group,
   by reacting the compound represented by formula III-4 with tetrabutylammonium benzoate. There are known conversion reactions, namely stereoinversions, from glucose -> mannose. However, no conversion has been reported when a 2-naphthylmethyl (Nap) group is the protecting group of the hydroxyl group attached to the carbon at position 3 of D-glucopyranoside of a glucose-glucosamine disaccharide linked by a β-glycosidic linkage. This method may be adopted to achieve the stereoinversion from glucose -> mannose, so that the mannose-glucosamine disaccharide skeleton linked by a β-glycosidic linkage can be constructed in high yield and selectivity.

The solvent in this step is not limited as long as the reaction proceeds. Examples include dimethyl sulfoxide, dimethylformamide, dimethylacetamide, N-methylpyrrolidone, N,N-dimethylimidazolidinone, sulfolane, tetrahydrofuran, or acetonitrile. Preferred is dimethylsulfoxide.

The reaction temperature in this step is not limited as long as the reaction proceeds, but may be, for example, from 20°C to 80°C, preferably from 23°C to 70°C, more preferably from 26°C to 60°C, and particularly preferably from 30°C to 50°C.

This step may be preferably performed using, for instance, the procedure shown in Example 9.

### <Substep 1-11>

Substep 1-11 is a step of producing a compound represented by the following formula III-6; 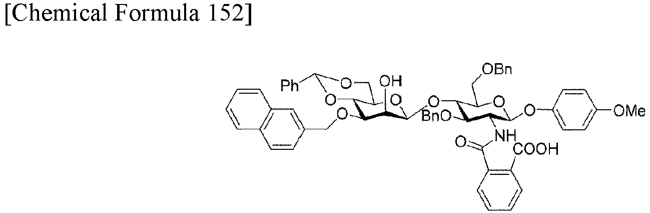 by eliminating the X₂ group and by opening the ring of the phthalimide group in the compound represented by formula III-5. This step may be performed by using or applying a known hydrolysis procedure. However, the procedure shown in Example 9, for example, may be preferably used.

The compound represented by formula III-6 that is produced in this step and dissolved in a solvent may be used as it is in the next step or may be isolated and purified by recrystallization. The major advantage of the compound represented by formula III-6 is that it can be isolated and purified by crystallization, which can almost completely remove impurities having similar structures that are difficult to remove by column purification. In this case, the compound represented by formula III-6 with HPLC purity of 99% or higher can be obtained.

The isolation and purification by recrystallization in this step are not limited as long as the reaction proceeds. Examples include a method for completely removing the solvent from the dissolved state by decompression drying; or a method for using tetrahydrofuran as a good solvent and adding dropwise isopropanol as a poor solvent in the presence of a small amount of water.

The recrystallization in this step may also be performed using seed crystals of the compound represented by formula III-6. In the case of using seed crystals, the crystallization may be conducted by, for instance, a method in which tetrahydrofuran is used as a good solvent; in the presence of a small amount of water, some of isopropanol is added dropwise as a poor solvent; seed crystals are added; crystal precipitation is observed; and the remaining isopropanol is then added dropwise.

### <Substep 1-12>

Substep 1-12 is a step of producing a compound represented by the following formula III-8; 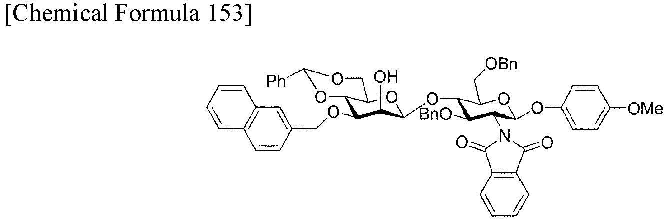 by subjecting the ring-opened phthalimide group in the compound represented by formula III-6 to dehydration condensation to close the ring. This step may be performed by using or applying a known procedure. However, the procedure shown in Example 10 or 20, for example, may be preferably used.

### <Substep 1-15>

Substep 1-15 is a step of producing a compound represented by the following formula III-9: 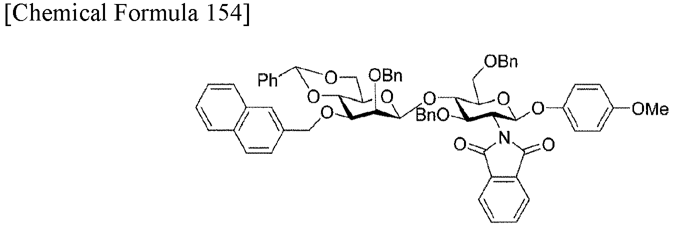 by protecting, by a benzyl group, the hydroxyl group attached to the carbon at position 2 of D-mannopyranoside in the compound represented by formula III-8. This step may be performed by using or applying a known procedure. However, the procedure shown in Example 11 or 21, for example, may be preferably used.

In an embodiment of the present invention, substep 1-15 includes a step of protecting, in the presence of lithium tert-butoxide or lithium tert-amoxide, the hydroxyl group attached to the carbon at position 2 of D-mannopyranoside in the compound represented by formula III-8 by a benzyl group to produce the compound represented by formula III-9. The ring-opening of phthalimide can be inhibited by performing substep 1-15 in the presence of lithium tert-butoxide or lithium tert-amoxide. In addition, it is safer and easier to scale up than the general conditions using sodium hydride.

The solvent in this step is not limited as long as the reaction proceeds. Examples include dimethylacetamide, dimethylformamide, N-methylpyrrolidone, or N,N-dimethylimidazolidinone. Preferred is dimethylacetamide.

The reaction temperature in this step is not limited as long as the reaction proceeds, but may be, for example, from -20°C to 100°C, preferably from -15°C to 70°C, and particularly preferably from -10°C to 50°C.

### <Substep 1-16>

Substep 1-16 is a step of producing a compound represented by the following formula III-10: 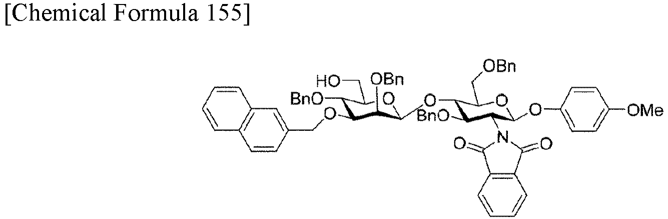 by selectively deprotecting only the hydroxyl group attached to the carbon at position 6 of D-mannopyranoside in the compound represented by formula III-9. This step may be performed by using or applying a known procedure. However, the procedure shown in Example 12 or 22, for example, may be preferably used.

The compound represented by formula III-10 that is produced in this step and dissolved in a solvent may be used as it is in the next step or may be isolated and purified by column or by recrystallization.

### <Substep 1-17>

Substep 1-17 is a step of producing a compound represented by the following formula III-11: 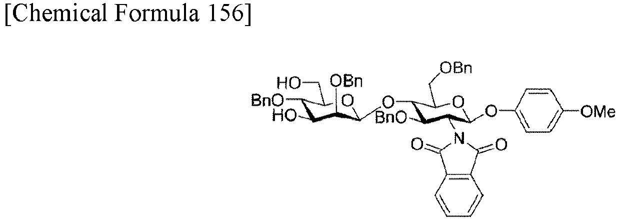 by eliminating the NAP group from the compound represented by formula III-10. This step may be performed by using or applying a known procedure. However, the procedure shown in Example 13, for example, may be preferably used.

### <Substep 1-18>

Substep 1-18 is a step of producing a compound represented by the following formula III-12: 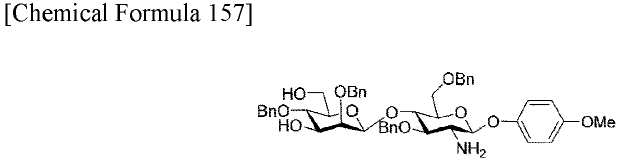 by eliminating the phthaloyl protecting group from the compound represented by formula III-11. This step may be performed by using or applying a known procedure. However, the procedure shown in Example 14, for example, may be preferably used.

### <Substep 1-19>

Substep 1-19 is a step of producing a compound represented by the following formula III-13: 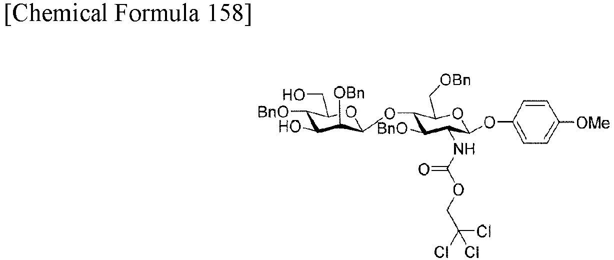 by protecting, by a 2,2,2-trichloroethoxycarbonyl group, the amino group in the compound represented by formula III-12. This step may be performed by using or applying a known procedure. However, the procedure shown in Example 15, for example, may be preferably used.

In an embodiment of the present invention, step 1 includes the following substeps 1-13 and 1-14 for stereoinversion from glucose -> mannose by using a redox reaction, instead of substeps 1-9 to 1-12 for the stereoinversion by using an S_{N}2 reaction.

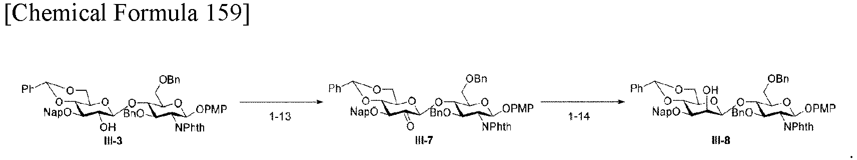

### <Substep 1-13>

Substep 1-13 is a step of producing a compound represented by the following formula III-7: 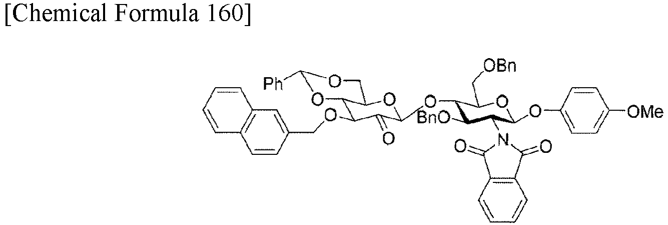 by oxidizing position 2 of D-glucopyranoside in the compound represented by formula III-3. This step may be performed by using or applying a known procedure. However, the procedure shown in Example 18, for example, may be preferably used.

### <Substep 1-14>

Substep 1-14 is a step of producing a compound represented by the following formula III-8: 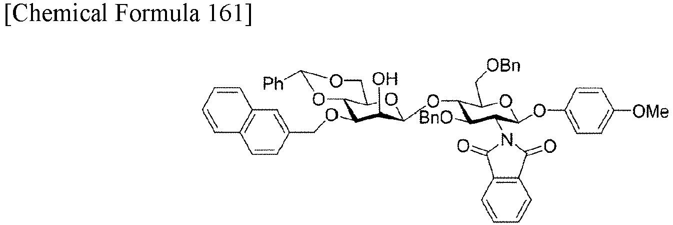 by reducing the oxo group attached to the carbon at position 2 of 2-keto-D-glucopyranoside in the compound represented by formula III-7. This step may be performed by using or applying a known procedure. However, the procedure shown in Example 19, for example, may be preferably used.

The solvent in this step is not limited as long as the reaction proceeds. Examples include diethyl ether, cyclopentyl methyl ether, tert-butyl methyl ether, diisopropyl ether, dipropyl ether, dibutyl ether, or 1,4-dioxane. Preferred is tetrahydrofuran.

The reaction temperature in this step is not limited as long as the reaction proceeds, but may be, for example, from -80°C to 20°C. Note that as described below, the optimum reaction temperature varies depending on the reducing agent used.

In an embodiment of the present invention, the oxo group attached to the carbon at position 2 of 2-keto-D-glucopyranoside in the compound represented by formula III-7 is reduced in the presence of a reducing agent selected from the group consisting of L-selectride; LS-selectride; lithium diisobutyl-tert-butoxyaluminum hydride (LDBBA); a compound represented by the following formula A: 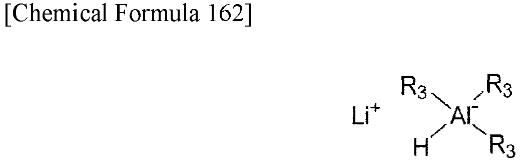
wherein R₃ is a di-tert-butylmethylphenoxide or hydride as shown in the following formula: 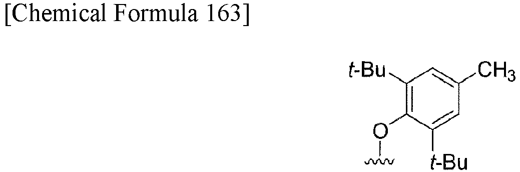
provided that at least two R₃ are each di-tert-butylmethylphenoxide; and a combination thereof. In the reduction step, NaBH₄, for example, may be used. In this case, the stereoselectivity was low (about 7:3), and it was difficult to efficiently obtain the desired stereoinversion from Gln -> Man (Org. Biomol. Chem., 2018, 16, 4720-4727). On the other hand, in the case of using the above listed reducing agents, the selectivity of stereoinversion from Gln -> Man is greatly improved (93.6:6.4 to 98.1:1.9) when compared to the case of using NaBH₄.

Among the compounds represented by formula A, a compound where three R₃ moieties are each di-tert-butylmethylphenoxide can be obtained, for example, by adding, to a tetrahydrofuran suspension (2 mL) containing lithium aluminum hydride (50.0 mg, 1.32 mmol), dibutylhydroxytoluene (885.41 mg, 4.02 mmol) at 0°C, followed by stirring at 25°C. Among the compounds represented by formula A, a compound where two R₃ moieties are each di-tert-butylmethylphenoxide can be obtained in a similar manner by using 2 molar equivalents of dibutylhydroxytoluene for 1 molar equivalent of lithium aluminum hydride.

As described above, the reaction temperature in this step is not limited as long as the reaction proceeds. When L-selectride, LS-selectride, or LDBBA is used as the reducing agent, the reaction temperature may be preferably from -80°C to -20°C, more preferably from -80°C to -30°C, still more preferably from -80°C to -40°C, and particularly preferably from -80°C to -50°C. When the compound represented by formula A is used as the reducing agent, the reaction temperature may be preferably from -20°C to 20°C, more preferably from -15°C to 15°C, and particularly preferably from -10°C to 10°C. Therefore, in terms of the fact that the reaction proceeds at an easy-to-handle temperature, the particularly suitable reducing agent used for this step is a compound represented by formula A.

### <Step 2>

Step 2 is a step of producing a compound represented by the following formula V-3: 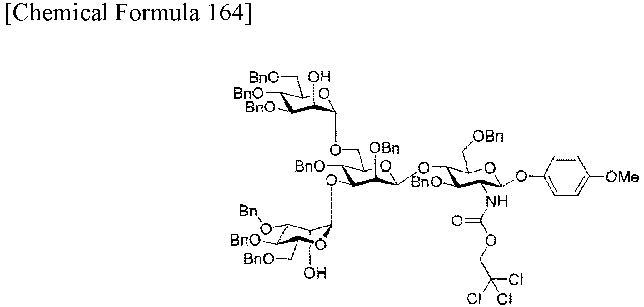 or a compound represented by the following formula V-5: 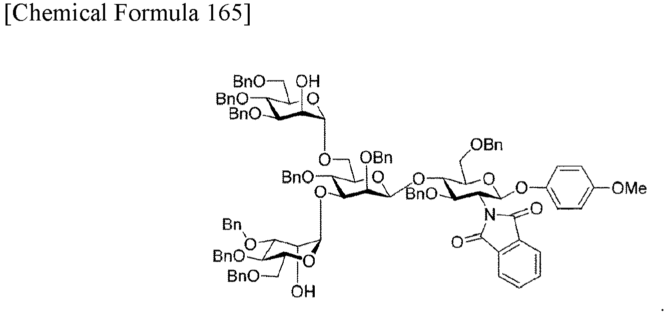 Step 2 includes essential substep 2-3 or 2-6 described below, in which two molecules of monosaccharide are regioselectively bonded to one molecule of disaccharide. The other substeps can be carried out by using or applying usual procedures for producing a monosaccharide or oligosaccharide.

In an embodiment of the present invention, step 2 includes the following substeps: 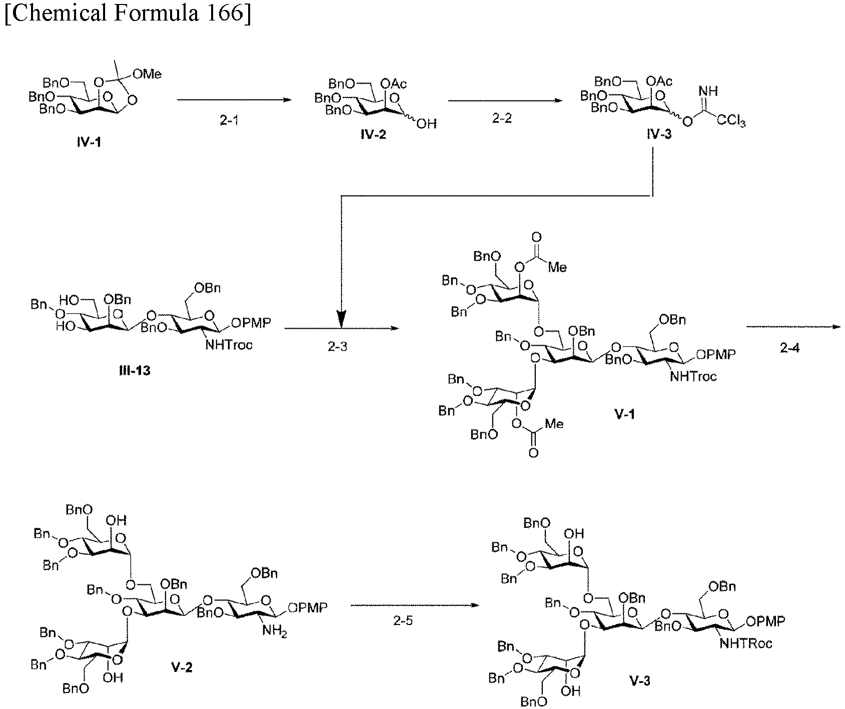 or

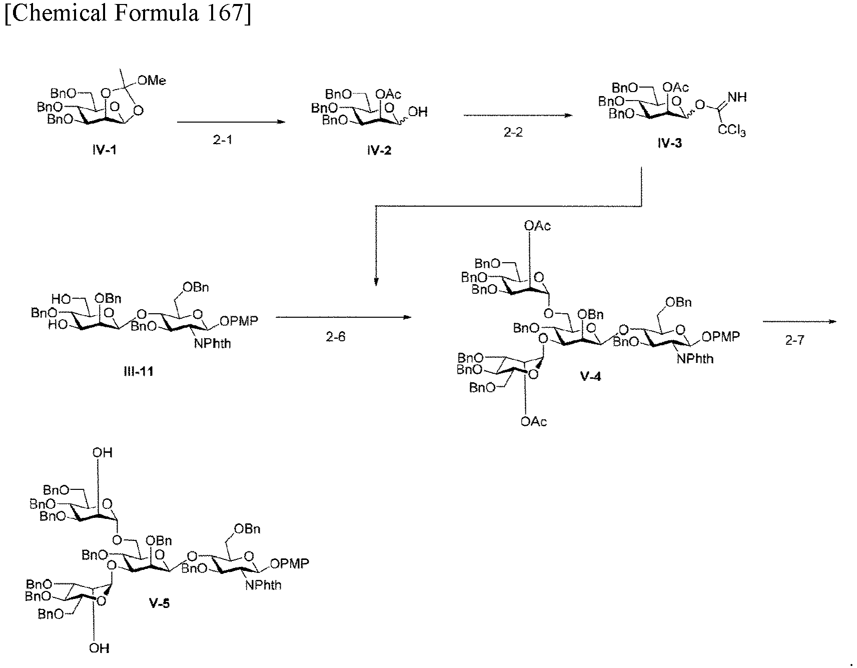

### <Substep 2-1>

Substep 2-1 is a step of producing a compound represented by the following formula IV-2: 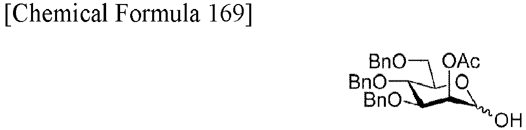 by selectively transferring an acetyl group in monoacetyl form as formed by hydrolysis of the ortho-ester moiety of the compound represented by the following formula IV-1: 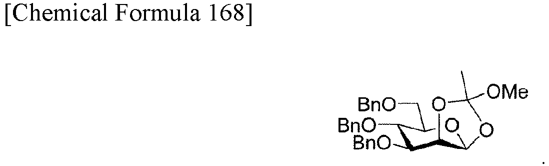 The compound represented by formula VI-1, which is the starting material for this step, may be produced by a known procedure or a commercially available product may be used. Examples of the commercially available compound represented by formula VI-1 include 3,4,6-Tri-O-benzyl-beta-D-mannopyranose-1,2-(methyl orthoacetate), manufactured by Combi-blocks. This step may be performed by using or applying a known procedure. However, the procedure shown in Example 23 or 26, for example, may be preferably used.

### <Substep 2-2>

Substep 2-2 is a step of producing a compound represented by the following formula IV-3: 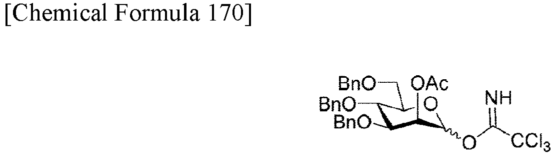 by reacting the compound represented by formula IV-2 with trichloroacetonitrile. This step may be performed by using or applying a known procedure. However, the procedure shown in Example 23 or 27, for example, may be preferably used.

### <Substep 2-3>

Substep 2-3 is a step of producing a compound represented by the following formula V-1: 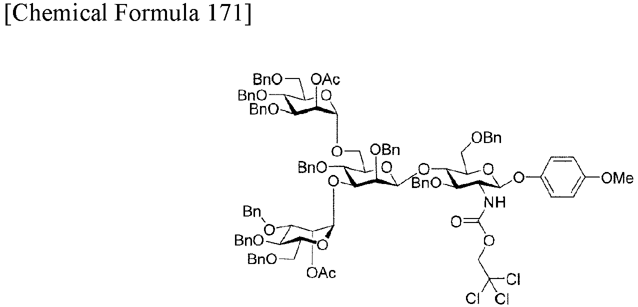 by reacting the compound represented by formula III-13 with the compound represented by formula IV-3. This step may be performed by using or applying a known procedure. However, the procedure shown in Example 24, for example, may be preferably used.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, tert-butyl dimethylsilyl trifluoromethanesulfonate, or a boron trifluoride diethyl ether complex. Preferred is trimethylsilyl trifluoromethanesulfonate.

The solvent in this step is not limited as long as the reaction proceeds. Examples include dichloromethane, toluene, trifluoromethylbenzene, or chlorobenzene. Preferred is dichloromethane or toluene.

The reaction temperature in this step is not limited as long as the reaction proceeds, but may be, for example, from -60°C to 0°C, preferably from -40°C to 0°C, more preferably from -30°C to 0°C, and particularly preferably from -20°C to 0°C.

In this step, it is preferable to use 2 to 4 equivalents of the compound represented by formula IV-3 with respect to 1 equivalent of the compound represented by formula III-13. It is more preferable to use 2.5 to 3 equivalents of the compound represented by formula IV-3 with respect to 1 equivalent of the compound represented by formula III-13.

### <Substep 2-4>

Substep 2-4 is a step of producing a compound represented by the following formula V-2; 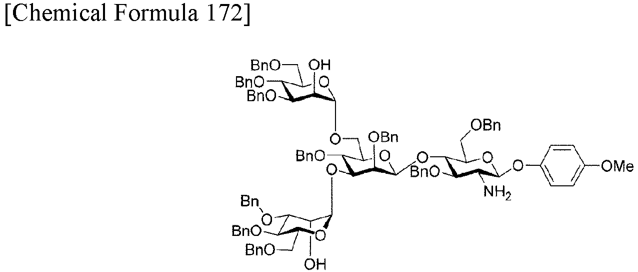 by eliminating the acetyl groups and the 2,2,2-trichloroethoxycarbonyl group from the compound represented by formula V-1. This step may be performed by using or applying a known procedure. However, the procedure shown in Example 25, for example, may be preferably used.

### <Substep 2-5>

Substep 2-5 is a step of producing a compound represented by the following formula V-3: 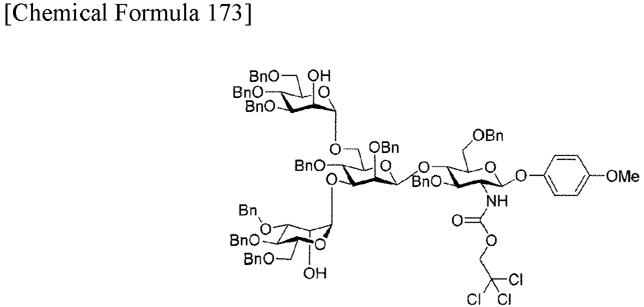 by protecting, by a 2,2,2-trichloroethoxycarbonyl group, the amino group in the compound represented by formula V-2. This step may be performed by using or applying a known procedure. However, the procedure shown in Example 25, for example, may be preferably used.

### <Substep 2-6>

Substep 2-6 is a step of producing a compound represented by the following formula V-4: 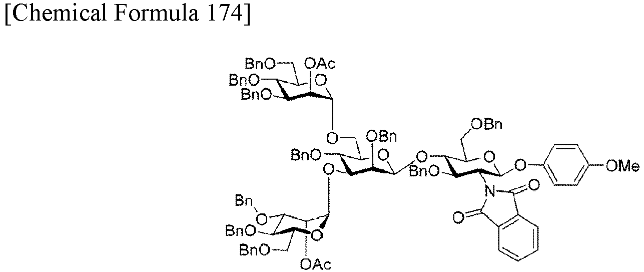 by reacting the compound represented by formula III-11 with the compound represented by formula IV-3. This step may be performed by using or applying a known procedure. However, the procedure shown in Example 28, for example, may be preferably used.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, tert-butyl dimethylsilyl trifluoromethanesulfonate, or a boron trifluoride diethyl ether complex. Preferred is trimethylsilyl trifluoromethanesulfonate.

The solvent in this step is not limited as long as the reaction proceeds. Examples include dichloromethane, toluene, trifluoromethylbenzene, or chlorobenzene. Preferred is dichloromethane or toluene.

The reaction temperature in this step is not limited as long as the reaction proceeds, but may be, for example, from -60°C to 0°C, preferably from -40°C to 0°C, more preferably from -30°C to 0°C, and particularly preferably from -20°C to 0°C.

In this step, it is preferable to use 2 to 4 equivalents of the compound represented by formula IV-3 with respect to 1 equivalent of the compound represented by formula III-11. It is more preferable to use 2.5 to 3 equivalents of the compound represented by formula IV-3 with respect to 1 equivalent of the compound represented by formula III-11.

### <Substep 2-7>

Substep 2-7 is a step of producing a compound represented by the following formula V-5: 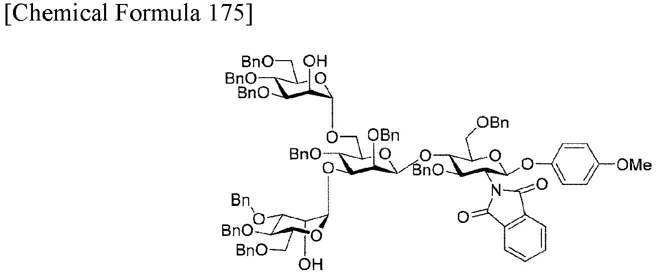 by eliminating the acetyl groups from the compound represented by formula V-4. This step may be performed by using or applying a known procedure. However, the procedure shown in Example 29, for example, may be preferably used.

In an embodiment of the present invention, substep 2-7 includes a step of producing the compound represented by formula V-5 by reacting the compound represented by formula V-4 with an alkoxide-based strong base in the presence of methyl trifluoroacetate. It is generally known that acetyl groups were deprotected using sodium methoxide in methanol. In this case, the phthalimide ring may be opened at the same time as an undesired side reaction. By contrast, a reaction with an alkoxide-based strong base may be carried out in the presence of methyl trifluoroacetate. This technique may be used to eliminate the acetyl groups while inhibiting the ring-opening of the phthalimide group.

The alkoxide-based strong base is not limited as long as the reaction proceeds. Examples include a sodium salt, a lithium salt, or a potassium salt of C1-C5 alkoxide, or a combination thereof. Preferable examples include lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide. Particularly preferable examples include sodium methoxide, lithium tert-butoxide, or potassium tert-butoxide.

The solvent in this step is not limited as long as the reaction proceeds. Examples include an alcohol solvent, e.g., methanol, ethanol, propanol, butanol, or a mixed solvent system of an alcohol solvent and, for instance, tetrahydrofuran, acetonitrile, cyclopentyl methyl ether, toluene, or dimethylacetamide. Preferred is methanol or a mixed solvent system of methanol and tetrahydrofuran.

The reaction temperature in this step is not limited as long as the reaction proceeds, but may be, for example, from -20°C to 80°C, preferably from 0°C to 70°C, more preferably from 15°C to 65°C, and particularly preferably from 30°C to 60°C.

### <Step 3>

Step 3 is a step of producing a compound represented by the following formula VI-3: 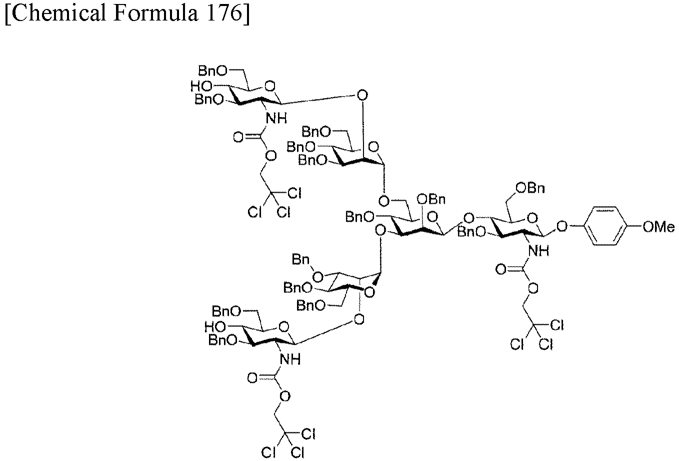 or a compound represented by the following formula VI-6: 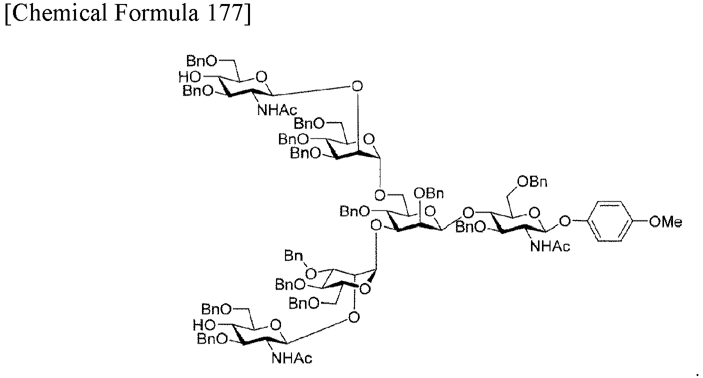 Step 3 includes essential substep 3-6 or 3-12 described below, in which two molecules of monosaccharide are regioselectively bonded to one molecule of tetrasaccharide. The other substeps can be carried out by using or applying usual procedures for producing a monosaccharide or oligosaccharide.

In an embodiment of the present invention, step 3 includes the following substeps: 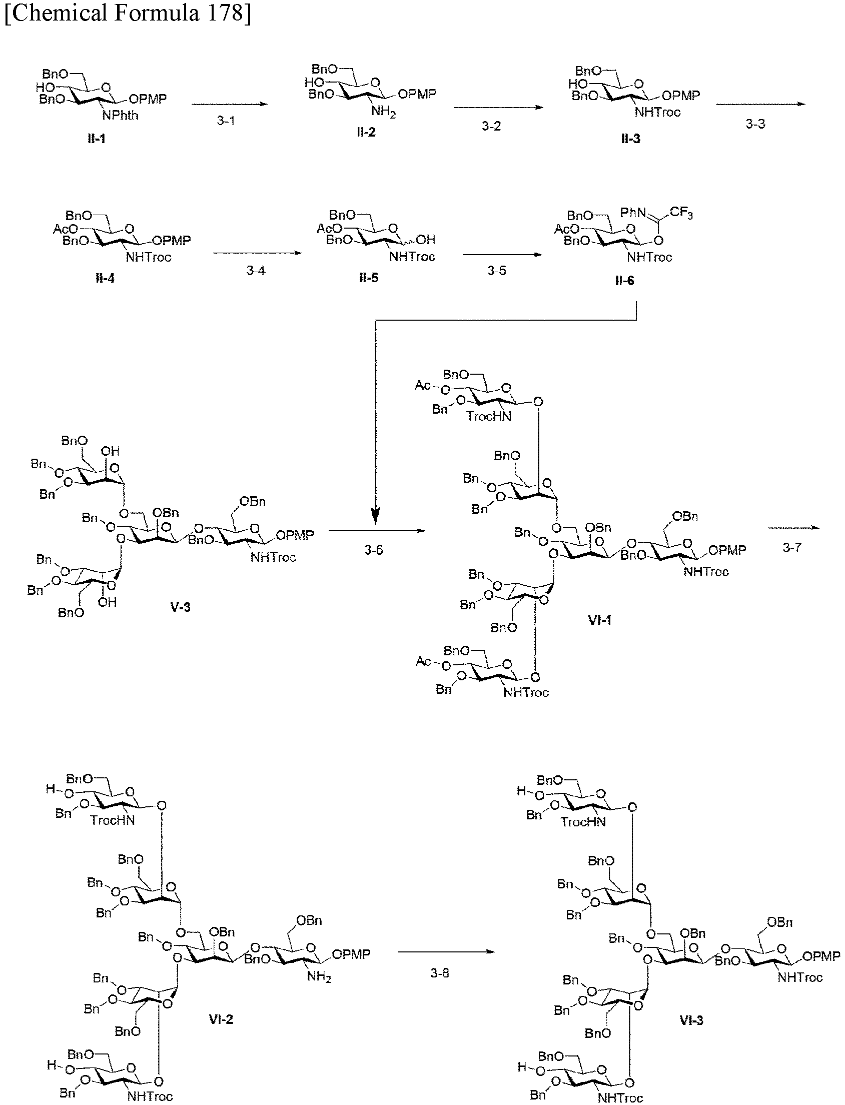 or

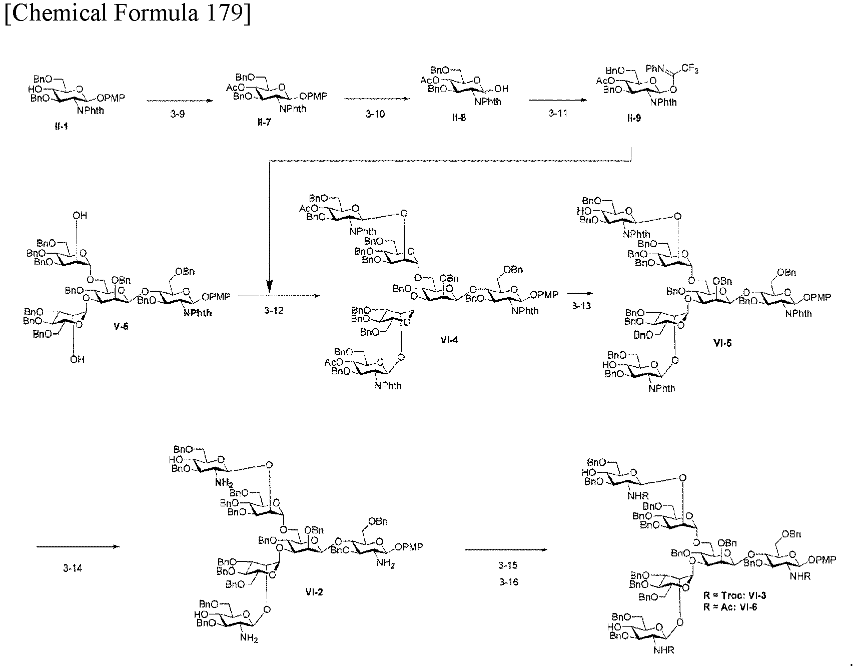

### <Substep 3-1>

Substep 3-1 is a step of producing a compound represented by the following formula II-2: 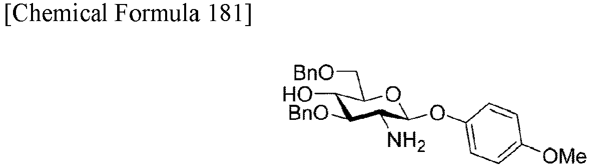 by eliminating the phthaloyl protecting group from a compound represented by the following formula II-1: 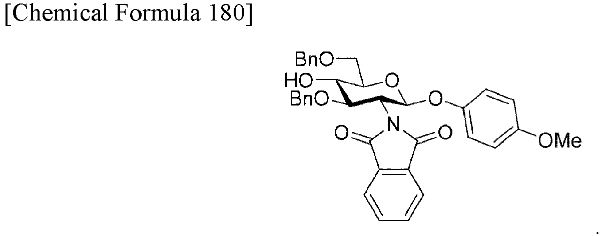 This step may be performed by using or applying a known procedure. However, the procedure shown in Example 30, for example, may be preferably used.

### <Substep 3-2>

Substep 3-2 is a step of producing a compound represented by the following formula II-3: 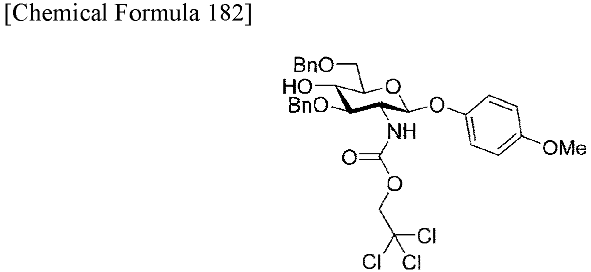 by protecting, by a 2,2,2-trichloroethoxycarbonyl group, the amino group in the compound represented by formula II-2. This step may be performed by using or applying a known procedure. However, the procedure shown in Example 31, for example, may be preferably used.

### <Substep 3-3>

Substep 3-3 is a step of producing a compound represented by the following formula II-4: 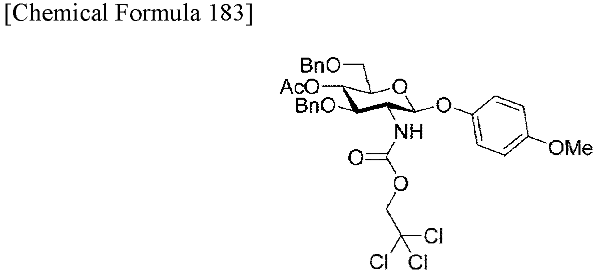 by protecting, by an acetyl group, the hydroxyl group on the compound represented by formula II-3. This step may be performed by using or applying a known procedure. However, the procedure shown in Example 32, for example, may be preferably used.

### <Substep 3-4>

Substep 3-4 is a step of producing a compound represented by the following formula II-5: 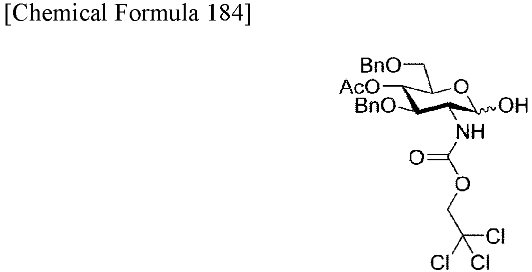 by eliminating the 4-methoxyphenyl group from the compound represented by formula II-4. This step may be performed by using or applying a known procedure. However, the procedure shown in Example 33, for example, may be preferably used.

In an embodiment of the present invention, this step is a step of producing the compound represented by formula II-5 by reacting the compound represented by formula II-4 with λ3-iodane in fluorous alcohol and water.

As used herein, the term "λ3-iodane" means a trivalent/hypervalent iodine compound. The use of λ3-iodane increases the yield of the compound represented by formula II-5 when compared to conventional deprotection processes. In addition, the reaction can be carried out under mild conditions. Here, since the reaction proceeds with a small excess of λ3-iodane, the purification of the deprotected product is easier than conventional deprotection procedures using, for example, an excess amount of cerium(IV) ammonium nitrate. Also, operation safety is high.

In an embodiment, λ3-iodane is a compound represented by formula R⁴-I(OR⁵)₂ (wherein R⁴ is an unsubstituted or substituted phenyl group and R⁵ is selected from the group consisting of H, acetoxy, trifluoroacetoxy, tosyloxy, methane sulfonyloxy, and a combination thereof). As defined in the above formula, R⁴ may be a "substituted phenyl group". Examples of the substituent include a linear or branched, saturated or unsaturated hydrocarbon group, an oxygen-containing group (e.g., alkoxy, ester), a nitrogen-containing group (e.g., cyano, azide), or a halogen (e.g., fluorine, chlorine, bromine, iodine). More preferred is a hydrocarbon group, an oxygen-containing substituent, or a halogen. When the above substituent contains carbon, for example, a C1-C5 or C1-C3 substituent may be preferably used. Specific examples of the λ3-iodane include, but are not limited to, [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, and [hydroxy(methanesulfonyloxy)iodo]benzene.

The amount of λ3-iodane can be set, if appropriate, but may be, for example, about 1 to 10 equivalents, about 1 to 7 equivalents, or about 1 to 5 equivalents, and preferably about 1 to 3 equivalents of the compound represented by formula II-4. Note that throughout this specification, the term "about" indicates a range of ±10% of the mentioned value.

As used herein, the term "fluorous alcohol" means a fluorine-containing alcohol compound in which all carbon atoms except those bonded to the alcohol have fluorine atoms. The fluorous alcohol preferably has a greater number of fluorine atoms as long as fluorine substitution is allowed. Examples of the fluorous alcohol include, but are not limited to, a fluorous aliphatic alcohol. The hydrocarbon moiety in the fluorous aliphatic alcohol may be saturated or unsaturated, linear or branched, or cyclic. The fluorous aliphatic alcohol is, for example, a fluorous C₂-C₈ aliphatic alcohol, preferably a fluorous C₂-C₅ aliphatic alcohol, and more preferably a fluorous C₂-C₃ aliphatic alcohol. Specific examples of the fluorous alcohol include, but are not limited to, the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.

Further, the fluorous alcohol and λ3-iodane may be preferably used in combination to give a deprotected product in higher yield. Such a combination can be selected, if appropriate, by a person skilled in the art. For example, PIFA is suitably used in combination with, for instance, hexafluoro-2-propanol (HFIP), 2,2,2-trifluoroethanol (TFE), or nonafluoro-tert-butyl alcohol. HTIB is suitably used in combination with, for instance, HFIP, TFE, or 2,2,3,3,4,4,5,5-octafluoro-1-pentanol. [Bis(trifluoroacetoxy)iodo]pentafluorobenzene is suitably used in combination with, for instance, hexafluoro-2-propanol (HFIP). [Hydroxy(methanesulfonyloxy)iodo]benzene is suitably used in combination with, for instance, hexafluoro-2-propanol (HFIP). The combination is not limited to the above examples.

The amount of fluorous alcohol can be set, if appropriate, from the viewpoint of, for instance, achieving a high yield of the product. For example, the amount may be about 1.0 equivalent or more, about 1.5 equivalents or more, about 2.0 equivalents or more, or 2.5 equivalents or more in a mole ratio based on the compound represented by formula II-4. The amount may be about 15 or less, about 10 or less, about 8 or less, or about 5 or less in a volume ratio based on the compound represented by formula II-4.

This step is carried out in the co-presence of the above-mentioned fluorous alcohol and "water". The amount of water can be set, if appropriate, from the viewpoint of, for instance, achieving a high yield of the product. For example, the amount may be about 1.0 equivalent or more, about 1.5 equivalents or more, about 2.0 equivalents or more, or about 2.5 equivalents or more in a mole ratio based on the compound represented by formula II-4. The amount may be about 10 or less, about 8 or less, about 5 or less, or about 3 or less in a volume ratio based on the compound represented by formula II-4.

In this step, an additional "solvent" may be added to the fluorous alcohol and water. The solvent may be selected from the group consisting of, but not limited to, dichloromethane, toluene, (trifluoromethyl)benzene, and a combination thereof. The type of solvent used can be selected, if appropriate, according to the λ3-iodane or the like used in order to achieve a high yield of the product. The amount of solvent can also be set, if appropriate, to achieve a high yield of the product, and may be, for example, about 0.5 to 50, about 1 to 20, or about 2 to 10 in a volume ratio based on the compound represented by formula II-4.

In this step, an additional "additive" may be added to the fluorous alcohol and water. The additive is preferably selected from the group consisting of sodium dihydrogen phosphate, potassium dihydrogen phosphate, disodium hydrogen phosphate, and a combination thereof. The acidity can increase as a de-4-methoxyphenylation reaction proceeds. In particular, λ3-iodane (e.g., HTIB), which is a byproduct of strongly acidic acid, may be used. In this case, a higher product yield can be obtained by adding an additive such as potassium dihydrogen phosphate mentioned above. The amount of additive can also be set, if appropriate, to achieve a high yield of the product, and may be, for example, about 0.5 to 8 equivalents, about 1 to 6 equivalents, or about 1.5 to 5 equivalents based on the compound represented by formula II-4.

When (diacetoxyiodo)benzene (PIDA) is used as λ3-iodane, trifluoroacetic acid (TFA) is preferably added to obtain the deprotected product in higher yield.

In this step, in the co-presence of fluorous alcohol and water, λ3-iodane acts as an oxidant for 4-methoxyphenyl (X group) in the compound represented by formula II-4. The OX group is then eliminated from the compound represented by formula II-4. After that, water (H₂O) is added thereto. As a result, the 4-methoxyphenoxy group (OX group) is easily eliminated from the compound represented by formula II-4. In this way, the effect seems to be achieved. Such an effect of λ3-iodane is generally achieved by, for instance, stirring and refluxing a solution containing the compound represented by formula II-4 and λ3-iodane in fluorous alcohol and water. Therefore, this step is simple to perform and can be scaled up relatively easily. The product, the compound represented by formula II-5, can be purified or isolated by any purification procedure known to those skilled in the art, such as crystallization or chromatography of the product.

The temperature at the time of reacting the compound represented by formula II-4 with λ3-iodane is preferably from about -20°C to the boiling point or lower of fluorous alcohols (e.g., about 58°C for hexafluoro-2-propanol (HFIP), about 78°C for 2,2,2-trifluoroethanol (TFE)), and may be, for example, about -20°C to 60°C, about 0°C to 60°C, or about 10°C to 30°C. In addition, the demethoxyphenylation reaction also proceeds at room temperature (15-30°C). Thus, the advantage is that neither cooling nor heating is needed.

Further, the reaction time can be set, if appropriate, to obtain the product in high yield.

### <Substep 3-5>

Substep 3-5 is a step of producing a compound represented by the following formula II-6: 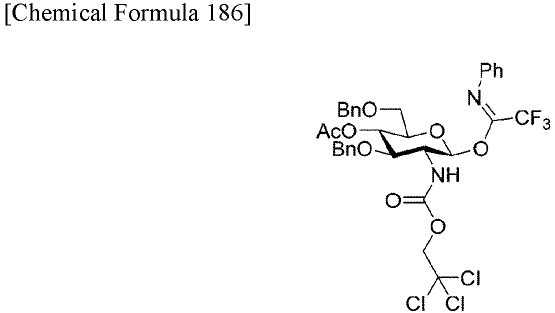 by reacting the compound represented by formula II-5 with 2,2,2-trifluoro-N-phenylacetimidoyl chloride (TFPC) represented by the following formula: 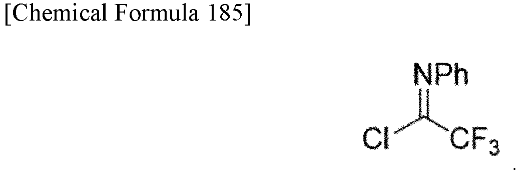 This step may be performed by using or applying a known procedure. However, the procedure shown in Example 34, for example, may be preferably used.

In an embodiment of the present invention, this step is a step of producing the compound represented by formula II-6 by reacting the compound represented by formula II-5 with TFPC in the presence of N-methylimidazole. For example, under certain conditions, in the case of using potassium carbonate as a base in this step, the yield is about 36%. However, in the case of using N-methylimidazole, the yield is significantly improved to about 76%. Since TFPC is an expensive reagent, an increase in the yield of this step is very beneficial for commercial production.

The solvent in this step is not limited as long as the reaction proceeds. Examples include dichloromethane, toluene, ethyl acetate, acetonitrile, or tetrahydrofuran. Preferred is dichloromethane.

The reaction temperature in this step is not limited as long as the reaction proceeds, but may be preferably from -20°C to 40°C, more preferably from -10°C to 35°C, and particularly preferably from 0°C to 30°C.

This step is suitably performed in the presence of a dehydrating agent. The dehydrating agent in this step is not limited as long as the reaction proceeds. Examples include Molecular Sieves. Preferred is Molecular Sieves 4A powder with a powder particle size of 10 µm or less.

The compound represented by formula II-6 that is produced in this step and dissolved in a solvent may be used as it is in the next step or may be isolated and purified by column or by recrystallization. Examples of the isolation and purification using a column include isolation and purification using silica gel as a stationary phase and a dichloromethane or toluene-ethyl acetate mixed solvent system as a mobile phase. On the other hand, examples of the isolation and purification by recrystallization include crystallization from a mixed solvent system containing diisopropyl ether and heptane. Note that the recrystallization may be preferably performed using seed crystals of the compound represented by formula II-6. In the case of using seed crystals, for example, the seed crystals are added into a diisopropyl ether solution, and after the crystals are found to precipitate, heptane is added dropwise to the solution. This operation may be used to obtain the compound.

### <Substep 3-6>

Substep 3-6 is a step of producing a compound represented by the following formula VI-1: 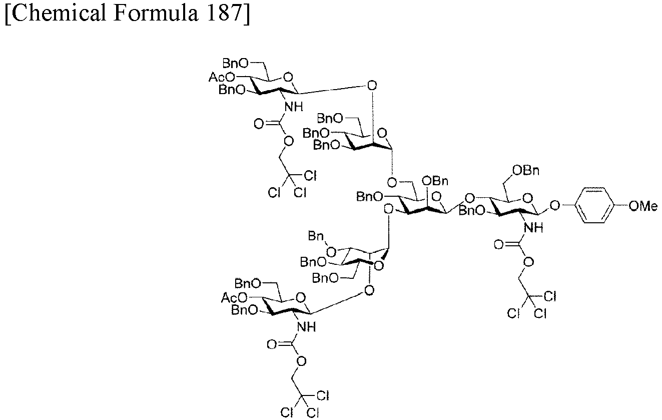 by reacting the compound represented by formula V-3 with the compound represented by formula II-6. This step may be performed by using or applying a known procedure. However, the procedure shown in Example 35, for example, may be preferably used.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, tert-butyl dimethylsilyl trifluoromethanesulfonate, or a boron trifluoride diethyl ether complex. Preferable examples include trimethylsilyl trifluoromethanesulfonate, triisopropylsilyl trifluoromethanesulfonate, or tert-butyl dimethylsilyl trifluoromethanesulfonate.

The solvent in this step is not limited as long as the reaction proceeds. Examples include dichloromethane, toluene, trifluoromethylbenzene, or chlorobenzene. Preferred is dichloromethane.

The reaction temperature in this step is not limited as long as the reaction proceeds, but may be, for example, from -78°C to 0°C, preferably from -78°C to - 20°C, more preferably from -78°C to -30°C, and particularly preferably from -78°C to -40°C.

In this step, it is preferable to use 2 to 5 equivalents of the compound represented by formula II-6 with respect to 1 equivalent of the compound represented by formula V-3. It is more preferable to use 3 to 5 equivalents of the compound represented by formula II-6 with respect to 1 equivalent of the compound represented by formula V-3.

### <Substep 3-7>

Substep 3-7 is a step of producing a compound represented by the following formula VI-2; 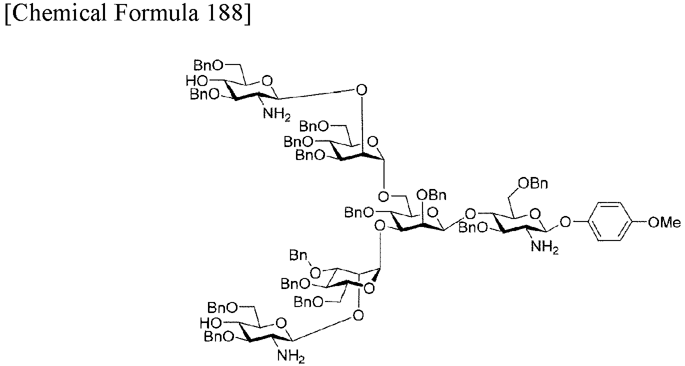 by eliminating the acetyl groups and the 2,2,2-trichloroethoxycarbonyl group from the compound represented by formula VI-1. This step may be performed by using or applying a known procedure. However, the procedure shown in Example 36, for example, may be preferably used.

### <Substep 3-8>

Substep 3-8 is a step of producing a compound represented by the following formula VI-3: 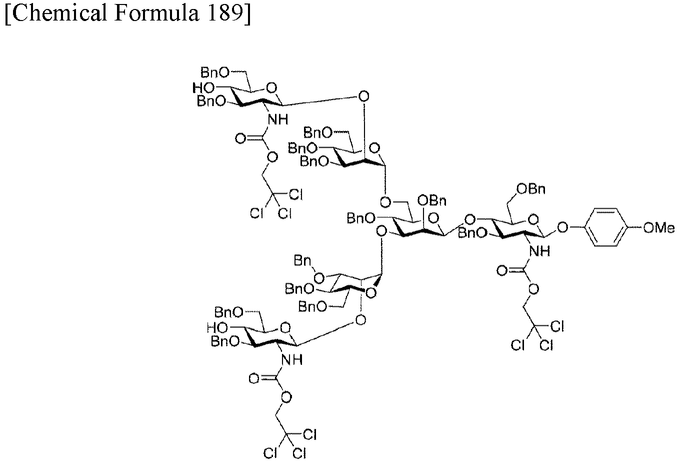 by protecting, by a 2,2,2-trichloroethoxycarbonyl group, the amino group in the compound represented by formula VI-2. This step may be performed by using or applying a known procedure. However, the procedure shown in Example 36, for example, may be preferably used.

### <Substep 3-9>

Substep 3-9 is a step of producing a compound represented by the following formula II-7: 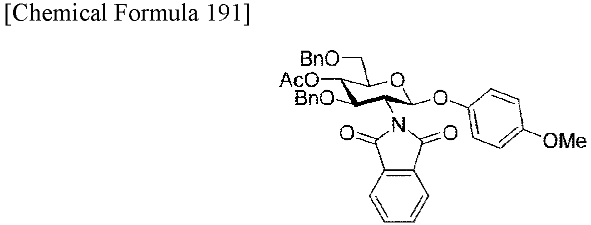 by protecting, by an acetyl group, the hydroxyl group on a compound represented by the following formula II-1: 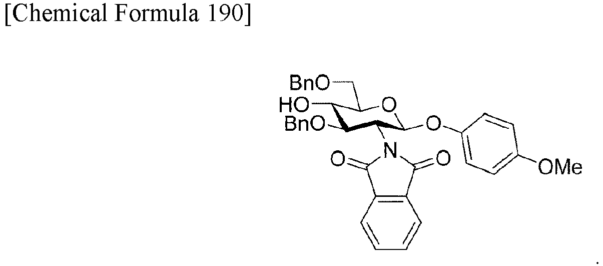 This step may be performed by using or applying a known procedure. However, the procedure shown in Example 37, for example, may be preferably used.

### <Substep 3-10>

Substep 3-10 is a step of producing a compound represented by the following formula II-8: 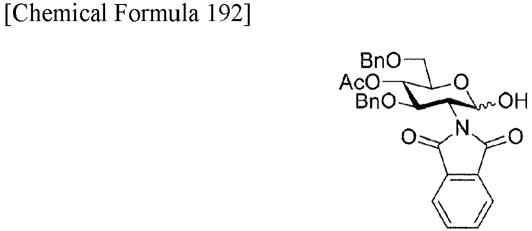 by eliminating the 4-methoxyphenyl group from the compound represented by formula II-7. This step may be performed by using or applying a known procedure. However, the procedure shown in Example 38, for example, may be preferably used.

In an embodiment of the present invention, this step is a step of producing the compound represented by formula II-8 by reacting the compound represented by formula II-7 with λ3-iodane in fluorous alcohol and water. For the details of this step, in the description of substep 3-4 herein, the "compound represented by formula II-4" and "compound represented by formula II-5" are read as and applied to the "compound represented by formula II-7" and "compound represented by formula II-8," respectively.

### <Substep 3-11>

Substep 3-11 is a step of producing a compound represented by the following formula II-9: 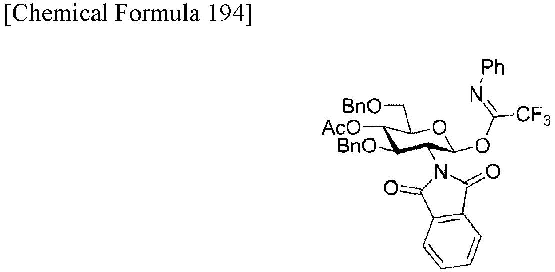 by reacting the compound represented by formula II-8 with 2,2,2-trifluoro-N-phenylacetimidoyl chloride (TFPC) represented by the following formula: 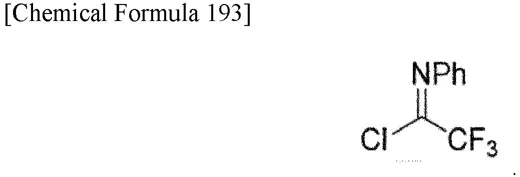 This step may be performed by using or applying a known procedure. However, the procedure shown in Example 39, for example, may be preferably used.

In an embodiment of the present invention, this step is a step of producing the compound represented by formula II-9 by reacting the compound represented by formula II-8 with TFPC in the presence of N-methylimidazole. The use of N-methylimidazole as a base allows the equivalent amount of TFPC to be reduced. In this case, the target product can still be obtained in high yield. Since TFPC is an expensive reagent, an increase in the yield of this step is very beneficial for commercial production.

The solvent in this step is not limited as long as the reaction proceeds. Examples include dichloromethane, toluene, ethyl acetate, acetonitrile, or tetrahydrofuran. Preferred is dichloromethane.

The reaction temperature in this step is not limited as long as the reaction proceeds, but may be preferably from -20°C to 40°C, more preferably from -10°C to 35°C, and particularly preferably from 0°C to 30°C.

This step is suitably performed in the presence of a dehydrating agent. The dehydrating agent in this step is not limited as long as the reaction proceeds. Examples include Molecular Sieves. Preferred is Molecular Sieves 4A powder with a powder particle size of 10 µm or less.

### <Substep 3-12>

Substep 3-12 is a step of producing a compound represented by the following formula VI-4: 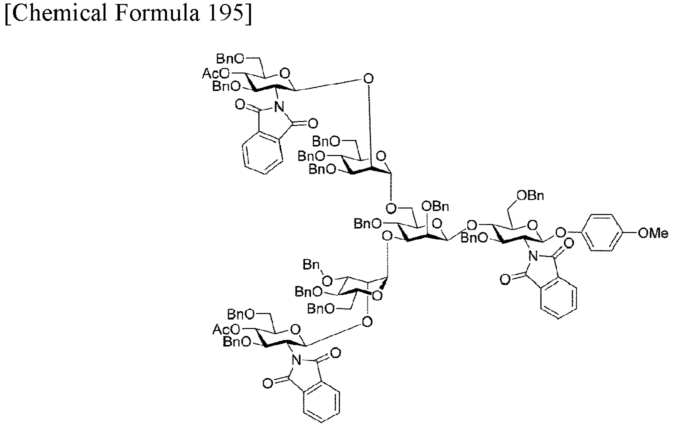 by reacting the compound represented by formula V-5 with the compound represented by formula II-9. This step may be performed by using or applying a known procedure. However, the procedure shown in Example 40, for example, may be preferably used.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, tert-butyl dimethylsilyl trifluoromethanesulfonate, or a boron trifluoride diethyl ether complex. Preferable examples include trimethylsilyl trifluoromethanesulfonate, triisopropyl silyl trifluoromethanesulfonate, or tert-butyl dimethylsilyl trifluoromethanesulfonate.

The solvent in this step is not limited as long as the reaction proceeds. Examples include dichloromethane, toluene, trifluoromethylbenzene, or chlorobenzene. Preferred is dichloromethane.

The reaction temperature in this step is not limited as long as the reaction proceeds, but may be, for example, from -78°C to 0°C, preferably from -78°C to - 20°C, more preferably from -78°C to -30°C, and particularly preferably from -78°C to -40°C.

In this step, it is preferable to use 2 to 5 equivalents of the compound represented by formula II-9 with respect to 1 equivalent of the compound represented by formula V-5. It is more preferable to use 3 to 5 equivalents of the compound represented by formula II-9 with respect to 1 equivalent of the compound represented by formula V-5.

### <Substep 3-13>

Substep 3-13 is a step of producing a compound represented by the following formula VI-5: 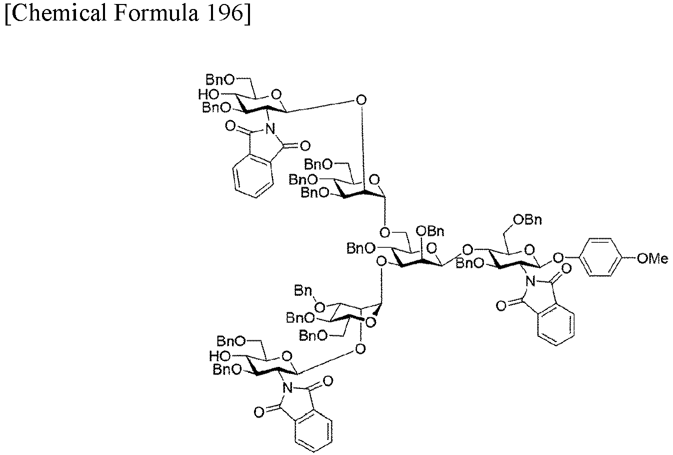 by eliminating the acetyl groups from the compound represented by formula VI-4. This step may be performed by using or applying a known procedure. However, the procedure shown in Example 41, for example, may be preferably used.

In an embodiment of the present invention, substep 3-13 is a step of producing the compound represented by formula VI-5 by reacting the compound represented by formula VI-4 with an alkoxide-based strong base in the presence of methyl trifluoroacetate. It is generally known that acetyl groups were deprotected using sodium methoxide in methanol. In this case, the phthalimide ring may be opened at the same time as an undesired side reaction. By contrast, a reaction with an alkoxide-based strong base may be carried out in the presence of methyl trifluoroacetate. This technique may be used to eliminate the acetyl groups while inhibiting the ring-opening of the phthalimide group.

The alkoxide-based strong base is not limited as long as the reaction proceeds. Examples include a sodium salt, a lithium salt, or a potassium salt of C1-C5 alkoxide, or a combination thereof. Preferable examples include lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide. Particularly preferable examples include sodium methoxide, lithium tert-butoxide, or potassium tert-butoxide.

The solvent in this step is not limited as long as the reaction proceeds. Examples include an alcohol solvent, e.g., methanol, ethanol, propanol, butanol, or a mixed solvent system of an alcohol solvent and, for instance, tetrahydrofuran, acetonitrile, cyclopentyl methyl ether, toluene, or dimethylacetamide. Preferred is methanol or a mixed solvent system of methanol and tetrahydrofuran.

The reaction temperature in this step is not limited as long as the reaction proceeds, but may be, for example, from -20°C to 80°C, preferably from 0°C to 70°C, more preferably from 15°C to 65°C, and particularly preferably from 30°C to 60°C.

### <Substep 3-14>

Substep 3-14 is a step of producing a compound represented by the following formula VI-2: 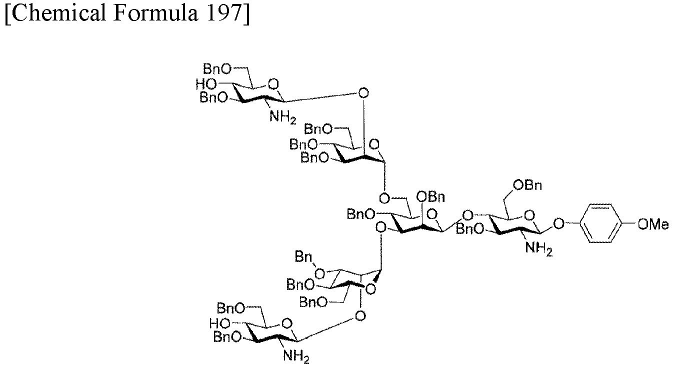 by eliminating the phthaloyl group from the compound represented by formula VI-5. This step may be performed by using or applying a known procedure. However, the procedure shown in Example 42, for example, may be preferably used.

### <Substep 3-15>

Substep 3-15 is a step of producing a compound represented by the following formula VI-3: 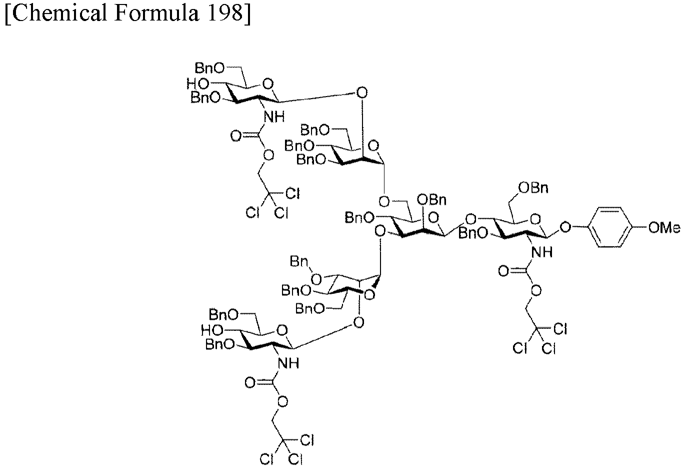 by protecting, by a 2,2,2-trichloroethoxycarbonyl group, the amino group in the compound represented by formula VI-2. This step may be performed by using or applying a known procedure. However, the procedure shown in Example 43, for example, may be preferably used.

### <Substep 3-16>

Substep 3-16 is a step of producing a compound represented by the following formula VI-6: 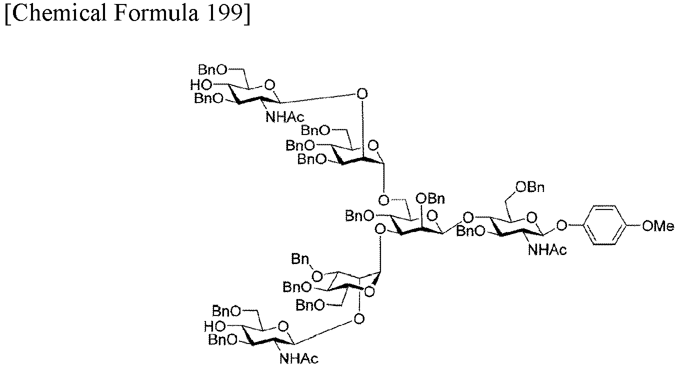 by protecting, by an acetyl group, the amino group in the compound represented by formula VI-2. This step may be performed by using or applying a known procedure. However, the procedure shown in Example 44, for example, may be preferably used.

### <Step 4>

Step 4 is a step of producing a compound represented by the following formula IX-5: 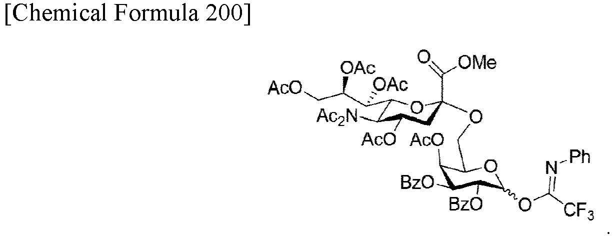 Step 4 includes essential substep 4-7 described below, in which two molecules of monosaccharide are subjected to α-2,6-glycosidic linkage to synthesize a disaccharide block. The other substeps can be carried out by using or applying usual procedures for producing a monosaccharide or oligosaccharide.

In an embodiment of the present invention, step 4 includes the following sub steps:

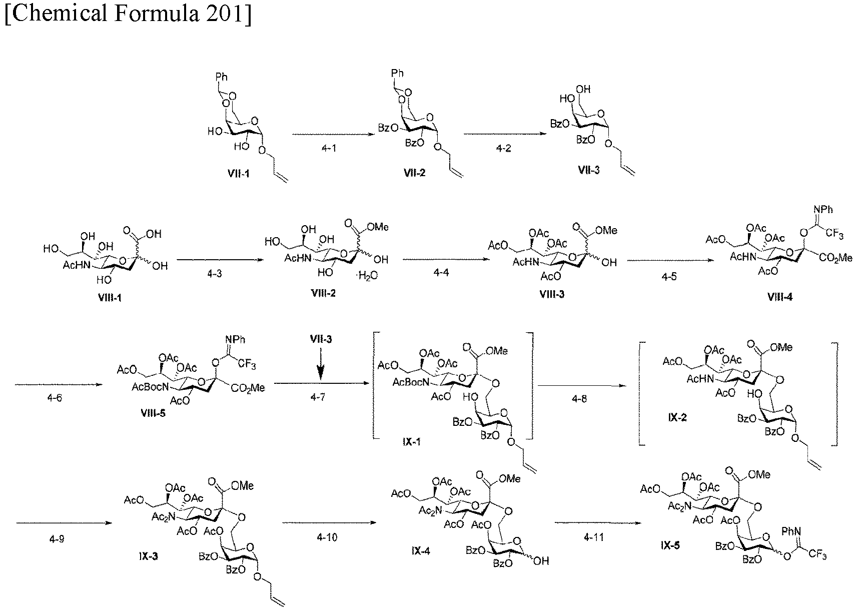

### <Substep 4-1>

Substep 4-1 is a step of producing a compound represented by the following formula VII-2: 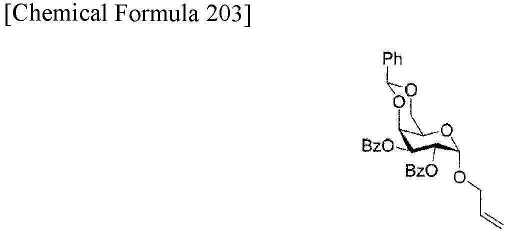 by protecting, by a benzoyl group, each hydroxyl group on the compound represented by formula VII-1: 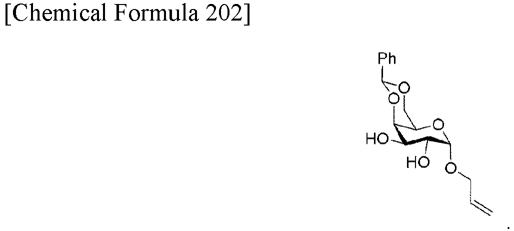 The compound represented by formula VII-1, which is the starting material for this step and is the compound specified as CAS No. 100759-10-2, may be produced by a known procedure. This step may be performed by using or applying a known procedure. However, the procedure shown in Example 45, for example, may be preferably used.

### <Substep 4-2>

Substep 4-2 is a step of producing a compound represented by the following formula VII-3: 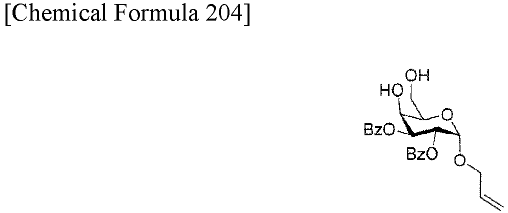 by eliminating the benzylidene protecting group from the compound represented by formula VII-2. This step may be performed by using or applying a known procedure. However, the procedure shown in Example 46, for example, may be preferably used.

In an embodiment of the present invention, this step includes a step of bringing a solvent dissolving the compound represented by formula VII-3 into contact with silica gel to perform solid-phase extraction of the compound represented by formula VII-3. Since an unreacted compound represented by formula VII-2 and eliminated benzaldehyde are not adsorbed on silica gel, the compound represented by formula VII-3 can be efficiently purified by this step.

Examples of the solvent used to dissolve the compound represented by formula VII-3 include toluene, heptane, dichloromethane, chloroform, or a combination thereof. Preferable examples include toluene, dichloromethane, chloroform, or a combination thereof. Particularly preferable examples include, but are not limited to, toluene.

As the silica gel in this step, silica gel in an amount 2 to 5 times the amount of the raw material, for example, may be used, silica gel in an amount 2 to 4 times the amount of the raw material may be preferably used, and silica gel in an amount about 3 times the amount of the raw material may be more preferably used.

In this step, the solvent used to elute the compound represented by formula VII-3 adsorbed on silica gel is not limited as long as the solvent does not dissolve the silica gel and can elute the target compound. Examples include cyclopentyl methyl ether, ethyl acetate, or tert-butyl methyl ether.

### <Substep 4-3>

Substep 4-3 is a step of producing a compound represented by the following formula VIII-2: 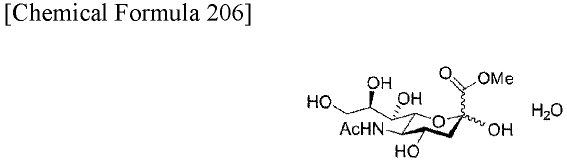 by subjecting the carboxylic acid of the compound represented by the following formula VIII-1: 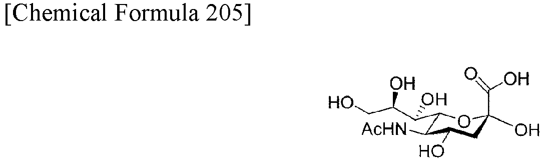 to esterification, followed by water pouring. The compound represented by formula VIII-1, which is the starting material for this step, may be produced by a known procedure or a commercially available product may be used. Examples of the commercially available compound represented by formula VIII-1 include N-acetylneuraminic acid, manufactured by TOKYO CHEMICAL INDUSTRY CO., LTD. This step may be performed by using or applying a known procedure. However, the procedure shown in Example 47, for example, may be preferably used.

### <Substep 4-4>

Substep 4-4 is a step of producing a compound represented by the following formula VIII-3: 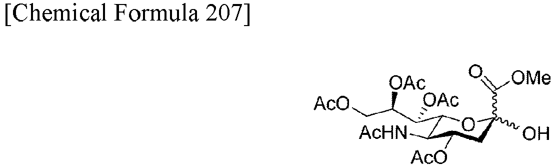 by protecting, by an acetyl group, each hydroxyl group other than the hydroxyl group attached to the carbon at position 1 of the compound represented by formula VIII-2. This step may be performed by using or applying a known procedure. However, the procedure shown in Example 48, for example, may be preferably used.

### <Substep 4-5>

Substep 4-5 is a step of producing a compound represented by the following formula VIII-4: 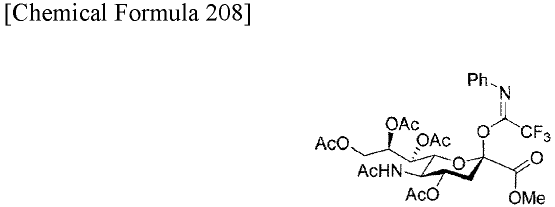 by reacting the compound represented by formula VIII-3 with 2,2,2-trifluoro-N-phenylacetimidoyl chloride (TFPC). This step may be performed by using or applying a known procedure. However, the procedure shown in Example 49, for example, may be preferably used.

In an embodiment of the present invention, this step is a step of producing the compound represented by formula VIII-4 by reacting the compound represented by formula VIII-3 with TFPC in the presence of N-methylimidazole. Compared to the case of using potassium carbonate, N-methylimidazole may be used as a base in this step. In this case, the equivalent amount of TFPC can be reduced, and the target product can be obtained in high yield. Since TFPC is an expensive reagent, an increase in the yield of this step is very beneficial for commercial production.

The solvent in this step is not limited as long as the reaction proceeds. Examples include dichloromethane, toluene, ethyl acetate, acetonitrile, or tetrahydrofuran. Preferred is dichloromethane.

The reaction temperature in this step is not limited as long as the reaction proceeds, but may be preferably from 20°C to 40°C, more preferably from 10°C to 35°C, and particularly preferably from 0°C to 30°C.

This step is suitably performed in the presence of a dehydrating agent. The dehydrating agent in this step is not limited as long as the reaction proceeds. Examples include Molecular Sieves. Preferred is Molecular Sieves 4A powder with a powder particle size of 10 µm or less.

### <Substep 4-6>

Substep 4-6 is a step of producing a compound represented by the following formula VIII-5: 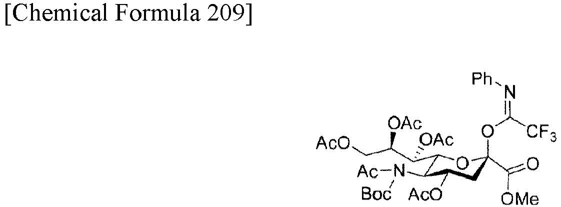 by protecting, by a tert-butoxycarbonyl group, the nitrogen atom of the acetamide group in the compound represented by formula VIII-4. This step may be performed by using or applying a known procedure. However, the procedure shown in Example 50, for example, may be preferably used.

The compound represented by formula VIII-5 that is produced in this step and dissolved in a solvent may be used as it is in the next step or may be isolated and purified by recrystallization. The major advantage of the compound represented by formula VIII-5 is that the compound can be isolated and purified by crystallization. The compound represented by formula VIII-5 with HPLC purity of 99% or higher can be obtained. Also, no impurities are included. Thus, a glycosylation reaction in the next step can be carried out stably. The isolation and purification by recrystallization may be performed by a procedure including adding heptane to a cyclopentyl methyl ether-containing solution for crystallization.

### <Substep 4-7>

Substep 4-7 is a step of producing a compound represented by the following formula IX-1: 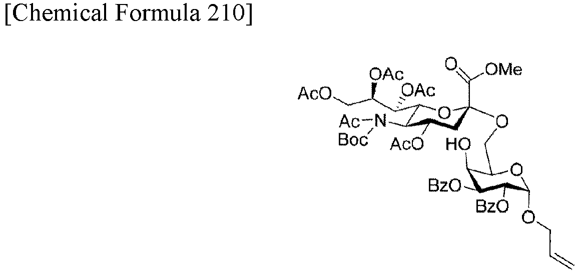 by subjecting the compound represented by formula VIII-5 and the compound represented by formula VII-3 to α-2,6-glycosidic linkage. It is difficult that an N-acetylneuraminic acid derivative and a galactose derivative are selectively linked by α-2,6-glycosidic linkage. For example, a method for synthesizing a disaccharide by reacting a compound represented by formula VIII-4 with a compound represented by formula VII-3 has been reported (J. Org. Chem., 2016, 81, 10600-10616). The reaction was not easy to reproduce, and the desired yield and selectivity could not be obtained. Unfortunately, in this reaction, the selectivity decreases as the scale increases, the reaction temperature tolerance is narrow, and the effect of reaction heat is significant. The compound represented by formula VIII-4, which is one of the raw material compounds in this reaction, is very expensive. Therefore, the low reproducibility, yield, and selectivity in this reaction are a major problem, especially in commercial production where scale-up is required. On the other hand, if the compound represented by formula VIII-5 with a tert-butoxycarbonyl group is used instead of the compound represented by formula VIII-4 as a raw material compound, the high selectivity for α-2,6-glycosidic linkage (α:β = 93:7) can be well-reproduced, and the yield is also improved. In addition, the temperature tolerance is wider, and high reproducibility, yield, and selectivity can be achieved even when scaled up. This has a very beneficial effect on commercial production.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, or tert-butyl dimethylsilyl trifluoromethanesulfonate. Preferred is trimethylsilyl trifluoromethanesulfonate.

The solvent in this step is not limited as long as the reaction proceeds. Examples include diisopropyl ether, tert-butyl methyl ether, diethyl ether, dibutyl ether, dipropyl ether, 1,4-dioxane, dichloromethane, 1,2-dichloroethane, toluene, chlorobenzene, trifluoromethylbenzene, propionitrile, or acetonitrile. Preferred is cyclopentyl methyl ether.

The reaction temperature in this step is not limited as long as the reaction proceeds, but may be, for example, from -78°C to 0°C, preferably from -78°C to - 20°C, more preferably from -78°C to -30°C, and particularly preferably from -78°C to -40°C.

In this step, it is preferable to use 1 to 3 equivalents of the compound represented by formula VII-3 with respect to 1 equivalent of the compound represented by formula VIII-5. It is more preferable to use 1.4 to 2 equivalents of the compound represented by formula VII-3 with respect to 1 equivalent of the compound represented by formula VIII-5.

This step is not limited as long as the reaction proceeds. For example, a mixed solution containing the compound represented by formula VIII-5 and the compound represented by formula VII-3 (suitably, a cyclopentyl methyl ether mixed solution) is added dropwise over a long time to a Lewis acid-containing solution (suitably, cyclopentyl methyl ether solution). Alternatively, a solution containing the compound represented by formula VIII-5 (suitably, a cyclopentyl methyl ether solution) is added dropwise over a long time to a solution containing a Lewis acid and the compound represented by formula VII-3 (suitably, cyclopentyl methyl ether solution). Preferably, a solution containing the compound represented by formula VIII-5 (suitably, a cyclopentyl methyl ether solution) is added dropwise over a long time to a solution containing a Lewis acid and the compound represented by formula VII-3 (suitably, cyclopentyl methyl ether solution). The dropping time is not limited as long as the reaction proceeds, but may be, for example, from 30 minutes to 5 hours, preferably from 1 to 4 hours, more preferably from 2 to 3.5 hours, and particularly preferably about 3 hours.

In an embodiment of the present invention, this step includes a step of bringing a solvent dissolving the compound represented by formula IX-1 into contact with silica gel to perform solid-phase extraction of the compound represented by formula IX-1. N-phenyl trifluoroacetamide, a byproduct of the glycosylation reaction, and other trace impurities in the toluene solvent that are not adsorbed on silica gel are not adsorbed on silica gel. Therefore, the compound represented by formula IX-1 can be efficiently purified by this step.

Examples of the solvent used to dissolve the compound represented by formula IX-1 include toluene, heptane, dichloromethane, chloroform, or a combination thereof. Preferable examples include toluene, dichloromethane, chloroform, or a combination thereof. Particularly preferable examples include, but are not limited to, toluene.

As the silica gel in this step, silica gel in an amount 2 to 5 times the amount of the raw material, for example, may be used, silica gel in an amount 2 to 4 times the amount of the raw material may be preferably used, and silica gel in an amount about 3.5 times the amount of the raw material may be more preferably used.

In this step, the solvent used to elute the compound represented by formula IX-1 adsorbed on silica gel is not limited as long as the solvent does not dissolve the silica gel and can elute the target compound. Examples include ethyl acetate, cyclopentyl methyl ether, or tert-butyl methyl ether. Preferred is ethyl acetate.

This step may be performed using, for instance, the procedure shown in Example 51.

### <Substep 4-8>

Substep 4-8 is a step of producing a compound represented by the following formula IX-2; 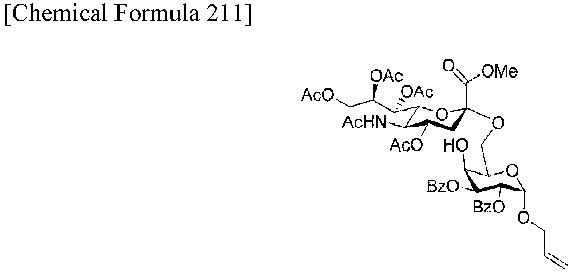 by eliminating the tert-butoxycarbonyl group from the compound represented by formula IX-1. This step may be performed by using or applying a known procedure. However, the procedure shown in Example 52, for example, may be preferably used.

### <Substep 4-9>

Substep 4-9 is a step of producing a compound represented by the following formula IX-3: 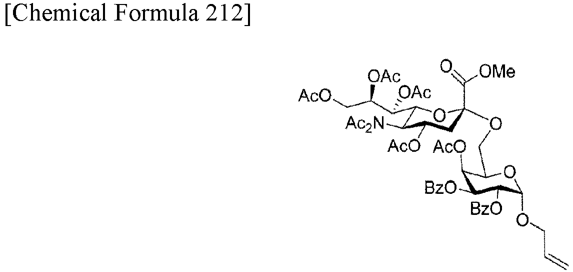 by further protecting, by acetyl groups, the hydroxyl group and the nitrogen atom of the acetamide group in the compound represented by formula IX-2. This step may be performed by using or applying a known procedure. However, the procedure shown in Example 53, for example, may be preferably used.

In an embodiment of the present invention, this step includes a step of bringing a solvent dissolving the compound represented by formula IX-3 into contact with silica gel to perform solid-phase extraction of the compound represented by formula IX-3. The compound represented by formula VII-3 used in excess during the upstream glycosylation reaction is acetylated to give, for instance, a diacetyl form of the compound represented by formula VII-3. This byproduct is not adsorbed on silica gel, so that this step allows the compound represented by formula IX-3 to be efficiency purified.

Examples of the solvent used to dissolve the compound represented by formula IX-3 include toluene, heptane, dichloromethane, chloroform, or a combination thereof. Preferable examples include toluene, dichloromethane, chloroform, or a combination thereof. Particularly preferable examples include, but are not limited to, toluene.

As the silica gel in this step, silica gel in an amount 2 to 5 times the amount of the raw material, for example, may be used, silica gel in an amount 2 to 4 times the amount of the raw material may be preferably used, and silica gel in an amount about 3.5 times the amount of the raw material may be more preferably used.

In this step, the solvent used to elute the compound represented by formula IX-3 adsorbed on silica gel is not limited as long as the solvent does not dissolve the silica gel and can elute the target compound. Examples include ethyl acetate, cyclopentyl methyl ether, or tert-butyl methyl ether. Preferred is ethyl acetate.

### <Substep 4-10>

Substep 4-10 is a step of producing a compound represented by the following formula IX-4: 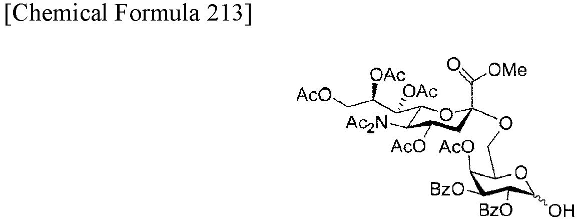 by eliminating the allyl group attached to the carbon at position 1 of D-galactopyranoside in the compound represented by formula IX-3. This step may be performed by using or applying a known procedure. However, the procedure shown in Example 54-1 and/or 54-2, for example, may be preferably used.

### <Substep 4-11>

Substep 4-11 is a step of producing a compound represented by the following formula IX-5: 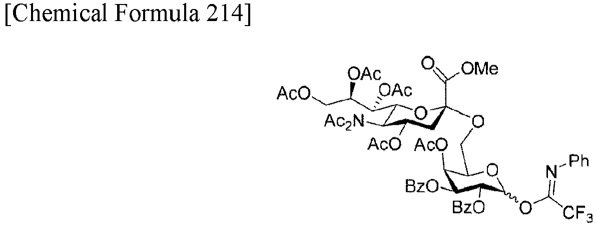 by reacting the compound represented by formula IX-4 with 2,2,2-trifluoro-N-phenylacetimidoyl chloride (TFPC). This step may be performed by using or applying a known procedure. However, the procedure shown in Example 55, for example, may be preferably used.

### <Step 5>

Step 5 is a step of producing an oligosaccharide represented by the following formula XI: 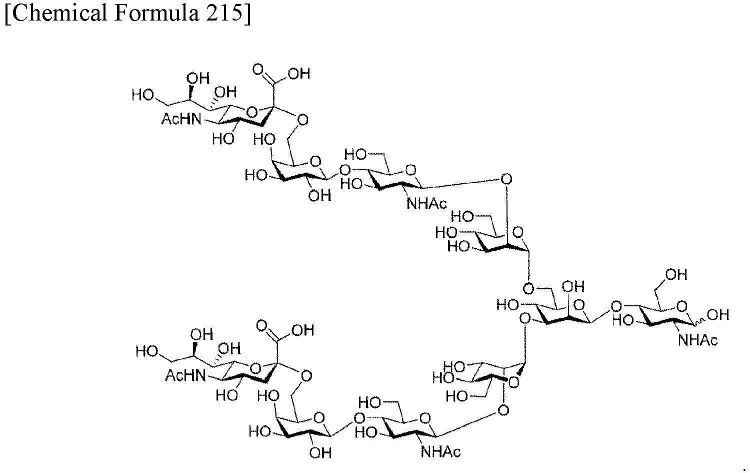 Step 5 includes essential substep 5-1 or 5-9 described below, in which two molecules of monosaccharide are linked to one molecule of hexasaccharide. The other substeps can be carried out by using or applying usual procedures for producing a monosaccharide or oligosaccharide.

In an embodiment of the present invention, step 5 includes the following sub steps:

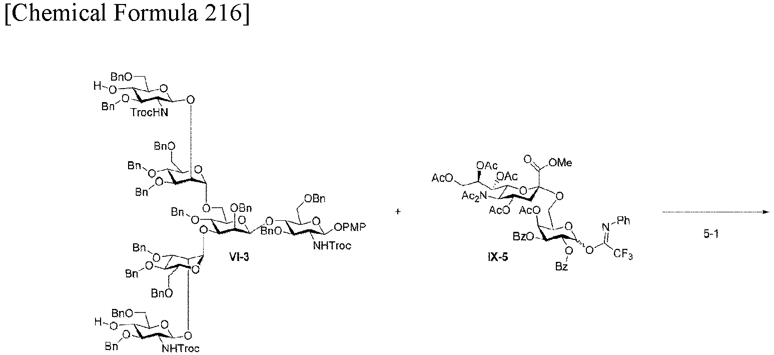

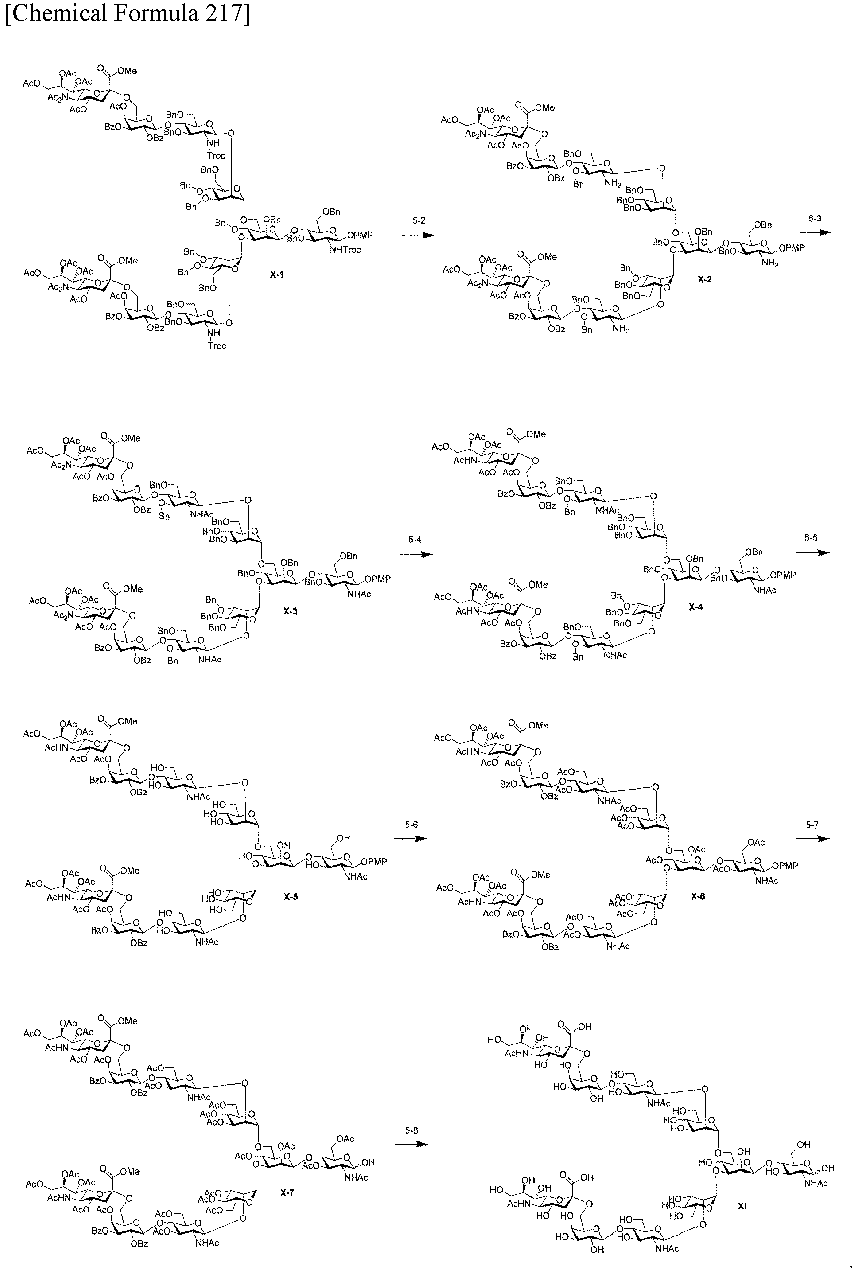

### <Substep 5-1>

Substep 5-1 is a step of producing a compound represented by the following formula X-1 : 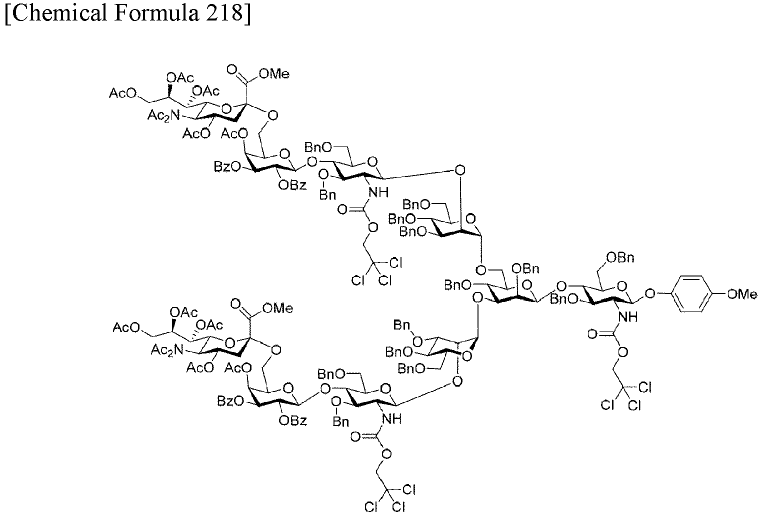 by reacting the compound represented by formula VI-3 with the compound represented by formula IX-5. This step may be performed by using or applying a known procedure. However, the procedure shown in Example 56, for example, may be preferably used.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, or tert-butyl dimethylsilyl trifluoromethanesulfonate. Preferred is tert-butyl dimethylsilyl trifluoromethanesulfonate.

The solvent in this step is not limited as long as the reaction proceeds. Examples include dichloromethane, toluene, trifluoromethylbenzene, or chlorobenzene.

The reaction temperature in this step is not limited as long as the reaction proceeds, but may be, for example, from -40°C to 20°C, preferably from -30°C to 10°C, more preferably from -20°C to 5°C, and particularly preferably from -10°C to 0°C.

In this step, it is preferable to use 2 to 5 equivalents of the compound represented by formula IX-5 with respect to 1 equivalent of the compound represented by formula VI-3. It is more preferable to use 3 to 4 equivalents of the compound represented by formula IX-5 with respect to 1 equivalent of the compound represented by formula VI-3.

### <Substep 5-2>

Substep 5-2 is a step of producing a compound represented by the following formula X-2; 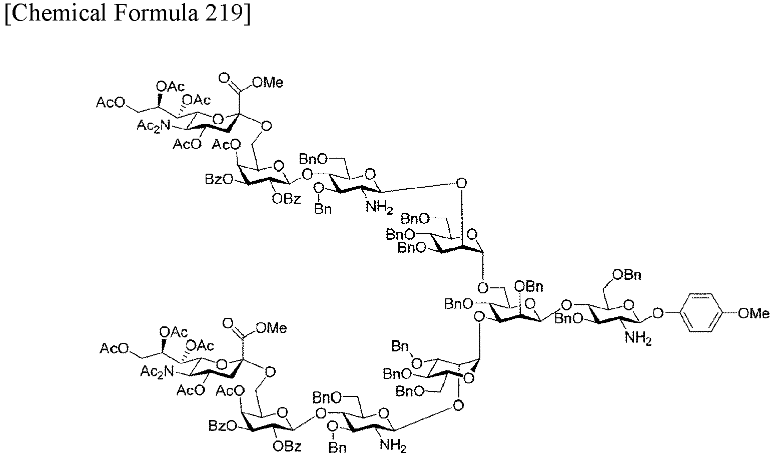 by eliminating the 2,2,2-trichloroethoxycarbonyl group from the compound represented by formula X-1. This step may be performed by using or applying a known procedure. However, the procedure shown in Example 57, for example, may be preferably used.

### <Substep 5-3>

Substep 5-3 is a step of producing a compound represented by the following formula X-3: 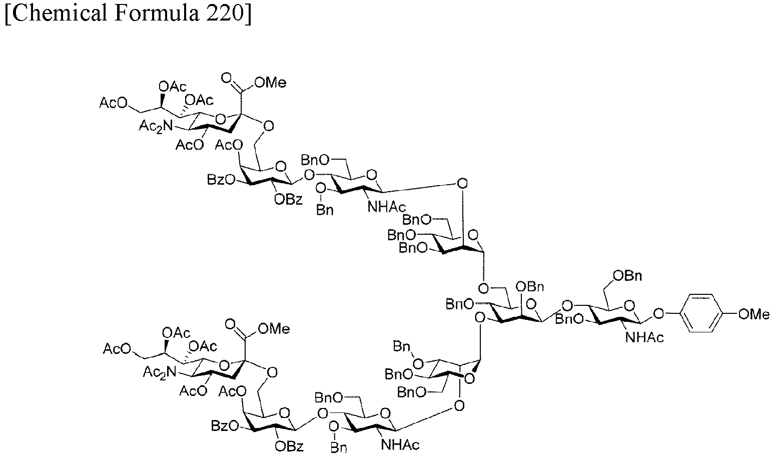 by protecting, by an acetyl group, the amino group in the compound represented by formula X-2. This step may be performed by using or applying a known procedure. However, the procedure shown in Example 58, for example, may be preferably used.

### <Substep 5-4>

Substep 5-4 is a step of producing a compound represented by the following formula X-4: 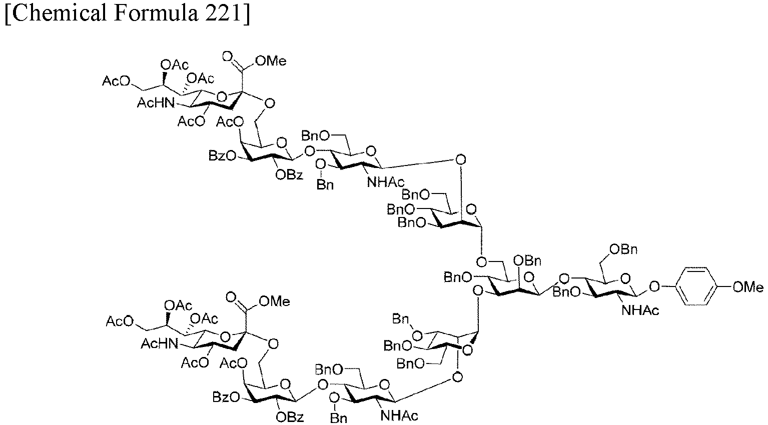 by eliminating only one acetyl group from the diacetylamino group on each D-galacto-non-2-uropyranoside in the compound represented by formula X-3. This step may be performed by using or applying a known procedure. However, the procedure shown in Example 59, for example, may be preferably used.

### <Substep 5-5>

Substep 5-5 is a step of producing a compound represented by the following formula X-5: 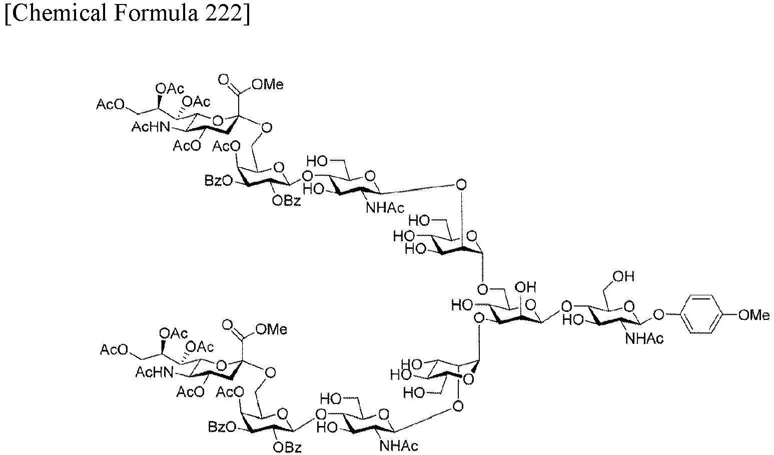 by eliminating the benzyl groups from the compound represented by formula X-4. This step may be performed by using or applying a known procedure. However, the procedure shown in Example 60, for example, may be preferably used.

### <Substep 5-6>

Substep 5-6 is a step of producing a compound represented by the following formula X-6: 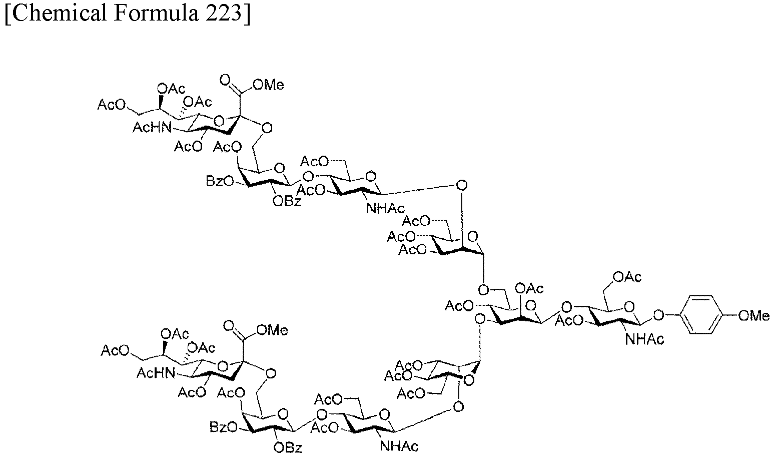 by protecting, by an acetyl group, each hydroxyl group in the compound represented by formula X-5. This step may be performed by using or applying a known procedure. However, the procedure shown in Example 61, for example, may be preferably used.

### <Substep 5-7>

Substep 5-7 is a step of producing a compound represented by the following formula X-7: 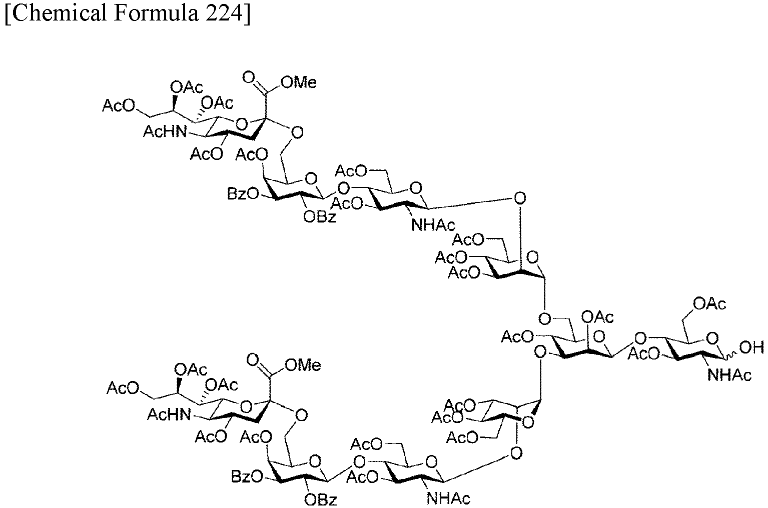 by eliminating the 4-methoxyphenyl group from the compound represented by formula X-6. This step may be performed by using or applying a known procedure. However, the procedure shown in Example 62, for example, may be preferably used.

### <Substep 5-8>

Substep 5-8 is a step of producing an oligosaccharide represented by the following formula XI: 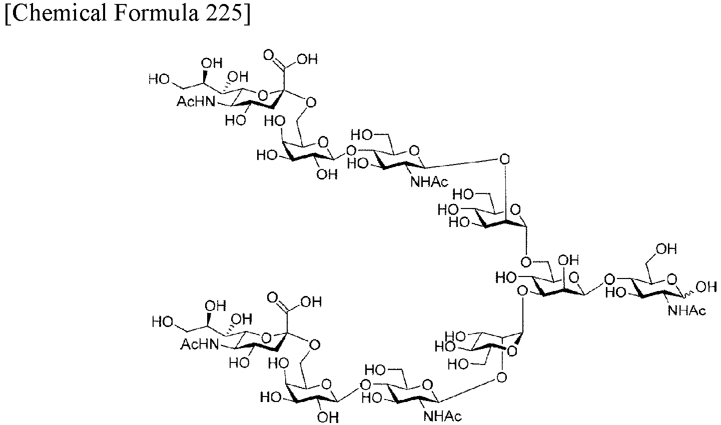 by eliminating the hydroxyl group-protecting acetyl groups and benzoyl groups and eliminating the carboxylic acid-protecting methyl groups from the compound represented by formula X-7. This step may be performed by using or applying a known procedure. However, the procedure shown in Example 63, for example, may be preferably used.

In an embodiment of the present invention, step 5 includes, instead of substeps 5-1 to 5-3, the following substep 5-9:

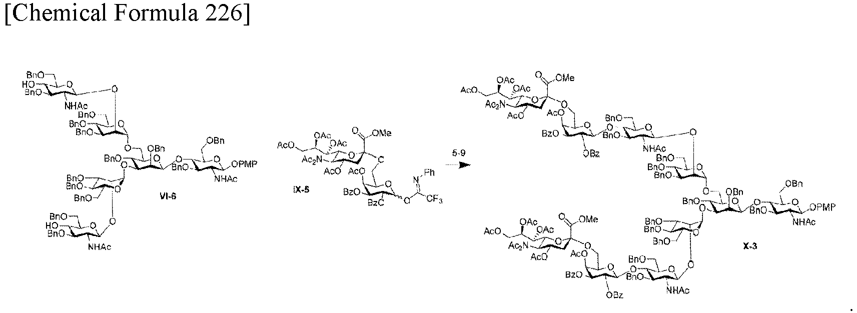

### <Substep 5-9>

Substep 5-9 is a step of producing the compound represented by formula X-3 by reacting the compound represented by formula VI-6 with the compound represented by formula IX-5. This step may be performed by using or applying a known procedure. However, the procedure shown in Example 64, for example, may be preferably used.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, or tert-butyl dimethylsilyl trifluoromethanesulfonate. Preferred is tert-butyl dimethylsilyl trifluoromethanesulfonate.

The solvent in this step is not limited as long as the reaction proceeds. Examples include dichloromethane, toluene, trifluoromethylbenzene, or chlorobenzene.

The reaction temperature in this step is not limited as long as the reaction proceeds, but may be, for example, from -40°C to 20°C, preferably from -30°C to 10°C, more preferably from -20°C to 5°C, and particularly preferably from -10°C to 0°C.

In this step, it is preferable to use 2 to 5 equivalents of the compound represented by formula IX-5 with respect to 1 equivalent of the compound represented by formula VI-6. It is more preferable to use 3 to 4 equivalents of the compound represented by formula IX-5 with respect to 1 equivalent of the compound represented by formula VI-6.

Note that step 1 through step 5 and each substep included therein are not necessarily carried out in the order as described herein. For example, substeps 2-1 and 2-2 in step 2 may be performed prior to step 1. In particular, step 4 may be performed prior to any of steps 1 to 3.

### <2. Novel method for producing monosaccharide or oligosaccharide>

An embodiment of the present invention provides a novel method for producing a monosaccharide or oligosaccharide useful in the novel method for pure-chemically producing a biantennary N-glycan with an α2,6-sialic acid structure at each non-reducing end. As used herein, the term "oligosaccharide" means a sugar oligomer in which two or more monosaccharides are bonded together via glycosidic linkage. In the present invention, the "monosaccharide" or "oligosaccharide" may have, for instance, a protecting group and/or an activating group introduced at each specific position.

The methods described below are useful in the method for producing an oligosaccharide shown in formula XI as detailed in <1> above, but are not limited to this application and can be applied to any of the applications. For example, the novel method for producing a monosaccharide or oligosaccharide according to the present invention can be used during the production of oligosaccharide represented by formula XI in a different way from the method for producing an oligosaccharide represented by formula XI as detailed above. The novel method for producing a monosaccharide or oligosaccharide according to the present invention can be used as the monosaccharide or oligosaccharide method as it is, or during the production of sugar other than the oligosaccharide represented by formula XI.

An embodiment of the present invention provides a method for producing a compound represented by the following formula III-2: 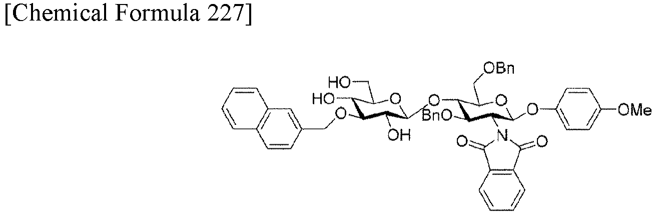 the method comprising a step of
reacting, in the presence of methyl trifluoroacetate, a compound represented by the following formula III-1: 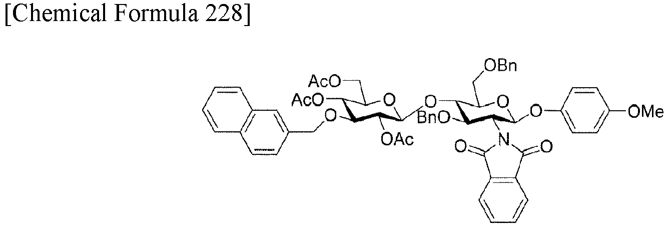
with an alkoxide-based strong base to give the compound represented by formula III-2.

A report (Org. Biomol. Chem., 2018, 16, 4720-4727) shows that the acetyl groups were eliminated using sodium methoxide in methanol. In this case, the phthalimide ring may be opened at the same time as an undesired side reaction. On the other hand, by using the technique of reaction with an alkoxide-based strong base in the presence of methyl trifluoroacetate, the acetyl group can be eliminated while the ring-opening of the phthalimide group is suppressed.

The alkoxide-based strong base is not limited as long as the reaction proceeds. Examples include a sodium salt, a lithium salt, or a potassium salt of C1-C5 alkoxide, or a combination thereof. Preferable examples include lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide. Particularly preferable examples include sodium methoxide, lithium tert-butoxide, or potassium tert-butoxide.

The solvent in this step is not limited as long as the reaction proceeds. Examples include an alcohol solvent, e.g., methanol, ethanol, propanol, butanol, or a mixed solvent system of an alcohol solvent and, for instance, tetrahydrofuran, acetonitrile, cyclopentyl methyl ether, toluene, or dimethylacetamide. Preferred is methanol or a mixed solvent system of methanol and tetrahydrofuran.

The reaction temperature in this step is not limited as long as the reaction proceeds, but may be, for example, from -20°C to 80°C, preferably from 0°C to 70°C, more preferably from 20°C to 65°C, and particularly preferably from 40°C to 60°C.

This method may be preferably performed using, for instance, the procedure shown in Example 6 or 16.

An embodiment of the present invention provides a method for producing a compound represented by the following formula III-5: 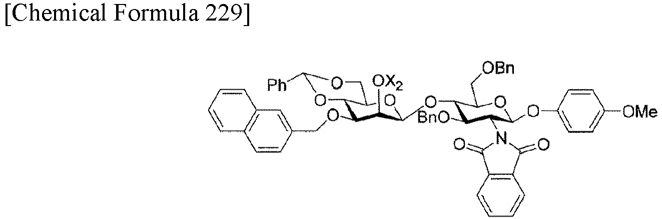
wherein X₂ is an acetyl group,
   the method comprising a step of
reacting a compound represented by the following formula III-4: 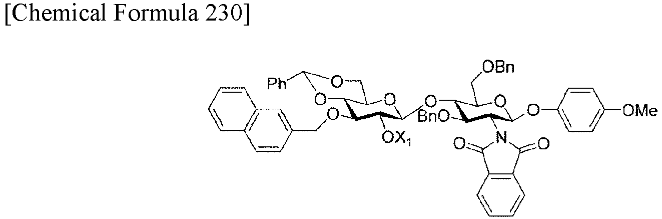
wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group
with cesium acetate or tetrabutylammonium acetate to give the compound represented by formula III-5, or
   the method for producing a compound represented by the following formula III-5: 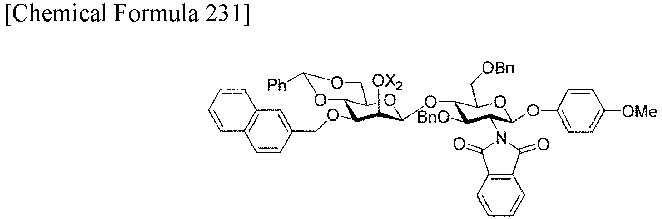
wherein X₂ is a benzoyl group,
   the method comprising a step of
reacting a compound represented by the following formula III-4: 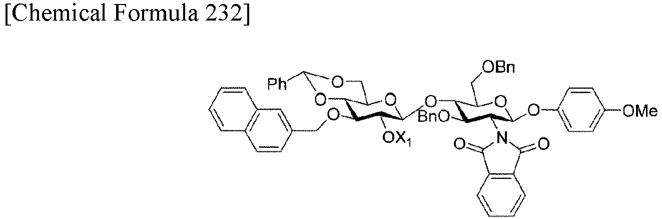
wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group
with tetrabutylammonium benzoate to give the compound represented by formula III-5.

There are known conversion reactions, namely stereoinversions, from glucose -> mannose. However, no conversion has been reported when a 2-naphthylmethyl (Nap) group is the protecting group of the hydroxyl group attached to the carbon at position 3 of D-glucopyranoside of a glucose-glucosamine disaccharide linked by a β-glycosidic linkage. This method may be adopted to achieve the stereoinversion from glucose -> mannose, so that the mannose-glucosamine disaccharide skeleton linked by a β-glycosidic linkage can be constructed in high yield and selectivity.

The solvent in this step is not limited as long as the reaction proceeds. Examples include dimethyl sulfoxide, dimethylformamide, dimethylacetamide, N-methylpyrrolidone, N,N-dimethylimidazolidinone, sulfolane, tetrahydrofuran, or acetonitrile. Preferred is dimethylsulfoxide.

The reaction temperature in this step is not limited as long as the reaction proceeds, but may be, for example, from 20°C to 80°C, preferably from 23°C to 70°C, more preferably from 26°C to 60°C, and particularly preferably from 30°C to 50°C.

This method may be preferably performed using, for instance, the procedure shown in Example 9.

An embodiment of the present invention provides a method for producing a compound represented by the following formula III-8: 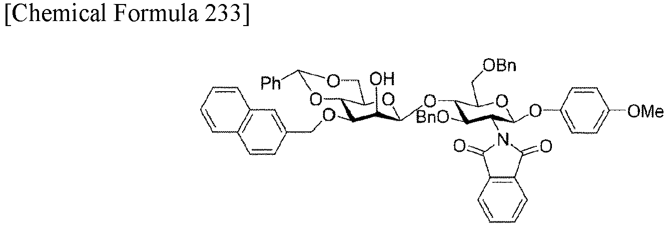 the method comprising a step of
reducing an oxo group attached to carbon at position 2 of 2-keto-D-glucopyranoside in a compound represented by the following formula III-7: 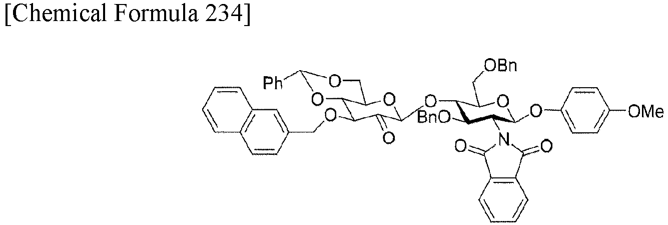
to give the compound represented by formula III-8.

The solvent in this step is not limited as long as the reaction proceeds. Examples include diethyl ether, cyclopentyl methyl ether, tert-butyl methyl ether, diisopropyl ether, dipropyl ether, dibutyl ether, or 1,4-dioxane. Preferred is tetrahydrofuran.

The reaction temperature in this step is not limited as long as the reaction proceeds, but may be, for example, from -80°C to 20°C. Note that as described below, the optimum reaction temperature varies depending on the reducing agent used.

In an embodiment of the present invention, the oxo group attached to the carbon at position 2 of 2-keto-D-glucopyranoside in the compound represented by formula III-7 is reduced in the presence of a reducing agent selected from the group consisting of L-selectride; LS-selectride; lithium diisobutyl-tert-butoxyaluminum hydride (LDBBA); a compound represented by the following formula A: 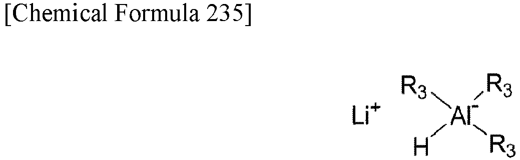
wherein R₃ is a di-tert-butylmethylphenoxide or hydride as shown in the following formula: 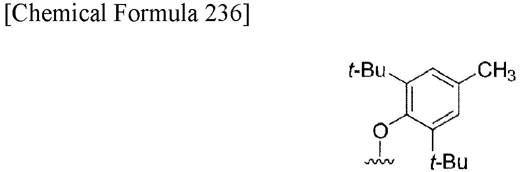
provided that at least two R₃ are each di-tert-butylmethylphenoxide; and a combination thereof. In the reduction step, NaBH₄, for example, may be used. In this case, the stereoselectivity was low (about 7:3), and it was difficult to efficiently obtain the desired stereoinversion from Gln -> Man (Org. Biomol. Chem., 2018, 16, 4720-4727). On the other hand, in the case of using the above listed reducing agents, the selectivity of stereoinversion from Gln -> Man is greatly improved (93.6:6.4 to 98.1:1.9) when compared to the case of using NaBH₄.

Among the compounds represented by formula A, a compound where three R₃ moieties are each di-tert-butylmethylphenoxide can be obtained, for example, by adding, to a tetrahydrofuran suspension (2 mL) containing lithium aluminum hydride (50.0 mg, 1.32 mmol), dibutylhydroxytoluene (885.41 mg, 4.02 mmol) at 0°C, followed by stirring at 25°C. Among the compounds represented by formula A, a compound where two R₃ moieties are each di-tert-butylmethylphenoxide can be obtained in a similar manner by using 2 molar equivalents of dibutylhydroxytoluene for 1 molar equivalent of lithium aluminum hydride.

As described above, the reaction temperature in this step is not limited as long as the reaction proceeds. When L-selectride, LS-selectride, or LDBBA is used as the reducing agent, the reaction temperature may be preferably from -80°C to -20°C, more preferably from -80°C to -30°C, still more preferably from -80°C to -40°C, and particularly preferably from -80°C to -50°C. When the compound represented by formula A is used as the reducing agent, the reaction temperature may be preferably from -20°C to 20°C, more preferably from -15°C to 15°C, and particularly preferably from -10°C to 10°C. Therefore, in terms of the fact that the reaction proceeds at an easy-to-handle temperature, the particularly suitable reducing agent used for this step is a compound represented by formula A.

This method may be preferably performed using, for instance, the procedure shown in Example 19.

An embodiment of the present invention provides a method for producing a compound represented by the following formula III-9: 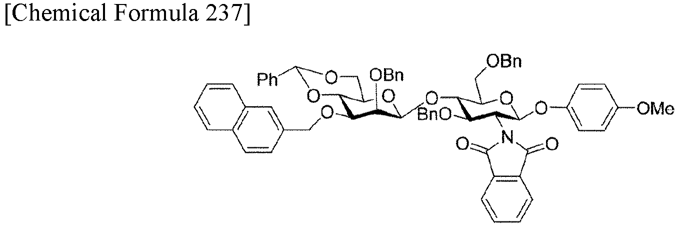 the method comprising a step of
protecting, by a benzyl group, in the presence of lithium tert-butoxide or lithium tert-amoxide, a hydroxyl group attached to carbon at position 2 of D-mannopyranoside in a compound represented by the following formula III-8: 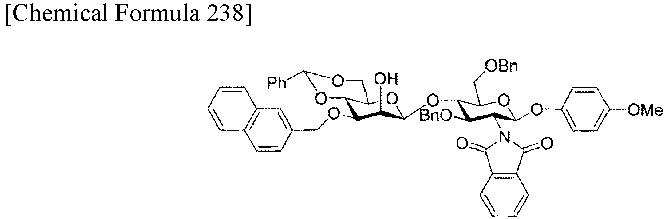
to give the compound represented by formula III-9.

The benzyl group may be protected in the presence of lithium tert-butoxide or lithium tert-amoxide to inhibit the ring-opening of the phthalimide. In addition, it is safer and easier to scale up than the general conditions using sodium hydride.

The solvent in this step is not limited as long as the reaction proceeds. Examples include dimethylacetamide, dimethylformamide, N-methylpyrrolidone, or N,N-dimethylimidazolidinone. Preferred is dimethylacetamide.

The reaction temperature in this step is not limited as long as the reaction proceeds, but may be, for example, from -20°C to 100°C, preferably from -15°C to 70°C, and particularly preferably from -10°C to 50°C.

This method may be preferably performed using, for instance, the procedure shown in Example 11 or 21.

An embodiment of the present invention provides a method for producing a compound represented by the following formula V-5: 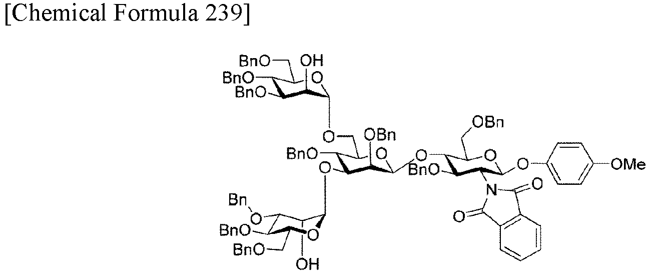 the method comprising a step of
reacting, in the presence of methyl trifluoroacetate, a compound represented by the following formula V-4: 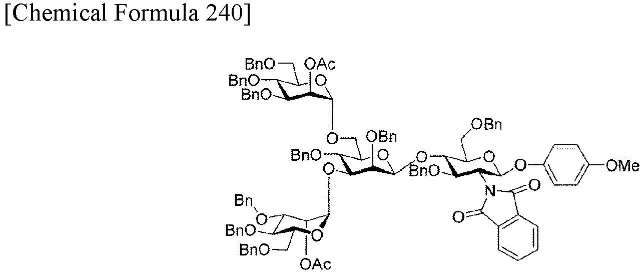
with an alkoxide-based strong base to give the compound represented by formula V-5.

It is generally known that acetyl groups were deprotected using sodium methoxide in methanol. In this case, the phthalimide ring may be opened at the same time as an undesired side reaction. By contrast, a reaction with an alkoxide-based strong base may be carried out in the presence of methyl trifluoroacetate. This technique may be used to eliminate the acetyl groups while inhibiting the ring-opening of the phthalimide group.

The alkoxide-based strong base is not limited as long as the reaction proceeds. Examples include a sodium salt, a lithium salt, or a potassium salt of C1-C5 alkoxide, or a combination thereof. Preferable examples include lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide. Particularly preferable examples include sodium methoxide, lithium tert-butoxide, or potassium tert-butoxide.

The solvent in this step is not limited as long as the reaction proceeds. Examples include an alcohol solvent (e.g., methanol, ethanol, propanol, butanol) or a mixed solvent system of an alcohol solvent and, for instance, tetrahydrofuran, acetonitrile, cyclopentyl methyl ether, toluene, or dimethylacetamide. Preferred is methanol or a mixed solvent system of methanol and tetrahydrofuran.

The reaction temperature in this step is not limited as long as the reaction proceeds, but may be, for example, from -20°C to 80°C, preferably from 0°C to 70°C, more preferably from 15°C to 65°C, and particularly preferably from 30°C to 60°C.

This method may be preferably performed using, for instance, the procedure shown in Example 29.

An embodiment of the present invention provides a method for producing a compound represented by the following formula II-5: 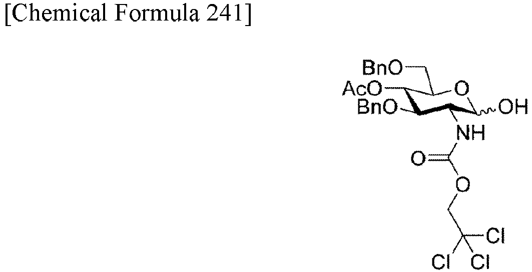 or a compound represented by the following formula II-8: 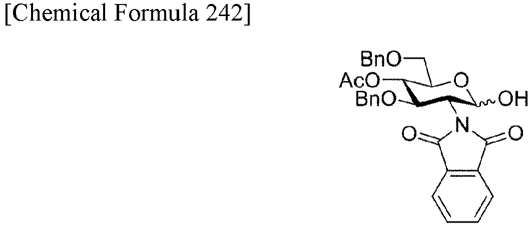 the method comprising a step of
reacting, in fluorous alcohol and water, a compound represented by the following formula II-4: 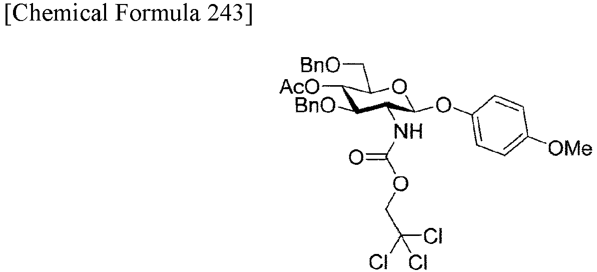
or a compound represented by the following formula II-7: 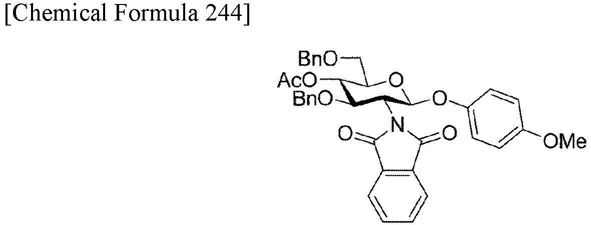
with λ3-iodane to give the compound represented by formula II-5 or the compound represented by formula II-8.

As used herein, the term "λ3-iodane" means a trivalent/hypervalent iodine compound. The use of λ3-iodane increases the yield of the compound represented by formula II-5 or the compound represented by formula II-8 when compared to conventional deprotection processes. In addition, the reaction can be carried out under mild conditions. Here, since the reaction proceeds with a small excess of λ3-iodane, the purification of the deprotected product is easier than conventional deprotection procedures using, for example, an excess amount of cerium(IV) ammonium nitrate. Also, operation safety is high.

In an embodiment, λ3-iodane is a compound represented by formula R⁴-I(OR⁵)₂ (wherein R⁴ is an unsubstituted or substituted phenyl group and R⁵ is selected from the group consisting of H, acetoxy, trifluoroacetoxy, tosyloxy, methane sulfonyloxy, and a combination thereof). As defined in the above formula, R⁴ may be a "substituted phenyl group". Examples of the substituent include a linear or branched, saturated or unsaturated hydrocarbon group, an oxygen-containing group (e.g., alkoxy, ester), a nitrogen-containing group (e.g., cyano, azide), or a halogen (e.g., fluorine, chlorine, bromine, iodine). More preferred is a hydrocarbon group, an oxygen-containing substituent, or a halogen. When the above substituent contains carbon, for example, a C1-C5 or C1-C3 substituent may be preferably used. Specific examples of the λ3-iodane include, but are not limited to, [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, and [hydroxy(methanesulfonyloxy)iodo]benzene.

The amount of λ3-iodane can be set, if appropriate, but may be, for example, about 0.1 to 10 equivalents, about 0.5 to 7 equivalents, or about 1 to 5 equivalents, and preferably about 1 to 3 equivalents of the compound represented by formula II-4 or the compound represented by formula II-7.

As used herein, the term "fluorous alcohol" means a fluorine-containing alcohol compound in which all carbon atoms except those bonded to the alcohol have fluorine atoms. The fluorous alcohol preferably has a greater number of fluorine atoms as long as fluorine substitution is allowed. Examples of the fluorous alcohol include, but are not limited to, a fluorous aliphatic alcohol. The hydrocarbon moiety in the fluorous aliphatic alcohol may be saturated or unsaturated, linear or branched, or cyclic. The fluorous aliphatic alcohol is, for example, a fluorous C₂-C₈ aliphatic alcohol, preferably a fluorous C₂-C₅ aliphatic alcohol, and more preferably a fluorous C₂-C₃ aliphatic alcohol. Specific examples of the fluorous alcohol include, but are not limited to, the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.

Further, the fluorous alcohol and λ3-iodane may be preferably used in combination to give a deprotected product in higher yield. Such a combination can be selected, if appropriate, by a person skilled in the art. For example, PIFA is suitably used in combination with, for instance, hexafluoro-2-propanol (HFIP), 2,2,2-trifluoroethanol (TFE), or nonafluoro-tert-butyl alcohol. HTIB is suitably used in combination with, for instance, HFIP, TFE, or 2,2,3,3,4,4,5,5-octafluoro-1-pentanol. [Bis(trifluoroacetoxy)iodo]pentafluorobenzene is suitably used in combination with, for instance, hexafluoro-2-propanol (HFIP). [Hydroxy(methanesulfonyloxy)iodo]benzene is suitably used in combination with, for instance, hexafluoro-2-propanol (HFIP). The combination is not limited to the above examples.

The amount of fluorous alcohol can be set, if appropriate, from the viewpoint of, for instance, achieving a high yield of the product. For example, the amount may be about 1.0 equivalent or more, about 1.5 equivalents or more, about 2.0 equivalents or more, or 2.5 equivalents or more in a mole ratio based on the compound represented by formula II-4 or the compound represented by formula II-7. The amount may be about 15 or less, about 10 or less, about 8 or less, or about 5 or less in a volume ratio based on the compound represented by formula II-4 or the compound represented by formula II-7.

This step is carried out in the co-presence of the above-mentioned fluorous alcohol and "water". The amount of water can be set, if appropriate, from the viewpoint of, for instance, achieving a high yield of the product. For example, the amount may be about 1.0 equivalent or more, about 1.5 equivalents or more, about 2.0 equivalents or more, or about 2.5 equivalents or more in a mole ratio based on the compound represented by formula II-4 or the compound represented by formula II-7. The amount may be about 10 or less, about 8 or less, about 5 or less, or about 3 or less in a volume ratio based on the compound represented by formula II-4 or the compound represented by formula II-7.

In this step, an additional "solvent" may be added to the fluorous alcohol and water. The solvent may be selected from the group consisting of, but not limited to, dichloromethane, toluene, (trifluoromethyl)benzene, and a combination thereof. The type of solvent used can be selected, if appropriate, according to the λ3-iodane or the like used in order to achieve a high yield of the product. The amount of solvent can also be set, if appropriate, to achieve a high yield of the product, and may be, for example, about 0.5 to 50, about 1 to 20, or about 2 to 10 in a volume ratio based on the compound represented by formula II-4 or the compound represented by formula II-7.

In this step, an additional "additive" may be added to the fluorous alcohol and water. The additive is preferably selected from the group consisting of sodium dihydrogen phosphate, potassium dihydrogen phosphate, disodium hydrogen phosphate, and a combination thereof. The acidity can increase as a de-4-methoxyphenylation reaction proceeds. In particular, λ3-iodane (e.g., HTIB), which is a byproduct of strongly acidic acid, may be used. In this case, a higher product yield can be obtained by adding an additive such as sodium dihydrogen phosphate mentioned above. The amount of additive can also be set, if appropriate, to achieve a high yield of the product, and may be, for example, about 0.5 to 8 equivalents, about 1 to 6 equivalents, or about 1.5 to 5 equivalents based on the compound represented by formula II-4 or the compound represented by formula II-7.

When (diacetoxyiodo)benzene (PIDA) is used as λ3-iodane, trifluoroacetic acid (TFA) is preferably added to obtain the deprotected product in higher yield.

In this step, in the co-presence of fluorous alcohol and water, λ3-iodane acts as an oxidant for 4-methoxyphenyl (X group) in the compound represented by formula II-4 or the compound represented by formula II-7. The OX group is then eliminated from the compound represented by formula II-4 or the compound represented by formula II-7. After that, water (H₂O) is added thereto. As a result, the 4-methoxyphenoxy group (OX group) is easily eliminated from the compound represented by formula II-4 or the compound represented by formula II-7. In this way, the effect seems to be achieved. Such an effect of λ3-iodane is generally achieved by, for instance, stirring and refluxing a solution containing the compound represented by formula II-4 or the compound represented by formula II-7 and λ3-iodane in fluorous alcohol and water. Therefore, this step is simple to perform and can be scaled up relatively easily. The product, the compound represented by formula II-5 or the compound represented by formula II-8 can be purified or isolated by any purification procedure known to those skilled in the art, such as crystallization or chromatography of the product.

The temperature at the time of reacting the compound represented by formula II-4 or the compound represented by formula II-7 with λ3-iodane is preferably from about -20°C to the boiling point or lower of fluorous alcohol (e.g., about 58°C for hexafluoro-2-propanol (HFIP), about 78°C for 2,2,2-trifluoroethanol (TFE)), and may be, for example, about -20°C to 60°C, about 0°C to 60°C, or about 10°C to 30°C. In addition, the demethoxyphenylation reaction also proceeds at room temperature (15-30°C). Thus, the advantage is that neither cooling nor heating is needed.

Further, the reaction time can be set, if appropriate, to obtain the product in high yield.

This method may be preferably performed using, for instance, the procedure shown in Example 33 or 38.

An embodiment of the present invention provides a method for producing a compound represented by the following formula II-6: 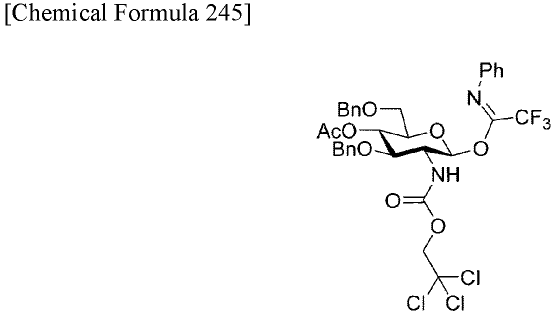 or a compound represented by the following formula II-9: 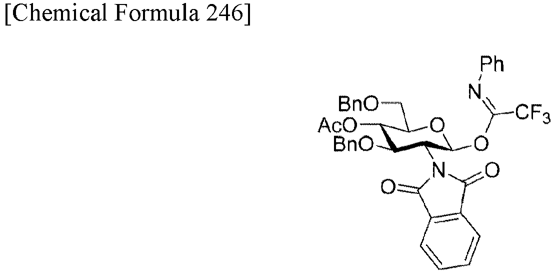 the method comprising a step of
reacting, in the presence of N-methylimidazole, a compound represented by the following formula II-5: 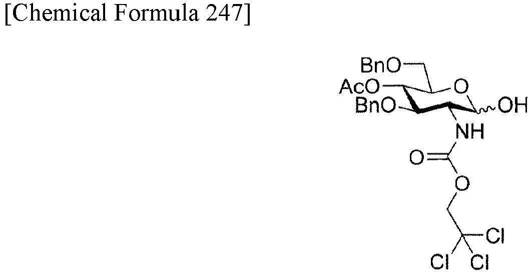
or a compound represented by the following formula II-8: 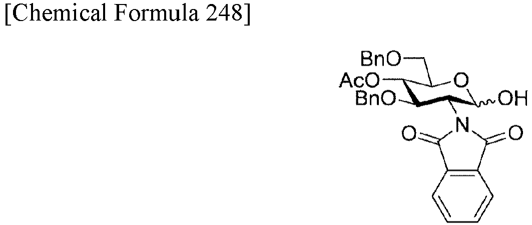
with 2,2,2-trifluoro-N-phenylacetimidoyl chloride (TFPC) represented by the following formula: 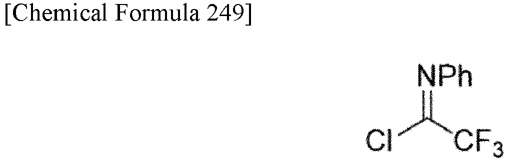
to give the compound represented by formula II-6 or the compound represented by formula II-9.

The use of N-methylimidazole as a base in this step significantly increases the yield. For example, the compound represented by formula II-6 may be produced from the compound represented by formula II-5. In the case of using potassium carbonate under certain conditions, the yield is about 36%. However, in the case of using N-methylimidazole, the yield is significantly improved to about 76%. Since TFPC is an expensive reagent, an increase in the yield of this step is very beneficial for commercial production.

The solvent in this step is not limited as long as the reaction proceeds. Examples include dichloromethane, toluene, ethyl acetate, acetonitrile, or tetrahydrofuran. Preferred is dichloromethane.

The reaction temperature in this step is not limited as long as the reaction proceeds, but may be preferably from -20°C to 40°C, more preferably from -10°C to 35°C, and particularly preferably from 0°C to 30°C.

This step is suitably performed in the presence of a dehydrating agent. The dehydrating agent in this step is not limited as long as the reaction proceeds. Examples include Molecular Sieves. Preferred is Molecular Sieves 4A powder with a powder particle size of 10 µm or less.

In this method, the compound represented by formula II-6 formed can be isolated and purified by column or by recrystallization. Examples of the isolation and purification using a column include isolation and purification using silica gel as a stationary phase and a dichloromethane or toluene-ethyl acetate mixed solvent system as a mobile phase. On the other hand, examples of the isolation and purification by recrystallization include crystallization from a mixed solvent system containing diisopropyl ether and heptane. Note that the recrystallization may be preferably performed using seed crystals of the compound represented by formula II-6. In the case of using seed crystals, for example, the seed crystals are added into a diisopropyl ether solution, and after the crystals are found to precipitate, heptane is added dropwise to the solution. This operation may be used to obtain the compound.

This method may be preferably performed using, for instance, the procedure shown in Example 34 or 39.

An embodiment of the present invention provides a method for producing a compound represented by the following formula VI-5: 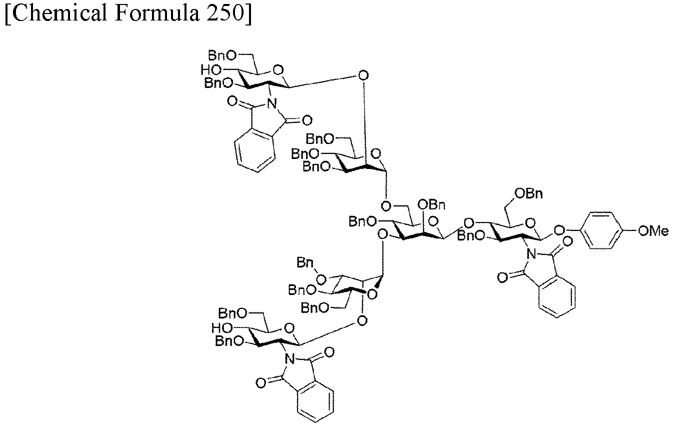 the method comprising a step of
reacting, in the presence of methyl trifluoroacetate, a compound represented by the following formula VI-4: 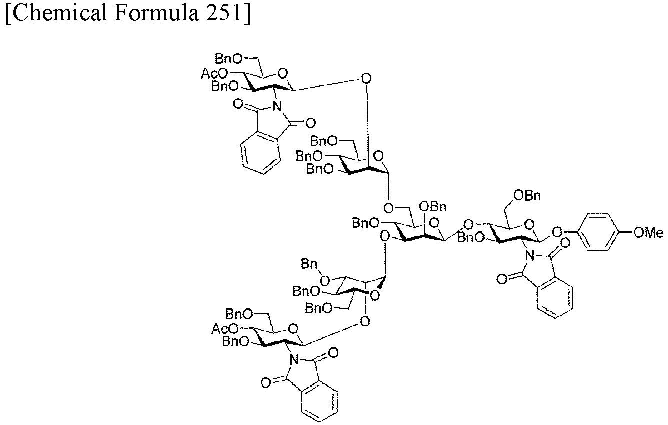
with an alkoxide-based strong base to give the compound represented by formula VI-5.

It is generally known that acetyl groups were deprotected using sodium methoxide in methanol. In this case, the phthalimide ring may be opened at the same time as an undesired side reaction. By contrast, a reaction with an alkoxide-based strong base may be carried out in the presence of methyl trifluoroacetate. This technique may be used to eliminate the acetyl groups while inhibiting the ring-opening of the phthalimide group.

The alkoxide-based strong base is not limited as long as the reaction proceeds. Examples include a sodium salt, a lithium salt, or a potassium salt of C1-C5 alkoxide, or a combination thereof. Preferable examples include lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide. Particularly preferable examples include sodium methoxide, lithium tert-butoxide, or potassium tert-butoxide.

The solvent in this step is not limited as long as the reaction proceeds. Examples include an alcohol solvent, e.g., methanol, ethanol, propanol, butanol, or a mixed solvent system of an alcohol solvent and, for instance, tetrahydrofuran, acetonitrile, cyclopentyl methyl ether, toluene, or dimethylacetamide. Preferred is methanol or a mixed solvent system of methanol and tetrahydrofuran.

The reaction temperature in this step is not limited as long as the reaction proceeds, but may be, for example, from -20°C to 80°C, preferably from 0°C to 70°C, more preferably from 15°C to 65°C, and particularly preferably from 30°C to 60°C.

This method may be preferably performed using, for instance, the procedure shown in Example 41.

An embodiment of the present invention provides a method for producing a compound represented by the following formula VII-3: 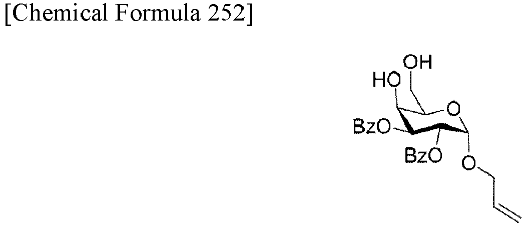 the method comprising a step of
bringing a solvent dissolving the compound represented by formula VII-3 into contact with silica gel to perform solid-phase extraction of the compound represented by formula VII-3.

Since an unreacted compound represented by formula VII-2, which may be used as a raw material at the time of producing the compound represented by formula VII-3, and eliminated benzaldehyde are not adsorbed on silica gel, the compound represented by formula VII-3 can be efficiently purified by this step.

Examples of the solvent used to dissolve the compound represented by formula VII-3 include toluene, heptane, dichloromethane, chloroform, or a combination thereof. Preferable examples include toluene, dichloromethane, chloroform, or a combination thereof. Particularly preferable examples include, but are not limited to, toluene.

As the silica gel in this step, silica gel in an amount 2 to 5 times the amount of the raw material, for example, may be used, silica gel in an amount 2 to 4 times the amount of the raw material may be preferably used, and silica gel in an amount about 3 times the amount of the raw material may be more preferably used.

In this step, the solvent used to elute the compound represented by formula VII-3 adsorbed on silica gel is not limited as long as the solvent does not dissolve the silica gel and can elute the target compound. Examples include cyclopentyl methyl ether, ethyl acetate, or tert-butyl methyl ether.

This method may be preferably performed using, for instance, the procedure shown in Example 46.

An embodiment of the present invention provides a method for producing a compound represented by the following formula VIII-4: 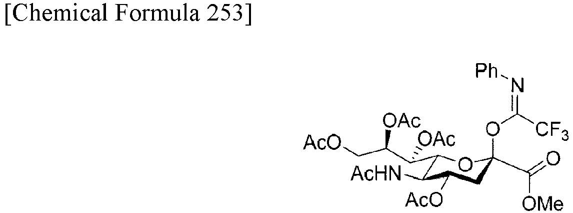 the process comprising a step of
reacting, in the presence of N-methylimidazole, a compound represented by the following formula VIII-3: 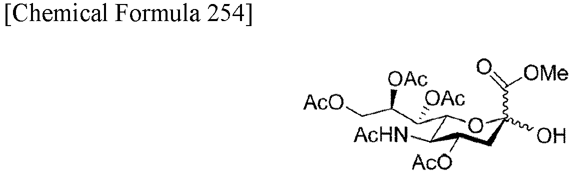
with 2,2,2-trifluoro-N-phenylacetimidoyl chloride (TFPC) represented by the following formula: 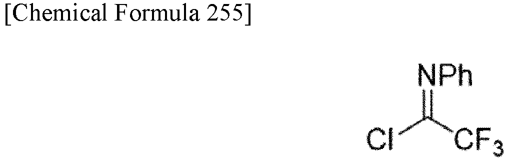
to give the compound represented by formula VIII-4.

Compared to the case of using potassium carbonate, N-methylimidazole may be used as a base in this step. Here, it is possible to reduce the equivalent amount of TFPC. In this case, the target product can still be obtained in high yield. Since TFPC is an expensive reagent, an increase in the yield of this step is very beneficial for commercial production.

The solvent in this step is not limited as long as the reaction proceeds. Examples include dichloromethane, toluene, ethyl acetate, acetonitrile, or tetrahydrofuran. Preferred is dichloromethane.

The reaction temperature in this step is not limited as long as the reaction proceeds, but may be preferably from -20°C to 40°C, more preferably from -10°C to 35°C, and particularly preferably from 0°C to 30°C.

This step is suitably performed in the presence of a dehydrating agent. The dehydrating agent in this step is not limited as long as the reaction proceeds. Examples include Molecular Sieves. Preferred is Molecular Sieves 4A powder with a powder particle size of 10 µm or less.

This step may be preferably performed using, for instance, the procedure shown in Example 49.

An embodiment of the present invention provides a process for producing a compound represented by the following formula IX-1: 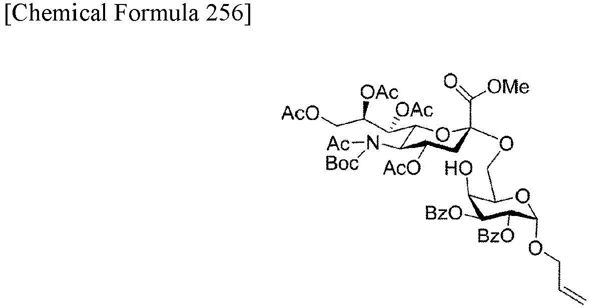 the method comprising a step of
subjecting a compound represented by the following formula VIII-5: 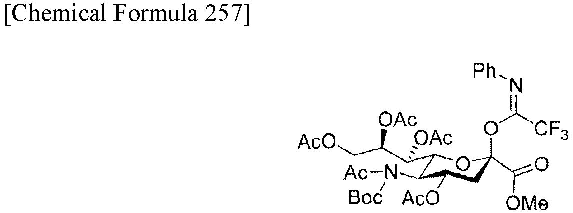
and a compound represented by the following formula VII-3: 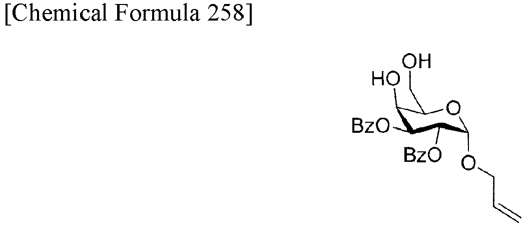
to α-2,6-glycosidic linkage to give the compound represented by formula IX-1.

It is difficult that an N-acetylneuraminic acid derivative and a galactose derivative are selectively linked by α-2,6-glycosidic linkage. For example, a method for synthesizing a disaccharide by reacting a compound represented by formula VIII-4 with a compound represented by formula VII-3 has been reported (J. Org. Chem., 2016, 81, 10600-10616). The reaction was not easy to reproduce, and the desired yield and selectivity could not be obtained. Unfortunately, in this reaction, the selectivity decreases as the scale increases, the reaction temperature tolerance is narrow, and the effect of reaction heat is significant. The compound represented by formula VIII-4, which is one of the raw material compounds in this reaction, is very expensive. Therefore, the low reproducibility, yield, and selectivity in this reaction are a major problem, especially in commercial production where scale-up is required. On the other hand, if the compound represented by formula VIII-5 with a tert-butoxycarbonyl group is used instead of the compound represented by formula VIII-4 as a raw material compound, the high selectivity for α-2,6-glycosidic linkage (α:β = 93:7) can be well-reproduced, and the yield is also improved. In addition, the temperature tolerance is wider, and high reproducibility, yield, and selectivity can be achieved even when scaled up. This has a very beneficial effect on commercial production.

This step can suitably be performed in the presence of a Lewis acid. The Lewis acid in this step is not limited as long as the reaction proceeds. Examples include trimethylsilyl trifluoromethanesulfonate, triisopropylsilyl trifluoromethanesulfonate, or tert-butyl dimethylsilyl trifluoromethanesulfonate. Preferred is trimethylsilyl trifluoromethanesulfonate.

The solvent in this step is not limited as long as the reaction proceeds. Examples include diisopropyl ether, tert-butyl methyl ether, diethyl ether, dibutyl ether, dipropyl ether, 1,4-dioxane, dichloromethane, 1,2-dichloroethane, toluene, chlorobenzene, trifluoromethylbenzene, propionitrile, or acetonitrile. Preferred is cyclopentyl methyl ether.

The reaction temperature in this step is not limited as long as the reaction proceeds, but may be, for example, from -78°C to 0°C, preferably from -78°C to - 20°C, more preferably from -78°C to -30°C, and particularly preferably from -78°C to -40°C.

In this step, it is preferable to use 1 to 3 equivalents of the compound represented by formula VII-3 with respect to 1 equivalent of the compound represented by formula VIII-5. It is more preferable to use 1.4 to 2 equivalents of the compound represented by formula VII-3 with respect to 1 equivalent of the compound represented by formula VIII-5.

This step is not limited as long as the reaction proceeds. For example, a mixed solution containing the compound represented by formula VIII-5 and the compound represented by formula VII-3 (suitably, a cyclopentyl methyl ether mixed solution) is added dropwise over a long time to a Lewis acid-containing solution (suitably, cyclopentyl methyl ether solution). Alternatively, a solution containing the compound represented by formula VIII-5 (suitably, a cyclopentyl methyl ether solution) is added dropwise over a long time to a solution containing a Lewis acid and the compound represented by formula VII-3 (suitably, cyclopentyl methyl ether solution). Preferably, a solution containing the compound represented by formula VIII-5 (suitably, a cyclopentyl methyl ether solution) is added dropwise over a long time to a solution containing a Lewis acid and the compound represented by formula VII-3 (suitably, cyclopentyl methyl ether solution). The dropping time is not limited as long as the reaction proceeds, but may be, for example, from 30 minutes to 5 hours, preferably from 1 to 4 hours, more preferably from 2 to 3.5 hours, and particularly preferably about 3 hours.

This method may be preferably performed using, for instance, the procedure shown in Example 51.

An embodiment of the present invention provides a method for producing a compound represented by the following formula IX-1: 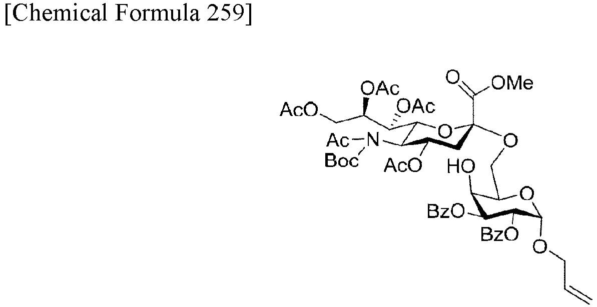 the method comprising a step of
bringing a solvent dissolving the compound represented by formula IX-1 into contact with silica gel to perform solid-phase extraction of the compound represented by formula IX-1.

In the step of producing the compound represented by formula IX-1, N-phenyl trifluoroacetamide, a byproduct of the glycosylation reaction, and other trace impurities in the toluene solvent that are not adsorbed on silica gel are not adsorbed on silica gel. Therefore, the compound represented by formula IX-1 can be efficiently purified by this step.

Examples of the solvent used to dissolve the compound represented by formula IX-1 include toluene, heptane, dichloromethane, chloroform, or a combination thereof. Preferable examples include toluene, dichloromethane, chloroform, or a combination thereof. Particularly preferable examples include, but are not limited to, toluene.

As the silica gel in this step, silica gel in an amount 2 to 5 times the amount of the raw material, for example, may be used, silica gel in an amount 2 to 4 times the amount of the raw material may be preferably used, and silica gel in an amount about 3.5 times the amount of the raw material may be more preferably used.

In this step, the solvent used to elute the compound represented by formula IX-1 adsorbed on silica gel is not limited as long as the solvent does not dissolve the silica gel and can elute the target compound. Examples include ethyl acetate, cyclopentyl methyl ether, or tert-butyl methyl ether. Preferred is ethyl acetate.

This method may be preferably performed using, for instance, the procedure shown in Example 51.

An embodiment of the present invention provides a method for producing a compound represented by the following formula IX-3: 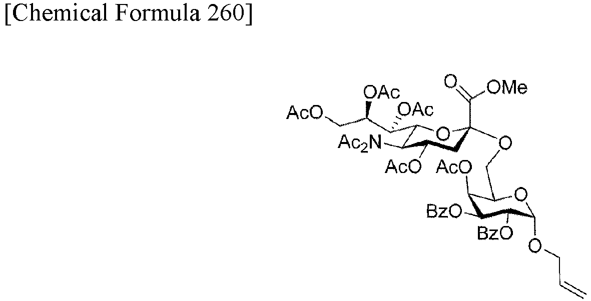 the method comprising a step of
bringing a solvent dissolving the compound represented by formula IX-3 into contact with silica gel to perform solid-phase extraction of the compound represented by formula IX-3.

In an embodiment of the step of producing the compound represented by formula IX-3, the compound represented by formula VII-3 used in excess during the upstream glycosylation reaction is acetylated to give, for instance, a diacetyl form of the compound represented by formula VII-3. These byproducts are not adsorbed on silica gel. Therefore, the compound represented by formula IX-3 can be efficiently purified by this step.

Examples of the solvent used to dissolve the compound represented by formula IX-3 include toluene, heptane, dichloromethane, chloroform, or a combination thereof. Preferable examples include toluene, dichloromethane, chloroform, or a combination thereof. Particularly preferable examples include, but are not limited to, toluene.

As the silica gel in this step, silica gel in an amount 2 to 5 times the amount of the raw material, for example, may be used, silica gel in an amount 2 to 4 times the amount of the raw material may be preferably used, and silica gel in an amount about 3.5 times the amount of the raw material may be more preferably used.

In this step, the solvent used to elute the compound represented by formula IX-3 adsorbed on silica gel is not limited as long as the solvent does not dissolve the silica gel and can elute the target compound. Examples include ethyl acetate, cyclopentyl methyl ether, or tert-butyl methyl ether. Preferred is ethyl acetate.

This method may be preferably performed using, for instance, the procedure shown in Example 53.

Any of the novel methods for producing monosaccharides or oligosaccharides as described above can be used in the method for producing the oligosaccharides represented by formula XI. Thus, an embodiment of the present invention provides a method for producing the oligosaccharide represented by formula XI, the method including any of the novel methods for producing monosaccharides or oligosaccharides as described above. In this context, the method for producing the oligosaccharide represented by formula XI may be the same as or different from the method detailed in section <1> herein. The method for producing the oligosaccharide represented by formula XI may be different from the method detailed in section <1> herein. In this method, a known method for producing the oligosaccharide represented by formula XI as well as each novel method for producing a monosaccharide or oligosaccharide as described above should be used or applied. In this way, a usual procedure for producing a monosaccharide or oligosaccharide may be used or such a usual procedure may be applied and/or the method detailed in section <1> herein may be partially used or applied for implementation.

### <3. Novel intermediate>

An embodiment of the present invention provides a novel intermediate useful in a novel method for pure-chemically producing a biantennary N-glycan with an a2,6-sialic acid structure at each non-reducing end. The intermediates described below are useful in the production of the oligosaccharide represented by formula XI as detailed in section <1> above, but are not limited to this application and can be applied to any of applications. For example, the novel intermediates in the present invention can be used in the production of sugar other than the oligosaccharide represented by formula XI.

An embodiment of the present invention provides a compound represented by the following formula III-6: 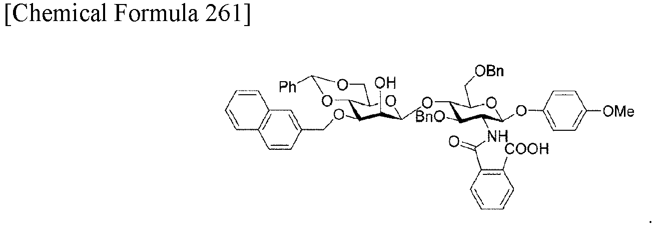 The compound can be produced by the method described in section <1>.

An embodiment of the present invention provides a compound represented by the following formula III-10: 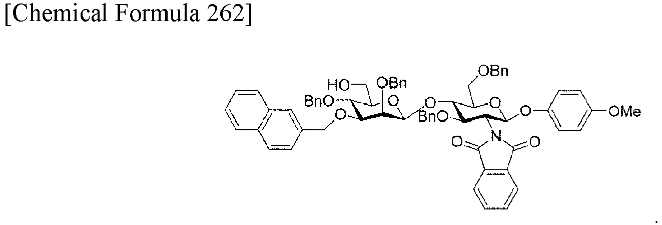 The compound can be produced by the method described in section <1>.

An embodiment of the present invention provides a compound represented by the following formula V-1: 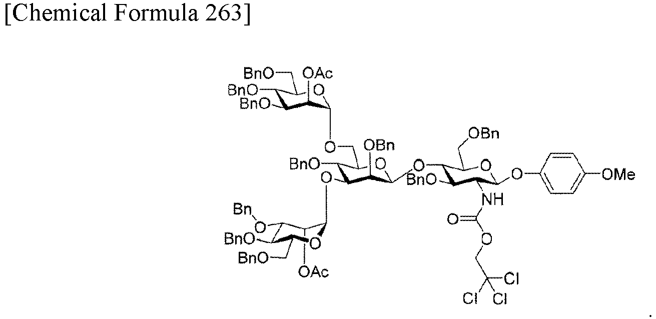 The compound can be produced by the method described in section <1>.

An embodiment of the present invention provides a compound represented by the following formula V-2: 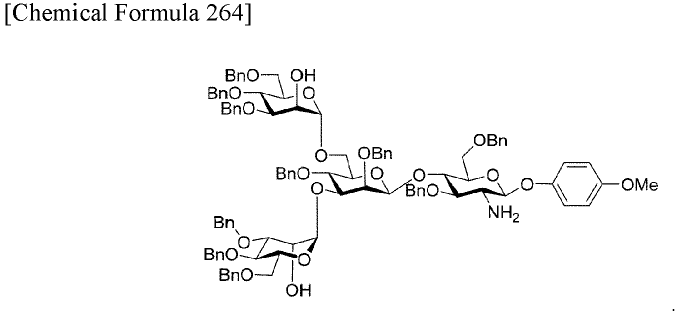 The compound can be produced by the method described in section <1>.

An embodiment of the present invention provides a compound represented by the following formula V-3: 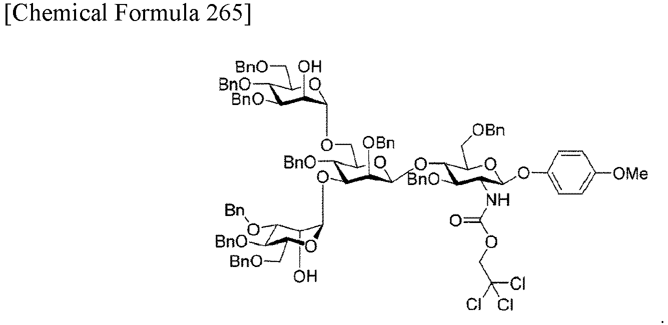 The compound can be produced by the method described in section <1>.

An embodiment of the present invention provides a compound represented by the following formula II-6: 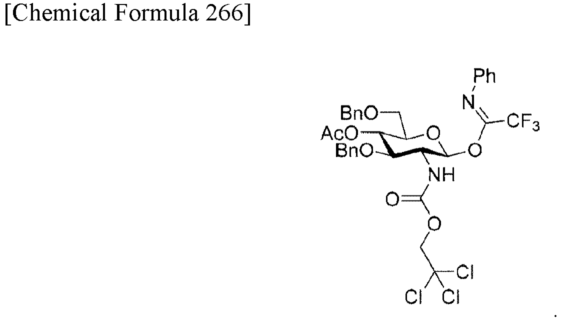 The compound can be produced by the method described in section <1>.

An embodiment of the present invention provides a compound represented by the following formula VI-A: 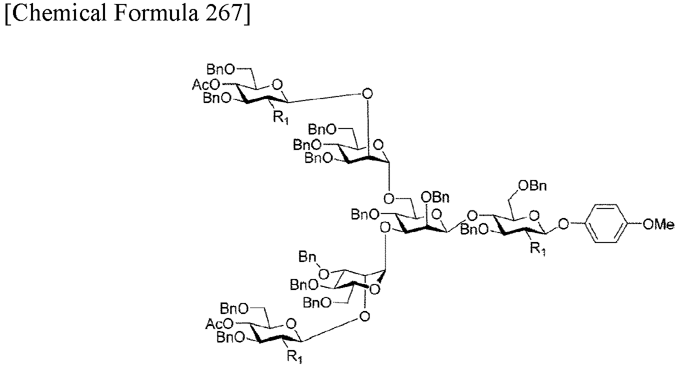 wherein R₁ represents a group selected from the group consisting of

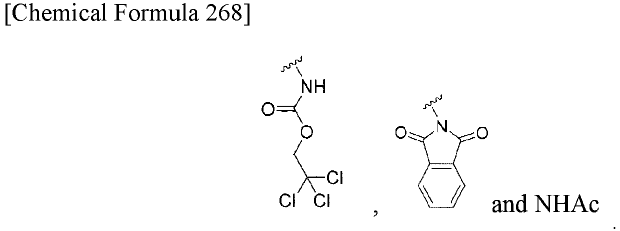

The compound where R₁ is a 2,2,2-trichloroethoxycarbonylamino group corresponds to the compound represented by formula VI-1 mentioned in section <1>. The compound where R₁ is a phthalimide group corresponds to the compound represented by formula VI-4 mentioned in section <1>. These compounds can be produced by the method described in section <1>. On the other hand, a compound where R₁ is NHAc can be obtained, for example, as an intermediate (compound represented by formula VI-7) in the production of the compound represented by formula VI-6 in Example 44.

An embodiment of the present invention provides a compound represented by the following formula VI-2: 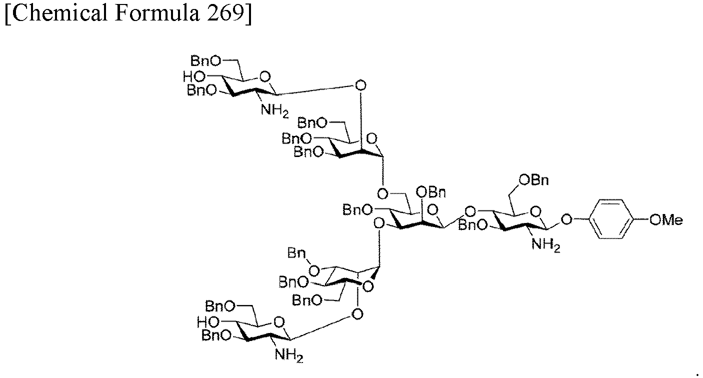 The compound can be produced by the method described in section <1>.

An embodiment of the present invention provides a compound represented by the following formula VI-B: 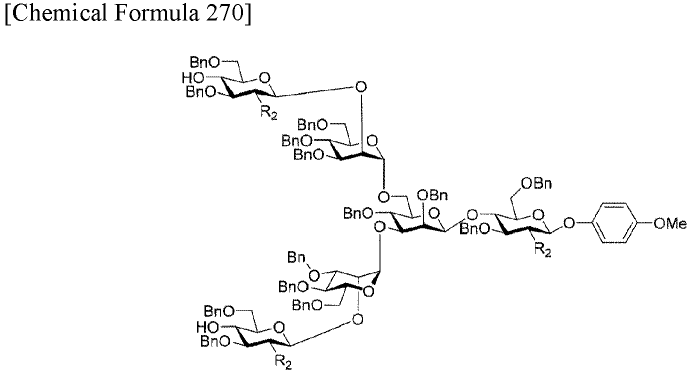 wherein R₂ represents a group selected from the group consisting of 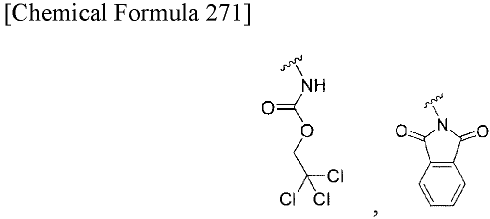 and NHAc The compound where R₂ is a 2,2,2-trichloroethoxycarbonylamino group corresponds to the compound represented by formula VI-3 mentioned in section <1>. The compound where R₁ is a phthalimide group corresponds to the compound represented by formula VI-5 mentioned in section <1>. Also, the compound where R₁ is an acetylamino group corresponds to the compound represented by formula VI-6 mentioned in section <1>. These compounds can be produced by the method described in section <1>.

An embodiment of the present invention provides a compound represented by the following formula VIII-5: 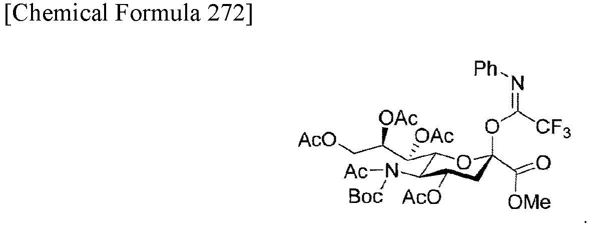 The compound can be produced by the method described in section <1>.

An embodiment of the present invention provides a compound represented by the following formula X-1: 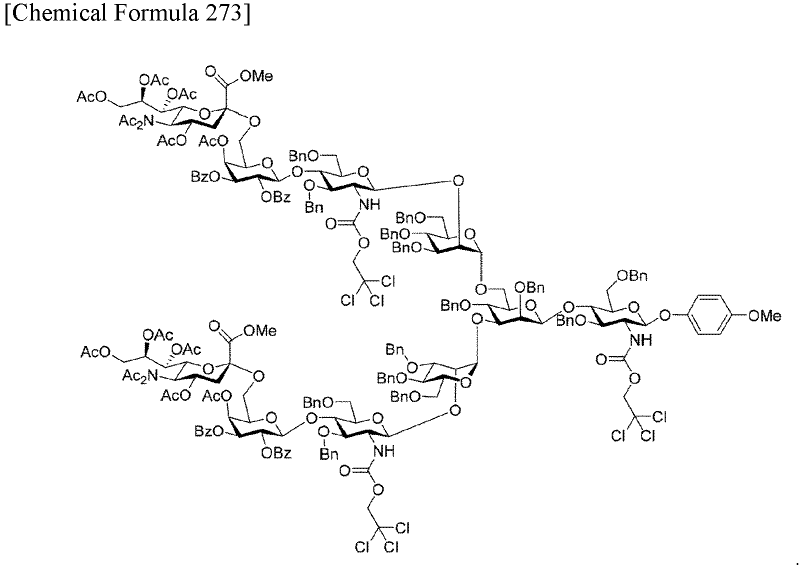 The compound can be produced by the method described in section <1>.

An embodiment of the present invention provides a compound represented by the following formula X-2: 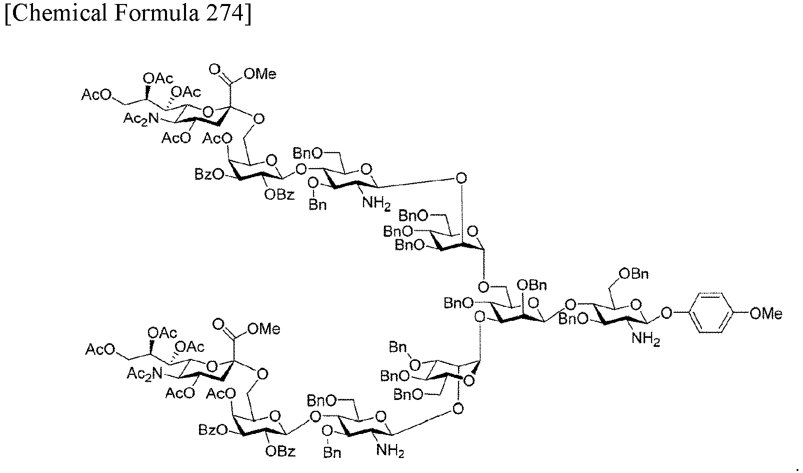 The compound can be produced by the method described in section <1>.

An embodiment of the present invention provides a compound represented by the following formula X-3: 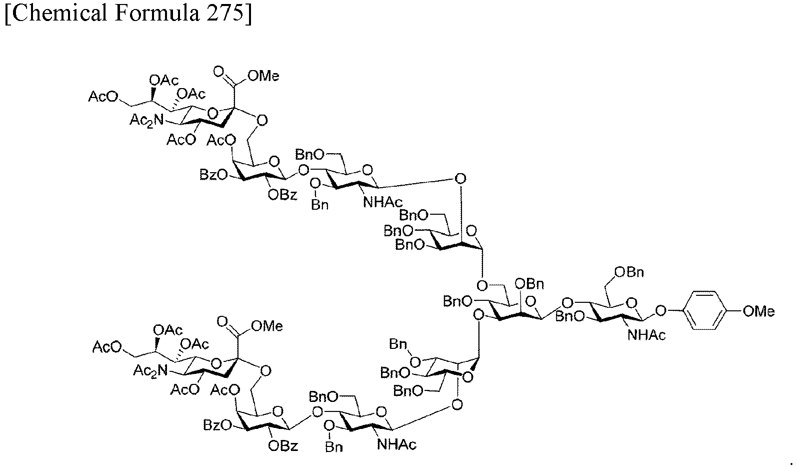 The compound can be produced by the method described in section <1>.

An embodiment of the present invention provides a compound represented by the following formula X-4: 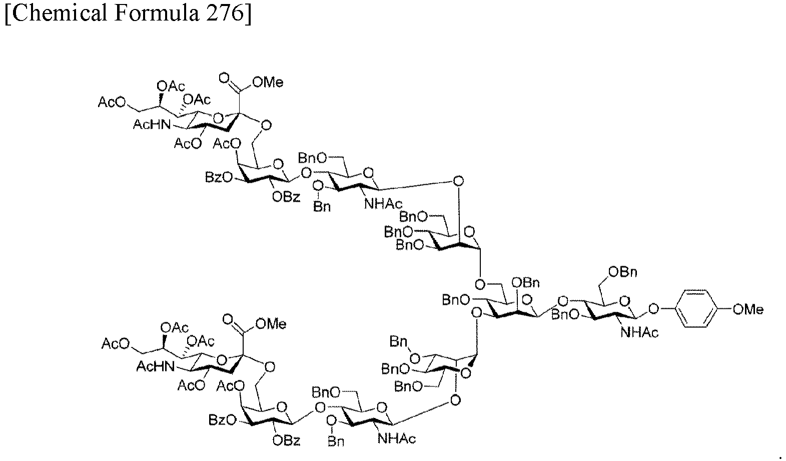 The compound can be produced by the method described in section <1>.

An embodiment of the present invention provides a compound represented by the following formula X-5: 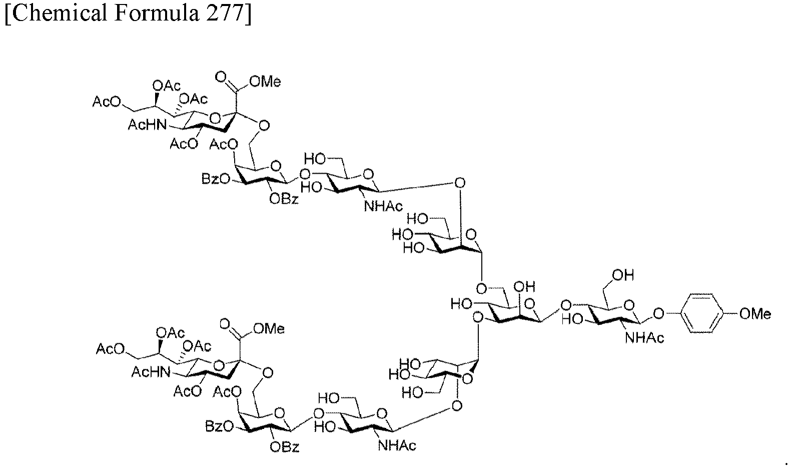 The compound can be produced by the method described in section <1>.

An embodiment of the present invention provides a compound represented by the following formula X-6: 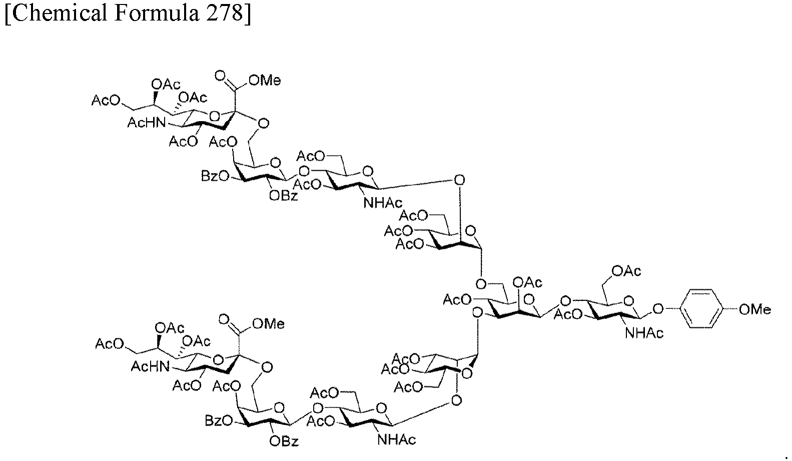 The compound can be produced by the method described in section <1>.

An embodiment of the present invention provides a compound represented by the following formula X-7: 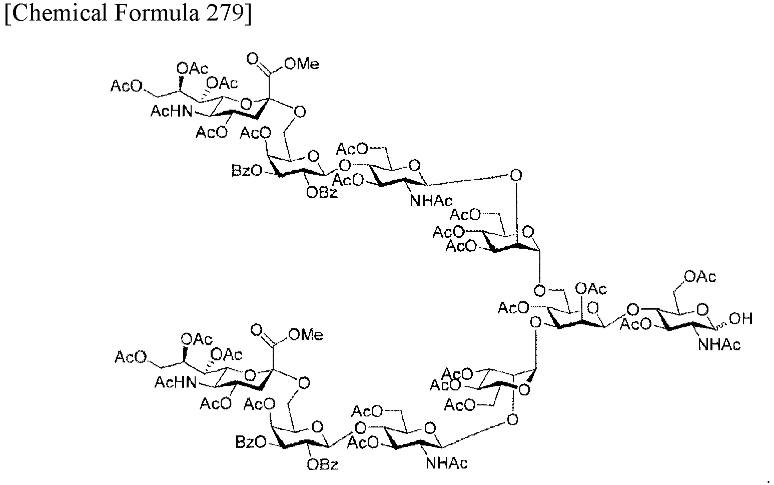 The compound can be produced by the method described in section <1>.

### <4. Novel method for producing glycoprotein, etc.>

In an embodiment of the present invention, the novel method for pure-chemically producing a biantennary N-glycan with an a2,6-sialic acid structure at each non-reducing end (i.e., an oligosaccharide represented by formula XI) or the novel N-glycan production method including novel methods for producing monosaccharides or oligosaccharides may be used to provide a novel method for producing, for instance, a glycoprotein (in particular, a glycan-remodeled antibody or a molecule containing an Fc region thereof, or an antibody-drug conjugate). As detailed below, the oligosaccharide represented by formula XI obtained by the production method of the present invention may be used for, but is not limited to, the production of a glycoprotein (in particular, a glycan-remodeled antibody or a molecule containing an Fc region thereof, or an antibody-drug conjugate) (e.g., WO2019/065964, WO2020/050406) or may be used for other applications.

It has recently been reported that the heterogeneous glycans of antibodies can be remodeled by enzymatic reactions to introduce functionalized glycans uniformly (ACS Chem. Biol. 2012, 7, 110-122, ACS Med. Chem. Lett. 2016, 7, 1005-1008). Using this glycan remodeling technology, attempts have been made to synthesize a homogeneous antibody-drug conjugate (ADC) by introducing a drug(s) in a site-specific manner (Bioconjugate Chem. 2015, 26, 2233-2242; Angew. Chem. Int. Ed. 2016, 55, 2361-2367, US2016361436).

In the glycan remodeling, heterogeneous glycans added to a protein (e.g., an antibody) are cleaved off, using hydrolase, to leave only N-acetylglucosamine (GlcNAc) at each terminus thereby producing a homogenous protein moiety with GlcNAc (hereinafter, referred to as an "acceptor molecule"). Subsequently, a given glycan separately prepared (hereinafter, referred to as a "donor molecule") is provided, and the acceptor molecule and the donor molecule are linked together by using glycosyltransferase. Thereby, a homogeneous glycoprotein with a given glycan structure can be synthesized.

In an embodiment of the present invention, the novel production method of the present invention may be used to produce an oligosaccharide represented by formula XI. Its terminal structure may then be activated, so that the oligosaccharide can be used as a donor molecule in the synthesis of the above homogeneous glycoprotein (in particular, a glycan-remodeled antibody or a molecule containing an Fc region thereof).

In the present invention, a "glycan" means a structural unit of two or more monosaccharides bonded together via glycosidic linkages. Specific monosaccharides and glycans are occasionally abbreviated, for example, as "GlcNAc-", "SG-", and so on. When any of these abbreviations is used in a structural formula, the abbreviation is shown with an intention that an oxygen atom or nitrogen atom involved in a glycosidic linkage at the reducing terminal to another structural unit is not included in the abbreviation indicating the glycan, unless specifically defined.

In the present invention, a monosaccharide as a basic unit of a glycan is indicated for convenience so that in the ring structure, the position of a carbon atom bonding to an oxygen atom constituting the ring and directly bonding to a hydroxy group (or an oxygen atom involved in a glycosidic linkage) is defined as the 1-position (the 2-position only for sialic acids), unless otherwise specified. The names of compounds in Examples are each provided in view of the chemical structure as a whole, and that rule is not necessarily applied.

When a glycan is indicated as a sign (e.g., SG, GlcNAc) in the present invention, the sign is intended, unless otherwise defined, to include carbon atoms ranging to the reducing terminal and not to include N or O involved in an N- or O-glycosidic linkage.

SGP (α2,6-SGP), an abbreviation for sialylglycopeptide, is a representative N-linked glycopeptide. As used herein, the glycan moiety of SGP is denoted as SG, and the glycan lacking one GlcNAc at the reducing end of SG is denoted as SG(10). SG(10) has the same meaning as the oligosaccharide represented by formula XI.

As used herein, the term "glycoprotein" means a protein with a glycan linked to a part of amino acids constituting the protein. As used herein, the "glycoprotein" is, for example, but not limited to, a glycan-remodeled antibody or a molecule containing an Fc region thereof.

In an embodiment of the present invention, the "glycoprotein" is a glycan-remodeled antibody or a molecule containing an Fc region thereof. Thus, an embodiment of the present invention provides a method for producing a glycan-remodeled antibody or a molecule containing an Fc region thereof, the method comprising the novel production method of the present invention, the method further comprising steps of
obtaining, from the obtained oligosaccharide represented by formula XI, a glycan donor molecule containing N-acetylglucosamine (GlcNAc) with a reducing end activated; and
reacting the glycan donor molecule with an acceptor molecule, namely an antibody with core GlcNAc optionally having a fucose as an N297-binding glycan or a molecule containing an Fc region thereof. As used herein, the "core GlcNAc" is GlcNAc at the reducing end of the N297-binding glycan.

As used herein, the term "glycan donor molecule" means a molecule that plays a role in adding a glycan to an acceptor molecule.

In the present invention, the wording "GlcNAc with an activated reducing end" means GlcNAc having an appropriate leaving group at the reducing end. Examples of the "GlcNAc with an activated reducing end" include oxazolinated GlcNAc, halogenated GlcNAc, phenylated GlcNAc, paranitrophenylated GlcNAc, or GlcNAc with dimethoxytriazine at the anomeric position (4,6-dimethoxy-1,3,5-triazin-2-yl-glycoside (DMT sugar)). Preferred is oxazolinated GlcNAc or fluorinated GlcNAc. For example, a glycan donor molecule containing oxazolinated GlcNAc is a compound (also referred to as "SG(10)-Ox") represented by the following formula XII:

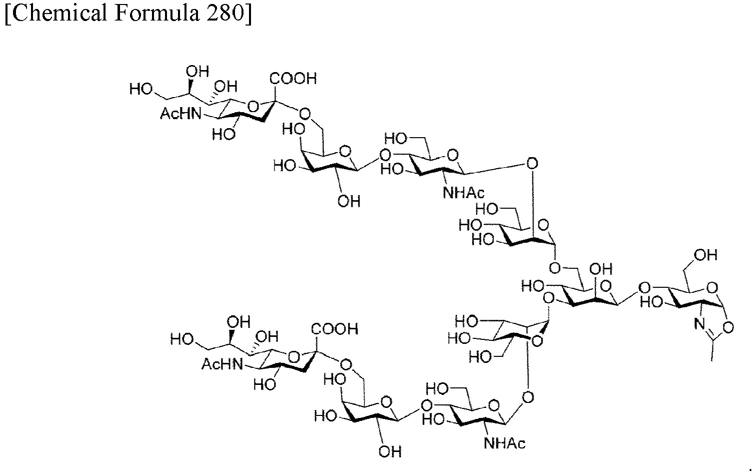

In the present invention, the non-reducing end of the "glycan donor molecule" may be chemically modified. It is possible to suitably select, depending on the purpose, a group(s) to be added by the chemical modification, a chemical reaction involved in the chemical modification, a functional group(s) on the glycosyl donor to be chemically modified, and so on. In an embodiment of the present invention, a glycan donor molecule may be chemically modified by reacting the carboxyl group at position 2 of sialic acid located at its non-reducing end with a compound having a terminal amino group to form an amide bond. Examples of the compound having a terminal amino group include, but are not particularly limited to, a compound having an azide group at the other end. Preferable examples include 11-azido-3,6,9-trioxaundecan-1-amine represented by the following formula:

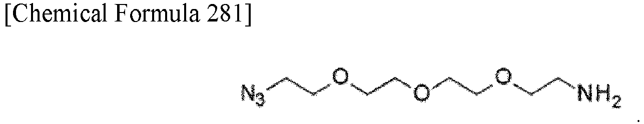

In an embodiment of the present invention, the "glycan donor molecule" is an azido-glycan oxazolinated moiety with an oxazolinated reducing end and an azide group-containing group at the non-reducing end. Note that the azido-glycan oxazolinated moiety can be synthesized according to the procedure described in WO2018/003983. As an example, how to synthesize [N₃-PEG(3)]₂-SG(10)-Ox (compound 1-10 as described in WO2018/003983) is illustrated in the following scheme.

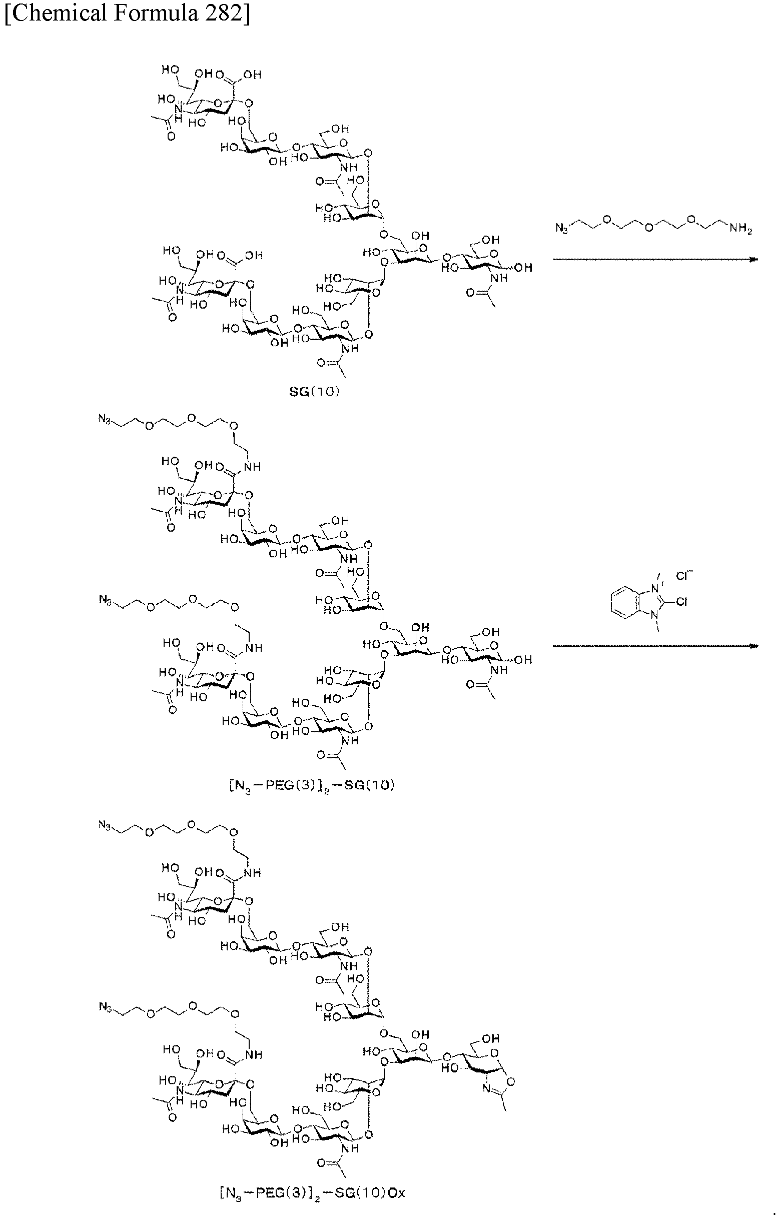

As used herein, the term "acceptor molecule" means a molecule that receives a glycan from the glycan donor molecule. The "acceptor molecule" is not particularly limited as long as the molecule can receive a glycan from the glycan donor molecule. Preferable examples include a glycan-cleaved antibody or molecule containing an Fc region thereof, in which most of the glycan is cleaved from the antibody. More preferable examples include an antibody with core GlcNAc or a molecule containing an Fc region thereof. The glycan that is partially cleaved in the glycan-cleaved antibody is an N-linked glycan or O-linked glycan, and preferably an N-linked glycan.

N-linked glycans and O-linked glycans each bond to an amino acid side chain of an antibody via an N-glycosidic linkage and an O-glycosidic linkage, respectively.

An antibody as the "acceptor molecule" is preferably IgG, and more preferably IgG1, IgG2 or IgG4.

IgG contains a well-conserved N-linked glycan (hereinafter, referred to as "Asn297-binding glycan or N297-binding glycan") at the 297th asparagine residue (hereinafter, referred to as "Asn297 or N297") in the Fc region of its heavy chain, and the glycan can contribute to, for example, the activity and dynamics of the antibody molecule (Eon-Duval, A. et al, Biotechnol. Prog. 2012, 28, 608-622; Sanglier-Cianferani, S., Anal. Chem. 2013, 85, 715-736).

The amino acid sequence in the constant region of IgG is well conserved, and each amino acid is specified by EU Index numbering in Edelman et al. (Proc. Natl. Acad. Sci. U.S. A., 63, 78-85, (1969)). For example, Asn297, to which an N-linked glycan is added, in the Fc region, corresponds to position 297 in the EU Index numbering, and even if the actual amino acid position changes due to fragmentation or loss of the region of the molecule, the amino acid can be uniquely identified by using the EU Index numbering.

In an embodiment of the present invention, the "acceptor molecule" is an antibody having core GlcNAc as an N297-binding glycan or a molecule containing an Fc region thereof. The core GlcNAc as an N297-binding glycan may have another monosaccharide or sugar chain attached. For example, fucose may be attached. Thus, in an embodiment of the present invention, the "acceptor molecule" is an antibody having optionally fucose-containing core GlcNAc as an N297-binding glycan or a molecule containing an Fc region thereof.

The glycan donor molecule may be reacted with an acceptor molecule which is an antibody with core GlcNAc optionally having a fucose as an N297-binding glycan or a molecule containing a Fc region thereof. As long as the reaction proceeds in this step, any reaction procedure and reaction conditions can be used.

In the present invention, a glycan-remodeled antibody or a molecule containing an Fc region thereof can be produced by the method illustrated in the following scheme in accordance with the procedure described in, for instance, WO2018/003983.

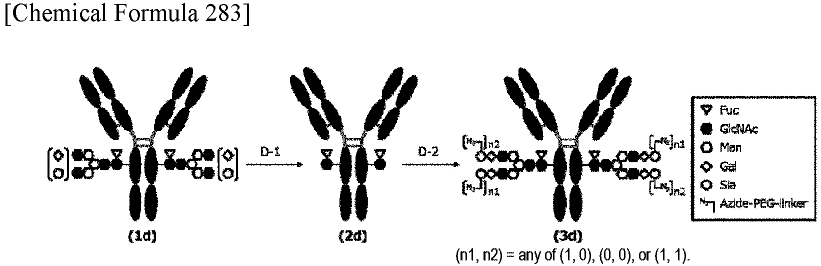

### (Step D-1)

This step is a step of producing a glycan-cleaved antibody by using a known enzymatic reaction to hydrolyze and cleave a glycosidic linkage between GlcNAcβ1-4GlcNAc of the chitobiose structure at the reducing end of the N-linked glycan (N297-binding glycan) attached to asparagine at position 297 of the amino acid sequence of an antibody. The hydrolysis reaction of the glycosidic linkage between GlcNAcβ1 and 4GlcNAc of the chitobiose structure at the reducing end of a desired antibody (1d) (10 mg/mL) is carried out using a hydrolase, e.g., wild-type EndoS enzyme, in a buffer solution (e.g., a phosphate buffer) at a temperature of 0°C to 40°C. The reaction time is from 10 minutes to 72 hours and preferably from 1 hour to 6 hours. The amount of wild-type EndoS enzyme used is from 0.1 mg to 10 mg and preferably from 0.1 mg to 3 mg per 100 mg of the antibody (1d). After completion of the reaction, the antibody is purified by affinity chromatography (HiTrap rProtein A FF (5 ml) (manufactured by GE Healthcare)) and/or hydroxyapatite column (Bio-Scale Mini CHT Type I cartridge (5 ml) (manufactured by BIO-RAD)). A (Fucα1,6)GlcNAc antibody (2d) is thus obtained.

### (Step D-2)

This step is a step of producing a glycan-remodeled antibody (3d) by using a known enzymatic reaction to link the (Fucα1,6)GlcNAc antibody (2d) obtained in step D-1 with an SG-type glycan oxazoline moiety having an azide group-containing PEG linker ("[N₃-PEG(3)]₂-SG(10)-Ox"). Note that the SG-type glycan oxazoline moiety having an azide group-containing PEG linker ("[N₃-PEG(3)]₂-SG(10)-Ox") can be synthesized according to the synthesis scheme for compound 1-10 described in WO2018/003983, as described above.

The glycosyltransfer is carried out by reacting the antibody (2d) with the SG-type glycan oxazoline moiety having an azide group-containing PEG linker ("[N₃-PEG(3)]₂-SG(10)-Ox") in the presence of glycosyltransferase, e.g., EndoS (D233Q/Q303L), in a buffer solution (e.g., a phosphate buffer) at a temperature of 0°C to 40°C. The reaction time is from 10 minutes to 72 hours and preferably from 1 hour to 6 hours. The amount of the EndoS enzyme (D233Q/Q303L) used is from 1 mg to 10 mg and preferably from 1 mg to 3 mg per 100 mg of the antibody; and 2 to excess equivalents and preferably 4 to 20 equivalents of the SG-type glycan oxazoline moiety having an azide group-containing PEG linker ("[N₃-PEG(3)]₂-SG(10)-Ox") is used. After completion of the reaction, the antibody is purified by affinity chromatography (HiTrap rProtein A FF (5 ml) (manufactured by GE Healthcare)) and hydroxyapatite column (Bio-Scale Mini CHT Type I cartridge (5 ml) (manufactured by BIO-RAD)). A glycan-remodeled antibody (3d) is thus obtained.

In the above preparation of the glycan-remodeled antibody, the common protocols A to C, described in WO2020/050406, may be used to concentrate the antibody aqueous solution, measure the concentration, and exchange the buffer.

As described above, a modified group containing an azide group may be added to the non-reducing end of the "glycan donor molecule" in the present invention. In this case, the method for producing a glycan-remodeled antibody or molecule containing an Fc region thereof according to the present invention may further include a step of reacting with a molecule having an alkyne structure on the azide group (N₃-). This reaction step may be carried out either before or after the reaction of the glycan donor molecules with the acceptor molecule. As used herein, the "molecule having an alkyne structure" may be any molecule as long as the alkyne structure is included. Examples include a chemotherapeutic agent, a molecular targeted drug, an immune activator, a toxin, an antimicrobial agent, an antiviral agent, a diagnostic agent, a protein, a peptide, an amino acid, a nucleic acid, an antigen, a vitamin, or a hormone. In the present invention, the "step of reacting with a molecule having an alkyne structure on an azide group (N₃-)" can use any reaction procedure and reaction conditions as long as the reaction proceeds. For example, the known procedure (e.g., WO2019/065964, WO2020/050406) may be used for implementation. For example, the "step of reacting with a molecule having an alkyne structure on an azide group (N₃-)" can be performed by using a SPAAC (strain-promoted azide-alkyne cycloaddition: J. Am. Chem. Soc. 2004, 126, 15046-15047) reaction to link the glycan-remodeled antibody (3d), as obtained in the above step D-2, and the molecule having an alkyne structure.

An embodiment of the present invention further provides a method for producing an antibody-drug conjugate, including the above method for producing a glycan-remodeled antibody or a molecule containing an Fc region thereof. In the present invention, the antibody and the drug included in the "antibody-drug conjugate" are not limited as long as they achieve the desired effect. Any of those can be used depending on the purpose. In the present invention, examples of the method for producing an antibody-drug conjugate include, but are not limited to, a method comprising step D-1, step D-2, and the SPAAC reaction described above.

As used herein, the term "antibody-drug conjugate" refers to a complex in which a drug(s) is conjugated via a linker to an antibody. By using this method, the drug(s) can be introduced in a site-specific manner and a uniform antibody-drug conjugate can be synthesized.

As used herein, an "antibody-drug conjugate" is represented by the following formula (XIII): 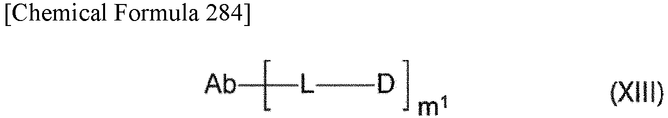 m¹ ranges from 1 to 10, and indicates the number of drugs, per antibody molecule, conjugated in the antibody-drug conjugate. Ab indicates an antibody or a functional fragment of the antibody, L indicates a linker connecting Ab and D, and D indicates a drug.

Examples of the antibody used herein include a full-body or a function fragment of antibody. The "functional fragment of antibody" is sometimes called an "antigen-binding fragment of antibody", and means a partial fragment of antibody having antigen-binding activity. Examples include an Fab, F(ab')₂, Fv, scFv, diabody, linear antibody and multispecific antibody formed using an antibody fragment(s). Also included is an Fab', which is a monovalent fragment of a variable region of antibody, obtained by treating F(ab')₂ under reducing conditions. Provided that the fragment is not limited to these molecules as long as having an ability to bind to an antigen. In addition, these antigen-binding fragments include not only those obtained by treating a full-length molecule of antibody protein with appropriate enzyme, but also proteins produced in appropriate host cells while a genetically engineered antibody gene is used.

In the present invention, the antibody may be derived from any species. Preferable examples include a human, a rat, a mouse, or a rabbit. If the antibody is derived from a species other than human, it is preferable to chimerize or humanize the antibody using well-known techniques. In addition, the antibody may be a polyclonal or monoclonal antibody, and is preferably a monoclonal antibody in the present invention. Examples of the monoclonal antibody include each monoclonal antibody derived from a non-human animal, e.g., a rat antibody, a mouse antibody, or a rabbit antibody, a chimeric antibody, a humanized antibody, a human antibody, a functional fragment thereof, or a modified antibody thereof.

The antibody used in the present invention is not limited as long as the desired effects can be exerted. Examples include anti-HER2 antibody, anti-HER3 antibody, anti-DLL3 antibody, anti-FAP antibody, anti-CDH11 antibody, anti-CDH6 antibody, anti-A33 antibody, anti-CanAg antibody, anti-CD19 antibody, anti-CD20 antibody, anti-CD22 antibody, anti-CD30 antibody, anti-CD33 antibody, anti-CD56 antibody, anti-CD70 antibody, anti-CD98 antibody, anti-TROP2 antibody, anti-CEA antibody, anti-Cripto antibody, anti-EphA2 antibody, anti-G250 antibody, anti-MUC1 antibody, anti-GPNMB antibody, anti-Integrin antibody, anti-PSMA antibody, anti-Tenascin-C antibody, anti-SLC44A4 antibody, anti-Mesothelin antibody, anti-ENPP3 antibody, anti-CD47 antibody, anti-EGFR antibody, anti-GPR20 antibody, or anti-DR5 antibody.

The antibody used for producing an antibody-drug conjugate of the present invention can be obtained by immunizing an animal with a polypeptide that serves as an antigen, and collecting and purifying the antibody produced in vivo, while using the protocols routinely implemented in the art. The origin of the antigen is not limited to a human, and each animal can also be immunized with the antigen derived from a non-human animal, e.g., a mouse, a rat. In this case, any antibody applicable to human diseases can be selected by testing the cross-reactivity between the obtained antibody that can bind to a heterologous antigen and the corresponding human antigen.

Alternatively, antibody-producing cells which produce antibodies against the antigen are fused with myeloma cells according to the known method (e.g., Kohler and Milstein, Nature (1975) 256, p.495-497; Kennett, R. ed., Monoclonal Antibodies, p.365-367, Plenum Press, N.Y. (1980)) to establish hybridomas, from which monoclonal antibodies can in turn be obtained.

Note that the antigen can be obtained by genetically engineering a host cell to produce a gene encoding an antigen protein of interest.

The humanized antibody used for producing an antibody-drug conjugate of the present invention can be obtained according to the known protocols (e.g., Proc. Natl. Acad. Sci. U.S.A., 81, 6851-6855, (1984); Nature (1986) 321, p.522-525, WO90/07861).

For example, anti-HER2 antibody (e.g., US5821337, WO2004/008099, WO2020/050406), anti-CD33 antibody (e.g., WO2014/057687, WO2020/050406), anti-EphA2 antibody (e.g., WO2009/028639, WO2020/050406), anti CDH6 antibody (e.g., WO2018/212136, WO2020/050406), anti-CD70 antibody (e.g., WO2004/073656, WO2007/038637), anti-TROP2 antibody (e.g., WO2015/098099), or anti-EGFR antibody (e.g., WO1998/050433, WO2002/092771) can be obtained by known means.

The antibody drug in the present invention is not limited as long as the desired effects can be exerted. Examples include a pharmacologically active compound such as a chemotherapeutic agent, a molecular targeted drug, an immune activator, a toxin, an antimicrobial agent, an antiviral agent, a diagnostic agent, a protein, a peptide, an amino acid, a nucleic acid, an antigen, a vitamin, or a hormone.

The drug D, for example, is represented by the following formula: 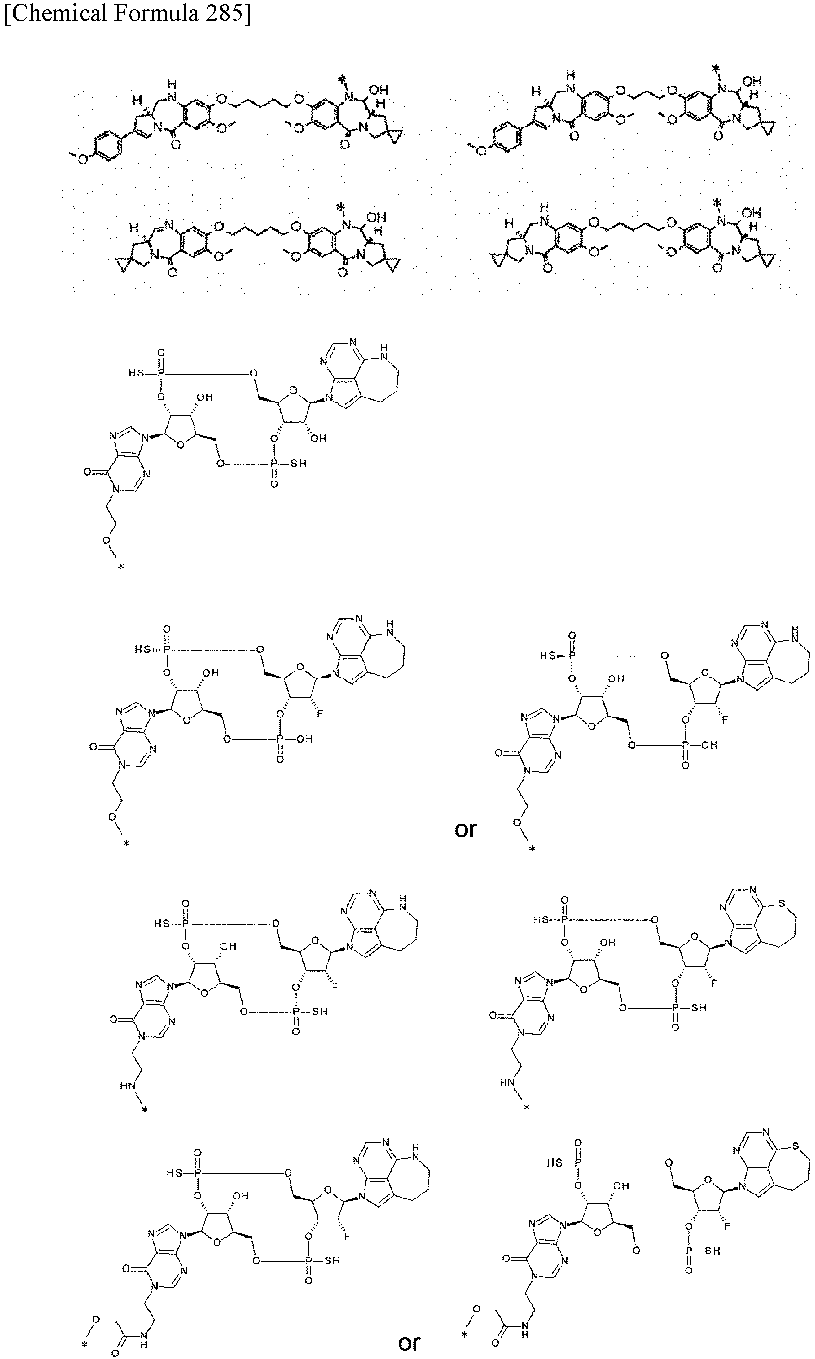 wherein * indicates bonding to the linker L.

In the present invention, the linker L linking antibody Ab to drug D is represented by the following formula:

-Lb-La-Lp-Lc-*

wherein * indicates bonding to the drug D.

Lp represents a linker consisting of an amino acid sequence cleavable in a target cell or is absent. Specific examples of Lp include -GGFG-, -GGPI-, - GGVA-, -GGFM-, -GGVCit-, -GGFCit-, -GGICit-, -GGPL-, -GGAQ-, or -GGPP-.

La is any one selected from

-C(=O)-(CH₂CH₂)n²-C(=O)-,

-C(=O)-(CH₂CH₂)n²-CH₂-C(=O)-,

-C(=O)-(CH₂CH₂)n²-C(=O)-NH-(CH₂CH₂)n³-C(=O)-,

-C(=O)-(CH₂CH₂)n²-C(=O)-NH-(CH₂CH₂)n³-CH₂-C(=O)-,

-C(=O)-(CH₂CH₂)n²-C(=O)-NH-(CH₂CH₂O)n³-CH₂-C(=O)-,

-(CH₂)n⁴-O-C(=O)-, or

-(CH₂)n⁹-C(=O)-,

wherein n² represents an integer of 1 to 3, n³ represents an integer of 1 to 5, n⁴ represents an integer of 0 to 2, and n⁹ represents an integer of 2 to 7.

Lb represents a spacer that links La to the glycan or remodeled glycan of Ab. Specific examples thereof include 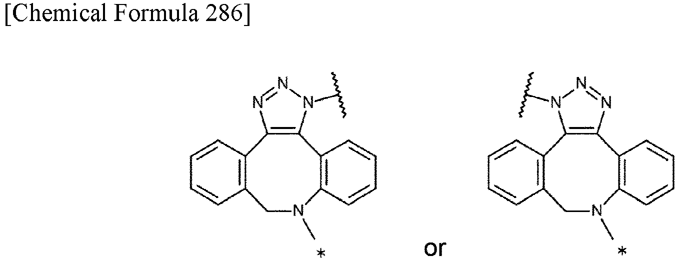 or 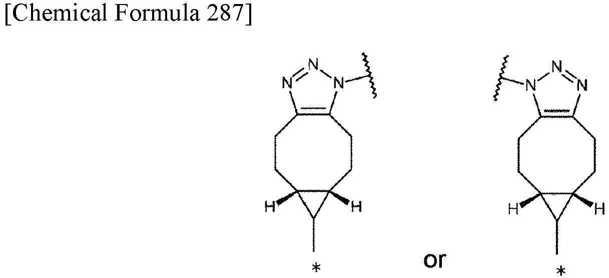 wherein in the structural formula of Lb shown above, * indicates bonding to La, and the wavy line indicates bonding to a glycan or remodeled glycan of Ab.

Lc represents -NH-CH₂- or -NH-B-CH2-O(C=O)-, or is absent. Here, B is a 1,4-phenyl, 2,5-pyridyl, 3,6-pyridyl, 2,5-pyrimidyl, or 2,5-thienyl group.

Hereinbelow, the present invention will be described with reference to Examples. However, the present invention is not limited to them. In the following, the term "compound I-1" or "I-1" in the synthesis scheme indicates that it corresponds to "compound represented by formula 1-1", and the same applies to subsequent compound numbers.

### Examples

In the following Examples, the room temperature indicated is from 15°C to 35°C. Silica gel chromatography was performed using Biotage Sfar HC D (20 µm, manufactured by Biotage), reverse-phase column chromatography was performed using Universal Column ODS Premium 30-µm L-size (manufactured by Yamazen Corporation) and Inject column ODS L-size (manufactured by Yamazen Corporation), and preparative HPLC was performed using Agilent Preparative HPLC System (manufactured by Agilent Technology). The preparative column used was XBridge Prep OBD (5 µm, C18, 130 Å, 250 × 30 mm; manufactured by Waters).

The following instruments were used to measure various spectral data. ¹H-NMR and ¹³C-NMR spectra were measured using JEOL ECZ500R and ECX400P. Mass spectra were measured using Shimadzu LCMS-2010 and LCMS-2020 (manufactured by Shimadzu Corporation), XEVO Q-Tof MS (Waters), and Q-Exactive (Thermo Fisher).

### <Synthesis of compound III-13>

Compound III-13 was synthesized according to the following synthesis scheme 1A.

### [Synthesis Scheme 1A]

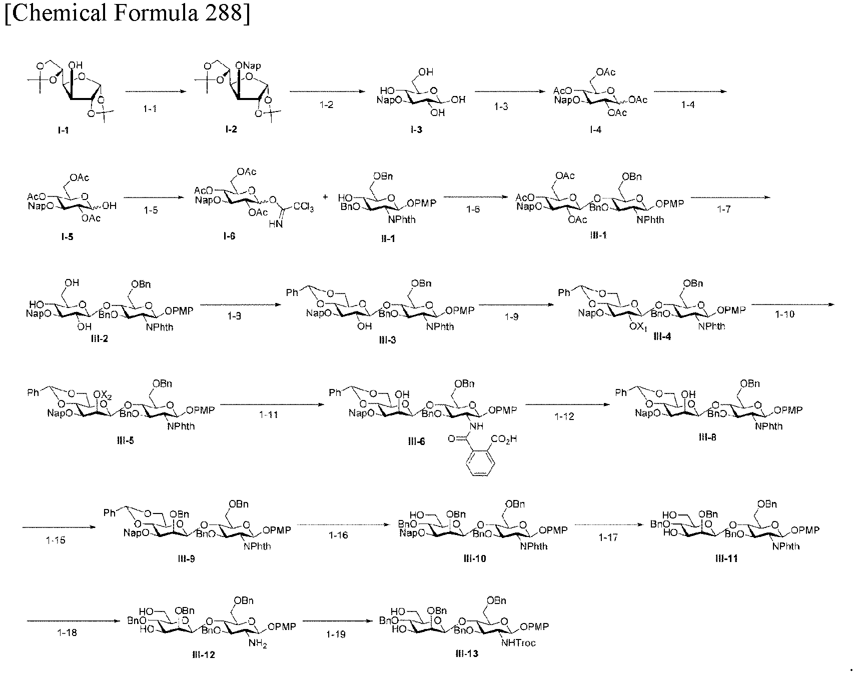

### Example 1:

### 1,2:5,6-Bis-O-(1-methylethyliden)-3-O-(2-naphthalenylmethyl)-α-D-glucofuranose (Compound I-2)

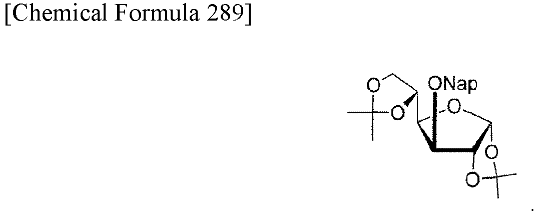

### (Substep 1-1)

A tetrahydrofuran (900 mL) solution containing sodium hydride (55.32 g, 1.38 mol, content: 50-72%) was cooled to 0°C. Next, a tetrahydrofuran (1.05 L) solution containing 1,2:5,6-bis-O-(1-methylethyliden)-α-D-glucofuranose (compound I-1) (300.00 g, 1.15 mol) was added dropwise over 1 hour. The temperature was then raised to 25°C and 1,3-dimethyl-2-imidazolidinone (150 mL) and 2-bromomethylnaphthalene (280.31 g, 1.27 mol) were added. After stirring at 25°C for 6 hours, the completion of the reaction was checked by HPLC. Ethylenediamine (anhydrous) (13.85 g, 230.52 mmol) was added, and the mixture was stirred for another 1 hour. The solution was cooled to 0°C and 10% aqueous citric acid solution (1.2 L) was added over 1 hour. The reaction liquid was diluted with heptane (3 L) and separated into an organic layer and an aqueous layer. The organic layer was washed with water (900 mL) and concentrated under reduced pressure until the liquid volume reached 900 mL. Acetonitrile (3 L) was further added, and the mixture was concentrated again until the liquid volume reached 900 mL to prepare, as an acetonitrile solution, crude 1,2:5,6-bis-O-(1-methylethyliden)-3 -O-(2-naphthalenylmethyl)-α-D-glucofuranose (compound I-2). This compound was used as it was in the next step.

### Example 2:

### 3-O-(2-Naphthalenylmethyl)-D-glucopyranose (Compound I-3)

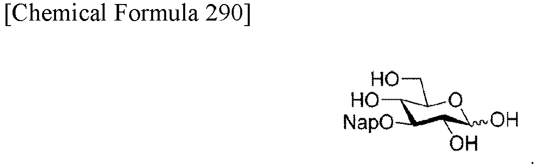

### (Substep 1-2)

To a solution containing the crude compound I-2 obtained in Example 1 were added acetonitrile (1.5 L), water (600 mL), and concentrated hydrochloric acid (17.51 g, 172.89 mmol), and the mixture was stirred at 55°C for 18.5 hours. After the completion of the reaction was checked by HPLC, the reaction liquid was cooled to 0°C and the pH in the system was adjusted to 6.25 with a 4 N aqueous sodium hydroxide solution (43.22 mL). The reaction liquid was diluted with heptane (900 mL) and separated into an acetonitrile layer and a heptane layer. Ethyl acetate (2.4 L) and water (600 mL) were added to the acetonitrile layer, and the mixture was liquid-separated to give an organic layer 1 and an aqueous layer. A mixed solution of ethyl acetate (1.5 L) and tetrahydrofuran (1.5 L) was added to the aqueous layer again, and the mixture was liquid-separated to give an organic layer 2 and an aqueous layer. The organic layers 1 and 2 were mixed, washed with saturated brine (600 mL), and concentrated under reduced pressure until the liquid volume reached 1.5 L (crystals were found to precipitate during the concentration step). Ethyl acetate (4.5 L) was further added, and the mixture was concentrated again until the liquid volume reached 3 L. To this suspension were added ethyl acetate (1.5 L) and cyclopentyl methyl ether (1.5 L), and the mixture was stirred at 55°C for 1 hour. Heptane (3 L) was added dropwise over 1.5 hours. After stirring for 1 hour, the mixture was cooled to 0°C. After that, the precipitated crystals were filtered and washed with an ethyl acetate (1.2 L)/heptane (600 mL) mixed solution cooled to 0°C. The resulting crystals were dried at 40°C under reduced pressure to afford 3-O-(2-naphthalenylmethyl)-D-glucopyranose (compound I-3) (356.95 g, yield 96.7%, HPLC area: 98.47%).

¹H-NMR (500 MHz, DMSO-d₆) δ 7.85-7.90 (m, 4H), 7.59 (dd, J = 8.0, 1.5 Hz, 1H), 7.46-7.51 (m, 2H), 6.69 (d, J = 6.0 Hz, 1H), 5.12 (dd, J = 5.0, 3.0 Hz, 2H), 4.94-5.00 (m, 2H), 4.53 (t, J = 6.0 Hz, 1H), 4.35 (dd, J = 8.0, 6.5 Hz, 1H), 3.70 (ddd, J = 11.5, 5.0, 2.0 Hz, 1H), 3.45-3.50 (m, 1H), 3.25-3.31 (m, 2H), 3.11-3.16 (m, 2H). ¹³C-NMR (125 MHz, DMSO-d₆) δ 137.3, 132.8, 132.3, 127.6, 127.5, 127.4, 126.1, 126.0, 125.6, 125.5, 96.9, 85.4, 76.7, 74.8, 73.7, 69.9, 61.1.
HRMS(ESI⁻)[M-H]⁻ calcd for C₁₇H₁₉O₆: 319.1187; found 319.1175.

### Example 3:

### 2,4,6-Tri-O-acetyl-3-O-(2-naphthalenylmethyl)-D-glucopyranose (Compound I-5)

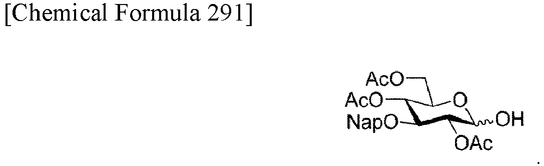

### (Substeps 1-3 and 1-4)

To a tetrahydrofuran (675 mL) solution containing 3-O-(2-naphthalenylmethyl)-D-glucopyranose (compound I-3) (150.00 g, 468.25 mmol) were added triethylamine (236.92 g, 2.34 mol) and 4-dimethylaminopyridine (0.29 g, 2.34 mmol). After the mixture was cooled to 0°C, acetic anhydride (195.99 g, 1.92 mol) was added dropwise over 30 minutes. The temperature was then raised to 25°C, and after stirring for 3 hour, 1,2,4,6-tetra-O-acetyl-3-O-(2-naphthalenylmethyl)-D-glucopyranose (compound I-4) was found using HPLC to be produced. The reaction liquid was cooled to 10°C and 1-methylpiperazine (60.97 g, 608.73 mmol) was added. After stirring at 35°C for 18 hours, the completion of the reaction was checked by HPLC. The mixture was then cooled to 0°C. The pH was adjusted to 6.36 with 6 N aqueous hydrochloric acid solution (480 mL), and the solution was diluted with heptane (375 mL) and separated into an organic layer and an aqueous layer. The organic layer was washed with saturated sodium bicarbonate water (450 mL) and then water (450 mL) and concentrated under reduced pressure until the liquid volume reached 450 mL. Ethyl acetate (2.25 L) was added, the mixture was concentrated again until the liquid volume reached 450 mL, and the same operation was repeated one more time. Dichloromethane (2.25 L) was added to this solution, the mixture was concentrated until the liquid volume reached 450 mL, and the same operation was repeated one more time to prepare, as a dichloromethane solution, crude 2,4,6-tri-O-acetyl-3-O-(2-naphthalenylmethyl)-D-glucopyranose (compound I-5). This compound was used as it was in the next step.

### Example 4:

### 2,4,6-Tri-O-acetyl-3-O-[(naphthalen-2-yl)methyl]-1-O-(2,2,2-trichloroethanimidoyl)-D-glycero-hexopyranose (Compound I-6)

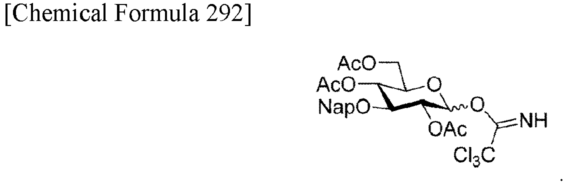

### (Substep 1-5)

To a solution containing the crude 2,4,6-tri-O-acetyl-3-O-(2-naphthalenylmethyl)-D-glucopyranose (compound I-5) obtained in Example 3 were added dichloromethane (450 mL) and trichloroacetonitrile (338.03 g, 2.34 mol), and the mixture was cooled to 0°C. Next, 1,8-diazabicyclo[5.4.0]-7-undecene (5.70 g, 37.46 mmol) was added dropwise. After stirring at 0°C for 14.5 hours, the completion of the reaction was checked by HPLC. Acetic acid (2.25 g, 37.46 mmol) was then added. Silica gel 60N (spherical, neutral) (150 g) was added to the solution, and the mixture was stirred for 1.5 hours and then filtered. The silica gel was washed with dichloromethane (1.5 L) and the filtrate was concentrated under reduced pressure until the liquid volume reached 450 mL. Dichloromethane (1.5 L) was further added, and the solution was concentrated until the liquid volume reached 450 mL to prepare, as a dichloromethane solution, crude 2,4,6-tri-O-acetyl-3-O-[(naphthalen-2-yl)methyl]-1-O-(2,2,2-trichloroethanimidoyl)-D-glycero-hexopyranose (compound I-6). This compound was used as it was in the next step.

### Example 5:

### 4-Methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (Compound III-1)

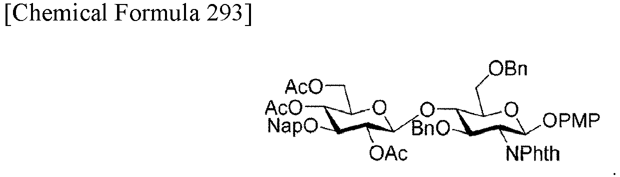

### (Substep 1-6)

To a solution containing the crude 2,4,6-tri-O-acetyl-3-O-[(naphthalen-2-yl)methyl]-1-O-(2,2,2-trichloroethanimidoyl)-D-glycero-hexopyranose (compound I-6) obtained in Example 4 were added 4-methoxyphenyl 3,6-di-O-benzyl-2-deoxy-2-(1,3-dioxo-1,3-dihydro-2H-isoindol-2-yl)-β-D-glucopyranoside (compound II-1) (276.66 g, 468.25 mmol), dichloromethane (4.2 L), and Molecular Sieves 4A (83.00 g), and the mixture was cooled to -5°C. Trimethylsilyl trifluoromethanesulfonate (10.41 g, 46.83 mmol) was added dropwise to this suspension over 20 minutes, and the mixture was then stirred for 3 hours. After the completion of the reaction was checked by HPLC, triethylamine (23.69 g, 234.13 mmol) was added. After the suspension was filtered, the filtrate was washed with ethyl acetate (2.8 L) and concentrated under reduced pressure until the liquid volume reached 1.4 L. Ethyl acetate (4.2 L) was further added, the mixture was concentrated until the liquid volume reached 1.4 L, and the same operation was repeated one more time. This solution was added with ethyl acetate (2.8 L), and the mixture was washed with saturated sodium bicarbonate water (830 mL) and water (830 mL). The resulting organic layer was then concentrated under reduced pressure until the liquid volume reached 830 mL. Next, 2-propanol (4.2 L) was added, and the mixture was concentrated until the liquid volume reached 1.4 L. The suspension was then warmed to 65°C. Ethyl acetate (830 mL) was added. After the mixture was stirred at 65°C for 2 hours, 2-propanol (5.53 L) was added dropwise over 2 hours. After this suspension was cooled to 0°C, the crystals were filtered and washed with 2-propanol (1.4 L) cooled to 0°C. The resulting crystals were dried under reduced pressure at 40°C to give crude 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound III-1) (355.34 g, yield 90.5%, HPLC area: 94.46%).

The resulting crude compound III-1 (350.00 g) was admixed with methyl isobutyl ketone (2.1 L). The compound was dissolved at 50°C, and ethyl cyclohexane (1.4 L) was added dropwise over 1 hour. Seed crystals (70.00 mg) of 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound III-1) were added, and the mixture was stirred for 1 hour. After the crystals were found to precipitate, ethylcyclohexane (4.9 L) was added dropwise over 2 hours. The suspension was cooled to room temperature and stirred for 14.5 hours. The precipitated crystals were then filtered, and washed with a mixed solution of methyl isobutyl ketone (350 mL) and ethyl cyclohexane (1.4 L). The resulting crystals were dried under reduced pressure at 40°C to give 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound III-1) (331.74 g, yield 94.8%, HPLC area: 98.07%).

¹H-NMR (500 MHz, CDCl₃) δ 7.81-7.84 (m, 4H), 7.69 (br, 1H), 7.65 (br, 3H), 7.46-7.51 (m, 2H), 7.28-7.36 (m, 6H), 7.00 (dd, J = 7.0, 1.5 Hz, 2H), 6.77-6.84 (m, 5H), 6.66-6.69 (m, 2H), 5.59, (d, J = 9.0 Hz, 1H), 5.09-5.15 (m, 2H), 4.82 (d, J = 12.5 Hz, 1H), 4.77 (d, J = 12.0 Hz, 1H), 4.71-4.77 (m, 2H), 4.60 (d, J = 8.0 Hz, 1H), 4.50 (d, J = 12.5 Hz, 1H), 4.45 (d, J = 13.0 Hz, 1H), 4.36 (dd, J = 11.0, 8.5 Hz, 1H), 4.28 (dd, J = 11.0, 8.5 Hz, 1H), 4.20 (dd, J = 12.5, 5.0 Hz, 1H), 4.10 (dd, J = 10.0, 8.5 Hz, 1H), 3.99 (dd, J = 12.0, 2.0 Hz, 1H), 3.80 (br, 2H), 3.69 (s, 3H), 3.58-3.62 (m, 2H), 3.44 (ddd, J = 10.0, 4.5, 2.5 Hz, 1H), 1.98 (s, 3H), 1.938 (s, 3H), 1.937 (s, 3H). ¹³C-NMR (125 MHz, CDCl₃) δ 171.0, 169.5, 169.1, 155.6, 151.0, 138.7, 138.2, 135.5, 133.9, 133.4, 133.2, 128.7, 128.4, 128.23, 128.20, 128.06, 128.05, 127.9, 127.2, 126.5, 126.2, 125.7, 123.5, 118.9, 114.5, 100.7, 97.8, 80.6, 78.5, 76.8, 75.2, 74.8, 74.1, 73.8, 73.1, 72.1, 69.9, 67.8, 62.2, 55.78, 55.75, 21.1, 20.94, 20.85. HRMS(ESI⁺)[M+H]⁺ calcd for C₅₈H₅₈NO₁₆: 1024.3750; found 1024.3706.

The obtained compound was found to correspond to the spectrum in the following literature: Reference 1) Org. Biomol. Chem., 2018, 16, 4720-4727.

### Example 6:

### 4-Methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (Compound III-2)

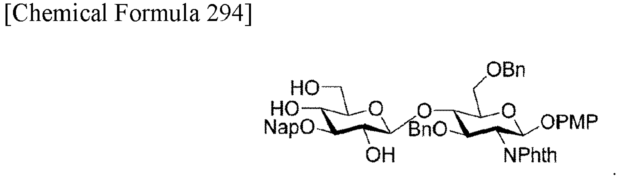

### (Substep 1-7)

To a tetrahydrofuran (150 mL) solution containing 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound III-1) (30.00 g, 29.29 mmol) were added methanol (90 mL) and methyl trifluoroacetate (3.75 g, 29.29 mmol). The mixture was stirred at 25°C for 10 min, and potassium tert-butoxide (1 mol/L tetrahydrofuran solution) (14.7 mL, 14.65 mmol) was then added. Next, the temperature was raised to 55°C. After stirring for 2 hours, the completion of the reaction was checked by HPLC. The reaction liquid was cooled to 25°C and acetic acid (1.76 g, 29.29 mmol) and ethyl acetate (300 mL) were added in this order. This solution was washed twice with 1% brine (300 mL) and concentrated under reduced pressure until the liquid volume reached 90 mL. Ethyl acetate (450 mL) was added, and the mixture was concentrated again until the liquid volume reached 90 mL. Acetonitrile (450 mL) was further added, and the mixture was concentrated until the liquid volume reached 90 mL to prepare, as an acetonitrile solution, crude 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (compound III-2). This compound was used as it was in the next step.

### Example 7:

### 4-Methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (Compound III-3)

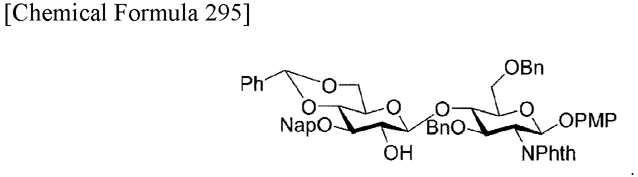

### (Substep 1-8)

To a solution containing the crude 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (compound III-2) obtained in Example 6 were added acetonitrile (210 mL), benzaldehyde dimethyl acetal (5.13 g, 33.69 mmol), and p-toluenesulfonic acid monohydrate (0.17 g, 0.88 mmol), and the mixture was stirred at 25°C for 30 minutes. Toluene (600 mL) was added to this solution, and the solution was concentrated until the liquid volume reached 300 mL. At this time point, the completion of the reaction was checked by HPLC. Further, 1-methylimidazole (12.03 g, 146.47 mmol) was added, and the mixture was concentrated until the liquid volume reached 90 mL to prepare, as a 1-methylimidazole-containing toluene solution, crude 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (compound III-3). This compound was used as it was in the next step.

### Example 8:

### 4-Methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside (Compound III-4, X₁ = trifluoromethanesulfonyl group)

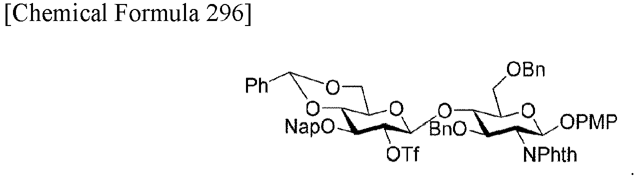

### (Substep 1-9)

To a (1-methylimidazole-containing) solution containing the crude 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (compound III-3)obtained in Example 7 was added ethyl acetate (210 mL), and the mixture was cooled to 0°C. Ttrifluoromethanesulfonic anhydride (16.53 g, 58.59 mmol) was added dropwise to this solution over 1 hour, followed by stirring for 30 minutes. After the completion of the reaction was checked by HPLC, water (300 mL) was added and organic and aqueous layers were separated. The organic layer was washed twice with water (300 mL) and once with saturated brine (150 mL), and then concentrated under reduced pressure until the liquid volume reached 90 mL. Ethyl acetate (300 mL) was added, and the mixture was concentrated again until the liquid volume reached 90 mL to prepare, as an ethyl acetate solution, crude 4-methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside (Compound III-4, X₁ = trifluoromethanesulfonyl group). This compound was used as it was in the next step.

### Example 9:

### 4-Methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (Compound III-6)

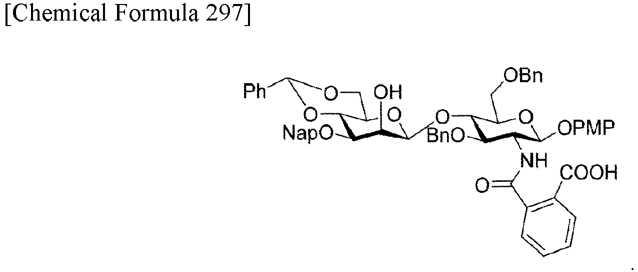

### (Substeps 1-10 and 1-11)

To a solution containing the crude 4-methoxyphenyl 3,6-di-O-benzyl-4-Of 4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside (Compound III-4, X₁ = trifluoromethanesulfonyl group) obtained in Example 8 were added dimethyl sulfoxide (150 mL) and tetrabutyl ammonium acetate (17.67 g, 58.59 mmol). Next, the temperature was raised to 30°C. After 17 hours of stirring, HPLC revealed the production of 4-methoxyphenyl 4-O-{2-O-acetyl-4,6-O-benzyliden-3-O-[(naphthalen-2-yl)methyl]-β-D-mannopyranosyl}-3,6-di-O-benzyl-2-deoxy-2-(1,3-dioxo-1,3-dihydro-2H-isoindol-2-yl)-β-D-glucopyranoside (compound III-5, X₂ = acetyl group). Toluene (150 mL) was added to this reaction liquid, and the mixture was concentrated under reduced pressure until the liquid volume reached 165 mL. Methanol (45 mL) and 50% aqueous sodium hydroxide solution (3.52 g, 87.88 mmol) were added, and the mixture was stirred at 25°C for 1.5 hours. After the completion of the reaction was checked by HPLC, ethyl acetate (450 mL) and water (300 mL) were added for liquid separation. Water (300 mL) was added to the resulting organic layer. The mixture was cooled to 0°C, and adjusted to pH 2.73 with 6 N hydrochloric acid under strong stirring. Tetrahydrofuran (300 mL) was added to the separated organic layer, and the mixture was concentrated under reduced pressure until the liquid volume reached 150 mL. Tetrahydrofuran (300 mL) was added, the mixture was concentrated again until the liquid volume reached 90 mL, and the internal temperature was then adjusted to 45°C. Tetrahydrofuran (60 mL) was added, and the mixture was then cooled to 25°C. Next, 2-propanol (150 mL) and water (15 mL) were added. Seed crystals of 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (compound III-6) (30 mg) were then added. The mixture was stirred at 25°C for 14 hours, and after the crystals were found to precipitate, 2-propanol (210 mL) was added dropwise over 1 hour. The mixture was then cooled to 0°C. After stirring for 2 hours, the precipitated crystals were filtered and washed with 2-propanol (150 mL) cooled to 0°C. The resulting crystals were dried under reduced pressure at 40°C to give 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (compound III-6) (27.74 g, yield 94.3%, HPLC area: 97.85%).

Note that the conversion of compound III-4 (X₁ = trifluoromethanesulfonyl group) to compound III-5 (X₂ = acetyl group) can also be performed under the conditions using cesium acetate (3 equivalents) and dimethyl sulfoxide at 50°C for 24 hours. The compound III-6 can then be obtained by a similar reaction (compound III-5 (X₂ = acetyl group) -> compound III-6) and post-treatment.

¹H-NMR (500 MHz, CDCl₃) δ 8.02 (dd, J = 6.0, 2.0 Hz, 1H), 7.69-7.83 (m, 4H), 7.38-7.49 (m, 12H), 7.32-7.34 (m, 2H), 7.16-7.29 (m, 8H), 6.97 (ddd, J = 9.0, 4.0, 2.5 Hz, 2H), 6.76 (ddd, J = 9.5, 3.5, 2.5 Hz, 2H), 5.51 (s, 1H), 5.40 (d, J = 6.0 Hz, 1H), 4.83-4.91 (m, 3H), 4.76 (d, J = 11.5 Hz, 1H), 4.55 (d, J = 0.5 Hz, 1H), 4.49 (d, J = 12.0 Hz, 1H), 4.36 (d, J = 12.0 Hz, 1H), 4.26-4.30 (m, 1H), 4.16 (t, J = 6.5 Hz, 1H), 4.05-4.09 (m, 2H), 3.99 (dd, J = 3.0, 0.5 Hz, 1H), 3.93 (t, J = 9.5 Hz, 1H), 3.80-3.86 (m, 2H), 3.71 (s, 3H), 3.65-3.69 (m, 1H), 3.56 (t, J = 10.0 Hz, 1H), 3.51 (dd, J = 10.0, 3.5 Hz, 1H), 3.13 (td, J = 9.5, 5.0 Hz, 1H).
¹³C-NMR (125 MHz, CDCl₃) δ 170.9, 168.4, 155.3, 151.4, 138.7, 138.0, 137.6, 136.2, 135.4, 133.4, 133.3., 132.2, 132.1, 130.7, 130.3, 129.2, 128.6, 128.49, 128.45, 128.11, 128.07, 128.0, 127.89, 127.87, 127.8, 126.8, 126.4, 126.3, 126.2, 125.8, 118.6, 114.7, 101.7, 100.4, 99.1, 78.3, 76.7, 76.4, 75.1, 73.7, 73.3, 72.5, 69.9, 69.4, 68.5, 67.0, 55.8, 54.4.
HRMS(ESI⁺)[M+H]⁺ calcd for C₅₉H₅₈NO₁₄: 1004.3852; found 1004.3873.

### Example 10:

### 4-Methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (Compound III-8)

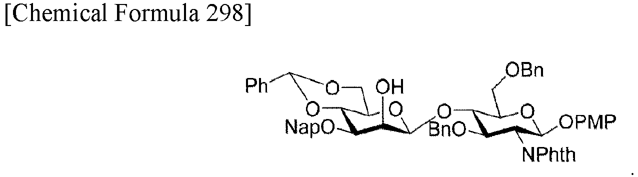

### (Substep 1-12)

To a dichloromethane (30 mL) solution containing 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (compound III-6) (3.00 g, 2.99 mmol) were added 1-hydroxybenzotriazole monohydrate (91.8 mg, 0.60 mmol), N,N-diisopropylethylamine (0.42 g, 3.29 mmol), 1-(3-dimethylaminopropyl)-3-ethylcarbodiimide hydrochloride (0.63 g, 3.29 mmol). The temperature was raised to 40°C, and the mixture was stirred for 24 hours. After the completion of the reaction was checked by HPLC, the reaction solution was cooled to 25°C and liquid-separated with water (30 mL). The resulting organic layer was then washed with saturated brine (15 mL). The solution was concentrated to dryness under reduced pressure to give crude 4-methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-8). This compound was used as it was in the next step.

### Example 11:

### 4-Methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (Compound III-9)

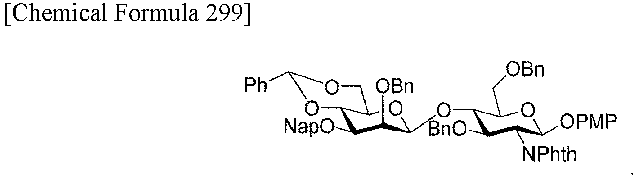

### (Substep 1-15)

To the crude 4-methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-8) obtained in Example 10 were added N,N-dimethylacetamide (30 mL), benzyl bromide (0.77 g, 4.48 mmol), and Molecular Sieves 4A (900 mg), and the mixture was cooled to 0°C. Sodium hydride (0.16 g, 3.88 mmol, content: 50-72%) was then added, and the mixture was stirred for 3 hours. After the completion of the reaction was checked by HPLC, the reaction solution was cooled to 25°C. Ethylenediamine (anhydrous) (0.18 g, 2.99 mmol) was added, and the mixture was stirred for another 1 hour. This solution was cooled to 0°C, acetic acid (0.36 g. 5.98 mmol) was added, and the mixture was then filtered. After the Molecular Sieves 4A was washed with N,N-dimethylacetamide (15 mL), ethyl acetate (60 mL) and water (30 mL) were added for liquid separation. The resulting organic layer was washed twice with water (30 mL) and once with saturated brine (15 mL). The solution was concentrated to dryness under reduced pressure, toluene (30 mL) and silica gel 60N (spherical) (3 g) were added, and the solution was stirred at 25°C for 30 minutes. The suspension was filtered, and the silica gel was washed with a mixed solution of toluene (82 mL) and ethyl acetate (8 mL). The filtrate was concentrated to dryness under reduced pressure to give crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-9).

### Example 12:

### 4-Methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (Compound III-10)

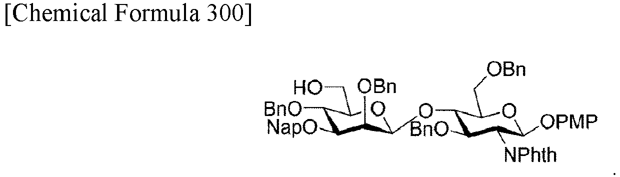

### (Substep 1-16)

To the crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-9) obtained in Example 11 were added dichloromethane (30 mL) and Molecular Sieves 4A (300 mg), and the mixture was cooled to 0°C. A borane-tetrahydrofuran complex (0.91 mol/L tetrahydrofuran solution) (16.42 mL, 14.94 mmol) and copper(II) trifluoromethanesulfonate (0.16 g, 0.45 mmol) were added, and the mixture was stirred for 6 hours. After the completion of the reaction was checked by HPLC, methanol (3 mL) was added and the mixture was stirred for another 1 hour. This solution was filtered, and the Molecular Sieves 4A was washed with ethyl acetate (30 mL). The resulting organic layer was then washed twice with 0.5 N hydrochloric acid (30 mL) and once with saturated brine (15 mL). The solution was concentrated under reduced pressure until the liquid volume reached 6 mL. Toluene (15 mL) was added, and the mixture was concentrated again until the liquid volume reached 6 mL to prepare a toluene solution containing crude 4-methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (compound III-10).

### Example 13:

### 4-Methoxyphenyl 3,6-di-O-benzyl-2-deoxy-4-O-{2,4-di-O-benzyl-β-D-mannopyranosyl}-2-(1,3-dioxo-1,3-dihydro-2H-isoindol-2-yl)-β-D-glucopyranoside (Compound III-11)

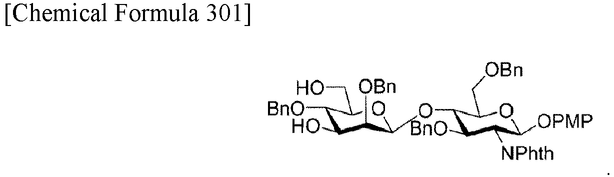

### (Substep 1-17)

To a solution containing crude 4-methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (compound III-10) were added dichloromethane (30 mL), water (1.5 mL), and potassium dihydrogen phosphate (0.81 g, 5.98 mmol). The mixture was cooled to 0°C, and 2,3-dichloro-5,6-dicyano-1,4-benzoquinone (0.81 g, 3.59 mmol) was added. Next, the temperature was raised to 25°C. After stirring for 8 hours, the completion of the reaction was checked by HPLC. The reaction solution was cooled to 0°C, and ethyl acetate (60 mL) and 5% aqueous sodium sulfite solution (30 mL) were added for liquid separation. The resulting organic layer was washed twice with 5% aqueous sodium sulfite solution (30 mL) and once with saturated brine (15 mL), and then concentrated to dryness under reduced pressure. The resulting material was purified by silica gel column chromatography (silica gel: 300 g; hexane:ethyl acetate = 50:50 -> 30:70) to give 4-methoxyphenyl 3,6-di-O-benzyl-2-deoxy-4-O-{2,4-di-O-benzyl-β-D-mannopyranosyl}-2-(1,3-dioxo-1,3-dihydro-2H-isoindol-2-yl)-β-D-glucopyranoside (compound III-11) (2.15 g, yield 76.7%, HPLC area: 98.67%).

¹H-NMR (500 MHz, CDCl₃) δ 7.67 (br, 4H), 7.27-7.37 (m, 15H), 6.89-6.96 (m, 5H), 6.83 (ddd, J = 9.5, 4.0, 2.5 Hz, 2H), 6.70 (ddd, J = 9.0, 4.0, 2.5 Hz, 2H), 5.64 (d, J = 9.0 Hz, 1H), 5.02 (d, J = 11.5 Hz, 1H), 4.94 (d, J = 12.0 Hz, 1H), 4.82 (d, J = 11.5 Hz, 1H), 4.70 (d, J = 12.0 Hz, 1H), 4.66 (d, J = 12.0 Hz, 1H), 4.62 (s, 1H), 4.57 (d, J = 11.0 Hz, 1H), 4.51 (d, J = 12.0 Hz, 1H), 4.42-4.46 (m, 2H), 4.33 (dd, J = 10.5, 8.5 Hz, 1H), 4.09 (t, J = 9.0 Hz, 1H), 3.69-3.79 (m, 4H), 3.71 (s, 3H), 3.65 (d, J = 3.5 Hz, 1H), 3.51 (td, J = 9.0, 3.5 Hz, 1H), 3.45 (t, J = 9.0 Hz, 1H), 3.44-3.48 (m, 1H), 3.17 (m, 1H), 2.29 (d, J = 9.0 Hz, 1H), 1.92 (t, J = 6.0 Hz, 1H).
¹³C-NMR (125 MHz, CDCl₃) δ 155.6, 151.0, 138.54, 138.46, 138.3, 137.8, 134.1, 131.8, 128.8, 128.6, 128.3, 128.21, 128.19, 128.15, 128.1, 128.0, 127.5, 127.4, 123.6, 118.9, 114.6, 101.5, 97.9, 79.1, 78.5, 77.3, 76.7, 75.6, 75.4, 75.3, 74.9, 74.8, 74.4, 73.9, 68.5, 62.5, 55.8.
HRMS(ESI⁻)[M-H]⁻ calcd for C₅₅H₅₄NO₁₃: 936.3601; found 936.3592.

The obtained compound was found to correspond to the spectrum in the following literature: Reference 1) Org. Biomol. Chem., 2018, 16, 4720-4727.

### Example 14:

### 4-Methoxyphenyl 2-amino-3,6-di-O-benzyl-2-deoxy-4-O-(2,4-di-O-benzyl-β-D-mannopyranosyl)-β-D-glucopyranoside (Compound III-12)

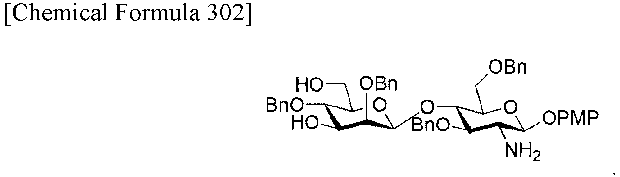

### (Substep 1-18)

To a 2-methyltetrahydrofuran (3.5 mL)/normal butanol (3.5 mL) mixed solution containing 4-methoxyphenyl 3,6-di-O-benzyl-2-deoxy-4-O-{2,4-di-O-benzyl-β-D-mannopyranosyl}-2-(1,3-dioxo-1,3-dihydro-2H-isoindol-2-yl)-β-D-glucopyranoside (compound III-11) (700 mg, 0.75 mmol) was added anhydrous ethylenediamine (0.25 mL, 3.73 mmol). The temperature was then raised to 80°C, and the mixture was stirred for 16.5 hours. After the completion of the reaction was checked by HPLC, the mixture was cooled to room temperature. Cyclopentyl methyl ether (7 mL) and 10% aqueous methanol solution (2.8 mL) were then added. The organic layer obtained by separating the aqueous layer was washed twice with 10% aqueous methanol solution (2.8 mL), and then concentrated to dryness under reduced pressure to give crude 4-methoxyphenyl 2-amino-3,6-di-O-benzyl-2-deoxy-4-O-(2,4-di-O-benzyl-β-D-mannopyranosyl)-β-D-glucopyranoside (compound III-12).

### Example 15:

### 4-Methoxyphenyl 3,6-di-O-benzyl-2-deoxy-4-O-(2,4-di-O-benzyl-β-D-mannopyranosyl)2-{[(2,2,2-trichloroethoxy)carbonyl]amino}-β-D-glucopyranoside (Compound III-13)

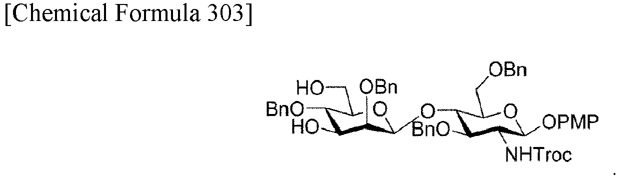

### (Substep 1-19)

To the crude 4-methoxyphenyl 2-amino-3,6-di-O-benzyl-2-deoxy-4-O-(2,4-di-O-benzyl-β-D-mannopyranosyl)-β-D-glucopyranoside (compound III-12) were added tetrahydrofuran (5.6 mL) and water (0.7 mL). Sodium hydrogen carbonate (125.4 mg, 1.49 mmol) and 2,2,2-trichloroethyl chloroformate (189.7 mg, 0.90 mmol) were then added. After stirring at 25°C for 2 hours, water (2.8 mL) and cyclopentyl methyl ether (7 mL) were added to the resulting suspension. The aqueous layer was removed. The crude product was then concentrated to dryness under reduced pressure, and purified by silica gel column chromatography (ethyl acetate/hexane 20% -> 65%) to give 4-methoxyphenyl 3,6-di-O-benzyl-2-deoxy-4-O-(2,4-di-O-benzyl-β-D-mannopyranosyl)2-{ [(2,2,2-trichloroethoxy)carbonyl]amino}-β-D-glucopyranoside (compound III-13) (702.6 mg, yield 96%).

¹H-NMR (500 MHz, CDCl₃) δ 1.94 (br, 1H), 2.34 (d, J = 9.0 Hz, 1H), 3.14 (m, 1H), 3.40-3.60 (m, 4H), 3.60-3.80 (m, 5H), 3.74 (s, 3H), 3.98 (t, J = 9.0 Hz, 1H), 4.02 (br, 1H), 4.45 (d, J = 7.0 Hz, 1H), 4.46 (brs, 1H), 4.58 (d, J = 11.5 Hz, 1H), 4.59-4.65 (m, 4H), 4.68 (d, J = 12.0 Hz, 1H), 4.82 (d, J = 11.0 Hz, 1H), 4.93 (d, J = 11.0 Hz, 1H), 5.01 (d, J = 10.5 Hz, 1H), 5.23 (d, J = 5.5 Hz, 1H), 5.54 (d, J = 7.0 Hz, 1H), 6.77 (d, J = 9.0 Hz, 2H), 6.96 (d, J = 9.0 Hz, 2H), 7.22-7.38 (m, 20H).
¹³C-NMR (125 MHz, CDCl₃) δ 55.4, 57.2, 62.1, 68.5, 73.6, 74.0, 74.2, 74.3, 74.6, 74.9, 75.1, 75.3, 76.4, 77.8, 77.9, 78.2, 95.4, 99.4, 101.4, 114.4, 118.5, 127.4, 127.65, 127.75, 127.82, 127.9, 128.3, 128.4, 128.46, 128.50, 137.5, 138.0, 138.2, 128.3, 151.2, 153.9, 155.3.
MS(ESI)(m/z): 999 ([M+NH₄]⁺).
= 999

### <Synthesis of compound III-8>

Compound III-8 was synthesized according to the following synthesis scheme 1B.

### [Synthesis Scheme 1B]

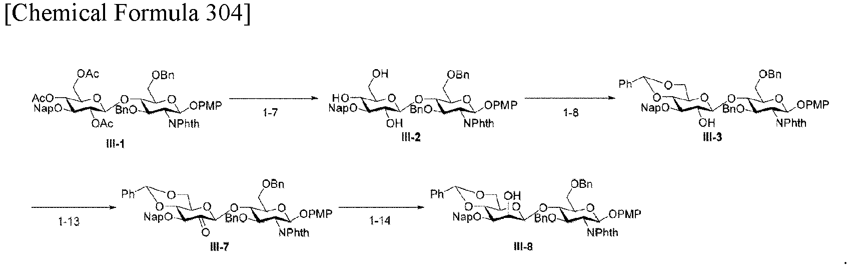

### Example 16:

### 4-Methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (Compound III-2)

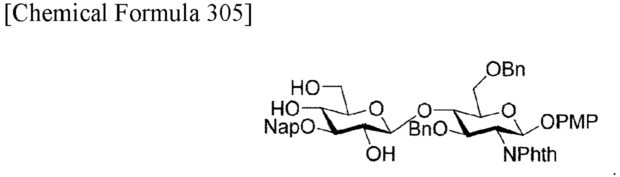

### (Substep 1-7)

To a tetrahydrofuran (250 mL) solution containing 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (compound III-1) (50.00 g, 48.82 mmol) were added methanol (75 mL) and methyl trifluoroacetate (6.25 g, 48.82 mmol), and the mixture was stirred at 25°C for 30 minutes. Potassium tert-butoxide (1 mol/L tetrahydrofuran solution) (24.41 mL, 24.41 mmol) was then added. Next, the temperature was raised to 55°C, and the mixture was stirred for 3.5 hours. The completion of the reaction was then checked by HPLC. The reaction liquid was cooled to 25°C, acetic acid (2.93 g, 48.82 mmol) was added, and the mixture was concentrated under reduced pressure until the liquid volume reached 150 mL. Ethyl acetate (500 mL) and water (150 mL) were then added for liquid separation. The resulting organic layer was concentrated under reduced pressure until the liquid volume reached 150 mL. Acetonitrile (750 mL) was added, and the mixture was concentrated again until the liquid volume reached 150 mL. Acetonitrile (500 mL) was further added, and the mixture was concentrated until the liquid volume reached 250 mL to prepare, as an acetonitrile solution, crude 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (compound III-2). This compound was used as it was in the next step.

### Example 17:

### 4-Methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (Compound III-3)

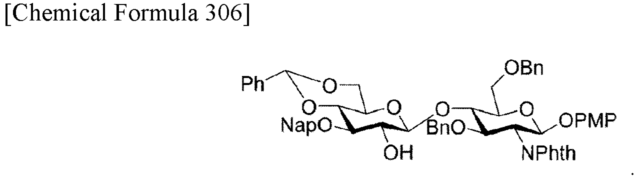

### (Substep 1-8)

To a solution containing the crude 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (compound III-2) obtained in Example 16 were added acetonitrile (250 mL), benzaldehyde dimethyl acetal (14.86 g, 97.65 mmol), and p-toluenesulfonic acid monohydrate (0.46 g, 2.44 mmol), and the mixture was stirred at 25°C for 30 minutes. The reaction solution was concentrated until the liquid volume reached 150 mL. At this time point, the completion of the reaction was checked by HPLC. Triethylamine (1.48 g, 14.65 mmol), ethyl acetate (500 mL), and saturated sodium bicarbonate water (150 mL) were added to this solution for liquid separation. The resulting organic layer was washed with 2 N hydrochloric acid (150 mL), and the separated organic layer was concentrated under reduced pressure until the liquid volume reached 150 mL. Ethyl acetate (500 mL) was further added, and the mixture was concentrated again until the liquid volume reached 250 mL to prepare, as an ethyl acetate solution, crude 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (compound III-3). This compound was used as it was in the next step.

### Example 18:

### 4-Methoxyphenyl 3,6-di-O-benzyl-4-O-{4,6-O-benzyliden-3-O-[(naphthalen-2-yl)methyl]hexopyranosyl-2-ulose}-2-deoxy-2-(1,3-dioxo-1,3-dihydro-2H-isoindol-2-yl)-β-D-glucopyranoside (Compound III-7)

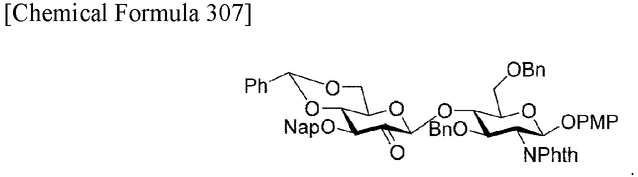

### (Substep 1-13)

To a solution containing the crude 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (compound III-3) were added dichloromethane (150 mL) and iodobenzene diacetate (18.87 g, 58.59 mmol), and the mixture was cooled to 0°C. This solution was admixed with 9-azanoradamantane N-oxyl (0.67 g, 4.88 mmol). The temperature was raised to 25°C, and the mixture was then stirred for 5 hours. After the completion of the reaction was checked by HPLC, the reaction liquid was cooled to 0°C. Ethyl acetate (600 mL) and 10% aqueous sodium sulfite solution (250 mL) were then added for liquid separation. The resulting organic layer was washed with 2 N hydrochloric acid (150 mL) and then saturated brine (100 mL), and concentrated under reduced pressure until the liquid volume reached 150 mL. The internal temperature was adjusted to 50°C, and 2-propanol (1 L) was added dropwise over 1 hour. The suspension was then cooled to 0°C and stirred for 3 hours. The precipitated crystals were filtered. The crystals were then washed with a mixed solvent of 2-propanol (200 mL)/ethyl acetate (50 mL) cooled to 0°C. The resulting crystals were dried under reduced pressure at 50°C to give 4-methoxyphenyl 3,6-di-O-benzyl-4-O-{4,6-O-benzyliden-3-O-[(naphthalen-2-yl)methyl]hexopyranosyl-2-ulose}-2-deoxy-2-(1,3-dioxo-1,3-dihydro-2H-isoindol-2-yl)-β-D-glucopyranoside (compound III-7) (41.61 g, yield 86.6%, HPLC area: 99.20%).

¹H-NMR (500 MHz, CDCl₃) δ 7.86 (br, 1H), 7.80-7.83 (m, 3H), 7.65-7.67 (m, 4H), 7.40-7.52 (m, 8H), 7.18-7.23 (m, 4H), 7.10-7.14 (m, 1H), 7.00-7.02 (m, 2H), 6.89-6.92 (m, 2H), 6.83-6.87 (m, 1H), 6.80 (ddd, J = 9.0, 4.0, 2.0 Hz, 2H), 6.70 (ddd, J = 9.0, 4.0, 2.5 Hz, 2H), 5.63 (d, J = 8.5 Hz, 1H), 5.49 (s, 1H), 5.06 (d, J = 13.0 Hz, 1H), 4.95 (d, J = 0.5 Hz, 1H), 4.85 (d, J = 12.5 Hz, 1H), 4.76 (d, J = 12.5 Hz, 1H), 4.68 (d, J = 12.0 Hz, 1H), 4.47 (dd, J = 10.5, 8.5 Hz, 1H), 4.40-4.42 (m, 1H), 4.39 (t, J = 4.5 Hz, 1H), 4.31 (d, J = 12.0 Hz, 1H), 4.25 (dd, J = 10.5, 5.0 Hz, 1H), 4.17 (t, J = 9.0 Hz, 1H), 3.99 (d, J = 11.0 Hz, 1H), 3.89 (dd, J = 11.5, 3.5 Hz, 1H), 3.78-3.86 (m, 3H), 3.71 (s, 3H), 3.57 (t, J = 10.0 Hz, 1H), 3.45 (td, J = 10.0, 5.0 Hz, 1H). ¹³C-NMR (125 MHz, CDCl₃) δ 197.2, 155.7, 151.0, 138.4, 138.0, 137.1, 135.0, 134.0, 133.5, 133.3131.8, 129.4, 128.7, 128.5, 128.4, 128.20, 128.18, 128.1, 128.0, 127.90, 127.87, 127.4, 126.7, 126.4, 126.3, 126.2, 125.8, 123.6, 119.0, 114.6, 102.2, 101.4, 98.0, 82.28, 82.25, 80.8, 77.3, 75.2, 74.7, 73.6, 73.4, 68.7, 68.3, 66.3, 56.0, 55.8.
HRMS(ESI⁻)[M+HCO₂]⁻ calcd for C₆₀H₅₄NO₁₅: 1028.3499; found 1028.3531.

### Example 19:

### 4-Methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (Compound III-8)

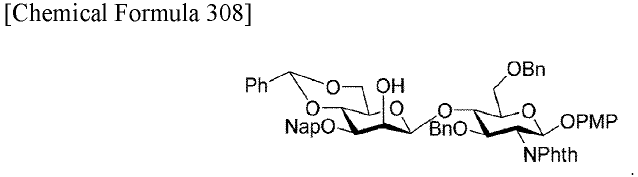

### (Step 1-14)

### (Condition 1: L-selectride)

A tetrahydrofuran solution (22.5 mL) containing compound III-7 (1.50 g, 1.52 mmol) was cooled to -50°C. After stirring for 50 min, an L-selectride-containing tetrahydrofuran solution (1.0 M, 1.68 mL, 1.68 mmol) was added over 5 minutes. After stirring for 1 hour, acetic acid (0.18 mL, 3.05 mmol) was added and the temperature was raised to room temperature (compound III-7: 2.41%PA and compound III-8: 76%PA@220 nm; dr = 96.5:3.5). Ethyl acetate (15 mL) was added to the resulting reaction liquid, 5% citric acid water (6 mL) was added, and the mixture was then stirred for 10 minutes. After heptane (4 mL) was added, the aqueous layer was removed and the resulting organic layer was washed with water (3 mL). The organic layer obtained was concentrated to dryness under reduced pressure. The residue was purified by silica gel chromatography (ethyl acetate/hexane: 20% -> 55%) to give the compound III-8 (1.15 g, yield 77%).

### (Condition 2: LS-selectride)

To a tetrahydrofuran solution (50 mL) containing 4-methoxyphenyl 3,6-di-O-benzyl-4-O-{4,6-O-benzyliden-3-O-[(naphthalen-2-yl)methyl]hexopyranosyl-2-ulose}-2-deoxy-2-(1,3-dioxo-1,3-dihydro-2H-isoindol-2-yl)-β-D-glucopyranoside (compound III-7) (5.00 g, 5.08 mmol) was added Molecular Sieves 4A (500 mg), and the mixture was then cooled to -45°C. The solution was stirred for 15 minutes. An LS-selectride-containing tetrahydrofuran solution (1.0 M, 5.59 mL, 5.59 mmol) was then added dropwise over 20 minutes. After stirring for 2 hours, the completion of the reaction was checked by HPLC. Acetic acid (0.38 mL, 6.61 mmol) was added and the temperature was raised to 25°C (compound III-8: 87%PA@220 nm; dr = 98.1:1.9). The Molecular Sieves 4A was filtered and washed with tetrahydrofuran (10 mL). Ethyl acetate (60 mL) was added to the resulting solution, 5% aqueous citric acid solution (20 mL) was added, and the mixture was then stirred for 10 minutes. The aqueous layer was removed. The resulting organic layer was washed with water (10 mL), 5% aqueous sodium bicarbonate solution (20 mL), and water (15 mL). The organic layer obtained was concentrated to dryness under reduced pressure, and then purified by silica gel column chromatography (ethyl acetate/hexane 20% -> 55%) to give 4-methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-8) (4.07 g, yield 81%).

### (Condition 3: lithium diisobutyl-tert-butoxyaluminum hydride: LDBBA)

To a tetrahydrofuran suspension (0.45 mL) containing compound III-7 (30.0 mg, 30.5 µmol) and Molecular Sieves 4A (3 mg, 0.1 wt) was added at -50°C a LDBBA-containing tetrahydrofuran/hexane solution (0. 25 M, 134.2 µL, 33.6 µmol). After stirring for 1 hour, the reaction liquid was analyzed by HPLC to confirm that the reaction was in progress (compound III-7: 5.7%PA and compound III-8: 83.49%PA@220 nm; dr = 93.6:6.4).

### (Condition 4: compound represented by formula A, where three R₃ moieties = di-tert-butylmethylphenoxide, hereafter referred to as "reagent A")

To a tetrahydrofuran suspension (2 mL) containing lithium aluminum hydride (50.0 mg, 1.32 mmol) was added at 0°C dibutylhydroxytoluene (885.41 mg, 4.02 mmol). The mixture was then stirred at 25°C to prepare reagent A represented by the following formula: 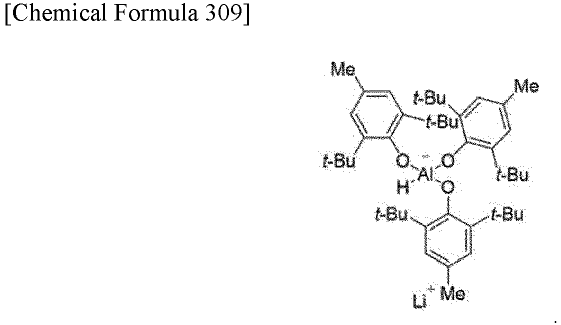 This reagent A-containing solution (80 µL) thus prepared was added to a tetrahydrofuran suspension (0.45 mL) containing 4-methoxyphenyl 3,6-di-O-benzyl-4-O-{4,6-O-benzyliden-3-O-[(naphthalen-2-yl)methyl]hexopyranosyl-2-ulose}-2-deoxy-2-(1,3-dioxo-1,3-dihydro-2H-isoindol-2-yl)-β-D-glucopyranoside (compound III-7) (30.0 mg, 30.5 µmοl) and Molecular Sieves 4A (3.0 mg) at 0°C, and the mixture was stirred for 1 hour. This reaction liquid was analyzed by HPLC to confirm that the reaction was in progress (compound III-7: undetected and compound III-8: 92.3%PA@220 nm (BHT was excluded); dr = 97.8:2.2).

### (Condition 5: diisobutylaluminum hydride: DIBALH)

To a tetrahydrofuran suspension (0.75 mL) containing compound III-7 (50.0 mg, 50.8 µmol) and Molecular Sieves 4A (5 mg, 0.1 wt) was added at -80°C a DIBALH-containing toluene solution (1.5 M, 37 µL, 37 µL, 55.5 µmol). The mixture was stirred overnight, and then stirred at -60°C for 90 minutes and -40°C for 30 minutes to confirm using HPLC that the reaction was in progress (compound III-7: N.D. and compound III-8: 40.92%PA@220 nm (toluene was excluded); dr = 41.9:58.1).

¹H-NMR (500 MHz, CDCl₃) δ 7.79-7.84 (m, 4H), 7.67-7.74 (m, 4H), 7.45-7.50 (m, 5H), 7.37-7.41 (m3H), 7.24-7.28 (m, 4H), 7.17-7.21 (m, 1H), 7.03-7.04 (m, 2H), 6.85-6.93 (m, 3H), 6.81 (ddd, J = 9.0, 4.0, 2.0 Hz, 2H), 6.69 (ddd, J = 9.0, 4.0, 2.0 Hz, 2H), 5.61 (d, J = 8.5 Hz, 1H), 5.53 (s, 1H), 4.92 (d, J = 13.0 Hz, 1H), 4.87 (d, J = 6.5 Hz, 1H), 4.84 (d, J = 6.0 Hz, 1H), 4.65-4.68 (m, 2H), 4.48 (d, J = 12.0 Hz, 1H), 4.39-4.46 (m, 3H), 4.14-4.20 (m, 2H), 4.03-4.07 (m, 2H), 3.79 (dd, J = 11.0, 3.5 Hz, 1H), 3.70-3.76 (m, 2H), 3.70 (s, 3H), 3.61 (t, J = 10.0 Hz, 1H), 3.51 (dd, J = 10.0, 3.5 Hz, 1H), 3.16 (td, J = 10.0, 5.0 Hz, 1H), 2.61 (d, J = 1.5 Hz, 1H).
¹³C-NMR (125 MHz, CDCl₃) δ 155.6, 151.0, 138.6, 137.9, 137.7, 135.6, 134.0, 133.5, 133.3, 131.8, 129.2, 128.7, 128.4, 128.2, 128.12, 128.09, 128.04, 127.92, 127.89, 127.4, 126.8, 126.4, 126.3, 126.2, 125.9, 123.6, 119.0, 114.6, 101.8, 101.0, 98.0, 78.9, 78.4, 77.7, 77.0, 74.94, 74.91, 73.8, 72.6, 69.9, 68.7, 68.6, 67.0, 55.9, 55.8.
HRMS(ESI⁻)[M+HCO₂]⁻ calcd for C₆₀H₅₆NO₁₅: 1030.3655; found 1030.3695.

### <Synthesis of compound III-10>

Compound III-10 was synthesized according to the following synthesis scheme 1C.

### [Synthesis Scheme 1C]

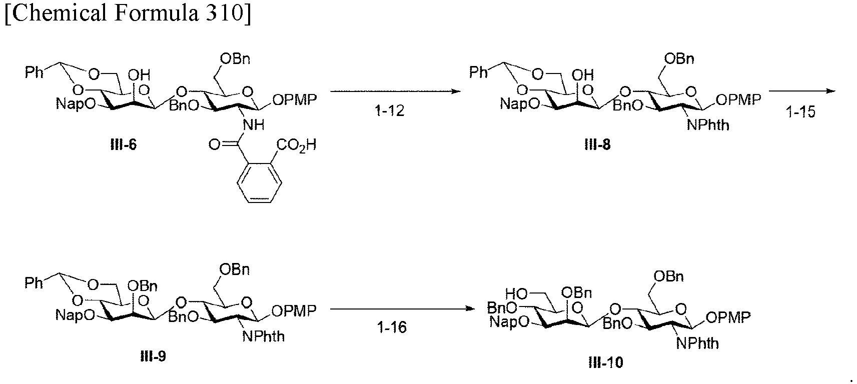

### Example 20:

### 4-Methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (Compound III-8)

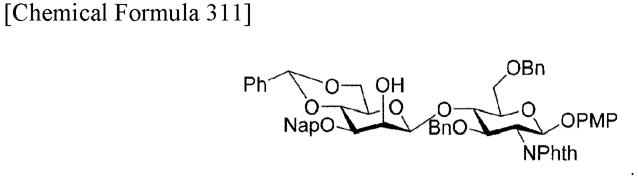

### (Substep 1-12)

To a dichloromethane (30 mL) solution containing 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (compound III-6) (6.00 g, 5.98 mmol) were added 1-hydroxybenzotriazole monohydrate (0.18 g, 1.20 mmol), N,N-diisopropylethylamine (0.85 g, 6.57 mmol), and 1-(3-dimethylaminopropyl)-3-ethylcarbodiimide hydrochloride (1.26 g, 6.57 mmol). The temperature was raised to 40°C, and the mixture was stirred for 31 hours. After the completion of the reaction was checked by HPLC, the reaction solution was cooled to 0°C. Ethyl acetate (90 mL) and water (60 mL) were then added, and under strong stirring, 6 N hydrochloric acid was added until pH 7 was reached. An organic layer and an aqueous layer were separated. The resulting organic layer was washed with water (60 mL) and then saturated brine (30 mL). The solution was concentrated under reduced pressure until the liquid volume reached 12 mL. Tetrahydrofuran (60 mL) was added, and the mixture was concentrated again until the liquid volume reached 9 mL to prepare a tetrahydrofuran solution containing crude 4-methoxyphenyl 3,6-di-O-benzyl-4-O- { 4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-8). This compound was used as it was in the next step.

### Example 21:

### 4-Methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (Compound III-9)

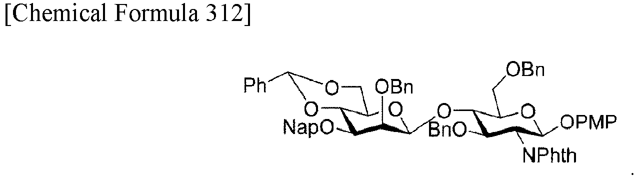

### (Substep 1-15)

To a solution containing the crude 4-methoxyphenyl 3,6-di-O-benzyl-4-O-{4,6-O-benzyliden-3-O-[(naphthalen-2-yl)methyl]-β-D-mannnopyranosyl}-2-deoxy-2-(1,3-dioxo-1,3-dihydro-2H-isoindol-2-yl)-β-D-glucopyranoside (compound III-8) were added N,N-dimethylacetamide (60 mL), benzyl bromide (1.53 g, 8.96 mmol), methyl trifluoroacetate (0.15 g, 1.20 mmol), and Molecular Sieves 4A (1.8 g). The mixture was cooled to 0°C. A lithium tert-butoxide-containing tetrahydrofuran solution (a mixed solution of 2-methyl-2-propanol (0.66 g, 8.96 mmol) and tetrahydrofuran (2.4 mL) was cooled to 0°C, a hexane solution containing normal butyl lithium (1.55 mol/L) (5.78 mL, 8.96 mmol) was added, and the mixture was stirred for 30 minutes and then used) was added, and the mixture was stirred for 3 hours. After the completion of the reaction was checked by HPLC, ethylenediamine (anhydrous) (0.18 g, 2.99 mmol) was added. The mixture was stirred for another 1 hour. Acetic acid (0.72 g. 11.95 mmol) was added to this solution, and the mixture was filtered. After the Molecular Sieves 4A was washed with ethyl acetate (90 mL), water (60 mL) was added for liquid separation. The organic layer was washed twice with water (60 mL) and then concentrated under reduced pressure until the liquid volume reached 12 mL. Toluene (30 mL) was added, and the mixture was concentrated again until the liquid volume reached 12 mL. Toluene (18 mL) and silica gel 60N (spherical) (9 g) were then added, and the mixture was stirred at 25°C for 30 minutes. The suspension was filtered, and the silica gel was washed with a mixed solution of toluene (191 mL) and ethyl acetate (19 mL). The filtrate was concentrated under reduced pressure until the liquid volume reached 9 mL to prepare a toluene solution containing crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-9).

### Example 22:

### 4-Methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (Compound III-10)

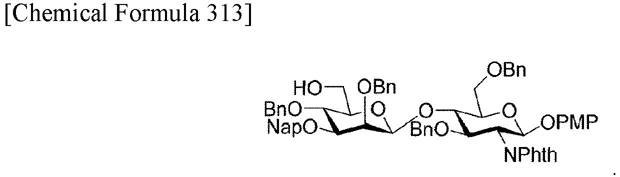

### (Substep 1-16)

To a solution containing the crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (compound III-9) obtained in Example 21 (5.50 g (5.48 mmol) equivalent in terms of compound III-8 was used) were added dichloromethane (16.5 mL) and Molecular Sieves 4A (550 mg), and the mixture was cooled to 0°C. A borane-tetrahydrofuran complex (0.91 mol/L tetrahydrofuran solution) (18.06 mL, 16.43 mmol) and copper(II) trifluoromethanesulfonate (0.59 g, 1.64 mmol) were added, and the mixture was then stirred for 3 hours. After the completion of the reaction was checked by HPLC, methanol (5.5 mL) was added and the mixture was stirred for another 30 minutes. The solution is filtered. After the Molecular Sieves 4A was washed with ethyl acetate (110 mL), 0.5 N hydrochloric acid (55 mL) was added, and the mixture was then stirred for 30 minutes. An organic layer and an aqueous layer were separated. The organic layer was washed with 0.5 N hydrochloric acid (55 mL) and saturated brine (27.5 mL), and the resulting solution was then concentrated to dryness under reduced pressure. The resulting material was purified by silica gel column chromatography (silica gel: 300 g; hexane:ethyl acetate = 55:45 -> 30:70) to give 4-methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (compound III-10) (4.94 g, yield 83.6%, HPLC area: 98.54%).

¹H-NMR (500 MHz, CDCl₃) δ 7.67-7.84 (m, 8H), 7.44-7.49 (m, 4H), 7.40 (dd, J = 8.0, 1.5 Hz, 1H), 7.21-7.33 (m, 13H), 6.87-6.93 (m, 5H), 6.82 (ddd, J = 9.5, 4.0, 2.5 Hz, 2H), 6.70 (ddd, J = 9.0, 4.0, 2.0 Hz, 2H), 5.64 (d, J = 8.5 Hz, 1H), 4.94 (d, J = 12.5 Hz, 1H), 4.91 (d, J = 10.0 Hz, 1H), 4.90 (s, 2H), 4.66 (s, 2H), 4.60 (d, J = 11.0 Hz, 1H), 4.59 (d, J = 12.0 Hz, 1H), 4.55 (s, 1H), 4.40-4.46 (m, 3H), 4.33 (dd, J = 11.0, 9.0 Hz, 1H), 4.06 (dd, J = 9.5, 8.5 Hz, 1H), 3.80-3.85 (m, 2H), 3.70-3.76 (m, 2H), 3.71 (s, 3H), 3.61-3.68 (m, 2H), 3.45-3.48 (m, 1H), 3.44 (dd, J = 9.5, 3.0 Hz, 1H), 3.23 (ddd, J = 9.5, 5.5, 2.5 Hz, 1H), 1.97 (br-t, 1H).
¹³C-NMR (125 MHz, CDCl₃) δ 155.6, 151.1, 138.9, 138.64, 138.56, 138.0, 135.9, 134.0, 133.5, 133.2, 131.8, 128.7, 128.6, 128.4, 128.3, 128.20, 128.19, 128.15, 128.1, 120.04, 128.01, 127.9, 127.7, 127.6, 127.3, 126.4, 126.3, 126.1, 125.8, 123.6, 119.0, 114.6, 101.2, 98.0, 82.6, 79.0, 77.2, 75.9, 75.4, 75.3, 75.2, 75.1, 74.8, 74.7, 73.8, 72.1, 68.7, 62.6, 55.82, 55.78, 34.4, 30.5.
HRMS(ESI⁻)[M+HCO₂]⁻ calcd for C₆₇H₆₄NO₁₅: 1122.4281; found 1122.4285.

### <Synthesis of compound V-3>

Compound V-3 was synthesized according to the following synthesis scheme 2A.

### [Synthesis Scheme 2A]

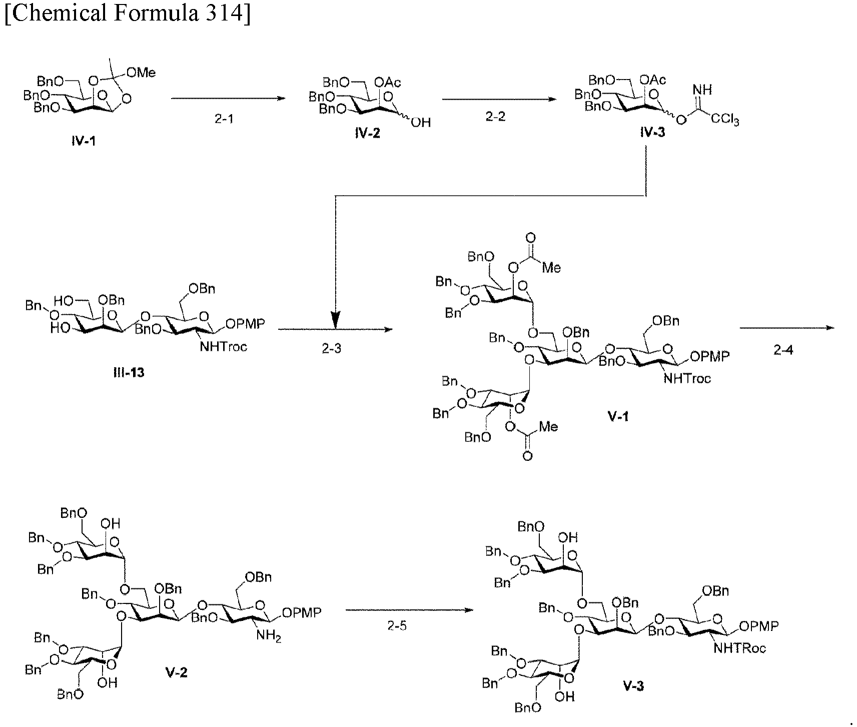

### Example 23:

### 2-O-Acetyl-3,4,6-tri-O-benzyl-1-O-(2,2,2-trichloroethanimidoyl)-D-mannopyranose (Compound IV-3)

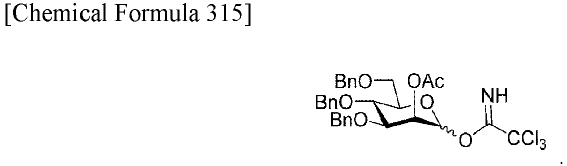

### (Substeps 2-1 and 2-2)

To a dichloromethane solution (30 mL) containing 3,4,6-tri-O-benzyl-1,2-O-(1-methoxyethyliden)-β-D-mannopyranose (compound IV-1) (3.00 g, 5.92 mmol) were added p-toluenesulfonic acid monohydrate (5.63 mg, 29.6 µmοl) and water (0.3 mL) at room temperature, and the mixture was stirred at the same temperature for 90 minutes. After the reaction, triethylamine (0.30 g, 2.96 mmol) was added. After stirring at room temperature for 2 hours, toluene (60 mL) was added to the concentrated and dried crude material to give 2-O-acetyl-3,4,6-tri- O-benzyl-D-mannopyranose (compound IV-2) as a toluene solution. To a toluene (15 mL)/2,2,2-trichloroacetonitrile (4.27 g, 29.6 mmol) mixture containing the obtained compound IV-2 was added 1,8-diazabicyclo[5.4.0]-7-undecene (7.1 µL, 47 µmol) over 30 minutes at room temperature. After stirred at the same temperature for 1 hour, the reaction liquid was concentrated to dryness. The resulting crude material was purified by silica gel chromatography (Wakogel 9 g; ethyl acetate/hexane 20%) to give 2-O-acetyl-3,4,6-tri-O-benzyl-1-O-(2,2,2-trichloroethanimidoyl)-D-mannopyranose (compound IV-3) (3.75 g, yield 99%). The compound was used in the next step.

### Example 24:

### 4-Methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-{[(2,2,2-trichloroethoxy)carbonyl]amino}-β-D-glucopyranoside (Compound V-1)

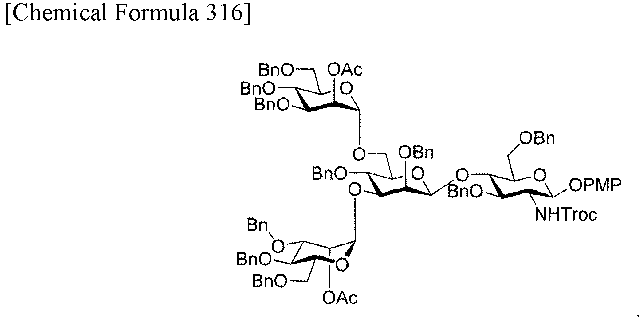

### (Substep 2-3)

Molecular Sieves 4A (10 mg, 0.2 wt) was added to a dichloromethane solution (0.8 mL) containing 4-methoxyphenyl 3,6-di-O-benzyl-2-deoxy-4-O-(2,4-di-O-benzyl-β-D-mannopyranosyl)2-{[(2,2,2-trichloroethoxy)carbonyl]amino}-β-D-glucopyranoside (compound III-13) (50 mg, 0.02 mmol) and compound IV-3 (113.4 mg, 0.18 mmol). The mixture was then cooled to -20°C. After stirring for 15 min, a dichloromethane solution containing trimethylsilyl trifluoromethanesulfonate (0.17 M, 30 µL, 5.0 µmol) was added. After stirring for 90 minutes, triethylamine (5 µL, 0.04 mmol) was added and the temperature was raised to room temperature (reaction solution 1).

To a dichloromethane solution (21 mL) containing compound III-13 (650 mg, 0.71 mmol) and compound IV-3 (1.59 g, 2.49 mmol) was added Molecular Sieves 4A (140 mg, 0.2 wt). The mixture was then cooled to -20°C. After stirring for 20 min, a dichloromethane solution containing trimethylsilyl trifluoromethanesulfonate (0.28 M, 239 µL, 0.07 mmol) was added. After 3 hours, trimethylsilyl trifluoromethanesulfonate (3 µL, 0.02 mmol) was added. After stirring for 45 minutes, triethylamine (30 µL, 0.21 mmol) was added and the temperature was raised to room temperature (reaction solution 2).

After confirming the progress of the reaction in both reaction solutions 1 and 2, both reaction suspensions were mixed, and the Molecular Sieves was filtered off and washed with dichloromethane (4 mL). The resulting solution was concentrated to dryness, and the resulting crude material (2.71 g) was purified by silica gel chromatography (ethyl acetate/hexane 20% -> 55% and ethyl acetate/dichloromethane 0% -> 20%) to afford compound V-1 (1.16 g, yield 85%).

¹H-NMR (500 MHz, CDCl₃) δ 2.07 (s, 3H), 2.08 (s, 3H), 3.01 (d, J = 12 Hz, 1H), 3.27-3.35 (m, 2H), 3.45-3.54 (m, 2H), 3.54-3.94 (m, 14H), 3.73 (s, 3H), 4.00 (t, J = 9.5 Hz, 1H), 4.09 (t, J = 8.5 Hz, 1H), 4.26 (d, J = 12 Hz, 1H), 4.32 (d, J = 11 Hz, 1H), 4.35-4.64 (m, 15H), 4.68 (d, J = 7.5 Hz, 1H), 4.71-4.80 (m, 3H), 4.81-4.85 (m, 2H), 4.93 (d, J = 8.5 Hz, 1H), 4.95 (d, J = 10 Hz, 1H), 5.14 (d, J = 8.0 Hz, 1H), 5.17 (brs, 1H), 5.35 (brs, 1H), 5.47 (dd, J = 2.0, 3.0 Hz, 1H), 6.76 (d, J = 9.0 Hz, 2H), 6.92 (d, J = 9.0 Hz, 2H), 7.06-7.38 (m, 50H).
¹³C-NMR (125 MHz, CDCl₃) δ 20.9, 21.0, 66.3, 68.3, 68.57, 68.63, 68.90, 68.92, 71.3, 71.8, 72.2, 73.2, 73.9, 74.1, 74.3, 74.5, 74.7, 74.8, 74.9, 75.3, 77.5, 77.9, 78.0, 78.5, 80.6, 95.6, 97.3, 99.2, 99.6, 99.9, 114.3, 118.5, 127.22, 127.27, 127.31, 127.38, 127.41, 127.45, 127.55, 127.59, 127.64, 127.66, 127.7, 127.8, 128.0, 128.13, 128.16, 128.19, 128.23, 128.29, 128.35, 128.44, 137.6, 137.7, 137.8, 137.96, 137.99, 138.3, 138.40, 138.44, 138.6, 138.9, 151.5, 153.5, 155.1, 170.0, 170.4.
MS(ESI)(m/z): 1949([M+NH₄]⁺).

### Example 25:

### 4-Methoxyphenyl 3,4,6-tri-O-benzyl-α-D-mannopyranosyl-(1→3)-[3,4,6-triO-benzyl-α-D-mannopyranosyl-(1→6)]-2,4-di-O-benzyl-β-D-mannopyranosyl-(1→4)-3,6-di-O-benzyl-2-deoxy-{[(2,2,2-trichloroethoxy)carbonyl]amino}-β-D-glucopyranoside (Compound V-3)

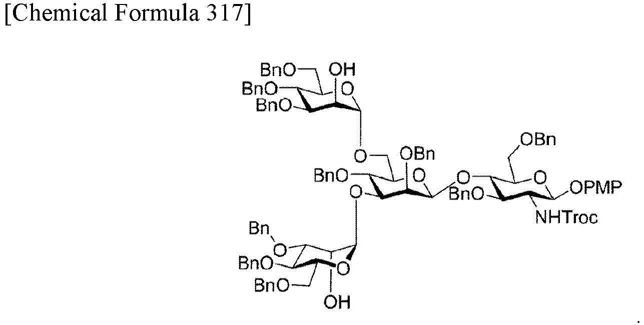

### (Substeps 2-4 and 2-5)

To a tetrahydrofuran (0.4 mL) solution containing compound V-1 (50 mg, 0.03 mmol) were added water (0.05 mL) and 50% aqueous sodium hydroxide solution (16.6 mg, 0.21 mmol), followed by stirring at 50°C for 27 hours. Next, methanol (0.05 mL) was added, and the mixture was stirred at 50°C for 18 hours. Then, the mixture was cooled to room temperature, and acetic acid (8.9 µL, 0.16 mmol) was added to give a solution containing 4-methoxyphenyl 3,4,6-tri-O-benzyl-α-D-mannopyranosyl-(1→3)-[3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranoside (compound V-2). The resulting solution was concentrated to dryness, and tetrahydrofuran (0.4 mL), water (0.05 mL), sodium hydrogen carbonate (8.7 mg, 0.10 mmol), and 2,2,2-trichloroethyl chloroformate (6.6 mg, 0.03 mmol) were then added. After stirring at room temperature for 1 hour, water (2 mL) and ethyl acetate (5 mL) were added to the resulting suspension. The aqueous layer was removed, and the concentrated and dried crude material was then purified by silica gel chromatography (ethyl acetate/hexane 20% -> 55% and ethyl acetate/dichloromethane 0% -> 20%) to afford compound V-3 (43.2 mg, yield 90%). Compound V-3 (821.6 mg, yield 82%) was obtained from compound V-1 (1.05 g, 0.54 mmol) by a similar procedure.

MS(ESI)(m/z): 1864([M+NH₄]⁺).

### <Synthesis of compound V-5>

Compound V-5 was synthesized according to the following synthesis scheme 2B.

### [Synthesis Scheme 2B]

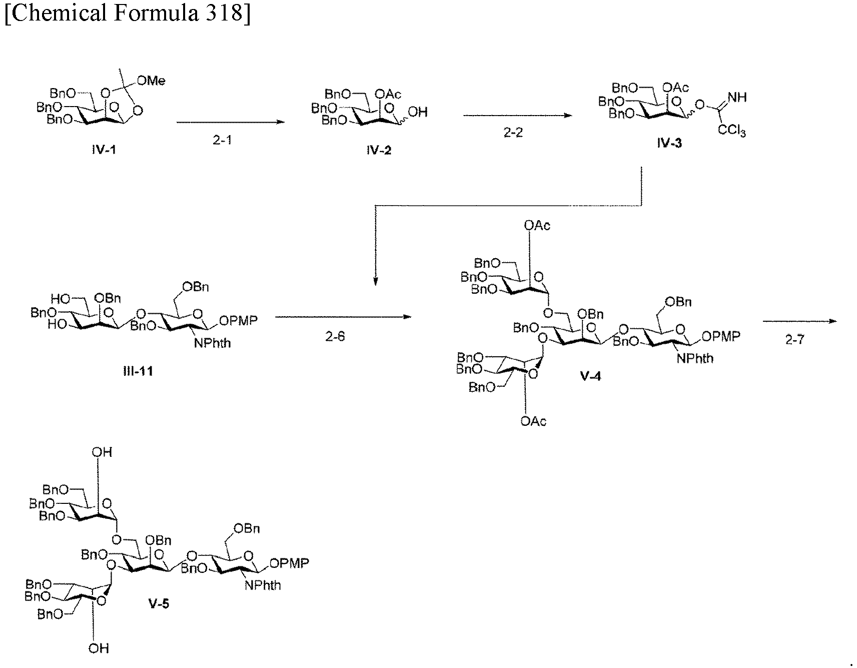

### Example 26:

### 2-O-Acetyl-3,4,6-tri-O-benzyl-D-mannopyranose (Compound IV-2)

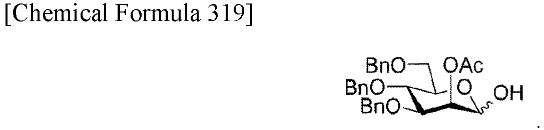

### (Substep 2-1)

First, 3,4,6-tri-O-benzyl-1,2-O-(1-methoxyethyliden)-β-D-mannopyranose (compound IV-1) (30.0 g, 59.2 mmol) was added to a 1-L recovery flask, and dichloromethane (300 mL) was added. Toluenesulfonic acid monohydrate (23.0 mg, 0.118 mmol) and water (3.0 mL) were added at room temperature under nitrogen, and the mixture was stirred at the same temperature for 3.5 hours. After the completion of the reaction was checked by HPLC, triethylamine (4.13 mL, 29.6 mmol) was added. The mixture was stirred at the same temperature for 1.5 hours. After the completion of the Ac-group transfer reaction was checked by HPLC, ethyl acetate (450 mL) was added to the reaction liquid. The mixture was liquid-separated using 5% sodium bicarbonate water (300 mL). Then, 20% brine (150 mL) was added to the organic layer for liquid-separation. The resulting organic layer was concentrated under reduced pressure until the liquid volume reached 60 mL, and toluene (450 mL) was added until the liquid volume reached 60 mL. Dehydrated dichloromethane (150 mL) was added to prepare, as a colorless solution, a toluene/dichloromethane solution containing 2-O-acetyl-3,4,6-tri- O-benzyl-D-mannopyranose (compound IV-2). This solution was used as it was in the next step.

### Example 27:

### 2-O-Acetyl-3,4,6-tri-O-benzyl-1-O-(2,2,2-trichloroethanimidoyl)-D-mannopyranose (Compound IV-3)

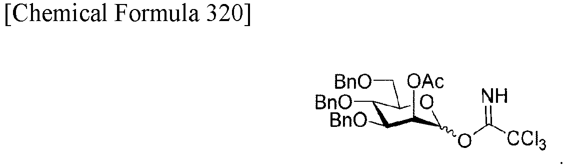

### (Substep 2-2)

A toluene/dichloromethane solution containing 2-O-acetyl-3,4,6-tri-O-benzyl-D-mannopyranose (compound IV-2) (59.2 mmol) was added to a 1-L recovery flask, and trichloroacetonitrile (9.03 mL, 89.0 mmol) and 1,8-diazabicyclo[5.4.0] undeca-7-ene (89.0 µL, 0.592 mmol) were added. The mixture was stirred under nitrogen at 0°C for 3 hours. Then, 1,8-diazabicyclo[5.4.0]undeca-7-ene (44.5 µL, 0.296 mmol) was added, and the mixture was stirred at 0°C for 2.5 hours. Further, 1,8-diazabicyclo[5.4.0]undeca-7-ene (44.5 µL, 0.296 mmol) was added, and the mixture was stirred at 0°C for 1 hour. The reaction liquid was then kept refrigerated overnight. After the completion of the reaction was checked by HPLC, acetic acid (68 µL, 1.18 mmol) was added to the reaction liquid at 0°C to prepare, as a brown solution, a dichloromethane solution containing 2-O-acetyl-3,4,6-tri-O-benzyl-1-O-(2,2,2-trichloroethanimidoyl)-D-mannopyranose (compound IV-3) (59.2 mmol). This solution was used as it was in the next step.

### Example 28:

### 4-Methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (Compound V-4)

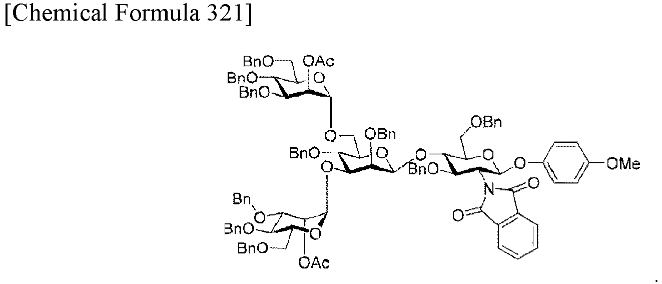

### (Substep 2-6)

A dichloromethane solution containing 2-O-acetyl-3,4,6-tri-O-benzyl-1-O-(2,2,2-trichloroethanimidoyl)-D-mannopyranose (compound IV-3) (32.5 mmol) and 4-methoxyphenyl 3,6-di-O-benzyl-2-deoxy-4-O-(2,4-di-O-benzyl-β-D-mannopyranosyl)-2-(1,3-dioxo-1,3-dihydro-2H-isoindol-2-yl)-β-D-glucopyranoside (compound III-11) (8.70 g, 9.27 mmol) was added to a 300-mL 3-neck flask. Dichloromethane (15 mL, total 131 mL as a reaction liquid) and Molecular Sieves 4A powder (1.31 g) were then added. Trimethylsilyl trifluoromethanesulfonate (0.335 mL, 1.86 mmol) was added dropwise over 10 minutes at -20°C under nitrogen, and the mixture was stirred at the same temperature for 3 hours. After the completion of the reaction was checked by HPLC, triethylamine (520 µL, 3.73 mmol) was added. The mixture was stirred at room temperature for 30 minutes. The reaction liquid was filtered through Celite and washed with dichloromethane (43.5 mL). The filtrate was concentrated, and the concentrated residue was purified by column chromatography (silica amount: 650 g; developing solvent: toluene/ethyl acetate = 1/0 to 4/1). The main residue was concentrated under reduced pressure to give 4-methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound V-4) (16.2 g, 93.0% isolation yield) as a white amorphous product.

¹H-NMR (500 MHz, CDCl₃) δ 7.63-6.60 (m, 58H), 5.54 (d, J = 8.4 Hz, 1H), 5.49 (dd, J = 3.2, 2.0 Hz, 1H), 5.34 (brt, J = 3.2 Hz, 1H), 5.13 (brd, J = 1.6 Hz, 1H), 5.05 (d, J = 12.0 Hz, 1H), 4.91-4.78 (m, 6H), 4.69 (d, J = 11.2 Hz, 1H), 4.63-4.55 (m, 6H), 4.48-4.32 (m, 9H), 4.21 (t, J = 11.2 Hz, 2H), 4.07 (t, J = 9.6 Hz, 1H), 4.00-3.73 (m, 9H), 3.70 (s, 3H), 3.68-3.51 (m, 8H), 3.44 (brd, J = 8.0 Hz, 1H), 3.21 (brd, J = 9.6 Hz, 1H), 2.10 (s, 3H), 1.86 (s, 3H). ¹³C-NMR (125 MHz, CDCl₃) δ 170.2, 170.0, 155.4, 151.0, 138.9, 138.7, 138.6, 138.5, 138.2, 138.1, 138.0, 137.9, 133.7, 131.7, 128.7, 128.5, 128.4, 128.3, 128.2, 127.9, 127.9, 127.8, 127.7, 127.6, 127.5, 127.5, 127.1, 123.4, 118.8, 114.4, 101.9, 99.8, 98.5, 97.7, 81.3, 79.5, 78.2, 78.0, 77.9, 76.9, 76.6, 75.2, 75.2, 75.1, 74.9, 74.7, 74.5, 74.4, 74.2, 74.2, 73.6, 73.5, 73.4, 72.4, 72.0, 71.9, 71.4, 69.4, 69.4, 68.8, 68.8, 68.3, 66.7, 55.7, 21.2, 20.9.
HRMS(ESI⁺)[M+NH₄]⁺ calcd for C₁₁₃H₁₁₉N₂O₂₅: 1903.8096; found 1903.8075.

### Example 29:

### 4-Methoxyphenyl 3,4,6-tri-O-benzyl-α-D-mannopyranosyl-(1→3)-[3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound V-5)

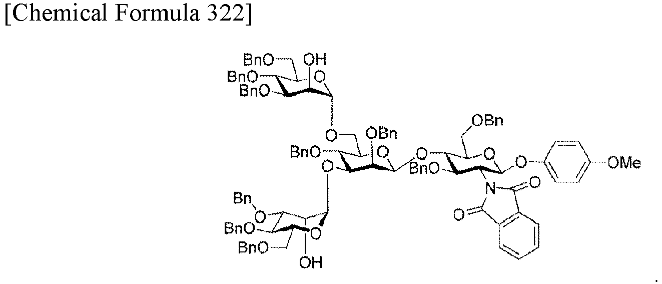

### (Substep 2-7)

To a 30-mL 2-neck flask was added 4-methoxyphenyl 2-O-acetyl-3,4,6-tri-O-benzyl-α-D-mannopyranosyl-(1→3)-[2-O-acetyl-3,4,6-tri-O-benzyl-α-D-mannopyranosyl-(1→6)]-2,4-di-O-benzyl-P-D-mannopyranosyl-(1→4)-3,6-di-O-benzyl-2-deoxy-(1,3-dioxo-1,3-dihydro-2H-isoindol-2-yl)-β-D-glucopyranoside (compound V-4) (1.0 g, 0.53 mmol). Tetrahydrofuran (5 mL), methanol (3 mL) and methyl trifluoroacetate (53 µL, 0.53 mmol) were then added. After stirring at room temperature under nitrogen for 15 minutes, 1 M tetrahydrofuran solution containing potassium tert-butoxide (0.265 mL, 0.265 mmol) was added dropwise. The mixture was stirred at 50°C for 1 hour. After the completion of the reaction was checked by HPLC, the mixture was cooled to room temperature. Acetic acid (30.3 µL, 0.53 mmol) was then added. After ethyl acetate (10 mL) was added, the mixture was liquid-separated three times with 5% brine (10 mL). The resulting organic layer was dried over sodium sulfate, filtered, and then concentrated. The concentrated residue was purified by column chromatography (silica amount: 20 g; developing solvent: toluene/ethyl acetate = 9/1 to 7/3). The main residue was concentrated under reduced pressure to give 4-methoxyphenyl 3,4,6-tri-O-benzyl-α-D-mannopyranosyl-(1→3)-[3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound V-5) (900 mg, 94% isolation yield) as a white amorphous product.

¹H-NMR (500 MHz, CDCl₃) δ 7.63 (brs, 2H), 7.42 (d, J = 7.2 Hz, 2H), 7.35-6.66 (m, 54H), 5.57 (d, J = 8.4 Hz, 1H), 5.14 (brd, J = 1.2 Hz, 1H), 5.03 (d, J = 12.0 Hz, 1H), 4.97 (brd, J = 1.2 Hz, 1H), 4.93 (d, J = 13.2 Hz, 1H), 4.86-4.75 (m, 3H), 4.64-4.33 (m, 17H), 4.21 (brt, J = 8.8 Hz, 1H), 4.09 (brt, J = 9.6 Hz, 1H), 3.97-3.51 (m, 22H), 3.43 (brd, J = 9.6 Hz, 1H), 3.22 (brd, J = 9.6 Hz, 1H), 2.34 (d, J = 2.4 Hz, 1H), 2.19 (d, J = 2.8 Hz, 1H).
¹³C-NMR (125 MHz, CDCl₃) δ 155.2, 150.8, 138.9, 138.6, 138.5, 138.3, 138.3, 138.0, 138.0, 137.8, 137.7, 133.6, 131.6, 128.5, 128.4, 128.4, 128.3, 128.2, 128.2, 128.2, 128.0, 127.9, 127.8, 127.8, 127.7, 127.7, 127.6, 127.5, 127.4, 127.4, 127.3, 127.3, 126.9, 123.2, 118.6, 114.2, 101.6, 101.4, 99.7, 97.6, 81.7, 80.0, 79.7, 79.0, 78.2, 76.7, 75.0, 75.0, 74.8, 74.7, 74.6, 74.3, 74.2, 74.2, 74.1, 74.1, 73.4, 73.3, 73.2, 72.0, 71.9, 71.4, 71.2, 69.4, 68.8, 68.5, 68.1, 67.7, 66.4, 55.5. HRMS(ESI⁺)[M+NH₄]⁺ calcd for C₁₀₉H₁₁₅N₂O₂₃: 1819.7885; found 1819.7886.

### <Synthesis of compound VI-3>

Compound VI-3 was synthesized according to the following synthesis scheme 3A.

### [Synthesis Scheme 3A]

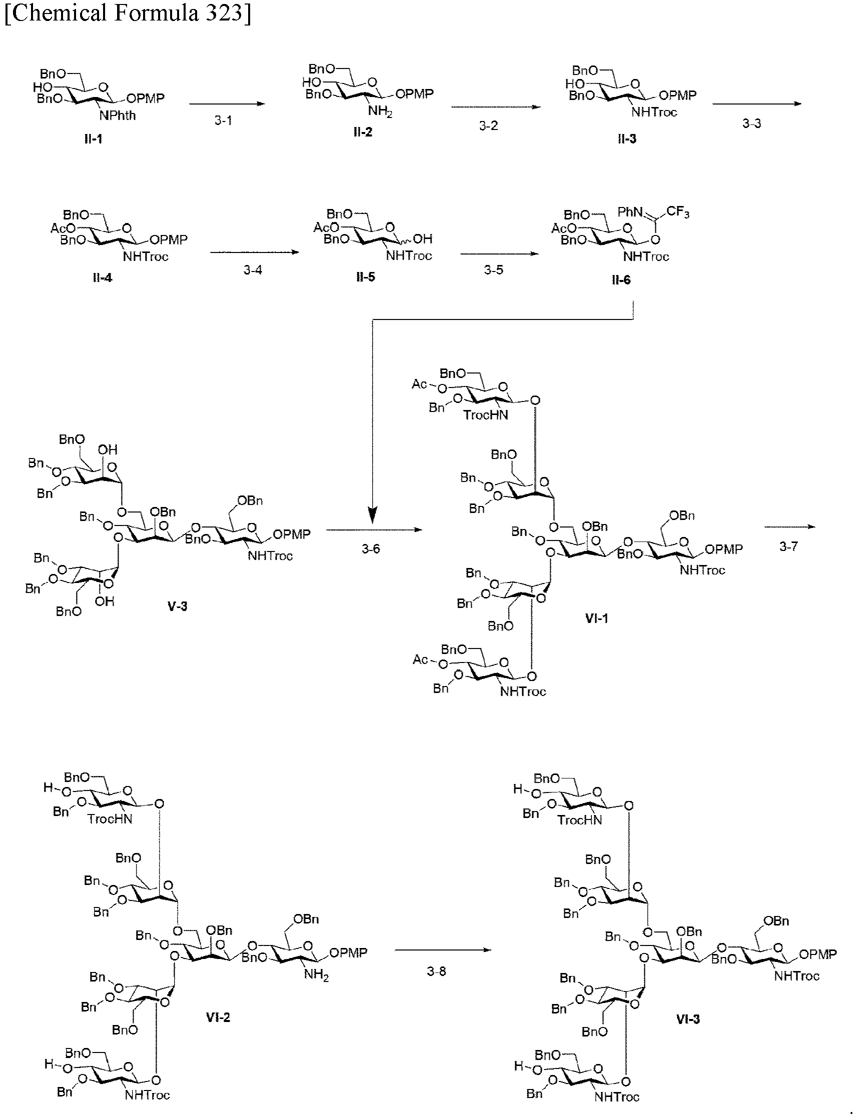

### Example 30:

### 4-Methoxyphenyl 2-amino-3,6-di-O-benzyl-2-deoxy-β-D-glucopyranoside (Compound II-2)

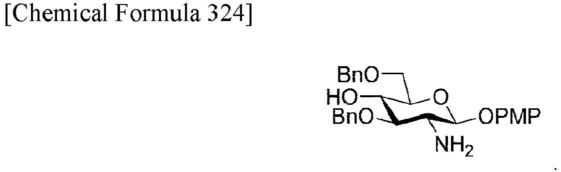

### (Substep 3-1)

To an n-butanol (80 mL) solution containing 4-methoxyphenyl 3,6-di-O-benzyl-2-deoxy-2-(1,3-dioxo-1,3-dihydro-2H-isoindol-2-yl)-β-D-glucopyranoside (compound II-1) (20.0 g, 33.58 mmol) was added ethylenediamine (10.09 g, 167.87 mmol). The mixture was heated to 90°C and stirred at the same temperature for 4 hours. After the completion of the reaction was checked by HPLC, the mixture was cooled to 30°C. Next, cyclopentyl methyl ether (300 mL) was poured, and the mixture was then stirred at the same temperature for 12 hours. The insoluble material derived from the phthaloyl group was filtered and washed with cyclopentyl methyl ether (60 mL). The resulting filtrate was washed three times with 25% methanol water (300 mL), and the organic layer was concentrated under reduced pressure to 40 mL. Isopropanol (400 mL) was added to the concentrated solution, and the mixture was concentrated under reduced pressure to 160 mL. Isopropanol (20 mL) and water (130 mL) were added to the concentrated solution, and the temperature was adjusted to 20°C. Seed crystals of compound II-2 were added. The mixture was stirred at the same temperature for 1 hour, and the crystals were found to precipitate. Water (230 mL) was then added dropwise over 1 hour. The resulting slurry solution was cooled to 0-5°C, and then stirred at the same temperature for 1 hour. The precipitated crystals were filtered. The filtered crystals were washed with a mixture of isopropanol and water (40/80 mL) at 0-5°C and dried under reduced pressure at 40°C to give 4-methoxyphenyl 2-amino-3,6-di-O-benzyl-2-deoxy-β-D-glucopyranoside (compound II-2) (14.4 g, yield 92%) as white crystals.

¹H-NMR (500 MHz, CDCl₃) δ 7.40-7.26 (m, 10H), 7.01-6.97 (m, 2H), 6.81-6.76 (m, 2H), 4.98 (d, J = 11.5 Hz, 1H), 4.79 (d, J = 12.0 Hz, 1H), 4.69 (d, J = 8.0 Hz, 1H), 4.56 (q, J= 11.5 Hz, 2H), 3.80-3.74 (m, 3H), 3.74 (s, 3H), 3.58-3.52 (m, 1H), 3.40 (t, J = 9.5 Hz, 1H), 3.10 (brs, 1H), 3.08 (dd, J = 9.5, 8.0 Hz, 1H), 1.63 (brs, 2H). ¹³C-NMR (125 MHz, CDCl₃) δ 155.3, 151.2, 138.5, 137.6, 128.6, 128.4, 127.9, 127.9, 127.8, 127.7, 118.4, 114.4, 103.1, 84.6, 74.9, 73.9, 73.7, 73.1, 70.8, 56.1, 55.6. HRMS(ESI⁺)[M+H]⁺ calcd for C₂₇H₃₂NO₆: 466.2224; found 466.2239.

### Example 31:

### 4-Methoxyphenyl 3,6-di-O-benzyl-2-deoxy-2-{[(2,2,2-trichloroethoxy)carbonyl]amino}-β-D-glucopyranoside (Compound II-3)

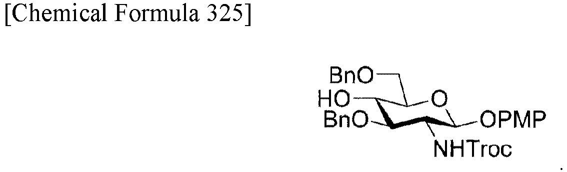

### (Substep 3-2)

To a tetrahydrofuran (100 mL) solution containing 4-methoxyphenyl 2-amino-3,6-di-O-benzyl-2-deoxy-β-D-glucopyranoside (compound II-2) (10.0 g, 21.48 mmol) was added water (50 mL) having dissolved sodium hydrogen carbonate (2.71 g, 32.26 mmol). Next, 2,2,2-trichloroethyl chloroformate (5.01 g, 23.65 mmol) was added dropwise at 25°C or below, and the mixture was stirred at the same temperature for 30 minutes. After the completion of the reaction was checked by HPLC, ethyl acetate (100 mL) and water (100 mL) were poured for liquid separation to obtain an organic layer. The organic layer obtained was washed again with water (100 mL) and further with 20% brine (50 mL). The resulting organic layer was concentrated under reduced pressure to 30 mL, and ethyl acetate (150 mL) was added. The mixture was concentrated under reduced pressure to 50 mL. Heptane (15 mL) was added to the concentrated solution, seed crystals of compound II-3 were added, and the mixture was stirred at 20°C for 12 hours. After the crystals were found to precipitate, heptane (150 mL) was added dropwise over 1 hour. The resulting slurry solution was cooled to 0-5°C, and then stirred at the same temperature for 1 hour. The precipitated crystals were then filtered. The filtered crystals were washed with a mixture of ethyl acetate and heptane (10/40 mL) at 0-5°C and dried under reduced pressure at 40°C to give 4-methoxyphenyl 3,6-di-O-benzyl-2-deoxy-2-{[(2,2,2-trichloroethoxy)carbonyl]amino}-β-D-glucopyranoside (compound II-3) (12.9 g, yield 94%) as white crystals.

¹H-NMR (500 MHz, CDCl₃) δ 7.35-7.26 (m, 10H), 6.95-6.91 (m, 2H), 6.76-6.72 (m, 2H), 5.35 (d, J = 8.0 Hz, 1H), 5.07 (d, J = 6.9 Hz, 1H), 4.78 (dd, J = 15.5, 11.5 Hz, 2H), 4.69 (brs, 2H), 4.55 (q, J = 11.5 Hz, 2H), 3.83 (brs, 1H), 3.79-3.71 (m, 3H), 3.72 (s, 3H), 3.58 (brs, 2H), 2.88 (d, J = 2.8 Hz, 1H). ¹³C-NMR (125 MHz, CDCl₃) δ 155.3, 154.1, 138.1, 137.6, 128.5, 128.4, 128.1, 127.9, 127.8, 127.7, 118.5, 114.4, 99.7, 95.4, 80.2, 74.3, 74.3, 73.9, 73.6, 72.7, 70.3, 57.2, 55.5.
HRMS(ESI⁻)[M-H]⁻ calcd for C₃₀H₃₁Cl₃NO₈: 638.1121; found 638.1099.

### Example 32:

### 4-Methoxyphenyl 4-O-acetyl-3,6-di-O-benzyl-2-deoxy-2-{[(2,2,2-trichloroethoxy)carbonyl]amino}-β-D-glucopyranoside (Compound II-4)

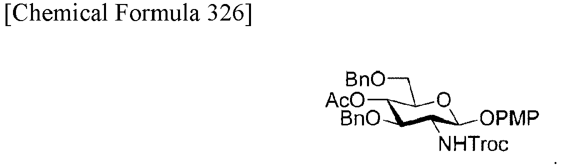

### (Substep 3-3)

To a pyridine (40 mL) solution containing 4-methoxyphenyl 3,6-di-O-benzyl-2-deoxy-2-{[(2,2,2-trichloroethoxy)carbonyl]amino}-β-D-glucopyranoside (compound II-3) (10.0 g, 15.60 mmol) were added dimethylaminopyridine (0.30 g, 2.46 mmol) and acetic anhydride (2.41 g, 23.61 mmol), and the mixture was stirred at 20°C for 4 hours. After the completion of the reaction was checked by HPLC, the mixture was heated to 50°C. Ethanol (40 mL) was added dropwise and the mixture was stirred at the same temperature for 30 minutes. After the crystals were found to precipitate, ethanol (60 mL) was added dropwise at 50°C over 30 minutes. The mixture was then stirred at the same temperature for 1 hour. The resulting slurry solution was cooled to 25°C and water (100 mL) was added dropwise over 1 hour. The slurry solution was cooled to 0-5°C, and then stirred at the same temperature for 1 hour. The precipitated crystals were filtered. The filtered crystals were washed with a mixture of ethanol and water (25/25 mL) at 0-5°C and dried under reduced pressure at 40°C to give 4-methoxyphenyl 4-O-acetyl-3,6-di-O-benzyl-2-deoxy-2-{[(2,2,2-trichloroethoxy)carbonyl]amino}-β-D-glucopyranoside (compound II-4) (10.4 g, yield 98%) as white crystals.

¹H-NMR (500 MHz, CDCl₃) δ 7.35-7.24 (m, 10H), 7.00-6.95 (m, 2H), 6.77-6.73 (m, 2H), 5.44 (brs, 1H), 5.27 (d, J = 6.9 Hz, 1H), 5.09 (t, J = 9.2 Hz, 1H), 4.74-4.62 (m, 4H), 4.50 (s, 2H), 4.19 (brs, 1H), 3.74 (s, 3H), 3.74 (brs, 1H), 3.63-3.56 (m, 3H), 1.91 (s, 3H). ¹³C-NMR (125 MHz, CDCl₃) δ 169.7, 155.4, 153.9, 151.1, 137.8, 137.7, 128.5, 128.3, 127.9, 127.7, 127.6, 118.4, 114.4, 99.1, 77.7, 74.3, 74.1, 73.6, 73.5, 71.4, 69.5, 57.7, 55.6, 20.8.
HRMS(ESI⁻)[M-H]⁻ calcd for C₃₂H₃₃Cl₃NO₉: 680.1226; found 680.1227.

### Example 33:

### 4-O-Acetyl-3,6-di-O-benzyl-2-deoxy-2-{[(2,2,2-trichloroethoxy)carbonyl]amino}-D-glucopyranoside (Compound II-5)

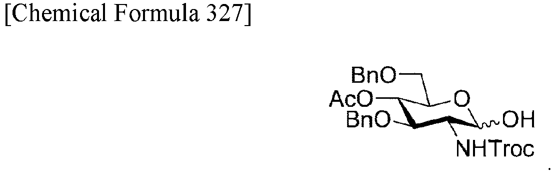

### (Substep 3-4)

To a solution containing 4-methoxyphenyl 4-O-acetyl-3,6-di-O-benzyl-2-deoxy-2-{[(2,2,2-trichloroethoxy)carbonyl]amino}-β-D-glucopyranoside (compound II-4) (10.0 g, 14.64 mmol) in dichloromethane (80 mL), hexafluoro-2-propanol (50 mL), and water (5 mL) was added [bis(trifluoroacetoxy)iodo]benzene (8.82 g, 20.51 mmol) at 25°C or below, and the mixture was stirred at the same temperature for 4 hours. After the completion of the reaction was checked by HPLC, ethyl acetate (250 mL) was added. The mixture was cooled on ice, water (100 mL) dissolving sodium hydrogen carbonate (5 g) and sodium sulfite (5 g) was poured, and the mixture was liquid-separated to obtain an organic layer. The organic layer obtained was washed again with water (100 mL) dissolving sodium hydrogen carbonate (5 g) and sodium sulfite (5 g), and further washed with 20% brine (50 mL). The resulting organic layer was concentrated under reduced pressure to 100 mL (crystals were found to precipitate during concentration), and heptane (150 mL) was added dropwise. The resulting slurry solution was cooled to 0-5°C, and then stirred at the same temperature for 1 hour. The precipitated crystals were then filtered. The filtered crystals were washed with a mixture of ethyl acetate and heptane (8/24 mL) at 0-5°C and dried under reduced pressure at 40°C to give 4-O-acetyl-3,6-di-O-benzyl-2-deoxy-2-{[(2,2,2-trichloroethoxy)carbonyl]amino}-D-glucopyranoside (compound II-5) (7.6 g, yield 90%) as white crystals.

¹H-NMR (500 MHz, CDCl₃) δ 7.38-7.21 (m, 10H), 5.17 (t, J = 3.5 Hz, 1H), 5.13 (d, J = 10.0 Hz, 1H), 4.98 (t, J = 10.0 Hz, 1H). 4.78 (d, J = 12.0 Hz, 1H), 4.65-4.55 (m, 3H), 4.50 (dd, J = 17.2, 12.0 Hz, 2H), 4.41 (d, J = 2.9 Hz, 1H), 4.13-4.09 (m, 1H), 3.97 (td, J = 10.2, 2.5 Hz, 1H), 3.72 (t, J = 10.0 Hz, 1H), 3.51 (dd, J = 10.6, 7.2 Hz, 1H), 3.44 (dd, J = 10.0, 2.5 Hz, 1H), 1.90 (3H, s). ¹³C-NMR (125 MHz, CDCl₃) δ 169.6, 154.1, 137.8, 137.2, 128.4, 128.1, 127.9, 127.7, 127.7, 95.3, 91.7, 77.3, 74.6, 73.7, 73.5, 70.9, 69.4, 68.8, 54.6, 20.7.
HRMS(ESI⁻)[M-H]⁻ calcd for C₂₅H₂₇Cl₃NO₈: 574.0808; found 574.0834.

### Example 34:

### 4-O-Acetyl-3,6-di-O-benzyl-2-deoxy-2-{[(2,2,2-trichloroethoxy)carbonyl]amino}-1-O-[2,2,2-trifluoro-N-phenylethanimidoyl]-β-D-glucopyranoside (Compound II-6)

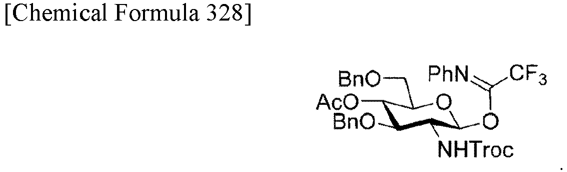

### (Substep 3-5)

A dichloromethane solution (75 mL) containing 4-O-acetyl-3,6-di-O-benzyl-2-deoxy-2- { [(2,2,2-trichloroethoxy)carbonyl]amino}-D-glucopyranoside (compound II-5) (3.0 g, 5.20 mmol) and Molecular Sieves 4A powder (powder particle size: 10 µm or less) (1.5 g) was cooled to 0°C. Next, 2,2,2-trifluoro-N-phenylacetimidoyl chloride (1.40 g, 6.74 mmol) and N-methylimidazole (0.68 g, 8.28 mmol) were added dropwise. The mixture was stirred at the same temperature for 15 hours, and the completion of the reaction was checked by HPLC. The reaction liquid was then filtered and washed with dichloromethane (15 mL) to obtain a filtrate. The filtrate was cooled to 0-5°C, washed twice with 5% brine (45 mL) at 0-5°C, and then washed further with 20% brine (15 mL). The resulting organic layer was concentrated under reduced pressure to 9 mL, and toluene (60 mL) was added. The mixture was concentrated under reduced pressure to 30 mL. Toluene (30 mL) was added to the concentrated solution, and the mixture was concentrated again under reduced pressure to 30 mL. The concentrated solution was cooled to 0-5°C, and a toluene (15 mL) slurry solution containing neutral silica gel (silica gel 60N, 40-50 µm, 3 g) cooled to 0-5°C was added. The mixture was stirred at the same temperature for 30 minutes, and then filtered. The filtered silica gel was washed with 1% ethyl acetate-containing toluene solution (75 mL). The resulting filtrate was concentrated under reduced pressure to 7.5 mL, isopropyl ether (45 mL) was added, seed crystals of compound II-6 were added, and the mixture was stirred at 20°C for 1 hour. After the crystals were found to precipitate, heptane (45 mL) was added dropwise and the mixture was stirred for 15 hours. The resulting slurry solution was cooled to 0-5°C, and then stirred at the same temperature for 1 hour. The resulting crystals were filtered. The filtered crystals were washed with a mixture of isopropyl ether and heptane (3/6 mL) at 0-5°C and dried under reduced pressure at 40°C to give 4-O-acetyl-3,6-di-O-benzyl-2-deoxy-2-{[(2,2,2-trichloroethoxy)carbonyl]amino}-1-O-[2,2,2-trifluoro-N-phenylethanimidoyl]-β-D-glucopyranoside (compound II-6) (3.2 g, yield 76%) as white crystals.

¹H-NMR (500 MHz, CDCl₃) δ 7.40-7.21 (m, 12H), 7.09 (t, J = 7.4 Hz, 1H), 6.74 (d, J = 7.4 Hz, 2H), 6.37 (brs, 1H), 5.26 (t, J = 9.7 Hz, 1H), 4.80-4.75 (m, 2H), 4.71-4.59 (m, 3H), 4.53 (dd, J = 15.2, 11.7 Hz, 2H), 4.17 (brs, 1H), 3.98 (brs, 1H), 3.85 (t, J = 10.0 Hz, 1H), 3.57 (brs, 2H), 1.96 (s, 3H).
¹³C-NMR (125 MHz, CDCl₃) δ 169.3, 154.0, 143.0, 137.6, 137.2, 128.7, 128.6, 128.3, 128.2, 128.0, 127.9, 127.9, 127.7, 124.5, 119.1, 95.2, 94.3, 76.1, 74.7, 73.6, 73.5, 71.8, 70.2, 68.6, 53.6, 20.8 (one carbon signal is missing). HRMS(ESI⁻)[M-H]⁻ calcd for C₃₃H₃₁Cl₃F₃N₂O₈: 745.1104; found 745.1096.

### Example 35:

### 4-Methoxyphenyl 4-O-acetyl-3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→3)-[4-O-acetyl-3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranoside (Compound VI-1)

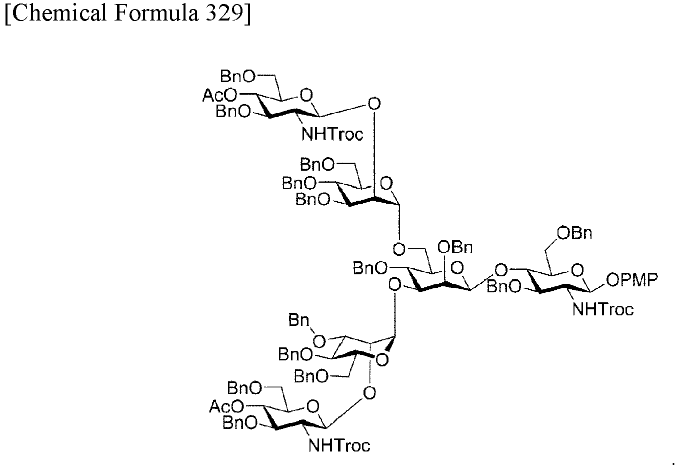

### (Substep 3-6)

To a dichloromethane solution (0.4 mL) containing compound V-3 (10 mg, 5.41 µmol) and compound II-6 (16.2 mg, 0.02 mmol) was added Molecular Sieves 4A (1 mg, 0.1 wt). The mixture was then cooled to -40°C. After stirring for 15 minutes, a dichloromethane solution containing t-butyldimethylsilyl trifluoromethanesulfonate (0.16 M, 10 µL, 1.62 µmol) was added. After stirring for 2 hours, triethylamine (10 µL, 0.08 mmol) was added, and the Molecular Sieves was filtered off at room temperature and washed with dichloromethane (2 mL) (reaction solution 1).

To a dichloromethane solution (26 mL) containing compound V-3 (650 mg, 0.35 mmol) and compound II-6 (1.05 g, 1.41 mmol) was added Molecular Sieves 4A (65 mg, 0.1 wt). The mixture was then cooled to -40°C. After stirring for 30 minutes, a dichloromethane solution containing t-butyldimethylsilyl trifluoromethanesulfonate (0.4 M, 100 µL, 0.04 mmol) was added. After stirring for 3.5 hours, triethylamine (14.7 µL, 0.11 mmol) was added, and the Molecular Sieves was filtered off at room temperature and washed with dichloromethane (2.6 mL) (reaction solution 2).

The progress of the reaction in both reaction solutions 1 and 2 was checked. Both solutions were then mixed and concentrated to dryness. The resulting crude product was purified by silica gel chromatography (ethyl acetate/hexane 20% -> 40%). To a mixture obtained by concentrating and drying the target substance-containing fractions was added t-butyl methyl ether (8.5 mL). The precipitated insoluble material was filtered off. The filtration cake was then washed with t-butyl methyl ether. The resulting solution was concentrated to dryness and purified by preparative HPLC (acetonitrile/water 90% -> 100%) to afford compound VI-1 (860 mg, yield 81%).

¹H-NMR (500 MHz, CD₃CN) δ 1.95 (s, 3H), 2.00 (s, 3H), 2.84 (m, 1H), 3.05 (d, J = 9.0 Hz, 1H), 3.20-4.03 (m, 29H), 3.73 (s, 3H), 4.06 (brs, 1H), 4.09 (brs, 1H), 4.15 (brs, 1H), 4.20-5.04 (m, 40H), 5.08 (t, J = 9.5 Hz, 1H), 5.20 (brs, 1H), 5.73 (brs, 1H), 5.89 (d, J = 7.0 Hz, 1H), 6.19 (d, J = 8.0 Hz, 1H), 6.84 (d, J = 9.0 Hz, 2H), 6.95 (d, J = 9.0 Hz, 2H), 7.14 (t, J = 7.5 Hz, 1H), 7.16-7.40 (m, 67H), 7.43 (d, J = 7.0 Hz, 2H). ¹³C-NMR (125 MHz, CD₃CN) δ 20.1, 21.0, 55.1, 68.5, 68.9, 69.4, 70.1, 70.4, 71.3, 72.1, 72.2, 72.3, 72.4, 72.6, 72.7, 73.1, 73.45, 73.52, 73.68, 73.75, 73.8, 74.1, 74.17, 74.24, 74.28, 75.0, 76.9, 77.7, 78.4, 78.6, 80.5, 95.7, 95.8, 98.0, 99.2, 99.6, 99.8, 100.3, 100.8, 114.3, 117.1, 117.9, 126.3, 127.0, 127.10, 127.15, 127.18, 127.20, 127.27, 127.28, 127.32, 127.37, 127.42, 127.48, 127.51, 127.55, 127.67, 127.72, 127.83, 127.90, 127.95, 128.0, 128.1, 128.2, 128.3, 128.4, 138.01, 138.04, 138.05, 138.07, 138.09, 138.12, 138.36, 138.46, 138.53, 138.7, 138.9, 151.1, 154.1, 154.2, 155.1, 169.2, 169.3.
MS(ESI)(m/z): 1501 ([M+2NH₄]²⁺).

### Example 36:

### 4-Methoxyphenyl 3,6-di-O-benzyl-2-deoxy-2-{ [(2,2,2-trichloroethoxy)carbonyl]amino}-β-D-glucopyranosyl-(1→2)-3,4,6-tri-O-benzyl-α-D-mannopyranosyl-(1→3)-[3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranoside (Compound VI-3)

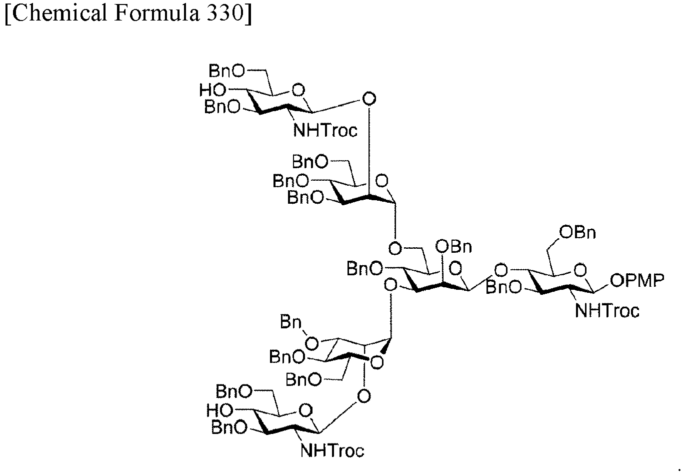

### (Substeps 3-7 and 3-8)

To a tetrahydrofuran solution (8.6 mL) containing compound VI-1 (860 mg, 0.29 mmol) were added water (0.9 mL) and 50% aqueous sodium hydroxide solution (186 mg, 2.3 mmol), followed by stirring at 50°C for 26 hours. Next, 50% aqueous sodium hydroxide solution (46 mg, 0.6 mmol) was added and the mixture was stirred for 18 hours. Methanol (0.86 mL) was then added. The mixture was stirred for 4 hours and cooled to 0°C. The pH of the reaction liquid was then adjusted to 10 by adding 4 N hydrochloric acid. In this way, a solution containing 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranoside (compound VI-2) was obtained. The resulting solution was concentrated to dryness, and tetrahydrofuran (8.6 mL), water (0.9 mL), sodium hydrogen carbonate (121.8 mg, 1.45 mmol), and 2,2,2-trichloroethyl chloroformate (202.7 mg, 0.96 mmol) were then added. After stirring at room temperature for 1 hour, water (4.3 mL) and ethyl acetate (8.6 mL) were added to the resulting suspension. The aqueous layer was removed, and the resulting organic layer was washed twice with 20% brine. The concentrated and dried crude material obtained was purified by silica gel chromatography (ethyl acetate/hexane: 15% -> 55%) to give a compound VI-3 (593.7 mg, yield 71%).

MS(ESI)(m/z): 1458 ([M+2NH₄]²⁺).

### <Synthesis of compound VI-3 or compound VI-6>

Compound VI-3 or compound VI-6 was synthesized according to the following synthesis scheme 3B.

### [Synthesis Scheme 3B]

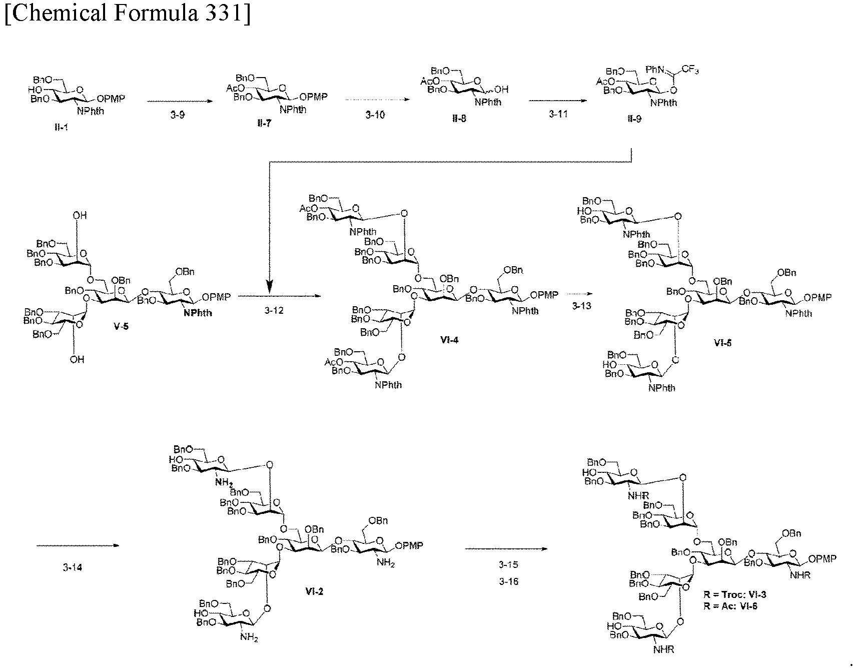

### Example 37:

### 4-Methoxyphenyl 4-O-acetyl-3,6-di-O-benzyl-2-deoxy-2-(1,3-dioxo-1,3-dihydro-2H-isoindol-2-yl)-β-D-glucopyranoside (Compound II-7)

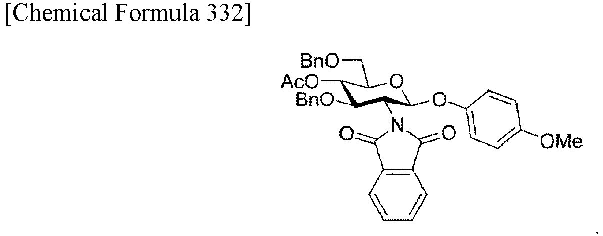

### (Substep 3-9)

To an ethyl acetate (200 mL) solution containing 4-methoxyphenyl 3,6-di-O-benzyl-2-deoxy-2-(1,3-dioxo-1,3-dihydro-2H-isoindol-2-yl)-β-D-glucopyranoside (compound II-1) (50.0 g, 83.94 mmol) were added triethylamine (11.04 g, 109.12 mmol), dimethylaminopyridine (0.31 g, 2.52 mmol), and acetic anhydride (11.10 g, 109.12 mmol), and the mixture was stirred at 20°C for 4 hours. After the completion of the reaction was checked by HPLC, ethanol (500 mL) and water (150 mL) were added dropwise. The slurry solution was stirred for 1 hour, and the precipitated crystals were filtered. The filtered crystals were washed with a mixture of ethanol and water (150/50 mL) and dried under reduced pressure at 40°C to give 4-methoxyphenyl 4-O-acetyl-3,6-di-O-benzyl-2-deoxy-2-(1,3-dioxo-1,3-dihydro-2H-isoindol-2-yl)-β-D-glucopyranoside (compound II-7) (49.0 g, yield 91%) as white crystals.

¹H-NMR (500 MHz, CDCl₃) δ 7.85-7.60 (m, 4H), 7.24-7.34 (m, 5H), 7.04-7.00 (m, 2H), 6.96-6.87 (m, 3H), 6.84 (dt, J = 9.0, 3.0 Hz, 2H), 6.67 (dt, J = 8.5, 2.5 Hz, 2H), 5.66 (t, J = 4.0 Hz, 1H), 5.22-5.18 (m, 1H), 4.64 (d, J = 12.0 Hz, 1H), 4.55-4.48 (m, 4H), 4.36 (d, J = 12.0 Hz, 1H), 3.90-3.84 (m, 1H), 3.68 (s, 3H), 3.68-3.64 (m, 2H), 1.98 (s, 3H). ¹³C-NMR (125 MHz, CDCl₃) δ 169.6, 155.3, 150.6, 137.8, 137.5, 133.9, 128.2, 128.0, 127.7, 127.7, 127.5, 127.4, 123.3, 118.4, 114.3, 97.4, 76.8, 73.9, 73.7, 73.5, 72.2, 69.4, 55.4, 55.3, 20.8.
HRMS(ESI⁺)[M+H]⁺ calcd for C₃₇H₃₆NO₉: 638.2385; found 638.2401.

### Example 38:

### 4-O-Acetyl-3,6-di-O-benzyl-2-deoxy-2-(1,3-dioxo-1,3-dihydro-2H-isoindol-2-yl)-D-glucopyranoside (Compound II-8)

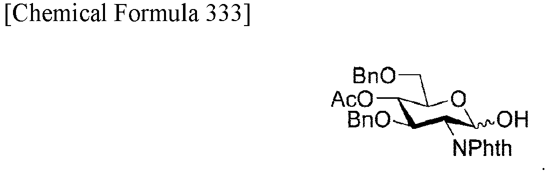

### (Substep 3-10)

To a solution containing 4-methoxyphenyl 4-O-acetyl-3,6-di-O-benzyl-2-deoxy-2-(1,3-dioxo-1,3-dihydro-2H-isoindol-2-yl)-β-D-glucopyranoside (compound II-7) (49.0 g, 76.84 mmol) in dichloromethane (392 mL), hexafluoro-2-propanol (245 mL), and water (25 mL) was added [bis(trifluoroacetoxy)iodo]benzene (46.3 g, 107.58 mmol) at 25°C or below, and the mixture was stirred at the same temperature for 4 hours. After the completion of the reaction was checked by HPLC, ethyl acetate (1225 mL) was added. After the mixture was cooled on ice, water (490 mL) dissolving sodium hydrogen carbonate (24.5 g) and sodium sulfite (24.5 g) was poured, and the mixture was then liquid-separated to obtain an organic layer. The organic layer obtained was washed again with water (490 mL) dissolving sodium hydrogen carbonate (24.5 g) and sodium sulfite (24.5 g), and further washed with 20% brine (245 g). The resulting organic layer was concentrated under reduced pressure to 490 mL (crystals were found to precipitate during concentration), and heptane (735 mL) was added dropwise. The resulting slurry solution was cooled to 0-5°C, and stirred at the same temperature for 1 hour. The precipitated crystals were then filtered. The filtered crystals were washed with a mixture of ethyl acetate and heptane (39/118 mL) at 0-5°C and dried under reduced pressure at 40°C to give 4-O-acetyl-3,6-di-O-benzyl-2-deoxy-2-(1,3-dioxo-1,3-dihydro-2H-isoindol-2-yl)-D-glucopyranoside (compound II-8) (37.5 g, yield 92%) as white crystals.

¹H-NMR (400 MHz, CDCl₃) δ 7.71-7.65 (m, 4H), 7.34-7.26 (m, 5H), 7.01-6.87 (m, 5H), 5.36 (dd, J = 8.0, 8.0 Hz, 1H), 5.13 (dd, J = 8.4, 10.0 Hz, 1H), 4.59 (d, J = 12.4 Hz, 1H), 4.54 (s, 2H), 4.50 (dd, J = 8.4, 10.4 Hz, 1H), 4.33 (d, J = 12.4 Hz, 1H), 4.17 (dd, J = 8.4, 10.4 Hz, 1H), 3.79 (ddd, J = 8.4, 5.2, 4.8 Hz, 1H), 3.61-3.53 (m, 2H), 3.41 (d, J = 8.0 Hz, 1H), 1.93 (s, 3H).
¹³C-NMR (100 MHz, CDCl₃) δ 169.8, 168.1, 137.7, 137.7, 134.0, 131.6, 128.4, 128.2, 128.0, 127.8. 127.7, 127.5, 123.4, 116.2, 92.9, 73.9, 73.7, 73.5, 72.2, 69.3, 57.1, 20.9.
HRMS(ESI⁻)[M-H]⁻ calcd for C₃₀H₂₈NO₈: 530.1820; found 530.1841.

### Example 39:

### 4-O-Acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (Compound II-9)

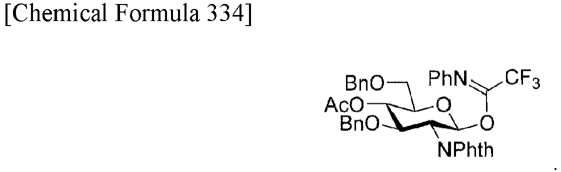

### (Substep 3-11)

A dichloromethane (125 mL) solution containing 4-O-acetyl-3,6-di-O-benzyl-2-deoxy-2-(1,3-dioxo-1,3-dihydro-2H-isoindol-2-yl)-D-glucopyranoside (compound II-8) (5.0 g, 9.41 mmol) and Molecular Sieves 4A powder (powder particle size: 10 µm or less) (2.5 g) was cooled to 0°C. Next, 2,2,2-trifluoro-N-phenylacetimidoyl chloride (1.95 g, 9.41 mmol) and N-methylimidazole (0.85 g, 10.35 mmol) were added dropwise. The mixture was then stirred at the same temperature for 15 hours. After the completion of the reaction was checked by HPLC, the reaction liquid at 0-5°C was filtered through a column packed with neutral silica gel (silica gel 60N, 40-50 µm, 15 g) in dichloromethane and washed with dichloromethane (125 mL). The filtrate obtained was concentrated to dryness to give 4-O-acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (compound II-9) (6.0 g, yield 90%) as an oily compound.

¹H-NMR (500 MHz, CDCl₃) δ 7.80-7.69 (m, 4H), 7.37-7.26 (m, 5H), 7.17 (t, J = 7.7 Hz, 2H), 7.04 (t, J = 7.5 Hz, 1H), 7.00 (d, J = 6.5 Hz, 2H), 6.95-6.88 (m, 3H), 6.59 (brs, 2H), 5.26-5.20 (m, 1H), 4.62 (d, J = 12.0 Hz, 1H), 4.56 (s, 2H), 4.49 (brs, 1H), 4.32 (d, J = 12.0 Hz, 2H), 3.61 (brs, 2H), 1.95 (s, 3H). ¹³C-NMR (125 MHz, CDCl₃) δ 169.7, 137.9, 137.6, 134.3, 131.5, 130.5, 129.2, 128.8, 128.6, 128.4, 128.2, 128.0, 127.9, 127.8, 124.6, 123.7, 119.4, 119.3, 76.8, 74.5, 74.2, 73.8, 71.9, 69.1, 54.7, 53.7, 21.1.
HRMS(ESI⁻)[M-H]⁻ calcd for C₃₈H₃₂F₃N₂O₈: 701.2116; found 701.2095.

### Example 40:

### 4-Methoxyphenyl 4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)3,6-di-O-benzyl-2-deoxy-2-(1,3-dioxo-1,3-dihydro-2H-isoindol-2-yl)-β-D-glucopyranoside (Compound VI-4)

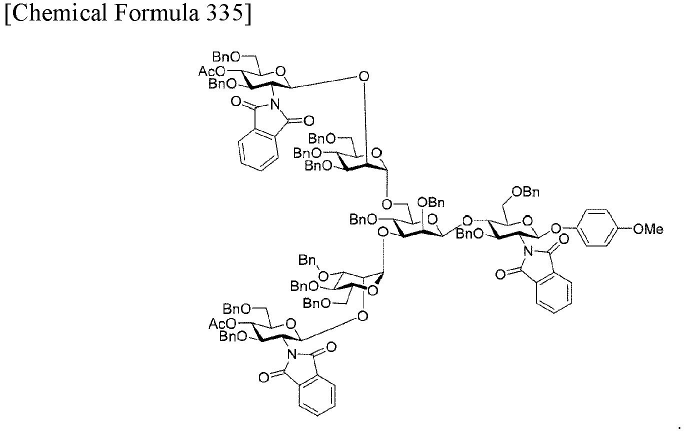

### (Substep 3-12)

First, 4-methoxyphenyl 3,4,6-tri-O-benzyl-α-D-mannopyranosyl-(1→3)-[3,4,6-tri-O-benzyl-α-D-mannopyranosyl-(1→6)]-2,4-di-O-benzyl-β-D-mannopyranosyl-(1→4)-3,6-di-O-benzyl-2-deoxy-(1,3-dioxo-1,3-dihydro-2H-isoindol-2-yl)-β-D-glucopyranoside (compound V-5) (410 mg, 0.227 mmol) and 4-O-acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (compound II-9) (800 mg, 1.14 mmol) were added to a 30-mL 2-neck flask. Dichloromethane (8.2 mL) and Molecular Sieves 4A powder (61.5 mg) were then added. Subsequently, tert-butyldimethylsilyl trifluoromethanesulfonate (10.4 µL, 45 µmol) was added dropwise over 5 minutes at -65°C under nitrogen, and the mixture was stirred at the same temperature for 1 hour. After the completion of the reaction was checked by HPLC, triethylamine (12.6 µL, 90 µmol) was added. The mixture was then stirred at room temperature for 1 hour. The reaction liquid was filtered through Celite and washed with dichloromethane (2.1 mL). The filtrate was liquid-separated twice with water (4.1 mL). The organic layer was dried over sodium sulfate, filtered, and then concentrated. The concentrated residue was purified by column chromatography (silica amount: 20 g; developing solvent: toluene/ethyl acetate = 9/1 to 8/2). The main residue was concentrated under reduced pressure to give 4-methoxyphenyl 4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)3,6-di-O-benzyl-2-deoxy-2-(1,3-dioxo-1,3-dihydro-2H-isoindol-2-yl)-β-D-glucopyranoside (compound VI-4) (534 mg, isolation yield 83%) as a white amorphous product.

¹H-NMR (500 MHz, CDCl₃) δ 7.76-7.50 (m, 12H), 7.38-7.59 (m, 74H), 5.51 (d, J = 8.4 Hz, 1H), 5.07 (t, J = 8.4 Hz, 2H), 4.95-4.09 (m, 35H), 4.01-3.98 (m, 3H), 3.79 (s, 2H), 3.73-3.66 (m, 7H), 3.55 (brd, J = 8.4 Hz, 1H), 3.47-3.16 (m, 14H), 2.88-2.80 (m, 3H), 2.62 (m, 1H), 2.51 (m, 1H), 1.92 (s, 3H), 1.83 (s, 3H).
¹³C-NMR (125 MHz, CDCl₃) δ 169.4, 169.3, 155.3, 150.9, 138.8, 138.6, 138.5, 138.4, 138.2, 138.0, 138.0, 138.0, 137.7, 133.6, 131.8, 131.6, 129.0, 128.9, 128.7, 128.6, 128.4, 128.3, 128.3, 128.3, 128.2, 128.1, 128.0, 127.9, 127.8, 127.7, 127.7, 127.5, 127.5, 127.4, 127.3, 127.3, 127.2, 127.1, 126.1, 125.3, 123.2, 118.6, 114.3, 101.7, 98.5, 97.6, 97.5, 97.1, 95.9, 80.4, 79.7, 78.0, 77.6, 77.2, 76.9, 76.6, 76.5, 75.0, 74.8, 74.5, 74.2, 74.1, 74.0, 73.7, 73.5, 73.4, 73.3, 73.2, 73.0, 72.9, 72.5, 72.4, 72.0, 71.0, 70.8, 70.7, 69.8, 69.7, 69.6, 69.4, 68.0, 66.6, 55.6, 5.6, 55.1, 55.0, 20.9, 20.9. HRMS(ESI⁺)[M+NH₄]⁺ calcd for C₁₆₉H₁₆₉N₄O₃₇: 2846.1460; found 2846.1404.

### Example 41:

### 4-Methoxyphenyl 3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[3,6-di-O-benzyl-2-deoxy-2-(1,3-dioxo-1,3-dihydro-2H-isoindol-2-yl)-β-D-glucopyranosyl-(1→2)-3,4, 6-tri-O-benzyl-α-D-mannopyranosyl-(1→6)]-2,4-di-O-benzyl-β-D-mannopyranosyl-(1→4)3,6-di-O-benzyl-2-deoxy-2-(1,3-dioxo-1,3-dihydro-2H-isoindol-2-yl)-β-D-glucopyranoside (Compound VI-5)

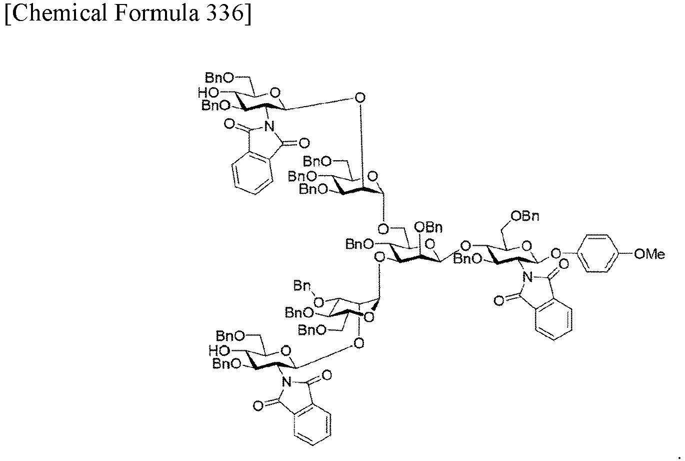

### (Substep 3-13)

First, 4-methoxyphenyl 4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[4-O-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)3,6-di-O-benzyl-2-deoxy-2-(1,3-dioxo-1,3-dihydro-2H-isoindol-2-yl)-β-D-glucopyranoside (compound VI-4) (530 mg, 0.187 mmol) was added to a 30-mL 2-neck flask. Tetrahydrofuran (2.1 mL), methanol (1.1 mL), and methyl trifluoroacetate (19 µL, 0.187 mmol) were then added. After stirring at room temperature under nitrogen for 20 minutes, 1 M tetrahydrofuran solution containing potassium tert-butoxide (94 µL, 94 µmol) was added dropwise. The mixture was stirred at 50°C for 3 hours. After the completion of the reaction was checked by HPLC, the mixture was cooled to room temperature. Acetic acid (11 µL, 0.187 mmol) was then added. After ethyl acetate (10.3 mL) was added, the mixture was liquid-separated twice with 5% brine (10 mL). The resulting organic layer was dried over sodium sulfate, filtered, and then concentrated to give 4-methoxyphenyl 3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)3,6-di-O-benzyl-2-deoxy-2-(1,3-dioxo-1,3-dihydro-2H-isoindol-2-yl)-β-D-glucopyranoside (compound VI-5) (460 mg, isolation yield 90%) as a white amorphous product. This product was not purified, and was used as it was in the next step.

¹H-NMR (500 MHz, CDCl₃) δ 7.78-7.49 (m, 12H), 7.38-6.56 (m, 74H), 5.51 (d, J = 8.8 Hz, 1H), 4.91-4.28 (m, 27H), 4.24-4.12 (m, 6H), 4.03-3.79 (m, 10H), 3.75-3.64 (m, 9H), 3.57-3.31 (m, 13H), 3.25 (dd, J = 10.0, 6.4 Hz, 1H), 3.17 (d, J = 10.0 Hz, 1H), 2.90-2.79 (m, 3H), 2.74 (d, J = 2.8 Hz, 1H), 2.64-2.57 (m, 2H), 2.40 (m, 1H).

### Example 42:

### 4-Methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranoside (Compound VI-2)

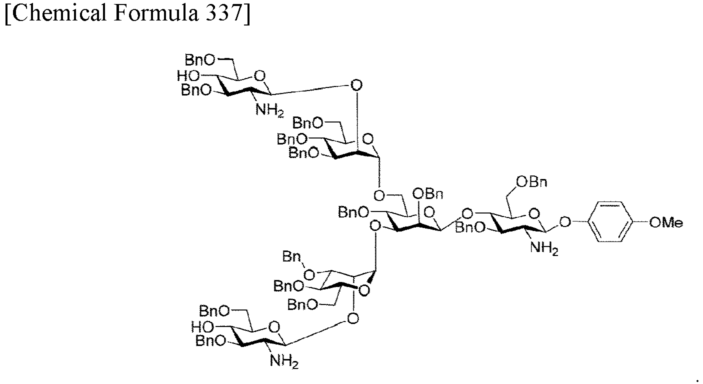

### (Substep 3-14)

A toluene solution (920 µL) containing 4-methoxyphenyl 3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)3,6-di-O-benzyl-2-deoxy-2-(1,3-dioxo-1,3-dihydro-2H-isoindol-2-yl)-β-D-glucopyranoside (compound VI-5) (460 mg, 0.168 mmol) was added to a 30-mL recovery flask. Next, 1-butanol (1.2 mL) and ethylenediamine (224 µL) were added. The mixture was stirred under nitrogen at 80°C for 22 hours. After the completion of the reaction was checked by HPLC, the mixture was cooled to room temperature. Cyclopentyl methyl ether (6.9 mL) was added, and the mixture was stirred for 17 hours. The precipitate was filtered off, and the filtrate was liquid-separated three times with 25% methanol solution (6.9 mL). The resulting organic layer was dried over sodium sulfate, filtered, and then concentrated to give 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranoside (compound VI-2) (360 mg, isolation yield 92%) as a white amorphous product. This product was not purified, and was used as it was in the next step.

¹H-NMR (500 MHz, CDCl₃) δ 7.41-7.04 (m, 76H), 6.96 (d, J = 9.0 Hz, 2H), 6.78 (d, J = 9.0 Hz, 2H), 5.15 (s, 1H), 5.09 (d, J = 12.0 Hz, 1H), 4.98-4.83 (m, 6H), 4.75-4.69 (m, 3H), 4.64-4.34 (m, 20H), 4.26 (d, J = 11.6 Hz, 1H), 4.12 (d, J = 8.4 Hz, 1H), 4.06-3.45 (m, 29H), 3.33-3.21 (m, 6H), 3.04-2.98 (m, 3H), 2.90-2.79 (m, 4H).

### Example 43:

### 4-Methoxyphenyl 3,6-di-O-benzyl-2-deoxy-2-{ [(2,2,2-trichloroethoxy)carbonyl]amino}-β-D-glucopyranosyl-(1→2)-3,4,6-tri-O-benzyl-α-D-mannopyranosyl-(1→3)-[3,6-di-O-benzyl-2-deoxy-2-{ [(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranoside (Compound VI-3)

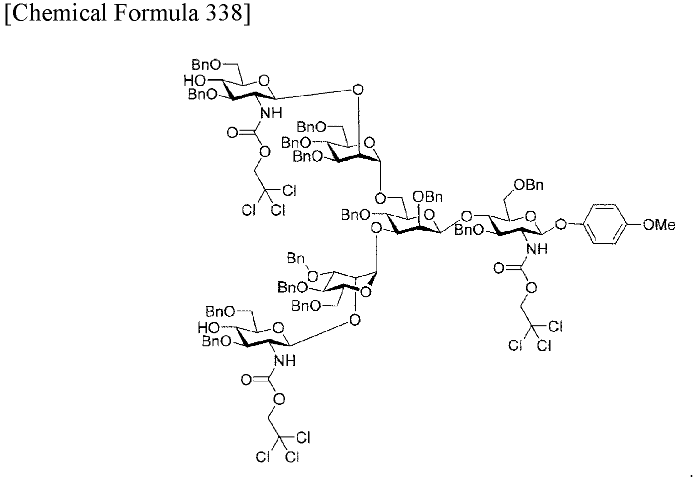

### (Substep 3-15)

A cyclopentyl methyl solution (600 µL) containing 4-methoxyphenyl 2-amino-3, 6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranoside (compound VI-2) (300 mg, 0.127 mmol) was added to a 30-mL recovery flask. Tetrahydrofuran (3 mL), water (1.5 mL), and sodium hydrogen carbonate (53 mg, 0.635 mmol) were added. Next, 2,2,2-trichloroethyl chloroformate (58 µL, 0.419 mmol) was added dropwise over 10 minutes, and the mixture was then stirred for 3 hours. After the completion of the reaction was checked by HPLC, ethyl acetate (3 mL) was added. The liquid was separated twice using water (3 mL). The resulting organic layer was then washed with 20% brine (1.5 mL). The organic layer was dried over sodium sulfate, filtered, and concentrated. The concentrated residue was purified by column chromatography (silica amount: 20 g; developing solvent: toluene/ethyl acetate = 9/1 to 8/2). The main residue was concentrated under reduced pressure to give 4-methoxyphenyl 3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→3)-[3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranoside (compound VI-3) (279 mg, isolation yield 76%) as a white amorphous product.

¹H-NMR (500 MHz, CDCl₃) δ 7.39-6.99 (m, 70H), 6.87 (d, J = 8.8 Hz, 2H), 6.74 (d, J = 8.8 Hz, 2H), 5.02 (s, 1H), 4.91-4.48 (m, 40H), 4.23 (d, J = 12.8 Hz, 1H), 4.08 (s, 1H), 3.93-3.35 (m, 34H), 3.22 (m, 4H), 2.93 (m, 1H), 2.75-2.69 (m, 2H). HRMS(ESI⁺)[M+Et₃N+H]⁺ calcd for C₁₅₆H₁₇₄Cl₉N₄O₃₅: 2982.9124; found 2982.9080.

1H-NMR was found to correspond to compound VI-3 synthesized in a different route.

### Example 44:

### 4-Methoxyphenyl 3,6-di-O-benzyl-2-deoxy-2-acetylamino-β-D-glucopyranosyl-(1→2)-3,4,6-tri-O-benzyl-α-D-mannopyranosyl-(1→3)-[3,6-di-O-benzyl-2-deoxy-2-acetylamino-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-acetylamino-β-D-glucopyranoside (Compound VI-6)

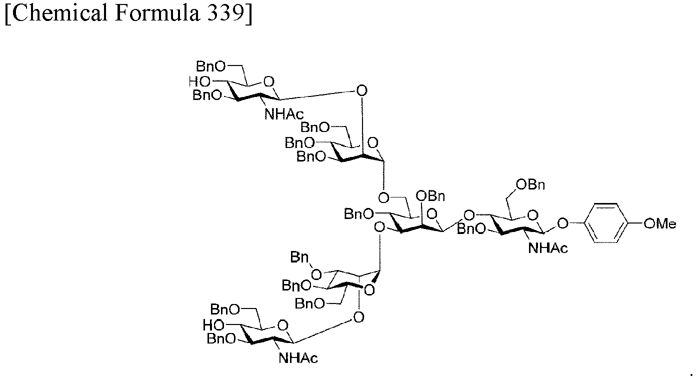

### (Substep 3-16)

A cyclopentyl solution (720 µL) containing 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranoside (compound VI-2) (360 mg, 0.153 mmol) was added to a 50-mL recovery flask. Tetrahydrofuran (3.6 mL), triethylamine (0.192 mL, 1.37 mmol), and dimethylaminopyridine (3.7 mg, 31 µmol) were then added. After acetic anhydride (101 µL, 1.07 mmol) was added, the mixture was stirred for 2 hours. HPLC revealed the production of a compound represented by the following formula VI-7: 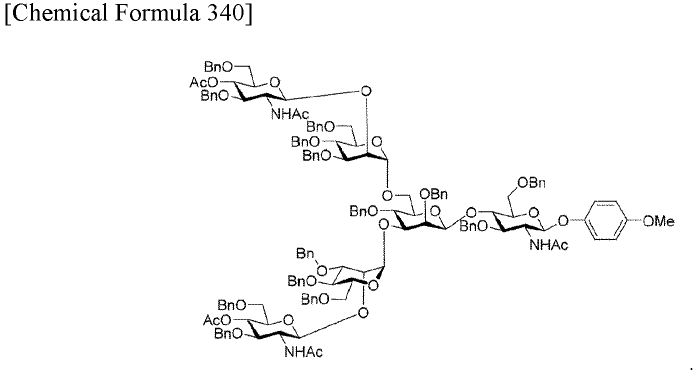 Ethyl acetate (5.4 mL) was then added. The liquid was separated using water (3.6 mL). Next, 5% aqueous sodium hydrogen carbonate solution (3.6 mL) was added to the organic layer and the liquid was separated. Water (3.6 mL) was added to the organic layer and the liquid was separated. The organic layer was dried over sodium sulfate, filtered, and then concentrated. A methanol (5.4 mL) solution containing the concentrated residue (410 mg) was added to a 50-mL recovery flask. A methanol solution containing 28% sodium methoxide (74 µL, 0.306 mmol) was added and the mixture was stirred for 3 hours. After the completion of the reaction was checked by HPLC, acetic acid (22 µL, 0.383 mmol) was added. The mixture was concentrated. The concentrated residue was purified by column chromatography (silica amount: 20 g; developing solvent: toluene/ethyl acetate = 3/1 to 1/1). The main residue was concentrated under reduced pressure to give 4-methoxyphenyl 3,6-di-O-benzyl-2-deoxy-2-acetylamino-β-D-glucopyranosyl-(1→2)-3,4,6-tri-O-benzyl-α-D-mannopyranosyl-(1→3)-[3,6-di-O-benzyl-2-deoxy-2-acetylamino-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-acetylamino-β-D-glucopyranoside (compound VI-6) (250 mg, isolated yield 66%) as a white amorphous product.

¹H-NMR (500 MHz, CDCl₃) δ 7.38-7.04 (m, 70H), 6.96 (d, J = 8.8 Hz, 2H), 6.67 (d, J = 8.8 Hz, 2H), 6.14 (d, J = 8.0 Hz, 1H), 5.78 (d, J = 6.4 Hz, 1H), 5.30 (d, J = 7.2 Hz, 1H), 5.16 (d, J = 8.0 Hz, 1H), 5.03-4.18 (m, 36H), 4.07-3.45 (m, 31H), 3.26-3.15 (m, 2H), 3.07 (brs, 1H), 2.99 (m, 1H), 2.84-2.76 (m, 2H), 2.56 (d, J = 2.4 Hz, 1H), 1.76-1.59 (m, 9H).
¹³C-NMR (125 MHz, CDCl₃) δ 171.7, 170.9, 170.1, 154.9, 151.5, 139.0, 138.8, 138.8, 138.7, 138.6, 138.3, 138.2, 138.2, 138.1, 138.0, 137.6, 137.5, 128.5, 128.4, 128.3, 128.2, 128.2, 128.1, 128.0, 127.7, 127.6, 127.6, 127.5, 127.4, 126.3, 118.0, 114.4, 100.9, 99.3, 99.1, 98.0, 97.4, 80.1, 79.5, 79.3, 78.7, 78.1, 77.9, 77.5, 77.2, 76.9, 75.3, 75.1, 74.8, 74.8, 74.6, 74.4, 74.2, 74.1, 73.9, 73.7, 73.6, 73.5, 73.3, 73.2, 73.0, 72.4, 71.6, 71.2, 71.0, 70.9, 70.6, 69.5, 69.3, 68.9, 66.6, 56.6, 55.5, 29.6, 43.5, 23.4, 23.2.
HRMS(ESI⁺)[M+H]⁺ calcd for C₁₄₇H₁₆₂N₃O₃₂: 2482.1170; found 2482.1124.

### <Synthesis of compound IX-5>

Compound IX-5 was synthesized according to the following synthesis scheme 4.

### [Synthetic Scheme 4]

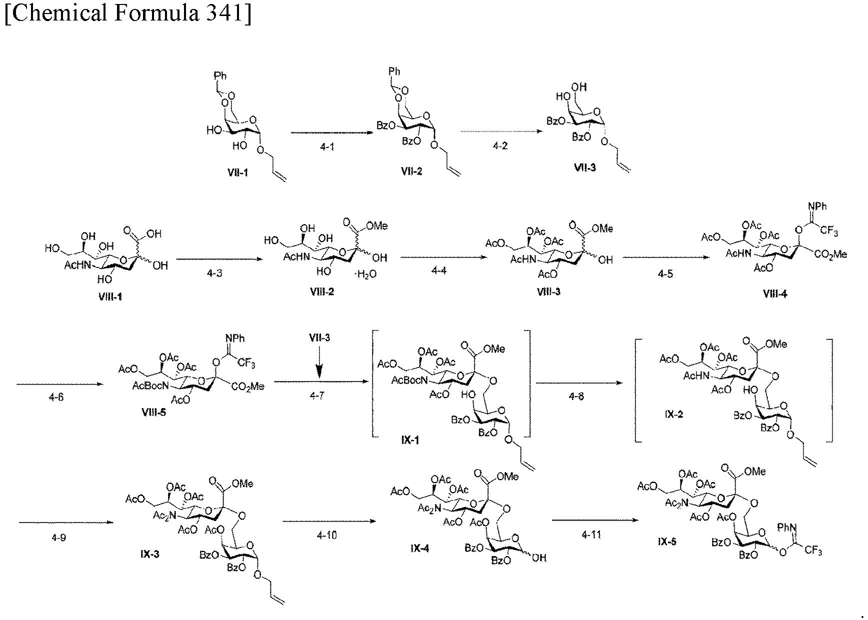

### Example 45:

### Prop-2-en-1-yl 2,3-di-O-benzoyl-4,6-O-benzyliden-α-D-galactopyranoside (Compound VII-2)

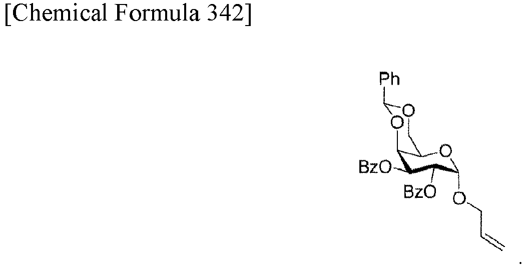

### (Substep 4-1)

To a pyridine solution (150 mL) containing prop-2-en-1-yl 4,6-0-benzylidene-α-D-galactopyranoside (compound VII-1) (30.0 g, 97.30 mmol) was added dropwise at 40°C benzoyl chloride (47.87 g, 340.54 mmol). The mixture was stirred at the same temperature for 2 hours. After the completion of the reaction was checked by HPLC, the mixture was cooled to 20-30°C. After ethanol (450 mL) was poured, water (300 mL) was added dropwise over 30 minutes. The resulting slurry solution was stirred at 20-30°C for 1 hour. The precipitated crystals were then filtered. The filtered crystals were washed with a mixture of ethanol and water (75/75 mL) and dried under reduced pressure at 40°C to give prop-2-en-1-yl 2,3-di-O-benzoyl-4,6-O-benzyliden-α-D-galactopyranoside (compound VII-2) (47.9 g, yield 95%) as white crystals.

¹H-NMR (500 MHz, CDCl₃) δ 8.03-7.98 (m, 4H), 7.55-7.46 (m, 4H), 7.40-7.30 (m, 7H), 5.90-5.78 (m, 2H), 5.82 (s, 1H), 5.57 (s, 1H), 5.42 (d, J = 1.7 Hz, 1H), 5.31 (dd, J = 17.2, 1.7 Hz, 1H), 5.15 (dd, J = 1.7, 10.5 Hz, 1H), 4.66 (s, 2H), 4.33 (d, J = 12.5 Hz, 1H), 4.26 (dd, J = 12.5, 4.5 Hz, 1H), 4.12 (dd, J = 12.5, 1.0 Hz, 1H), 4.08 (dd, J = 6.5, 13.5 Hz, 1H), 3.95 (s, 1H). ¹³C-NMR (125 MHz, CDCl₃) δ 166.1, 165.8, 137.5, 133.4, 133.2, 133.1, 129.8, 129.7, 129.5, 129.4, 128.8, 128.3, 128.1, 126.1, 117.5, 100.6, 96.2, 74.2, 69.3, 69.1, 68.7, 68.6, 62.4.
HRMS(ESI⁺)[M+H]⁺ calcd for C₃₀H₂₉O₈: 517.1857; found 517.1880.

### Example 46:

### Prop-2-en-1-yl 2,3-di-O-benzoyl-α-D-galactopyranoside (Compound VII-3) [0547]

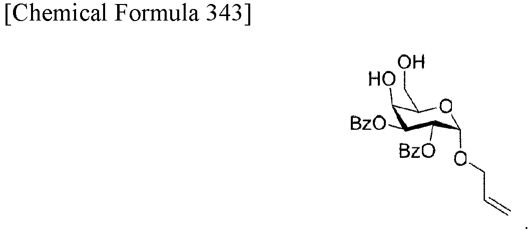

### (Substep 4-2)

An acetonitrile (377 mL) solution containing prop-2-en-1-yl 2,3-di-O-benzoyl-4,6-O-benzyliden-α-D-galactopyranoside (Compound VII-2) (47.1 g, 91.18 mmol) was heated to 45°C. Water (24 mL) and concentrated hydrochloric acid (9.2 g, 91.18 mmol) were added, and the mixture was stirred at the same temperature for 30 minutes. Water (353 mL) was added dropwise at 45-50°C over 3 hours, and the mixture was stirred for another 30 minutes. After the completion of the reaction was checked by HPLC, sodium acetate (11.22 g, 136.78 mmol) was added and ethyl acetate (942 mL) and water (471 mL) were poured. After cooling to 25°C or below, the mixture was liquid-separated to obtain an organic layer. The organic layer obtained was washed twice with water (471 mL) and further washed with 20% brine (236 mL). The organic layer was concentrated under reduced pressure to 141 mL, and toluene (707 mL) was added. The mixture was concentrated again under reduced pressure to 141 mL. Toluene (236 mL) was added to the concentrated solution obtained, and the mixture was concentrated under reduced pressure to 141 mL. The concentrated solution was cooled to 0-5°C, and a toluene (330 mL) slurry solution containing neutral silica gel (silica gel 60N, 40-50 µm, 141 g) cooled to 0-5°C was poured. The mixture was stirred at the same temperature for 15 minutes and the product was adsorbed on silica gel, followed by filtration. The silica gel solid phase containing the product was washed with toluene (942 mL) at 0-5°C (the filtrate at the time of the toluene wash was discarded). The target substance was then desorbed from silica gel by using cyclopentyl methyl ether (707 mL) to give a cyclopentyl methyl ether solution containing prop-2-en-1-yl 2,3-di-O-benzoyl-α-D-galactopyranoside (compound VII-3) (quantitative value 36.2 g, quantitative yield 93%). This solution was used in the next step.

### Example 47:

### Methyl 5-acetamido-3,5-dideoxy-D-glycero-D-galacto-non-2-uropyranosonate monohydrate (Compound VIII-2)

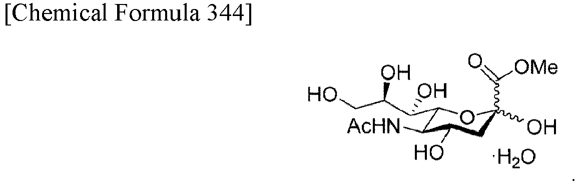

### (Substep 4-3)

To a methanol (321 mL) solution containing 5-acetamido-3,5-dideoxy-D-glycero-D-galacto-non-2-uropyranosonic acid (compound VIII-1) (40.1 g, 129.66 mmol) and methyl orthoformate (15.60 mL, 142.59 mmol) was added sulfuric acid (1.0 g, 10.20 mmol), and the mixture was heated to 40°C and stirred for 3 hours. After the completion of the reaction was checked by HPLC, the mixture was cooled to 25°C. Dimethylacetamide (40 mL) was added, and the mixture was concentrated under reduced pressure to 160 mL. The temperature of the resulting concentrated solution was adjusted to 15°C. Water (20 mL) and ethyl acetate (722 mL) were poured. The mixture was then stirred at 25°C for 1 hour. The slurry solution was cooled to 0-5°C, and then stirred at the same temperature for 2 hours. The precipitated crystals were filtered. The filtered crystals were washed with ethyl acetate (80 mL) at 0-5°C and dried under reduced pressure at 40°C to give methyl 5-acetamido-3,5-dideoxy-D-glycero-D-galacto-non-2-uropyranosonate monohydrate (compound VIII-2) (41.1 g, yield 93%) as white crystals.

¹H-NMR (500 MHz, CD₃OD) δ 4.07-3.98 (m, 2H), 3.85-3.77 (m, 2H), 3.78 (s, 3H), 3.72-3.68 (m, 1H), 3.62 (dd, J = 10.9, 5.7 Hz, 1H), 3.48 (dd, J = 9.2, 1.1 Hz, 1H), 2.22 (dd, J = 12.9, 4.9 Hz, 1H), 2.02 (s, 3H), 1.89 (dd, J = 12.6, 11.5 Hz, 1H). ¹³C-NMR (125 MHz, CD₃OD) δ 175.2, 175.1, 171.8, 96.6, 72.1, 72.0, 71.6, 70.1, 67.9, 64.8, 54.4, 54.3, 53.2, 40.7, 22.7, 22.7.
HRMS(ESI⁺)[M+H]⁺ calcd for C₁₂H₂₂NO₉: 324.1289; found 324.1288.

### Example 48:

### Methyl 5-acetamido-4,7,8,9-tetra-O-acetyl-3,5-dideoxy-D-glycero-D-galacto-non-2-uropyranosonate (Compound VIII-3)

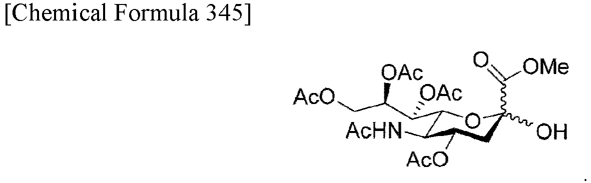

### (Substep 4-4)

The temperature of an acetonitrile (403 mL) slurry solution containing methyl 5-acetamido-3,5-dideoxy-D-glycero-D-galacto-non-2-uropyranosonate monohydrate (compound VIII-2) (40.3 g, 118.07 mmol) was adjusted to 25°C. Acetic anhydride (60.27 g, 590.36 mmol) and paratoluenesulfonic acid monohydrate (1.12 g, 5.89 mmol) were added, and the mixture was stirred at 25°C for 24 hours. The reaction liquid was cooled to 15°C, acetic anhydride (12.05 g, 118.03 mmol) was added, and the mixture was then stirred at the same temperature for 47 hours. After the completion of the reaction was checked by HPLC, methanol (40 mL) was added. The temperature was adjusted to 25°C, and the mixture was stirred at the same temperature for 2 hours. Next, sodium acetate (0.97 g, 11.82 mmol) was added, and the mixture was stirred at the same temperature for another 1 hour. The reaction liquid was concentrated under reduced pressure to 120 mL, and cooled to 0-5°C. Ethyl acetate (403 mL) and water (161 mL) were then poured, and under stirring at 0-5°C, triethylamine was added to adjust the pH to 7.0. The liquid-separated organic layer was washed twice with 10% brine (121 mL), and concentrated under reduced pressure to 200 mL. Ethyl acetate (605 mL) was added to the concentrated solution, and the mixture was concentrated again under reduced pressure to 200 mL. Ethyl acetate (40 mL) was added to the concentrated solution. Seed crystals were added, and the mixture was stirred at 25°C for 4 hours. Heptane (302 mL) was then added dropwise over 30 minutes. The resulting slurry solution was stirred at 25°C for 2 hours, and the precipitated crystals were then filtered. The filtered crystals were washed with a mixture of ethyl acetate and heptane (67/135 mL) and dried under reduced pressure at 35°C to give methyl 5-acetamido-4,7,8,9-tetra-O-acetyl-3,5-dideoxy-D-glycero-D-galacto-non-2-uropyranosonate (compound VIII-3) (44.1 g, yield 76%) as white crystals.

¹H-NMR (500 MHz, CDCl₃) δ 6.28 (d, J = 10.3 Hz, 1H), 5.41 (dd, J = 4.6, 2.3 Hz, 1H), 5.28-5.23 (m, 1H), 5.21-5.14 (m, 1H), 5.09 (s, 1H), 4.62 (dd, J = 12.3, 2.6 Hz, 1H), 4.28 (dd, J = 10.3, 2.3 Hz, 1H), 4.21-4.11 (m, 1H), 4.04 (dd, J = 12.3, 8.3 Hz, 1H), 3.85 (s, 3H), 2.24-2.20 (m, 2H), 2.16 (s, 3H), 2.12 (s, 3H), 2.03 (s, 3H), 2.01 (s, 3H), 1.91 (s, 3H). ¹³C-NMR (125 MHz, CDCl₃) δ 171.5, 171.1, 170.8, 170.3, 170.2, 168.9, 94.9, 72.1, 71.4, 69.1, 68.3, 62.5, 53.2, 49.1, 36.1, 23.0, 21.0, 20.8, 20.7, 20.7.
HRMS(ESI⁺)[M+H]⁺ calcd for C₂₀H₃₀NO₁₃: 492.1712; found 492.1712.

### Example 49:

### Methyl 5-acetamido-4,7,8,9-tetra-O-acetyl-3,5-dideoxy-2-O-(2,2,2-trifluoro-N-phenylethanimidoyl)-D-glycero-β-D-galacto-non-2-uropyranosonate (Compound VIII-4)

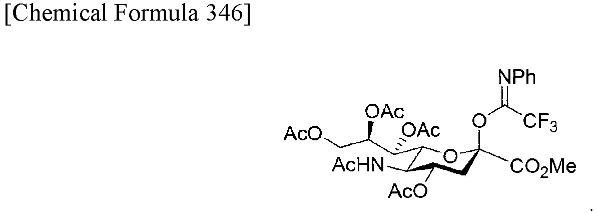

### (Substep 4-5)

The temperature of a dichloromethane (352 mL) slurry solution containing methyl 5-acetamido-4,7,8,9-tetra-O-acetyl-3,5-dideoxy-D-glycero-D-galacto-non-2-uropyranosonate (compound VIII-3) (44.0 g, 89.53 mmol) and Molecular Sieves 4A powder (powder particle size: 10 µm or less) (22 g) was adjusted to 20°C. After stirring at the same temperature for 30 minutes, 2,2,2-trifluoro-N-phenylacetimidoyl chloride (26.02 g, 125.35 mmol) was added. Next, N-methylimidazole (11.03 g, 134.33 mmol) was added dropwise, and the mixture was stirred at 20°C for 7.5 hours. The completion of the reaction was checked by HPLC. The reaction liquid was then filtered and washed with dichloromethane (88 mL) to obtain a filtrate. The filtrate obtained was cooled to 0°C and admixed with cold water (440 mL). Under stirring at 0-5°C, triethylamine was added to adjust the pH to 7.5. The mixture was stirred at 0-5°C for 30 minutes, and then liquid-separated. The resulting organic layer was washed twice with cold water (440 mL) and then cooled 20% brine (220 mL), and concentrated under reduced pressure to 88 mL. Ethyl acetate (440 mL) was added to the concentrated solution, and the mixture was concentrated again under reduced pressure to 88 mL. The concentrated solution was admixed with t-butyl methyl ether (308 mL). Seed crystals were then added, and the mixture was stirred at 20°C for 4 hours. Heptane (264 mL) was added dropwise to the resulting slurry solution over 1 hour. The mixture was stirred at the same temperature for 2 hours. The precipitated crystals were then filtered. The filtered crystals were washed with a mixture of t-butyl methyl ether and heptane (132/88 mL) and dried under reduced pressure at 35°C to give methyl 5-acetamido-4,7, 8,9-tetra-O-acetyl-3, 5-dideoxy-2-O-(2,2,2-trifluoro-N-phenylethanimidoyl)-D-glycero-β-D-galacto-non-2-uropyranosonate (compound VIII-4) (39.5 g, yield 67%) as white crystals.

¹H-NMR (500 MHz, CDCl₃) δ 7.30-7.24 (m, 2H), 7.09 (t, J = 7.4 Hz, 1H), 6.72 (d, J = 8.0 Hz, 2H), 5.76 (d, J = 9.7 Hz, 1H), 5.48-5.45 (m, 1H), 5.30 (td, J = 10.9, 4.8 Hz, 1H), 5.15-5.10 (m, 1H), 4.60 (dd, J = 12.6, 2.3 Hz, 1H), 4.30 (q, J = 10.3 Hz, 1H), 4.23 (dd, J = 10.3, 2.3 Hz, 1H), 4.11 (dd, J = 12.3 Hz, 7.7 Hz, 1H), 3.81 (s, 3H), 2.79 (dd, J = 13.5, 4.9 Hz, 1H), 2.21-2.15 (m, 1H), 2.16 (s, 3H), 2.10 (s, 3H), 2.07 (s, 3H), 1.90 (s, 3H), 1.75 (s, 3H).
¹³C-NMR (125 MHz, CDCl₃) δ 171.0, 170.7, 170.4, 170.2, 170.1, 165.3, 142.6, 141.0 (q, ²J_{C-F} = 36.0 Hz), 128.8, 124.6, 119.0, 115.1 (q, ¹J_{C-F} = 284.4 Hz), 99.7, 73.6, 71.9, 68.3, 68.0, 62.4, 53.1, 48.6, 35.6, 23.0, 20.8, 20.8, 20.7, 20.3. HRMS(ESI⁺)[M+NH₄]⁺ calcd for C₂₈H₃₇F₃N₃O₁₃: 680.2273; found 680.2314.

### Example 50:

### Methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-uropyranosonate (Compound VIII-5)

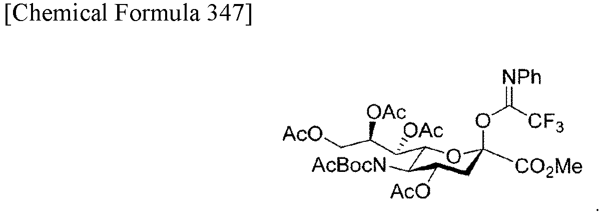

### (Substep 4-6)

To a tetrahydrofuran (390 mL) solution containing methyl 5-acetamido-4,7, 8,9-tetra-O-acetyl-3, 5-dideoxy-2-O-(2,2,2-trifluoro-N-phenylethanimidoyl)-D-glycero-β-D-galacto-non-2-uropyranosonate (compound VIII-4) (39.0 g, 58.86 mmol) were added di-tert-butyl bicarbonate (27.05 g, 123.94 mmol) and dimethylaminopyridine (1.80 g, 14.73 mmol), and the mixture was heated to reflux temperature. The mixture was stirred under reflux for 30 min. After the completion of the reaction was then checked by HPLC, the reaction liquid was concentrated under reduced pressure to 117 mL. Toluene (195 mL) was added to the concentrated solution, and the mixture was concentrated again under reduced pressure to 117 mL. The concentrated solution was filtered using a silica gel-filled funnel (silica gel 60N, 40-50 µm, 117 g; toluene wet packed) and washed with a toluene-ethyl acetate mixture (8/2) (975 mL) to obtain a filtrate. The resulting filtrate was concentrated under reduced pressure (to a weight of 59 g). Cyclopentyl methyl ether (23 mL) was then added. The temperature of the solution was adjusted to 20°C, heptane (156 mL) was added dropwise over 15 minutes, and the mixture was stirred at the same temperature for 1 hour. After the crystals were found to precipitate, heptane (312 mL) was added dropwise over 1 hour. The precipitated crystals were filtered. The filtered crystals were washed with heptane (78 mL) and dried under reduced pressure at 35°C to give methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-uropyranosonate (compound VIII-5) (37.0 g, yield 82%) as white crystals.

### ¹H-NMR (500 MHz, CDCl₃)

Note: Detected as a ca. 1/4 isomer mixture.
Major isomer: δ 7.27 (t, J = 8.9 Hz, 2H), 7.09 (t, J = 7.2 Hz, 1H), 6.73 (d, J = 8.0 Hz, 2H), 5.75-5.65 (m, 1H), 5.31 (d, J = 4.6 Hz, 1H), 5.18-5.14 (m, 1H), 5.15 (d, J = 6.0 Hz, 2H), 4.54 (dd, J = 12.0, 2.0 Hz, 1H), 4.08 (dd, J = 12.6, 6.9 Hz, 1H), 3.84 (s, 3H), 2.90 (dd, J = 13.7, 5.2 Hz, 1H), 2.39 (s, 3H), 2.25 (dd, J = 13.7, 11.2 Hz, 1H), 2.09 (s, 3H), 2.07 (s, 3H), 1.99 (s, 3H), 1.77 (s, 3H), 1.62 (s, 9H). Minor isomer: δ inter alia 6.76 (d, J = 8.0 Hz, 2H), 5.85-5.80 (m, 1H), 5.29-5.25 (m, 1H), 5.22-5.19 (m, 1H), 4.44 (d, J = 11.0 Hz, 1H), 4.15-4.11 (m, 1H), 3.03 (dd, J = 14.0, 5.0 Hz, 1H), 2.41 (s, 3H), 2.12 (s, 3H), 2.00 (s, 3H), 1.88 (s, 3H), 1.74 (s, 3H), 1.54 (s, 9H). ¹³C-NMR (125 MHz, CDCl₃) Mixture: δ 173.7, 170.4, 170.2, 170.0, 169.9, 165.4, 151.7, 142.8, 128.7, 124.5, 119.1, 100.6, 85.2, 72.8, 71.3, 67.7, 65.9, 62.0, 53.1, 52.0, 36.7, 27.9, 27.7, 26.6, 20.8, 20.7, 20.6, 20.3.
HRMS(ESI⁺)[M+NH₄]⁺ calcd for C₃₃H₄₅F₃N₃O₁₅: 780.2797; found 780.2801.

### Example 51:

### Prop-2-en-1-yl 2,3-di-O-benzoyl-6-O-{4,7,8,9-tetra-O-acetyl-5-[acetyl(tert-butoxycarbonyl)amino]-3,5-dideoxy-1-methyl-D-glycero-α-D-galacto-non-2-uropyranosyl}-α-D-galactopyranoside (Compound IX-1)

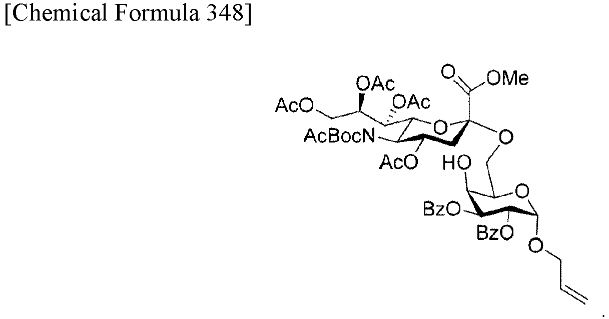

### (Substep 4-7)

A cyclopentyl methyl ether solution containing prop-2-en-1-yl 2,3-di-O-benzoyl-α-D-galactopyranoside (compound VII-3) (quantitative value 31.46 g, 73.43 mmol) was concentrated under reduced pressure to 105 mL. This solution was added to a cyclopentyl methyl ether (175 mL) solution containing methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-uropyranosonate (compound III-5) (35.0 g, 45.89 mmol). Next, cyclopentyl methyl ether was added to the resulting mixed solution, and the total volume was adjusted to 350 mL (cyclopentyl methyl ether mixed solution containing compound VII-3 and compound III-5). Cyclopentyl methyl ether (525 mL) and Molecular Sieves 4A powder (powder particle size: 10 µm or less) (17.5 g) were added to another vessel. After the mixed solution was cooled to -60°C, trimethylsilyl trifluoromethanesulfonate (4.2 mL, 23.24 mmol) was added. To this solution, a cyclopentyl methyl ether mixed solution containing compound VII-3 and compound III-5 was added dropwise over 4.5 hours at -60°C under strong stirring, and the mixture was stirred at the same temperature for 2 hours. After the completion of the reaction was checked by HPLC, triethylamine (4.5 mL, 32.12 mmol) was added. The reaction liquid was heated to 0°C. After that, Celite 545 (35.00 g) was added, and the reaction liquid was filtered and washed with cyclopentyl methyl ether (175 mL). Water (350 mL) was added to the filtrate and the liquid was separated. Next, 0.5 N hydrochloric acid water (350 mL) was added to the organic layer, and the mixture was stirred at 20°C for 2 hours. After the decomposition of byproducts was checked by HPLC, the liquid was separated to obtain an organic layer. The organic layer was washed with water (350 mL) and then 20% brine (175 mL), and concentrated under reduced pressure to 70 mL. Toluene (700 mL) was added to the concentrated solution, and the mixture was concentrated under reduced pressure to 70 mL. The concentrated solution was admixed again with toluene (700 mL) and neutral silica gel (silica gel 60N, 40-50 µm, 158 g) and stirred at 20°C for 30 minutes. The product was adsorbed on silica gel, and then filtered. The silica gel solid phase containing the product was washed with toluene (1575 mL) (the filtrate at the time of the toluene wash was discarded). The target substance was desorbed from silica gel by using ethyl acetate (875 mL). The resulting ethyl acetate solution was concentrated under reduced pressure to 70 mL. Toluene (175 mL) was added, and the mixture was concentrated again under reduced pressure to 70 mL to give a toluene solution containing prop-2-en-1-yl 2,3-di-O-benzoyl-6-O-{4,7,8,9-tetra-O-acetyl-5-[acetyl(tert-butoxycarbonyl)amino]-3,5-dideoxy-1-methyl-D-glycero-α-D-galacto-non-2-uropyranosyl}-α-D-galactopyranoside (compound IX-1). This solution was used in the next step.

### Example 52:

### Prop-2-en-1-yl 6-O-{5-acetamido-4,7,8,9-tetra-O-acetyl-3,5-dideoxy-1-methyl-D-glycero-α-D-galacto-non-2-uropyranosyl}-2,3-di-O-benzoyl-α-D-galactopyranoside (Compound IX-2)

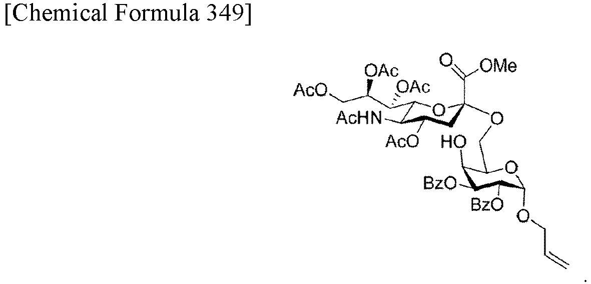

### (Substep 4-8)

To the toluene solution containing prop-2-en-1-yl 2,3-di-O-benzoyl-6-Of 4,7,8,9-tetra-O-acetyl-5-[acetyl(tert-butoxycarbonyl)amino]-3,5-dideoxy-1-methyl-D-glycero-α-D-galacto-non-2-uropyranosyl}-α-D-galactopyranoside (compound IX-1) as so obtained were added dichloromethane (525 mL) and copper(II) trifluoromethanesulfonate (8.30 g, 22.95 mmol). The temperature was raised to 40°C and the mixture was stirred at the same temperature for 2 hours. After the completion of the reaction was then checked by HPLC, the mixture was cooled to 25°C and concentrated under reduced pressure to 70 mL. The concentrated solution was admixed with ethyl acetate (525 mL), and washed three times with 5% brine (350 mL). Heptane (263 mL) was then added to the organic layer, and the mixture was washed four times with 20% methanol water (525 mL). HPLC was used to check whether impurities derived from the β-eliminated form of compound 8, a byproduct of the glycosylation reaction, were removed into the aqueous layer. The organic layer was then concentrated under reduced pressure to 70 mL. Isopropenyl acetate (525 mL) was added to the concentrated solution, and the mixture was concentrated under reduced pressure to 350 mL to give an isopropenyl acetate solution containing prop-2-en-1-yl 6-O- { 5-acetamido-4,7,8,9-tetra-O-acetyl-3,5-dideoxy-1-methyl-D-glycero-α-D-galacto-non-2-uropyranosyl}-2,3-di-O-benzoyl-α-D-galactopyranoside (compound IX-2). This solution was used in the next step.

### Example 53:

### Prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-α-D-galactopyranoside (Compound IX-3)

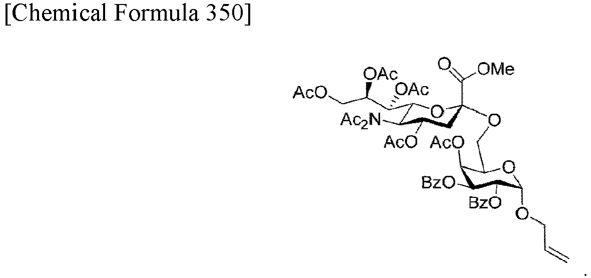

### (Substep 4-9)

To the isopropenyl acetate solution containing prop-2-en-1-yl 6-O-{5-acetamide-4,7,8,9-tetra-O-acetyl-3,5-dideoxy-1-methyl-D-glycero-α-D-galacto-non-2-uropyranosyl}-2,3-di-O-benzoyl-α-D-galactopyranoside (compound IX-2) as so obtained was added paratoluenesulfonic acid monohydrate (0.88 g, 4.62 mmol). The temperature was raised to reflux temperature (internal temperature at or near 90°C) and the mixture was stirred at the same temperature for 3 hours. After the completion of the reaction was checked by HPLC, the mixture was cooled to 25°C. Triethylamine (0.95 mL, 6.85 mmol) was added, and the mixture was concentrated under reduced pressure to 70 mL. Toluene (350 mL) was added to the concentrated solution, and the mixture was concentrated again under reduced pressure to 70 mL. The concentrated solution was admixed with toluene (630 mL) and then neutral silica gel (silica gel 60N, 40-50 µm, 123 g). The mixture was stirred at the same temperature for 30 minutes. The product was adsorbed on the silica gel, and then filtered. The silica gel solid phase containing the product was washed with toluene (1925 mL) and then a toluene/ethyl acetate mixture (97/3, 1400 mL) (the filtrate at the time of the wash was discarded). The target substance was desorbed from the silica gel solid phase containing the product by using ethyl acetate (1050 mL). The resulting ethyl acetate solution was concentrated under reduced pressure to give prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-α-D-galactopyranoside (compound IX-3) (30.1 g, yield 67% (in terms of compound VIII-5)) as a white foamy solid (containing 0.42 equivalents of toluene (about 4% by weight)).

¹H-NMR (500 MHz, CDCl₃) δ 7.99 (d, J = 8.4 Hz, 2H), 7.88 (dd, J = 8.4, 1.4 Hz, 2H), 7.53-7.47 (m, 2H), 7.37 (dt, J = 14.7, 6.9 Hz, 4H), 5.90-5.82 (m, 1H), 5.82 (dd, J = 10.9, 3.4 Hz, 1H), 5.73 (d, J = 2.9 Hz, 1H), 5.58 (dd, J = 10.9, 4.0 Hz, 1H), 5.51 (td, J = 10.6, 5.0 Hz, 1H), 5.35-5.30 (m, 3H), 5.17-5.15 (m, 2H), 4.94 (dd, J = 10.3, 1.7 Hz, 1H), 4.38-4.27 (m, 3H), 4.20-4.13 (m, 2H), 4.08 (dd, J = 13.2, 5.7 Hz, 1H), 3.94 (dd, J = 10.3, 6.3 Hz, 1H), 3.82 (s, 3H), 3.50 (dd, J = 9.7, 7.4 Hz, 1H), 2.73 (dd, J = 13.2, 5.2 Hz, 1H), 2.37 (s, 3H), 2.31 (s, 3H), 2.19 (s, 3H), 2.15 (s, 3H), 2.14 (s, 3H), 2.03 (s, 3H), 1.97 (s, 3H), 1.86 (dd, J = 13.2, 10.9 Hz, 1H). ¹³C-NMR (125 MHz, CDCl₃) δ 174.5, 173.6, 170.5, 170.1, 169.9, 169.8, 169.6, 167.3, 166.0, 165.5, 133.5, 133.3, 133.1, 129.8, 129.5, 129.4, 128.4, 128.3, 117.5, 98.6, 95.5, 77.2, 69.8, 68.9, 68.6, 68.6, 68.3, 68.3, 67.5, 67.0, 66.7, 62.4, 61.8, 57.0, 52.9, 38.7, 27.9, 25.9, 21.0, 20.9, 20.7, 20.7, 20.6.
HRMS(ESI⁺)[M+H]⁺ calcd for C₄₇H₅₆NO₂₂: 986.3288; found 986.3277.

The obtained compound was found to correspond to the spectrum in the following literature: Reference 4) J. Org. Chem., 2016, 81, 10600-10616.

### Example 54-1:

### 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (Compound IX-4)

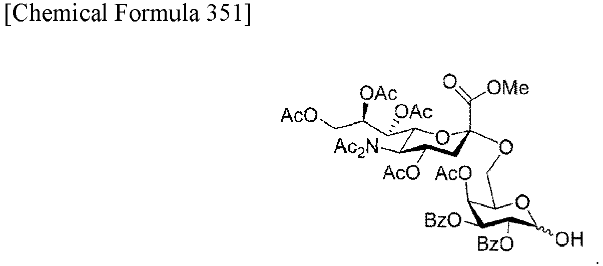

### (Substep 4-10)

The pressure of a methanol solution (290 mL) containing prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-α-D-galactopyranoside (compound IX-3) (29.00 g, 29.41 mmol), 1,3-dimethylbarbituric acid (9.19 g, 58.86 mmol), and triphenylphosphine (2.31 g, 8.81 mmol) was reduced. The solution was subjected to nitrogen substitution. This operation was repeated five times for deaeration. Palladium(II) acetate (0.66 g, 2.94 mmol) was then added, and the mixture was stirred at 40°C for 12 hours. After the completion of the reaction was checked by HPLC, toluene (580 mL) and water (1015 mL) were added. The resulting liquid was separated to obtain an organic layer. The organic layer was washed four times with 20% methanol water (580 mL) to remove 1,3-dimethylbarbituric acid into the aqueous layer. The organic layer was concentrated under reduced pressure to 58 mL, and toluene (435 mL) was added. The mixture was concentrated again under reduced pressure to 58 mL. The concentrated solution was admixed with toluene (383 mL), chloroform (197 mL), and neutral silica gel (silica gel 60N, 40-50 µm, 145 g). The mixture was stirred for 30 minutes. The product was adsorbed on the silica gel, and then filtered. The silica gel solid phase containing the product was washed with toluene/chloroform mixture (2/1, 4350 mL) (the filtrate at the time of the wash was discarded). The target substance was desorbed from the silica gel solid phase containing the product by using ethyl acetate (870 mL). SH silica gel (29.00 g) was added to the resulting ethyl acetate solution. The mixture was stirred for 30 minutes, filtered, and then washed with ethyl acetate (145 mL) to obtain an ethyl acetate solution containing the target product. The resulting solution was concentrated under reduced pressure to 58 mL, and toluene (145 mL) was added. The mixture was concentrated again under reduced pressure to 58 mL. The concentrated solution was purified by silica gel column purification (silica gel 60N, 40-50 µm, 290 g; mobile phase hexane/ethyl acetate 50/50 to 30/70) and the selected fractions were concentrated under reduced pressure to 29 mL. The concentrated solution was admixed with ethyl acetate (290 mL) and activated carbon (Shirasagi A, 14.5 g), stirred for 30 minutes, filtered, and then washed with ethyl acetate (87 mL) to obtain a purified ethyl acetate solution containing the target product. The resulting solution was concentrated under reduced pressure to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (compound IX-4) (18.70 g, 67% yield) as a white foamy solid.

¹H-NMR (500 MHz, CDCl₃) Major isomer: δ 7.98-8.03 (m, 2H), 7.89 (t, 2H, J = 8.9 Hz), 7.52-7.48 (m, 2H), 7.39-7.34 (m, 4H), 5.92 (dd, 1H, J = 10.3, 2.9 Hz), 5.82 (d, 1H, J = 3.4 Hz), 5.68 (t, 1H, J = 2.3 Hz), 5.59-5.48 (m, 2H), 5.37 (td, 1H, J = 7.5, 2.5 Hz), 5.17 (d, 1H, J = 7.5 Hz), 5.02 (d, 1H, J = 10.5 Hz), 4.74-4.71 (m, 1H), 4.44-4.38 (m, 1H), 4.16-4.08 (m, 2H), 3.85 (s, 3H), 3.80 (m, 1H), 3.60-3.54 (m, 1H), 2.76 (dd, 1H, J = 13.0, 6.0 Hz), 2.38 (s, 3H), 2.33 (s, 3H), 2.31 (s, 3H), 2.15 (s, 3H), 2.12 (s, 3H), 2.04 (s, 3H), 1.98 (s, 3H), 1.93-1.87 (m, 1H). ¹³C-NMR (125 MHz, CDCl₃) α/β mixture: 174.5, 173.8, 173.6, 171.7, 171.0, 170.5, 170.3, 170.3, 169.9, 169.8, 169.8, 169.6, 167.6, 167.3, 166.3, 166.0, 165.6, 165.4, 133.3, 133.2, 133.2, 133.1, 129.8, 129.7, 129.5, 129.4, 129.4, 129.3, 129.0, 128.4, 128.3, 99.1, 98.8, 95.9, 91.0, 72.2, 71.6, 71.5, 69.9, 69.9, 69.3, 69.3, 68.8, 68.5, 68.1, 67.5, 67.5, 67.3, 67.0, 66.6, 62.9, 62.6, 62.4, 60.3, 57.2, 56.8, 53.0, 52.9, 38.6, 38.3, 27.9, 27.8, 26.1, 25.7, 21.0, 20.9, 20.8, 20.7, 20.7, 20.6, 20.5.
HRMS(ESI⁻)[M+HCOO]⁻ calcd for C₄₅H₅₂NO₂₄: 990.2885; found 990.2873.

### Example 54-2:

### Process for purifying 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (compound IX-4) by crystallization

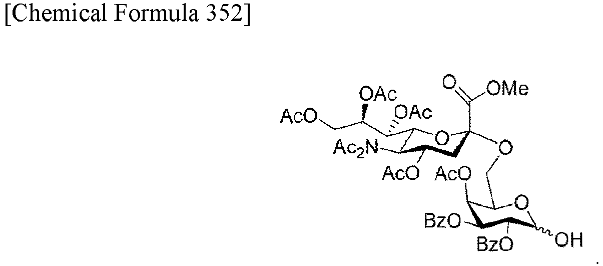

First, 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (compound IX-4) (3.00 g, 3.17 mmol; sialyl moiety α/β ratio = 95.7/4.3) was dissolved in ethyl acetate (4 mL). Next, 2-propanol (60 mL) was added, and the mixture was stirred at 25°C and then concentrated under reduced pressure to 18 mL. The slurry solution was stirred at 0°C for 3 hours, and the precipitated crystals were then filtered. The filtered crystals were washed with cold 2-propanol (9 mL) and dried under reduced pressure at 40°C to give 4-O-acetyl-2,3-di-O-benzoyl-6-O-[4,7, 8,9-tetra-O-acetyl-3, 5-dideoxy-5-(diacetylamino)-1-methyl-D-glycero-α-D-galacto-non-2-uropyranosyl]-D-galactopyranose (compound IX-4) (2.66 g, yield 88.7%; sialyl moiety α/β ratio => 99.9/N.D.) as white crystals.

### [Analytical conditions].

Column: CAPCELL PAK ADME ϕ4.6 × 150 mm, film thickness 3 µm
Wavelength: 220 nm
Oven: 40°C
Eluent: (A) 0.1% aqueous trifluoroacetic acid solution, (B) acetonitrile
Gradient: 0-150 min (B) conc. 40%
   150.1 min (B) conc. 95%
   155 min (B) conc. 95%
   155.1 min (B) conc. 40%
   160 min (B) conc. 40%
Flow rate: 1 mL/min
Injection: 5 µL
Sialyl moiety α-form: 131.0 min, Sialyl moiety β-form: 126.2 min

### Example 55:

### 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (Compound IX-5)

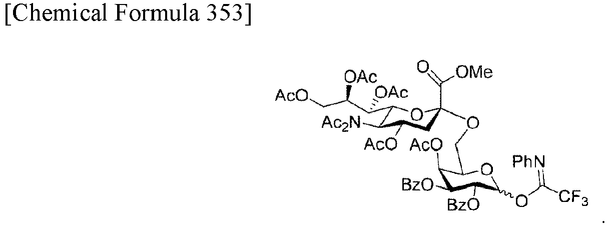

### (Substep 4-11)

To an acetone solution (500 mL) containing 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (compound IX-4) (42.9 g, 45.4 mmol) were added 2,2,2-trifluoro-N-phenylacetimidoyl chloride (19.0 g, 91.5 mmol) and potassium carbonate (19.0 g, 137 mmol). The mixture was stirred at room temperature for 18 hours. The slurry solution was filtered, washed with a small amount of acetone, and then concentrated under reduced pressure. The concentrated solution was purified by silica gel column purification (silica gel 60N, 40-50 µm, 400 g; mobile phase hexane/ethyl acetate 75/25 to 30/70). The selected fractions were concentrated under reduced pressure to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (compound IX-5) (49.5 g, yield 98%) as a white foamy solid.

¹H-NMR (500 MHz, CDCl₃) δ 7.99 (dd, 2H, J = 8.3, 1.4 Hz), 7.91-7.88 (m, 2H), 7.59-7.56 (m, 1H), 7.51 (tt, 1H, J = 9.7, 2.4 Hz), 7.44-7.34 (m, 4H), 7.12 (t, 2H, J = 7.7 Hz), 7.01 (t, 1H, J = 7.4 Hz), 6.82 (brs, 1H), 6.43 (brs, 2H), 5.87-5.77 (m, 3H), 5.52 (td, 1H, J = 11.0, 5.0 Hz), 5.38-5.34 (m, 1H), 5.18 (dd, 1H, J = 8.6, 1.7 Hz), 4.95 (dd, 1H, J = 10.0, 2.0 Hz), 4.53 (brs, 1H), 4.29 (dd, 1H, J = 12.6, 2.9 Hz), 4.21-4.09 (m, 3H), 4.04 (dd, 1H, J = 10.0, 6.0 Hz), 3.84 (s, 3H), 3.53 (dd, 1H, J = 10.3, 7.4 Hz), 2.76 (dd, 1H, J = 12.9, 5.4 Hz), 2.39 (s, 3H), 2.31 (s, 3H), 2.18 (s, 3H), 2.17 (s, 3H), 2.14 (s, 3H), 2.02 (s, 3H), 1.98 (s, 3H), 1.86 (dd, 1H, J = 13.0, 11.0 Hz). ¹³C-NMR (125 MHz, CDCl₃) δ 174.5, 173.5, 170.5, 170.1, 169.8, 169.7, 169.6, 167.2, 165.5, 165.4, 165.3, 142.9, 133.6, 133.3, 129.8, 129.7, 129.6, 129.5, 129.0, 128.7, 128.6, 128.5, 128.4, 124.2, 119.0, 98.7, 98.5, 70.4, 69.8, 68.3, 68.2, 67.5, 67.5, 66.9, 66.7, 62.0, 61.7, 60.3, 56.9, 53.7, 52.9, 38.7, 31.7, 29.2, 27.9, 26.0, 25.8, 21.0, 21.0, 20.9, 20.8, 20.7, 20.6, 20.5.
HRMS(ESI⁺)[M+NH₄]⁺ calcd for C₅₂H₅₉F₃N₃O₂₂: 1134.3537; found 1134.3564.

The obtained compound was found to correspond to the spectrum in the following literature: Reference 4) J. Org. Chem., 2016, 81, 10600-10616.

### <Synthesis of compound XI>

Compound XI was synthesized according to the following synthesis scheme 5A.

### [Synthesis Scheme 5A]

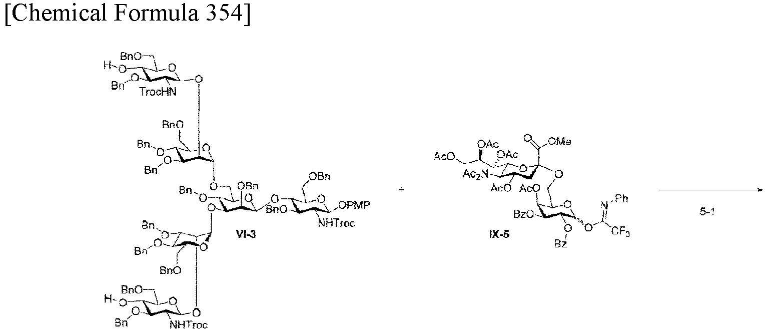

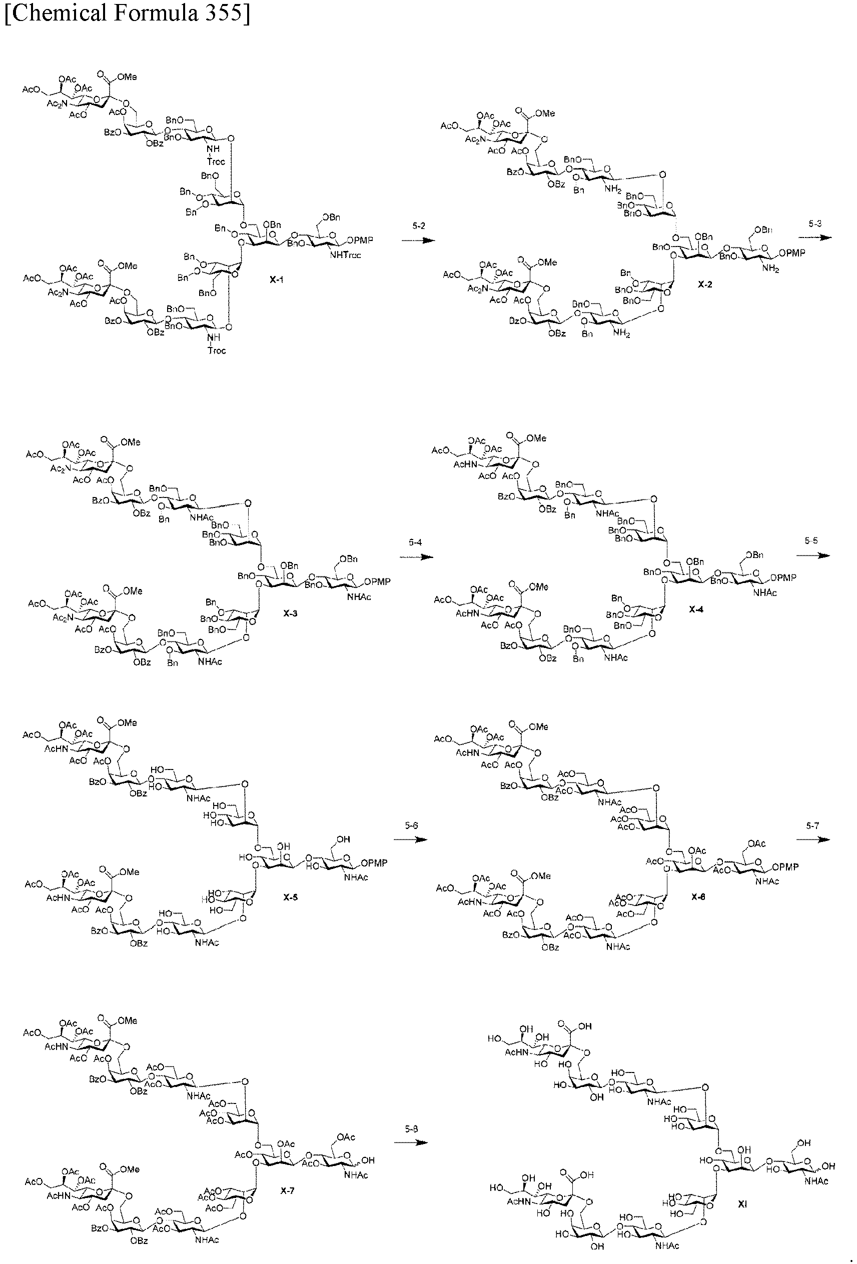

### Example 56:

### 4-Methoxyphenyl 4,7,8,9-tetra-O-acetyl-3,5-dideoxy-5-(N,N-diacetylamino)-1-O-methyl-D-glycero-α-D-galacto-non-2-uropyranosyl-(2→6)-4-O-acetyl-2,3-di-O-4-methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-{ [(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranoside (Compound X-1)

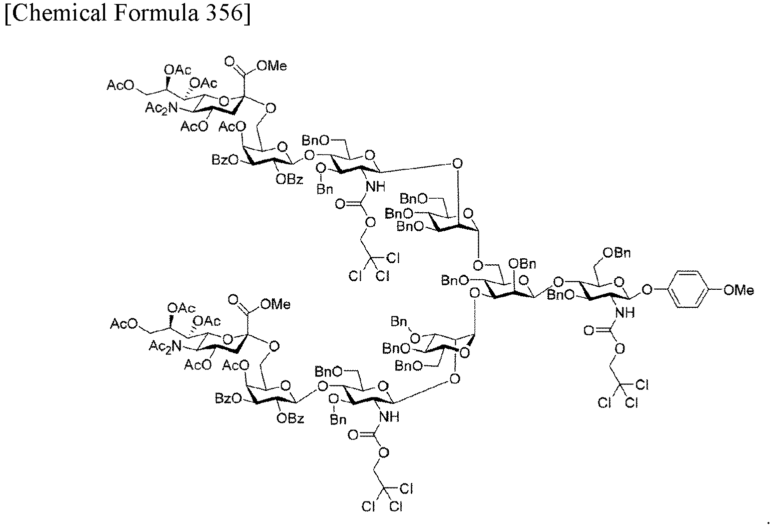

### (Substep 5-1A)

To a dichloromethane solution (17 mL) containing compound VI-3 (556 mg, 0.19 mmol) and compound IX-5 (646.0 mg, 0.58 mmol) was added Molecular Sieves 4A (111.1 mg, 0.2 wt). The mixture was then cooled to 0°C. After stirring for 1 hour, a dichloromethane solution containing t-butyldimethylsilyl trifluoromethanesulfonate (0.3 M, 100 µL, 0.03 mmol) was added. After stirring for 60 minutes, a dichloromethane solution containing t-butyldimethylsilyl trifluoromethanesulfonate (0.3 M, 33 µL, 0.01 mmol) was added. After 20 minutes, a dichloromethane solution containing t-butyldimethylsilyl trifluoromethanesulfonate (0.3 M, 33 µL, 0.01 mmol) was further added. The resulting reaction liquid was admixed with compound IX-5 (43.1 mg, 0.04 mmol) and a dichloromethane solution containing t-butyldimethylsilyl trifluoromethanesulfonate (0.3 M, 33 µL, 0.01 mmol), and stirred for 3 hours. Triethylamine (27 µL, 0.19 mmol) was then added and the temperature was raised to room temperature. The Molecular Sieves 4A was filtered off from the reaction liquid, and washed with dichloromethane (2.2 mL). The resulting solution was concentrated to dryness, and the crude product was purified by reverse phase chromatography (acetonitrile/water 93% -> 100%) to afford compound X-1 (836.2 mg, yield 92%).

¹H-NMR (500 MHz, CD₂Cl₂) δ 1.79 (dd, J = 11.5, 13.5 Hz, 1H), 1.85 (s, 3H), 1.87 (brs, 6H), 1.89 (brs, 6H), 1.92 (brs, 3H), 2.00 (brs, 3H), 2.01 (s, 3H), 2.02 (s, 3H), 2.03 (s, 3H), 2.18 (s, 3H), 2.20 (s, 3H), 2.23 (s, 3H), 2.25 (s, 3H), 2.38 (br, 1H), 2.55 (br, 1H), 2.65 (dd, J = 4.5, 13.5 Hz, 1H), 3.00-4.07 (m, 40H), 3.63 (s, 3H), 3.77 (s, 6H), 4.08-5.00 (m, 51H), 5.02-5.10 (m, 2H), 5.15-5.30 (m, 4H), 5.32-5.46 (m, 2H), 5.50-5.65 (m, 4H), 5.92 (br, 1H), 6.76 (d, J = 8.0 Hz, 2H), 6.80-7.50 (m, 84H), 7.66 (br, 2H), 7.80 (d, J = 8.0 Hz, 2H), 7.83 (d, J = 7.5 Hz, 2H), 7.89 (d, J = 8.0 Hz, 2H). ¹³C-NMR (125 MHz, CD₂Cl₂) δ 20.5, 20.7, 20.8, 20.9, 21.08, 21.15, 21.2, 26.1, 26.2, 28.1, 30.0, 38.8, 38.9, 53.1, 53.2, 53.4, 53.6, 53.8, 54.0, 54.2, 55.7, 57.0, 57.2, 57.3, 57.6, 62.1, 62.2, 62.3, 66.7, 66.8, 67.45, 67.49, 67.57, 68.6, 69.0, 69.7, 70.3, 70.4, 70.7, 71.1, 72.1, 72.2, 72.3, 72.5, 72.7, 73.0, 73.3, 73.4, 73.5, 73.7, 73.8, 73.9, 74.0, 74.3, 74.6, 74.8, 75.1, 75.2, 77.2, 78.2, 78.3, 78.6, 80.3, 96.2, 99.2, 99.3, 99.8, 100.2, 100.6, 101.3, 114.8, 118.4, 126.1, 127.5, 127.71, 127.76, 127.79, 127.86, 127.89, 127.95, 128.1, 128.32, 128.39, 128.44, 128.52, 128.57, 128.7, 128.80, 128.82, 128.86, 128.90, 128.96, 129.03, 129.55, 129.66, 129.75, 129.83, 130.0, 133.65, 133.72, 133.8, 138.4, 138.6, 138.82, 138.88, 138.92, 139.0, 139.17, 139.21, 139.3, 139.8, 151.9, 154.2, 154.3, 154.5, 155.6, 165.1, 165.3, 165.5, 165.7, 167.7, 167.8, 169.86, 169.92, 170.04, 170.1, 170.2, 170.3, 170.4, 170.76, 170.81, 173.9, 174.81, 174.85.
HRMS(ESI)[M+3NH₄]³⁺(m/z): calcd for C₂₃₈H₂₆₈Cl₉N₈O₇₇: 1596.8163; found 1596.8134 [M+H]⁺).

### (Substep 5-1B)

First, 4-methoxyphenyl 3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→3)-[3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranoside (compound VI-3) (279 mg, 97 µmol) and 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (compound IX-5) (320 mg, 0.291 mmol) were added to a 30-mL recovery flask. Dichloromethane (4.2 mL) and Molecular Sieves 4A powder (42 mg) were then added. Subsequently, tert-butyldimethylsilyl trifluoromethanesulfonate (4.5 µL, 19 µmol) was added dropwise over 5 minutes at -20°C under nitrogen, and the mixture was stirred at -20 to 0°C for 1 hour. After the completion of the reaction was checked by HPLC, triethylamine (5.4 µL, 38 µmol) was added. The mixture was stirred at room temperature for 1 hour. The reaction liquid was filtered through Celite and washed with dichloromethane (1.4 mL). The filtrate was liquid-separated twice with water (2.8 mL). The organic layer was dried over sodium sulfate, filtered, and then concentrated. The concentrated residue was purified by column chromatography (silica amount: 10 g; developing solvent: toluene/ethyl acetate = 9/1 to 3/1). The main residue was concentrated under reduced pressure to give 4-methoxyphenyl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl D-galactopyranosyl-(1→4)-3,6-di-O-benzyl-2-deoxy-2-{ [(2,2,2-trichloroethoxy)carbonyl]amino}-β-D-glucopyranosyl-(1→2)-3,4,6-tri-O-benzyl-α-D-mannopyranosyl-(1→3)-[4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-β-D-galactopyranosyl-(1→4)-3,6-di-O-benzyl-2-deoxy-2-{[(2,2,2-trichloroethoxy)carbonyl]amino}-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-{[(2,2,2-trichloroethoxy)carbonyl]amino}-β-D-glucopyranoside (Compound X-1) (450 mg, isolation yield 98%) as a white amorphous product.

¹H-NMR (500 MHz, CDCl₃) δ 8.01-7.74 (m, 10H), 7.53-6.97 (m, 82H), 6.80 (d, J = 9.2 Hz, 2H), 5.68-5.58 (m, 5H), 5.50-5.25 (m, 7H), 5.16-4.26 (m, 46H), 4.20-3.16 (m, 56H), 2.79-2.50 (m, 4H), 2.37-2.28 (m, 15H), 2.23-1.96 (m, 36H), 1.85-1.79 (m, 2H).
HRMS(ESI⁺)[M+Et₃N+H]⁺ calcd for C₂₄₄H₂₇₂Cl₉N₆O₇₇: 4838.4752; found 4838.3530.

¹H-NMR was found to correspond to compound X-1 synthesized in a different route.

### Example 57:

### 4-Methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-b enzoyl-β-D-galactopyranosyl-(1→4)-3,6-di-O-benzyl-2-deoxy-2-amino-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-amino-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-amino-β-D-glucopyranoside (Compound X-2)

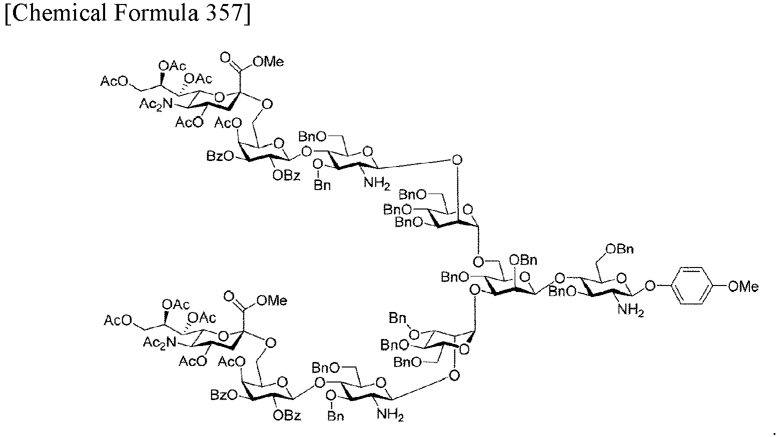

### (Substep 5-2)

To a tetrahydrofuran/acetic acid solution (1:1, 1.9 mL) containing compound X-1 (80 mg, 16.9 µmol) was added zinc powder (33.1 mg, 0.51 mmol). After stirring for 24 hours, the insoluble material was filtered off, and the solution obtained after washed with tetrahydrofuran (3 mL) was concentrated to dryness to obtain compound X-2 as a crude product.

### Compound X-2: MS(ESI)(m/z): 1404 ([M+3H]³⁺)

### Example 58:

### 4-Methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-(acetylamino)-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-(acetylamino)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(acetylamino)-β-D-glucopyranoside (Compound X-3)

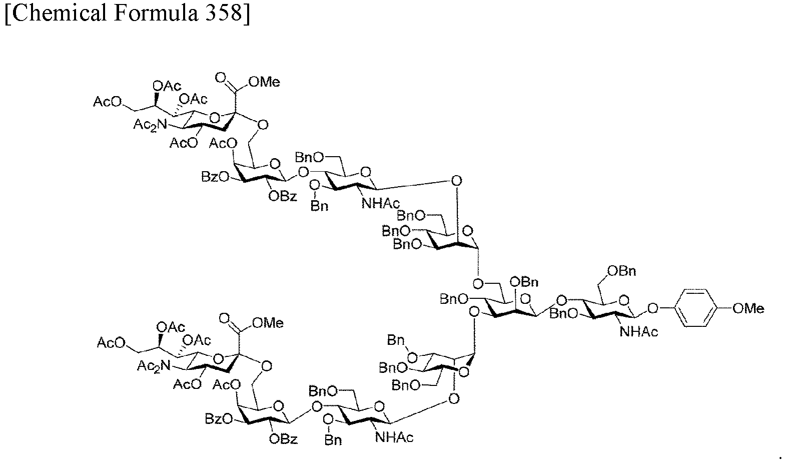

### (Substep 5-3)

The crude compound X-2 was admixed with tetrahydrofuran (0.8 mL), triethylamine (28.8 µL, 0.20 mmol) and acetic anhydride (16.0 µL, 0.17 mmol) in this order. After stirring for 2 hours, ethyl acetate (5 mL) and water (3 mL) were added. The aqueous layer was removed. The resulting organic layer was washed twice with 5% sodium bicarbonate water (3 mL) and further washed with water (3 mL), and the organic layer was concentrated to dryness to afford crude compound X-3 (91.2 mg). Here, 28.5 mg of this sample (equivalent to 25 mg of compound X-1) was used for the next step.

### Example 59:

### 4-Methoxyphenyl 4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-(acetylamino)-β-D-glucopyranosyl-(1→2)-3,4,6-tri-O-benzyl-α-D-mannopyranosyl-(1→3)-[4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-(acetylamino)-β-D-glucopyranosyl-(1→2)-3,4,6-tri-O-benzyl-a-D-mannopyranosyl-(1→6)]-2,4-di-O-benzyl-β-D-mannopyranosyl-(1→4)-3,6-di-O-benzyl-2-deoxy-2-(acetylamino)- β-D-glucopyranoside (Compound X-4)

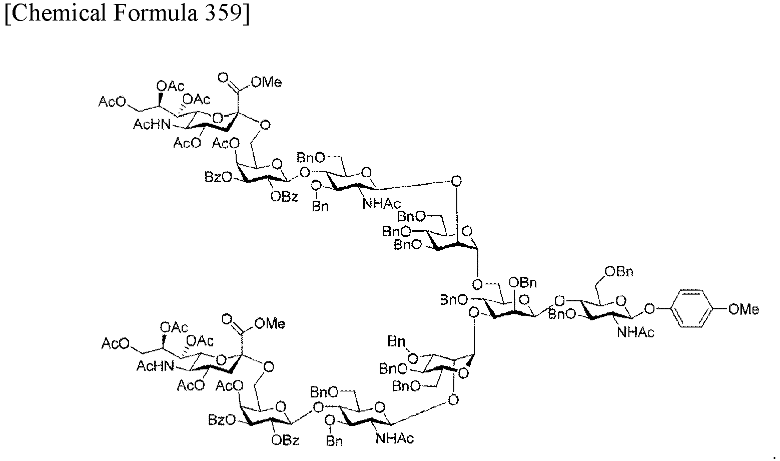

### (Substep 5-4)

To a tetrahydrofuran solution (0.25 mL) containing the crude compound X-3 (28.5 mg, 5.3 µmol) was added a tetrabutylammonium fluoride-containing tetrahydrofuran solution (1.0 M, 15.9 µL, 15.9 µmol) at 0°C. The temperature was raised to room temperature and the mixture was stirred for 2 hours. A tetrabutylammonium fluoride-containing tetrahydrofuran solution (1.0 M, 26.5 µL, 26.5 µmol) was added, and the mixture was further stirred for 3 hours. Then, acetic acid (2 µL), triethylamine (5 µL), and acetic anhydride (2 µL) were added sequentially, and the mixture was stirred at room temperature for 1.5 hours. Ethyl acetate (5 mL) and water (2 mL) were added to the resulting reaction liquid, and the aqueous layer was then removed. The resulting organic layer was washed with 5% sodium bicarbonate water (3 mL) and water (3 mL) in this order. Subsequently, the organic layer was concentrated to dryness. The crude product was purified by preparative HPLC (acetonitrile/water 80% -> 100%) to afford compound X-4 (17.7 mg, yield 77%).

MS(ESI)(m/z): 2128 ([M+2H]²⁺).

### Example 60:

### 4-Methoxyphenyl 4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1→4)-2-deoxy-2-(acetylamino)-β-D-glucopyranosyl-(1→2)-α-D-mannopyranosyl-(1→3)-[4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1→4)-2-deoxy-2-(acetylamino)-β-D-glucopyranosyl-(1→2)-α-D-mannopyranosyl-(1→6)]-β-D-mannopyranosyl-(1→4)-2-deoxy-2-(acetylamino)-β-D-glucopyranoside (Compound X-5)

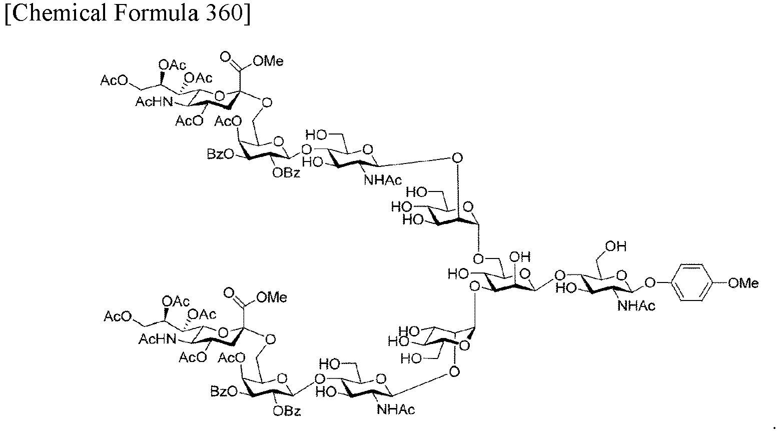

### (Substep 5-5)

To a tetrahydrofuran solution (0.46 mL) containing compound X-4 (15.2 mg, 3.57 µmol) were added palladium carbon (30.4 mg, 2.0 Wt) and acetic acid (18.5 µL). After stirring at room temperature, the mixture was stirred under hydrogen pressure (+0.4 MPa) for 5 days. HPLC analysis demonstrated the formation of compound X-5 (compound X-5: 34 PA%, containing an excessively reduced product in which the Bz group portion of 58%PA appears to have been nuclear reduced).

### Compound X-5: MS(ESI)(m/z): 1496 ([M+2H]²⁺).

Excessively reduced product: MS(ESI)(m/z):1499 ([M+2H]²⁺, MS(ESI)(m/z): 1502 ([M+2H]²⁺).

### Example 61:

### 4-Methoxyphenyl 4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-acetyl-2-deoxy-2-(acetylamino)-β-D-glucopyranosyl-(1→2)-α-3,4,6-tri-O-acetyl-D-mannopyranosyl-(1→3)-[4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-acetyl-2-deoxy-2-(acetylamino)-β-D-glucopyranosyl-(1→2)-3,4,6-tri-O-acetyl-α-D-mannopyranosyl-(1→6)]-2,4-di-O-acetyl-β-D-mannopyranosyl-(1→4)-3,6-di-O-acetyl-2-deoxy-2-(acetylamino)-β-D-glucopyranoside (Compound X-6)

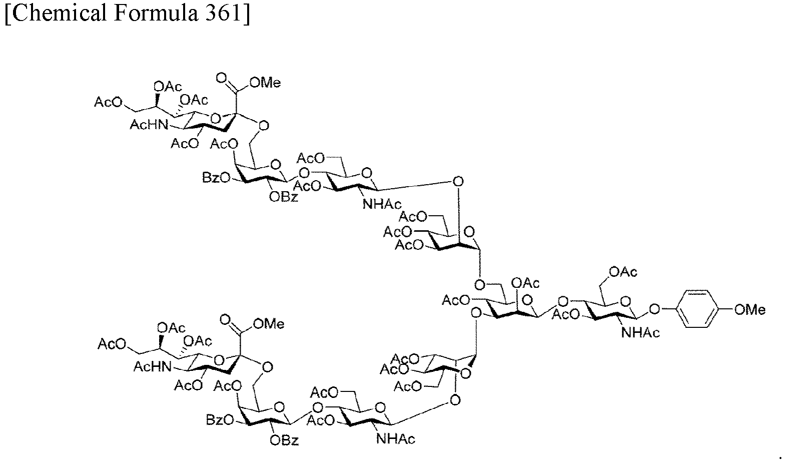

### (Substep 5-6)

The reaction liquid containing compound X-5 was subjected to nitrogen substitution. The resulting suspension was then admixed with triethylamine (30 µL, 0.21 mmol), acetic anhydride (10 µL, 0.11 mmol), and dimethylaminopyridine (0.1 mg) at 0°C. The temperature was raised to room temperature and the mixture was stirred for 19 hours. Triethylamine (200 µL) and acetic anhydride (150 µL) were then added, and the mixture was further stirred for 7 hours. After the insoluble material was filtered off, 5% sodium bicarbonate water (5 mL) was added to the solution obtained by washing with ethyl acetate (8 mL). After the aqueous layer was removed, the organic layer was washed sequentially with water (2 mL), 10% aqueous ammonium chloride solution (3 mL), and water (2 mL). Subsequently, the resulting organic layer was concentrated to dryness. The crude product was purified by preparative HPLC (acetonitrile/water 80% -> 100%) to afford compound X-6 (11.8 mg (35%PA, containing an excessively reduced product in which the Bz group portion of 65%PA appears to have been nuclear reduced).

### Compound X-6: MS(ESI)(m/z): 1790 ([M+2H]²⁺).

Excessively reduced product: MS(ESI)(m/z): 1793 ([M+2H]²⁺), MS(ESI)(m/z): 1796 ([M+2H]²⁺).

### Example 62:

### 4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1→4)-3, 6-di-O-acetyl-2-deoxy-2-(acetylamino)-β-D-glucopyranosyl-(1→2)-α-3,4,6-tri-O-acetyl-D-mannopyranosyl-(1→3)-[4,7,8,9-tetra-O-acetyl-3,5-dideoxy-5-(acetylamino)-1-O-methyl-D-glycero-α-D-galacto-non-2-uropyranosyl-(2→6)-4-O-acetyl-2,3-di-O-benzoyl-β-D-galactopyranosyl-(1->4)-3,6-di-O-acetyl-2-deoxy-2-(acetylamino)-β-D-glucopyranosyl-(1→2)-3,4,6-tri-O-acetyl-α-D-mannopyranosyl-(1→6)]-2,4-di-O-acetyl-β-D-mannopyranosyl-(1→4)-3,6-di-O-acetyl-2-deoxy-2-(acetylamino)-D-glucopyranoside (Compound X-7)

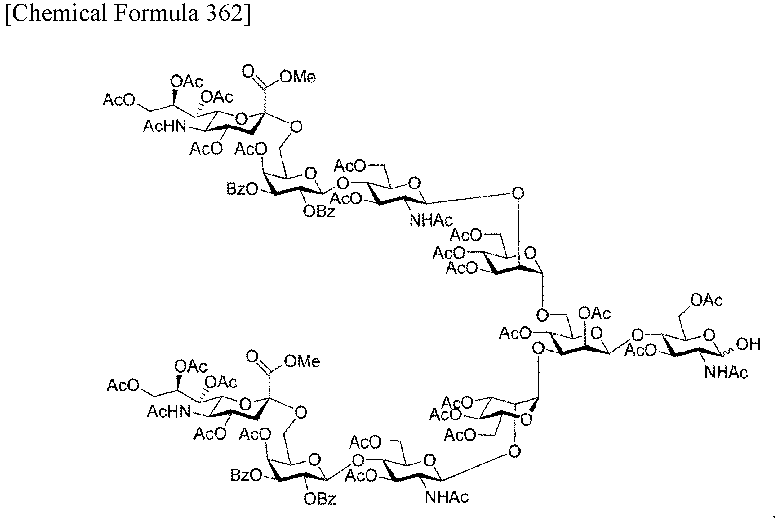

### (Substep 5-7)

To an acetonitrile/aqueous solution (20:1, 0.71 mL) containing compound X-6 (11.8 mg) was added cerium ammonium nitrate (15.2 mg, 3.57 µmol) at 0°C. After stirring for 2 hours, ethyl acetate (5 mL) and water (2 mL) were added. After the aqueous layer was removed, the organic layer was washed twice with water (2 mL), twice with 5% sodium sulfite (2 mL), and once with water (2 mL). The resulting organic layer was concentrated to dryness to give the crude compound X-7. This sample was used for the reaction in the next step.

### Example 63:

O-(N-Acetyl-α-neuraminosyl)-(2→6)-O-β-D-galactopyranosyl-(1→4)-O-2-(acetylamino)-2-deoxy-β-D-glucopyranosyl-(1→2)-O-α-D-mannopyranosyl-(1→3)-O-[O-(N-acetyl-α-neuraminosyl)-(2→6)-O-β-D-galactopyranosyl-(1→4)-O-2-(acetylamino)-2-deoxy-β-D-glucopyranosyl-(1→2)-α-D-mannopyranosyl-(1→6)]-O-β-D-mannopyranosyl-(1→4)-2-(acetylamino)-2-deoxy-D-glucose (Compound XI)

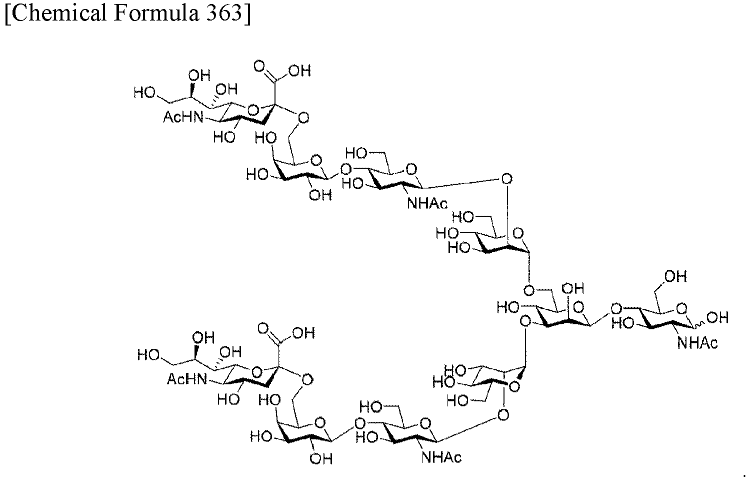

### (Substep 5-8)

To a methanol (0.5 mL) solution containing the crude compound X-7 obtained in Example 62 was added a 28% sodium methoxide-containing methanol solution (8.6 µL). After stirring at room temperature for 13 hours, the reaction liquid was concentrated to dryness. The crude material obtained was then dissolved in water (0.2 mL). This solution was purified with Hitrap(TM) Desalting 5 mL (water: 100%). The resulting fraction was admixed with an aqueous sodium hydroxide solution (4 M, 3.3 µL, 13.2 µmol) and methanol (3 mL). The mixture was then concentrated to dryness at 25°C or below to give compound XI (5.41 mg, yield 92%).

¹H-NMR (500 MHz, D₂O+NaOD) δ 1.57 (t, J = 12.0 Hz, 2H), 1.85 (s, 6H), 1.90 (brs, 9H), 2.53 (dd, 1H), 2.65 (dd, J = 4.0, 12.0 Hz, 1H), 3.30-3.85 (m, 62H), 3.98 (brs, 1H), 4.06 (brs, 1H), 4.12 (d, J = 8.5 Hz, 1H), 4.30 (d, J = 8.0 Hz, 2H), 4.47 (brs, 1H), 4.49 (brs, 1H), 5.14 (d, J = 4.0 Hz, 1H).
MS(ESI)(m/z): 1009 ([M-2H]²⁻).

For the obtained product, ¹H-NMR (500 MHz) was measured using a standard (disialyl octasaccharide (TCI, D4065)) under the same conditions, and a match was confirmed.

### <Synthesis of compound X-3>

Compound X-3 was synthesized according to the following synthesis scheme 5B.

### [Synthesis Scheme 5B]

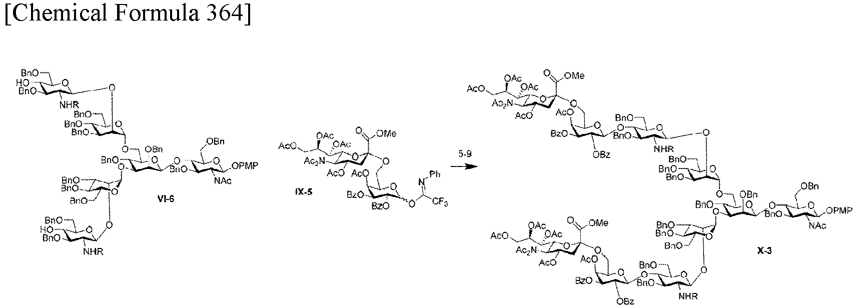

### Example 64:

### 4-Methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-(acetylamino)-β-D-glucopyranosyl-(1→2)-3,4,6-tri-O-benzyl-α-D-mannopyranosyl-(1→3)-[4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-(acetylamino)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(acetylamino)-β-D-glucopyranoside (Compound X-3)

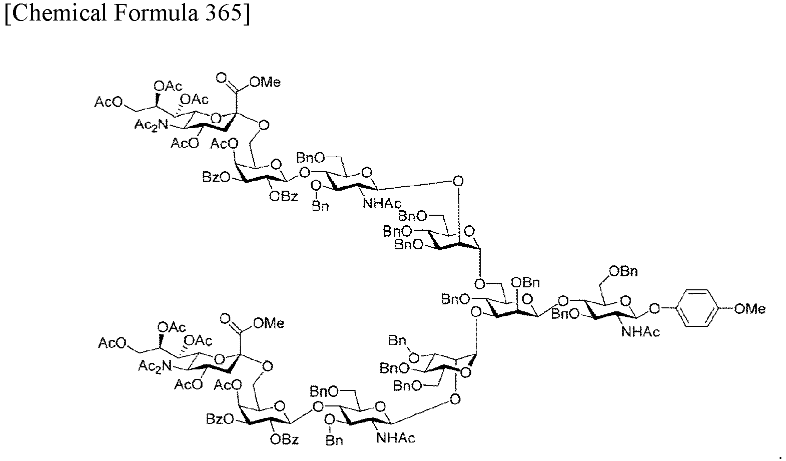

### (Substep 5-9)

First, 4-methoxyphenyl 3,6-di-O-benzyl-2-deoxy-2-acetylamino-β-D-glucopyranosyl-(1→2)-3,4,6-tri-O-benzyl-α-D-mannopyranosyl-(1→3)-[3,6-di-O-benzyl-2-deoxy-2-acetylamino-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-acetylamino-β-D-glucopyranoside (compound VI-6) (50 mg, 20.2 µmol) and 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (Compound IX-5) (90 mg, 80.8 µmol) were added to a 30-mL recovery flask. Dichloromethane (0.15 mL) and Molecular Sieves 4A powder (7.5 mg) were then added. Subsequently, tert-butyldimethylsilyl trifluoromethanesulfonate (4.6 µL, 20.2 µmol) was added dropwise over 5 minutes at 0°C under nitrogen, and the mixture was stirred at 0°C for 3 hours. The completion of the reaction was unable to be observed by HPLC. Thus, tert-butyldimethylsilyl trifluoromethanesulfonate (2.3 µL, 10.1 µmol) was added dropwise over 5 minutes at 0°C, and the mixture was stirred at 0°C for 19 hours. After the completion of the reaction was checked by HPLC, and triethylamine (13.8 µL, 101 µmol) was added. The mixture was then stirred at room temperature for 1 hour. The reaction liquid was filtered through Celite and washed with dichloromethane (0.25 mL). The filtrate was liquid-separated twice with water (0.5 mL). The organic layer was dried over sodium sulfate, filtered, and then concentrated. The concentrated residue was purified by preparative thin layer chromatography (Merck PLC silica gel 60F₂₅₄; developing solvent: heptane/ethyl acetate = 1/4; developed 3 times). The main residue was concentrated under reduced pressure to give 4-methoxyphenyl 4-O-acetyl-2,3-di-O-benzoyl-6-O-[4,7,8,9-tetra-O-acetyl-3,5-dideoxy-5-(acetylamino)-1-methyl-D-glycero-α-D-galacto-non-2-uropyranosyl]-β-D-galactopyranosyl-(1→4)-2-(acetylamino)-3,6-di-O-benzyl-2-deoxy-β-D-glucopyranosyl-(1→2)-3,4,6-tri-O-benzyl-α-D-mannopyranosyl-(1→3)-[4-O-acetyl-2,3-di-O-benzoyl-6-O-[4,7,8,9-tetra-O-acetyl-3,5-dideoxy-5-(acetylamino)-1-methyl-D-glycero-α-D-galacto-non-2-uropyranosyl]-β-D-galactopyranosyl-(1→4)-2-(acetylamino)-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-(acetylamino)-3,6-di-O-benzyl-2-deoxy-β-D-glucopyranoside (compound X-3) (30.7 mg, isolation yield 35.2%) as a white amorphous product.

¹H-NMR (500 MHz, CDCl₃) δ 7.96 (d, J = 7.6 Hz, 2H), 7.88-7.85 (m, 6H), 6.91 (m, 84H), 6.80 (d, J = 9.2 Hz, 2H), 5.76-5.58 (m, 6H), 5.52-5.26 (m, 8H), 5.16-5.10 (m, 3H), 4.82-4.67 (m, 9H), 4.78-4.27 (m, 27H), 4.20-4.06 (m, 10H), 4.06-3.22 (m, 46H), 3.22-3.05 (m, 3H), 2.69 (ddd, J = 12.8, 6.4, 5.4 Hz, 2H), 2.50 (brd, J = 8.8 Hz, 1H), 2.38 (s, 3H), 2.37 (s, 3H), 2.31 (s, 3H), 2.30 (s, 3H), 2.12 (s, 3H), 2.11 (s, 3H), 2.09 (m, 6H), 2.07 (s, 3H), 2.06 (s, 3H), 1.99-1.98 (m, 15H), 1.76 (s, 3H), 1.68 (s, 3H). ¹³C-NMR (125 MHz, CDCl₃) δ 174.5, 173.6, 170.6, 170.4, 170.3, 170.2, 169.9, 169.8, 169.7, 167.1, 165.4, 165.1, 165.0, 155.0, 151.6, 139.1, 138.8, 138.5, 138.3, 138.2, 138.2, 133.5, 133.2, 129.7, 129.6, 129.2, 129.2, 129.1, 128.5, 128.4, 128.3, 128.2, 128.2, 128.1, 128.0, 127.9, 127.8, 127.7, 127.5, 127.3, 127.2, 125.8, 118.3, 114.4, 100.1, 100.0, 98.9, 98.8, 98.5, 77.2, 74.8, 74.2, 73.9, 73.7, 73.5, 73.4, 73.2, 73.0, 72.5, 72.0, 71.7, 70.7, 70.3, 70.2, 69.8, 68.9, 68.4, 68.3, 67.3, 67.1, 66.7, 62.2, 62.1, 61.9, 57.0, 55.6, 53.0, 52.9, 38.7, 28.0, 26.0, 23.4, 23.2, 21.1, 21.0, 20.8, 20.6, 20.5, 14.2.
HRMS(ESI⁺)[M+H]⁺ calcd for C₂₃₅H₂₅₉N₅O₇₄: 4337.6758; found 4337.6572.

## Claims

1. A method for producing an oligosaccharide represented by the following formula XI: 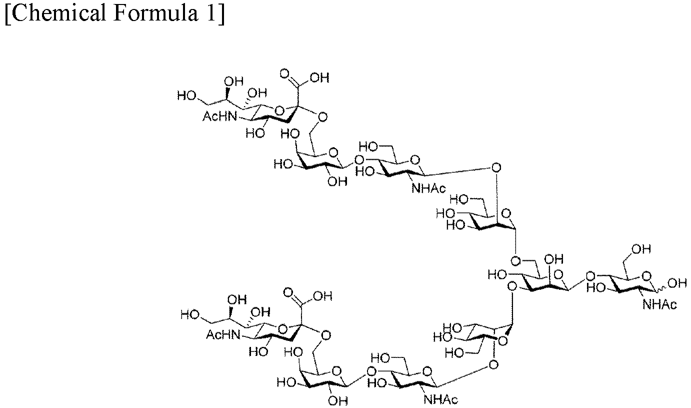 the method comprising steps of:
(step 1) producing a compound represented by the following formula III-11: 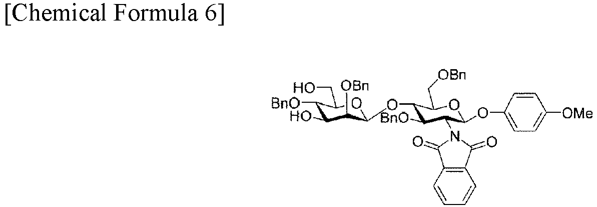
or a compound represented by the following formula III-13: 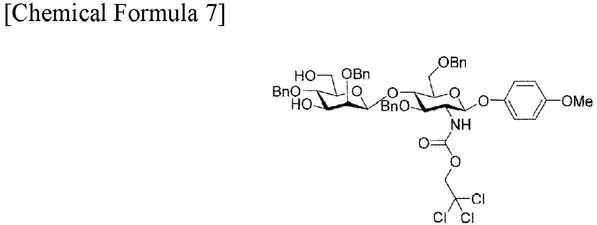
comprising a step of
reacting a compound represented by the following formula III-3: 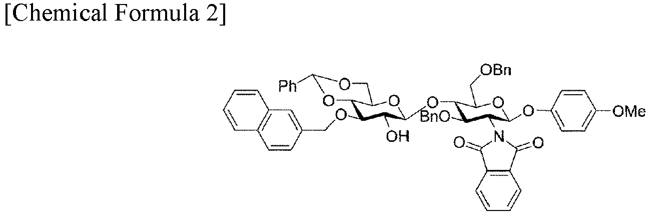
with a compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group to give a compound represented by the following formula III-4: 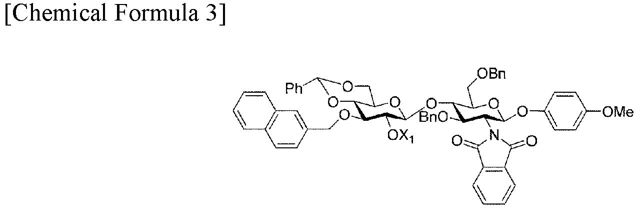
wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group, and
then reacting the compound represented by formula III-4 with cesium acetate or tetrabutylammonium acetate to give a compound represented by the following formula III-5: 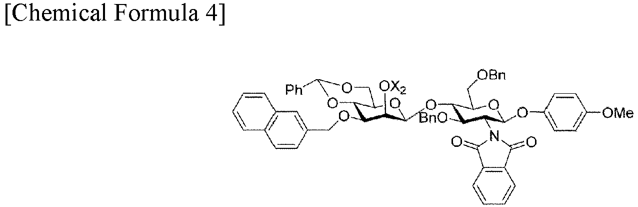
wherein X₂ is an acetyl group,
or reacting the compound represented by formula III-4 with tetrabutylammonium benzoate to give a compound represented by the following formula III-5: 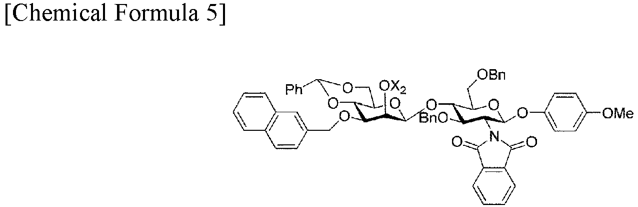
wherein X₂ is a benzoyl group;
(step 2) producing a compound represented by the following formula V-3: 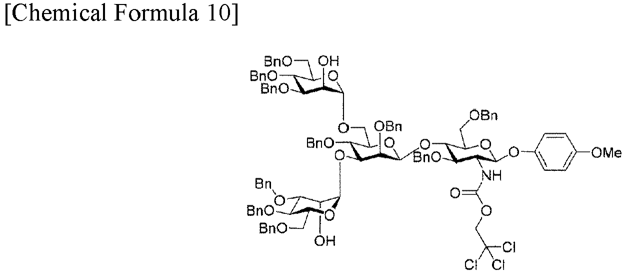
comprising a step of
reacting the compound represented by formula III-13 with a compound represented by the following formula IV-3: 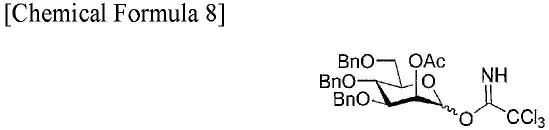
to give a compound represented by the following formula V-1: 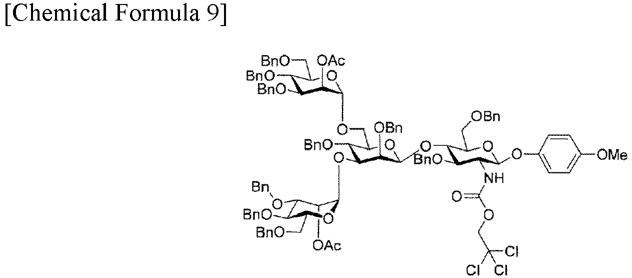 or producing a compound represented by the following formula V-5: 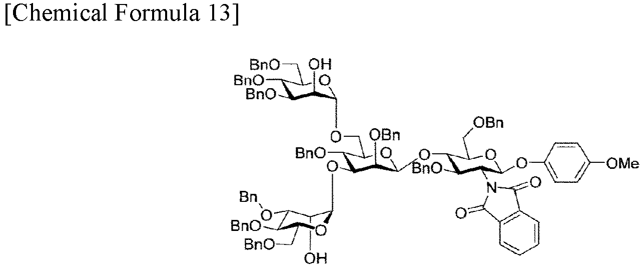
comprising a step of
reacting the compound represented by formula III-11 with a compound represented by the following formula IV-3: 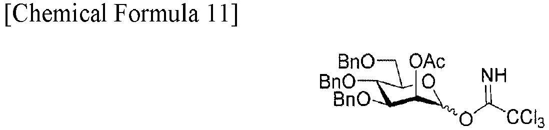
to give a compound represented by the following formula V-4: 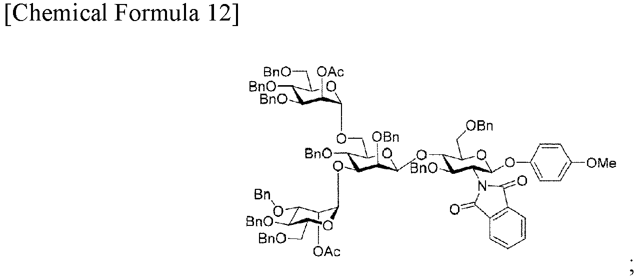 (step 3) producing a compound represented by the following formula VI-3: 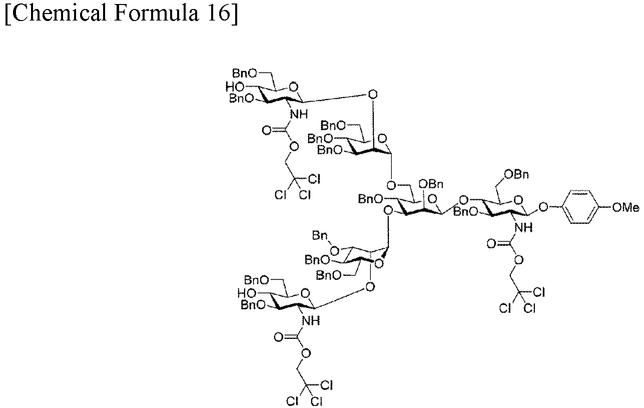
comprising a step of
reacting the compound represented by formula V-3 with a compound represented by the following formula II-6: 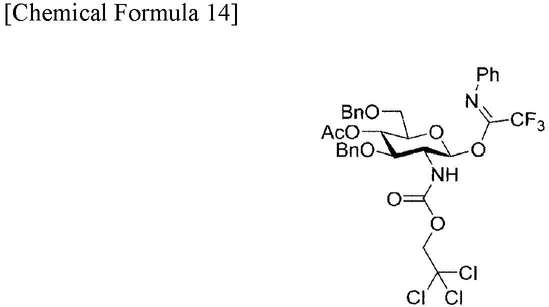
to give a compound represented by the following formula VI-1: 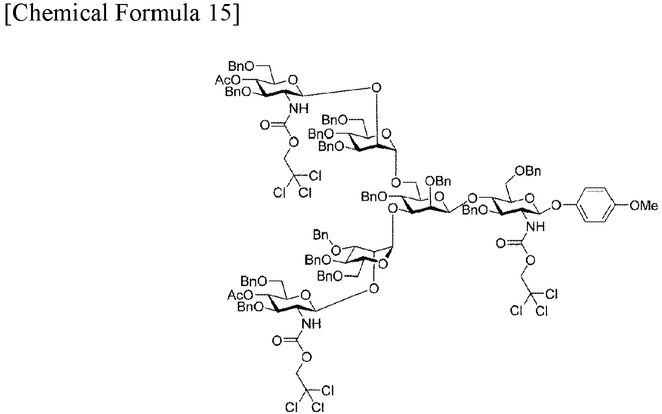 or producing a compound represented by the following formula VI-3: 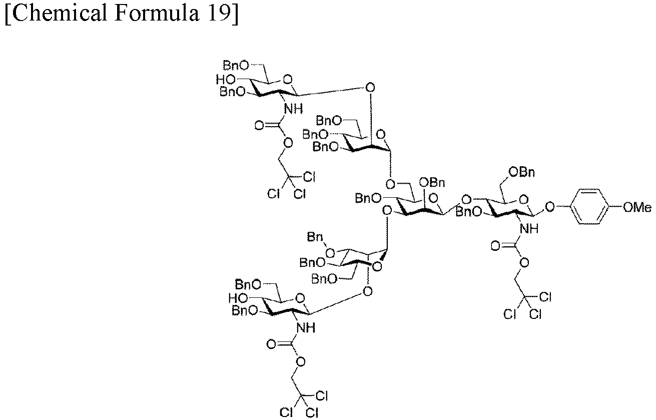 or a compound represented by the following formula VI-6: 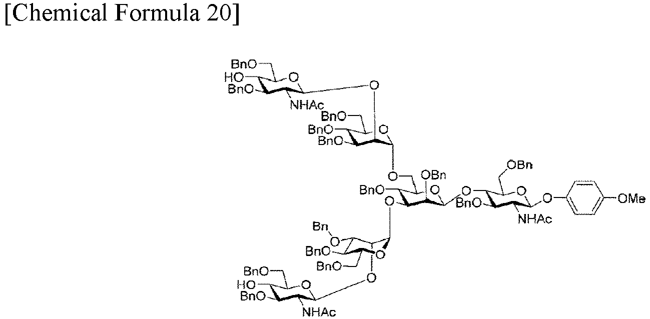
comprising a step of
reacting the compound represented by formula V-5 with a compound represented by the following formula II-9: 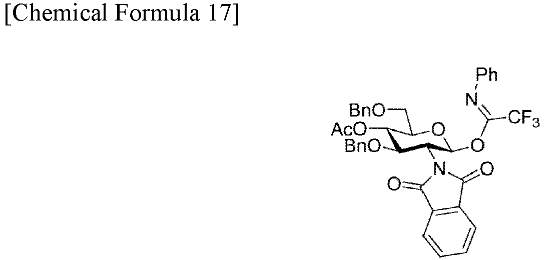
to give a compound represented by the following formula VI-4: 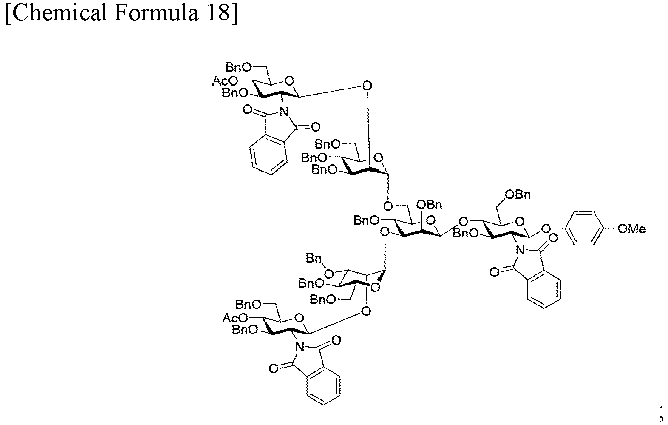 (step 4) producing a compound represented by the following formula IX-5: 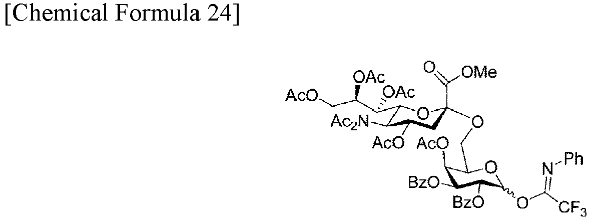
comprising a step of
subjecting a compound represented by the following formula VIII-5: 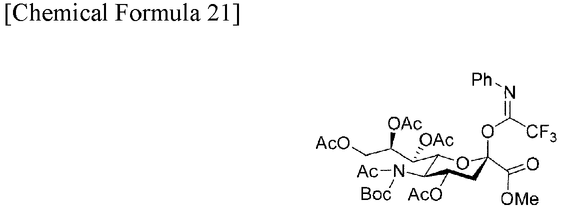
and a compound represented by the following formula VII-3: 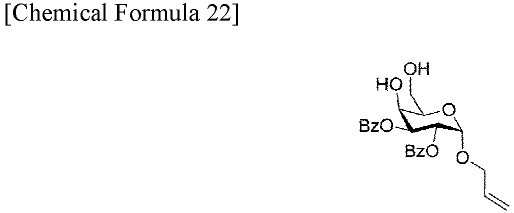
to α-2,6-glycosidic linkage to give a compound represented by the following formula IX-1: 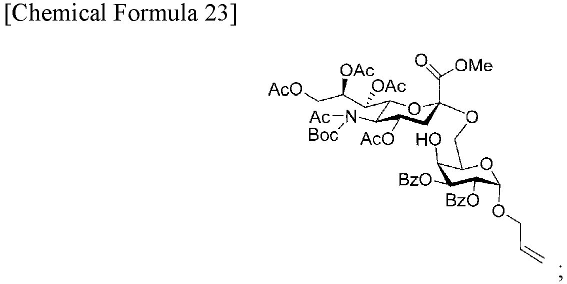 and
(step 5) producing the oligosaccharide represented by formula XI
comprising a step of
reacting the compound represented by formula VI-3 with the compound represented by formula IX-5 to give a compound represented by the following formula X-1: 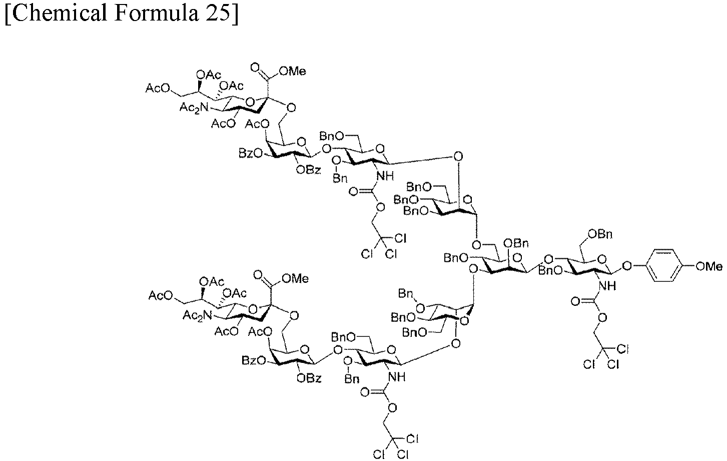
or reacting the compound represented by formula VI-6 with the compound represented by formula IX-5 to give a compound represented by the following formula X-3:

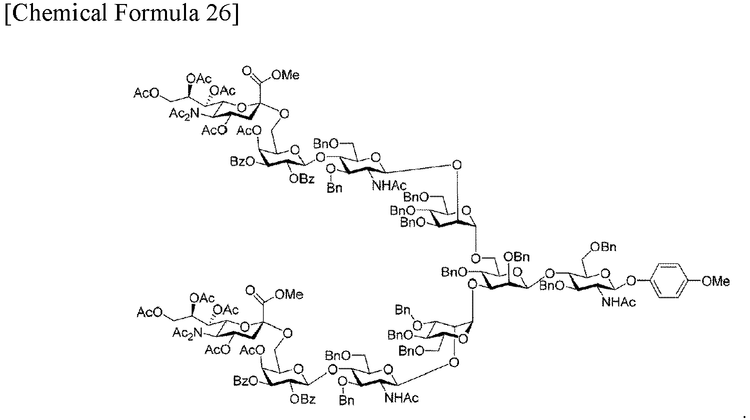

2. The method according to claim 1, wherein step 1 comprises a step of reacting a compound represented by the following formula III-1: 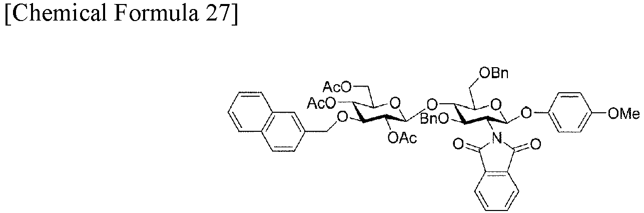 with an alkoxide-based strong base in the presence of methyl trifluoroacetate to give a compound represented by the following formula III-2:

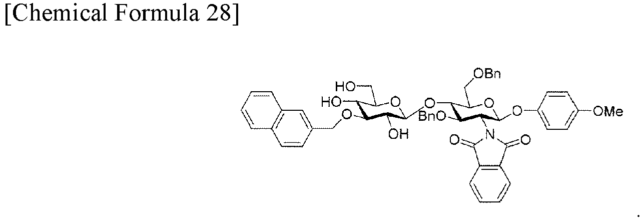

3. The method according to claim 2, wherein the alkoxide-based strong base is selected from the group consisting of a sodium salt, lithium salt, and potassium salt of C1-C5 alkoxide, and a combination thereof.

4. A method for producing an oligosaccharide represented by the following formula XI: 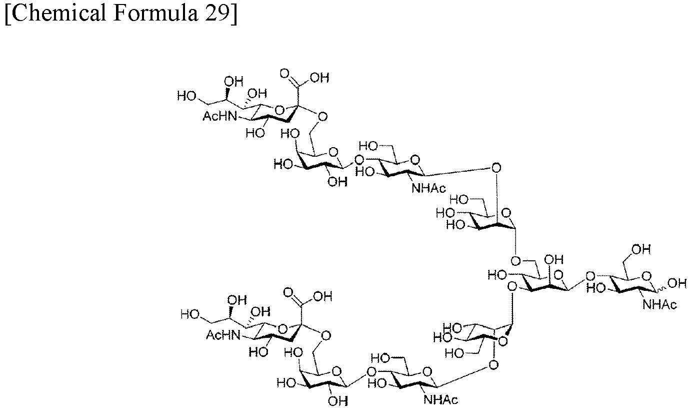 the method comprising steps of:
(step 1) producing a compound represented by the following formula III-11: 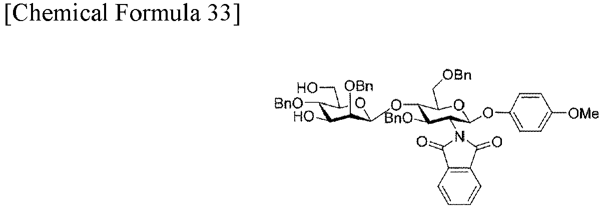
or a compound represented by the following formula III-13: 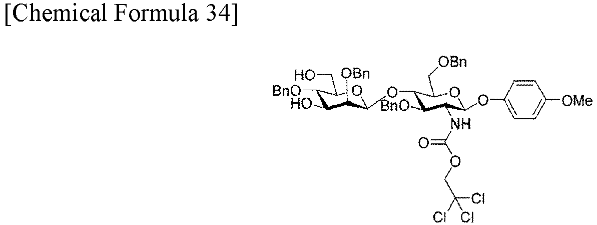
comprising a step of
oxidizing position 2 of D-glucopyranoside in a compound represented by the following formula III-3: 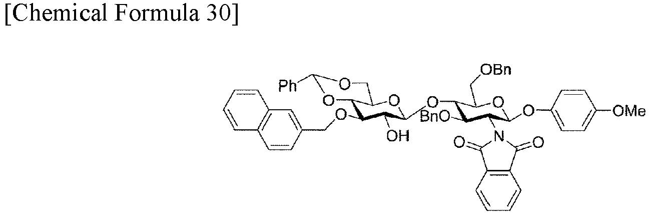
to give a compound represented by the following formula III-7: 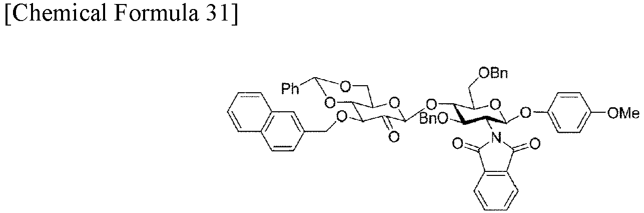
then reducing the oxo group attached to the carbon at position 2 of 2-keto-D-glucopyranoside in the compound represented by formula III-7 to give a compound represented by the following formula III-8: 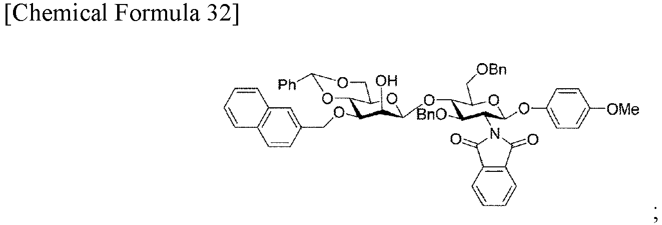
(step 2) producing a compound represented by the following formula V-3: 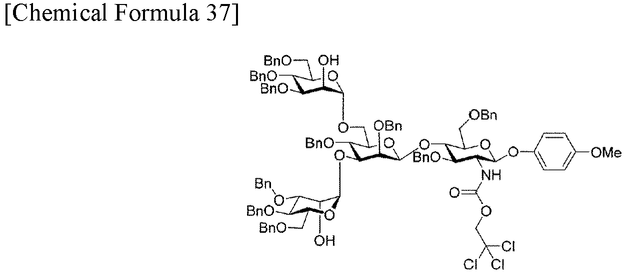
comprising a step of
reacting the compound represented by formula III-13 with a compound represented by the following formula IV-3: 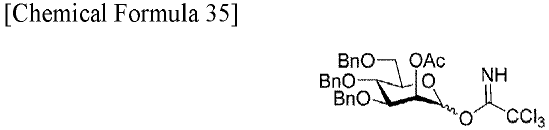
to give a compound represented by the following formula V-1: 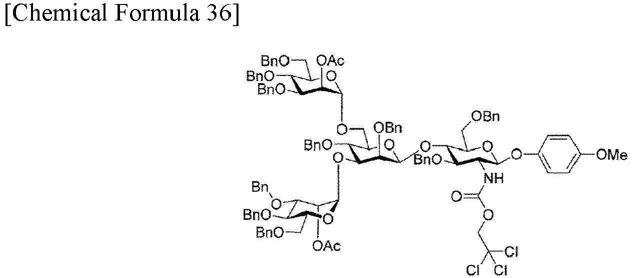
or producing a compound represented by the following formula V-5: 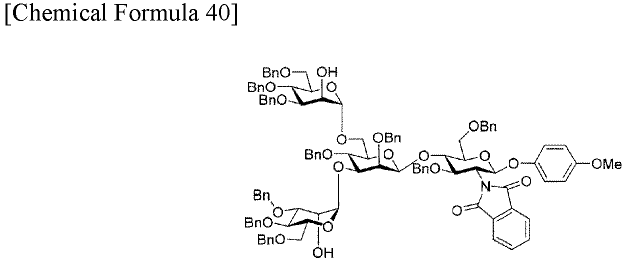
comprising a step of
reacting the compound represented by formula III-11 with a compound represented by the following formula IV-3: 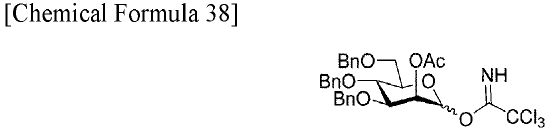
to give a compound represented by the following formula V-4: 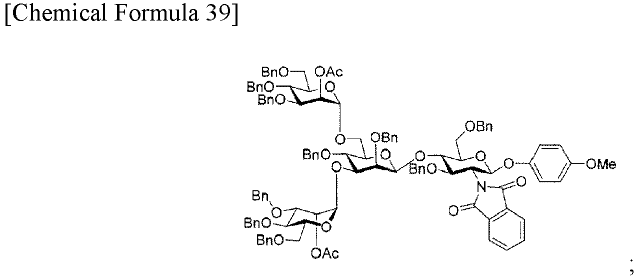
(step 3) producing a compound represented by the following formula VI-3: 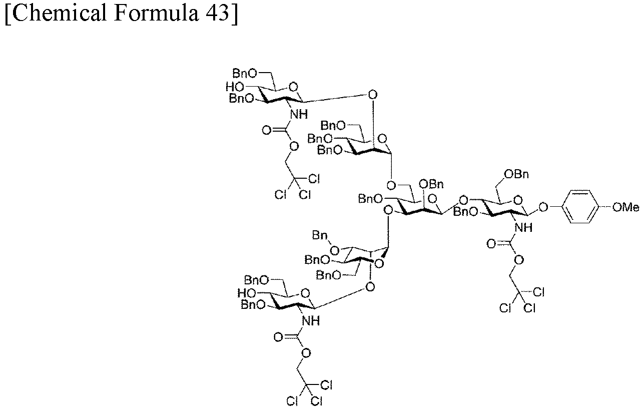
comprising a step of
reacting the compound represented by formula V-3 with a compound represented by the following formula II-6: 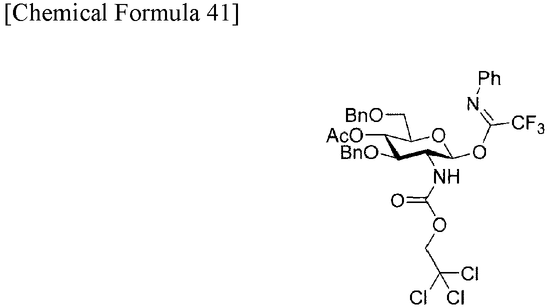
to give a compound represented by the following formula VI-1: 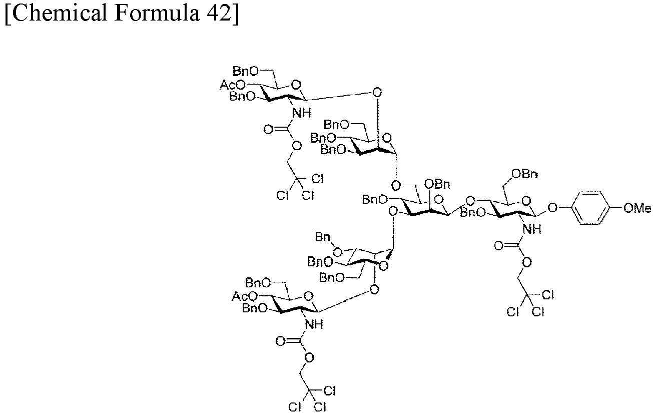
or producing a compound represented by the following formula VI-3: 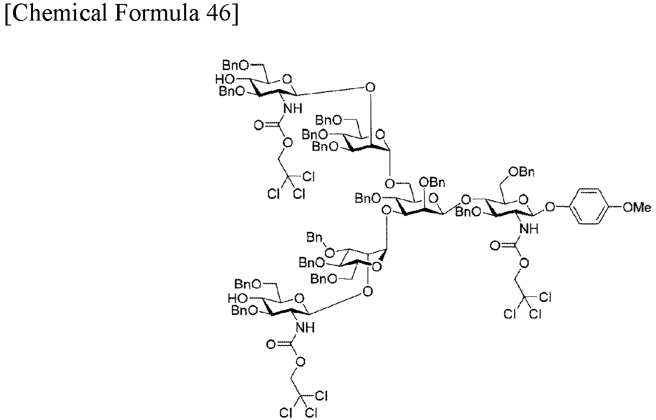
or a compound represented by the following formula VI-6: 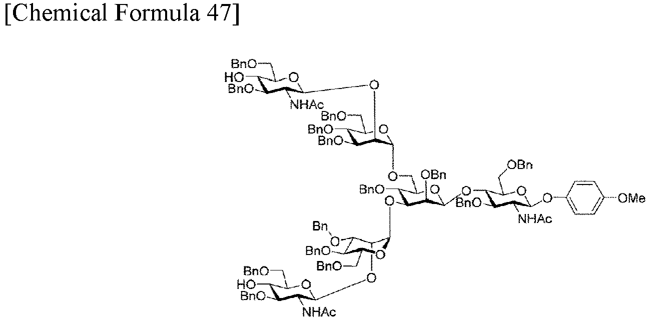
comprising a step of
reacting the compound represented by formula V-5 with a compound represented by the following formula II-9: 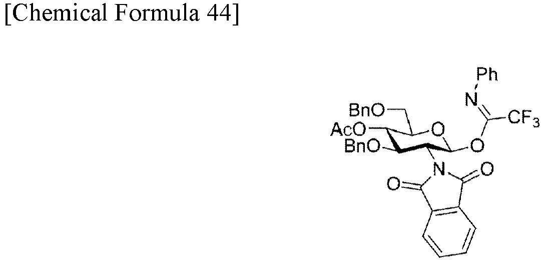
to give a compound represented by the following formula VI-4: 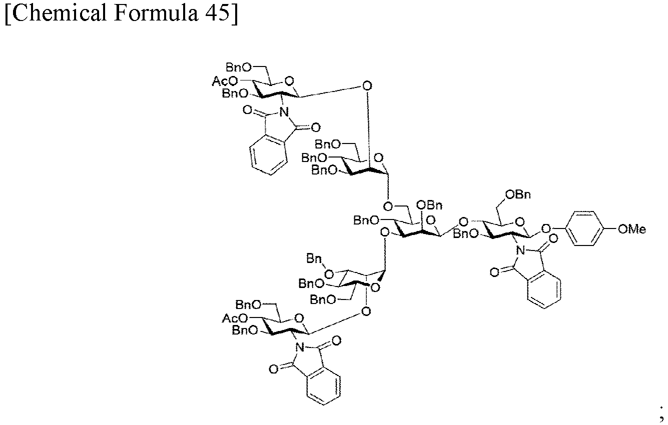
(step 4) producing a compound represented by the following formula IX-5: 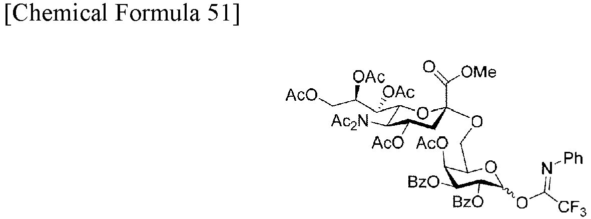
comprising a step of
subjecting a compound represented by the following formula VIII-5: 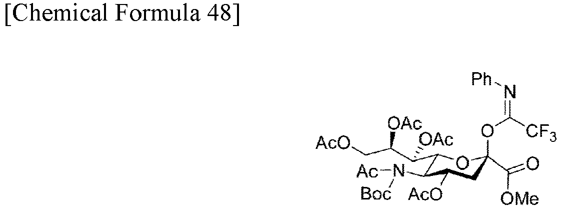
and a compound represented by the following formula VII-3: 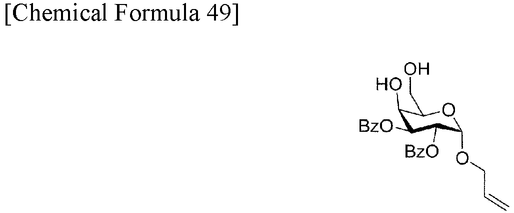
to α-2,6-glycosidic linkage to give a compound represented by the following formula IX-1: 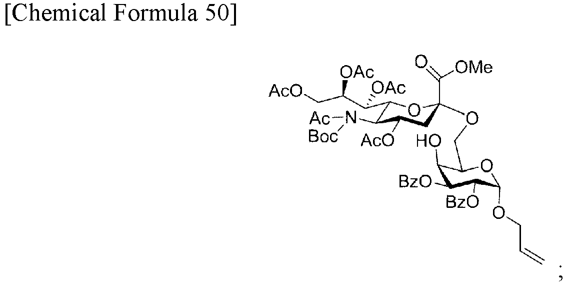 and
(step 5) producing the oligosaccharide represented by the formula XI
comprising a step of
reacting the compound represented by formula VI-3 with the compound represented by formula IX-5 to give a compound represented by the following formula X-1: 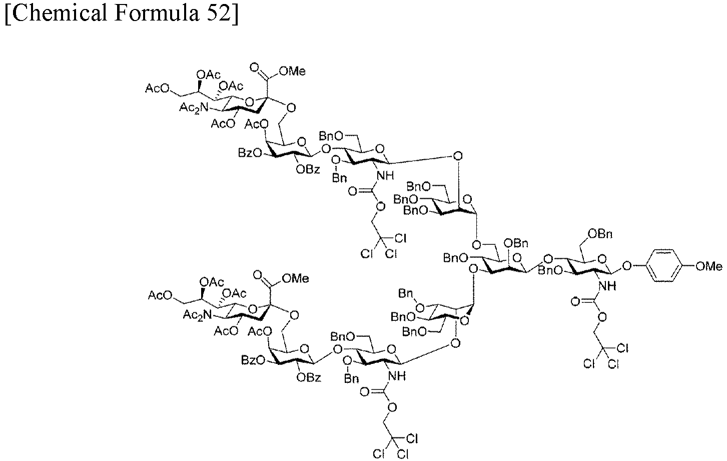
or reacting the compound represented by formula VI-6 with the compound represented by formula IX-5 to give a compound represented by the following formula X-3:

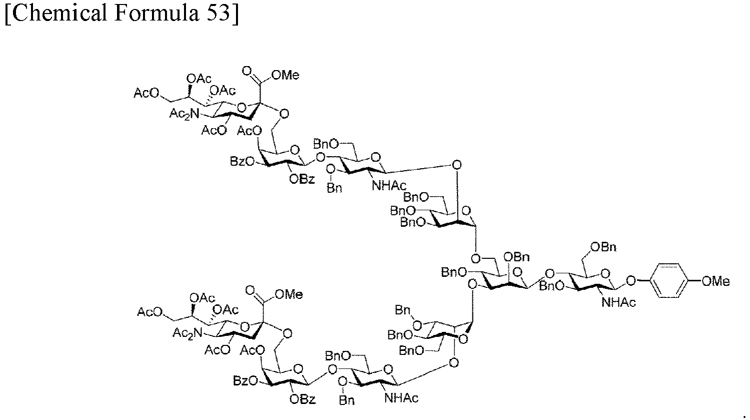

5. The method according to claim 4, wherein step 1 comprises a step of reacting a compound represented by the following formula III-1: 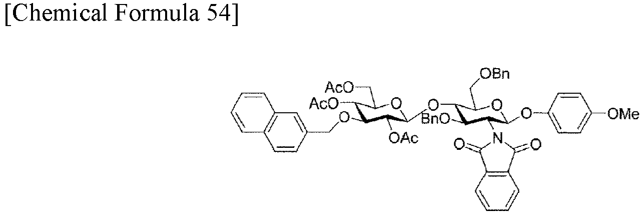 with an alkoxide-based strong base in the presence of methyl trifluoroacetate to give a compound represented by the following formula III-2:

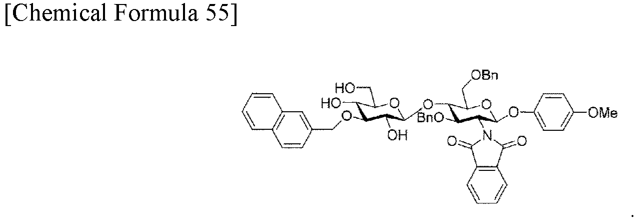

6. The method according to claim 5, wherein the alkoxide-based strong base is selected from the group consisting of a sodium salt, lithium salt, and potassium salt of C1-C5 alkoxide, and a combination thereof.

7. The method according to any one of claims 4 to 6, wherein in step 1, the compound represented by formula III-7 is reduced in the presence of a reducing agent selected from the group consisting of L-selectride; LS-selectride; lithium diisobutyl-tert-butoxyaluminum hydride (LDBBA); a compound represented by the following formula A: 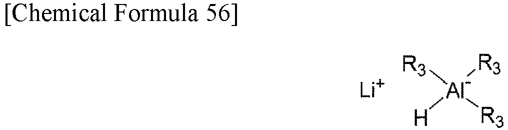
wherein R₃ is a di-tert-butylmethylphenoxide as shown in the following formula: 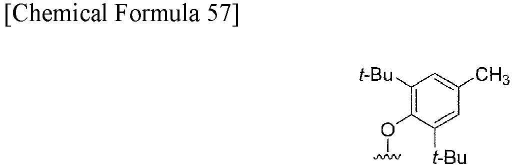
or hydride, provided that at least two R₃ are each di-tert-butylmethylphenoxide; and a combination thereof.

8. The method according to any one of claims 4 to 7, wherein in step 1, the compound represented by formula III-7 is reduced in the presence of a reducing agent that is a compound represented by the following formula A: 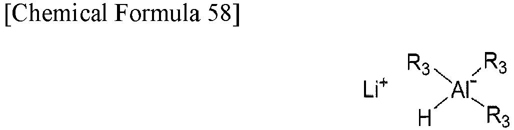
wherein R₃ is a di-tert-butylmethylphenoxide as shown in the following formula: 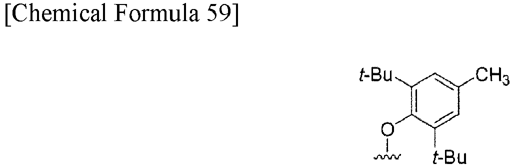
or hydride, provided that at least two R₃ are each di-tert-butylmethylphenoxide.

9. The method according to any one of claims 1 to 8, wherein step 1 further comprises a step of
producing a compound represented by the following formula III-9: 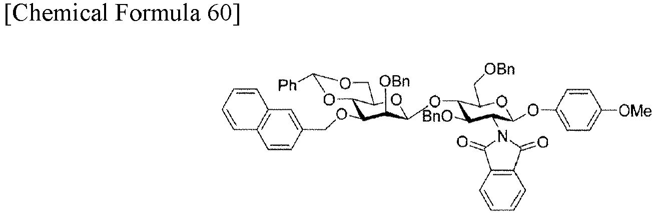
and the step of producing the compound represented by formula III-9 comprises a step of
protecting, in the presence of lithium tert-butoxide or lithium tert-amoxide, a hydroxyl group attached to the carbon at position 2 of D-mannopyranoside in a compound represented by the following formula III-8: 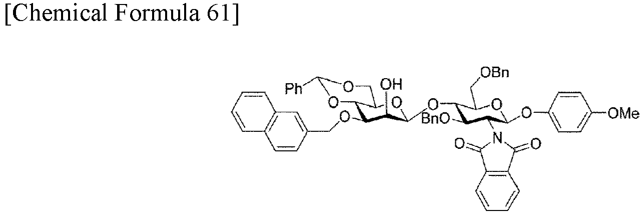
by a benzyl group to give the compound represented by formula III-9.

10. The method according to any one of claims 1 to 9, wherein step 2 further comprises a step of
producing a compound represented by the following formula V-5: 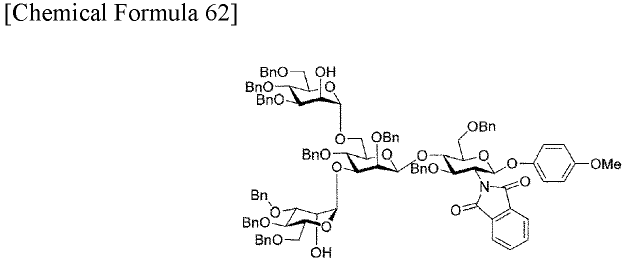
and the step of producing the compound represented by formula V-5 comprises a step of
reacting, in the presence of methyl trifluoroacetate, a compound represented by the following formula V-4: 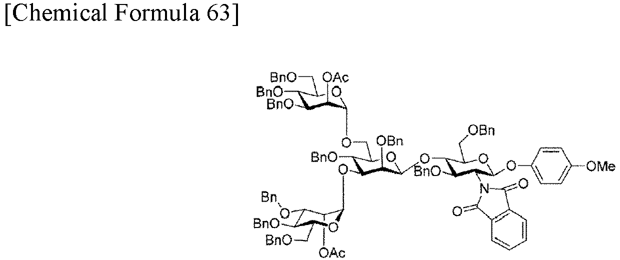
with an alkoxide-based strong base to give the compound represented by formula V-5.

11. The method according to claim 10, wherein the alkoxide-based strong base is selected from the group consisting of a sodium salt, lithium salt, and potassium salt of C1-C5 alkoxide, and a combination thereof.

12. The method according to any one of claims 1 to 11, wherein step 3 further comprises a step of producing the compound represented by formula II-6 or the compound represented by formula II-9,
and the step of producing the compound represented by formula II-6 comprises a step of
reacting, in fluorous alcohol and water, a compound represented by the following formula II-4: 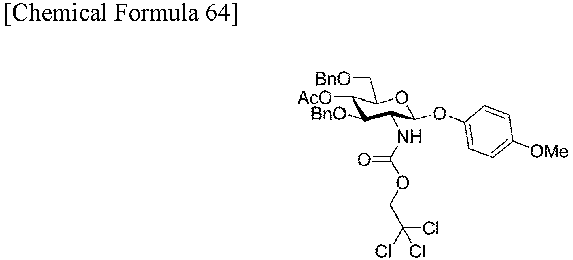
with λ3-iodane to give a compound represented by the following formula II-5 : 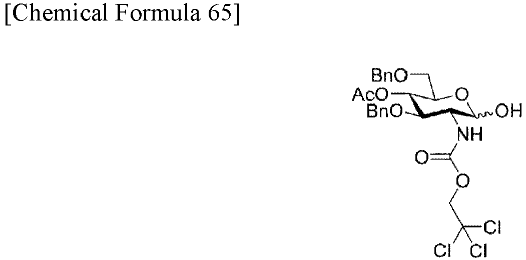
and the step of producing the compound represented by formula II-9 comprises a step of
reacting, in fluorous alcohol and water, a compound represented by the following formula II-7: 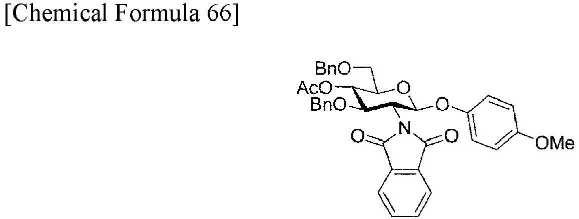
with λ3-iodane to give a compound represented by the following formula II-8:

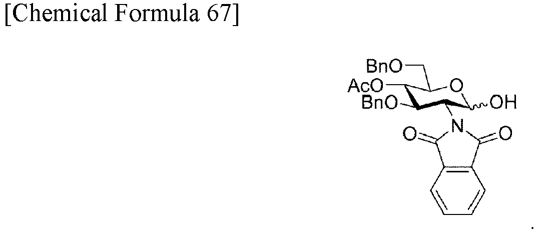

13. The method according to claim 12, wherein the λ3-iodane is selected from the group consisting of [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, [hydroxy(methanesulfonyloxy)iodo]benzene, and a combination thereof.

14. The method according to claim 12 or 13, wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.

15. The method according any one of claims 1 to 14, wherein step 3 further comprises a step of
producing the compound represented by formula II-6 or the compound represented by formula II-9,
and the step of producing the compound represented by formula II-6 comprises a step of
reacting, in the presence of N-methylimidazole, a compound represented by the following formula II-5: 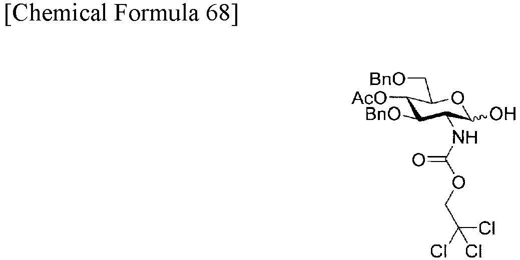
with 2,2,2-trifluoro-N-phenylacetimidoyl chloride (TFPC) represented by the following formula: 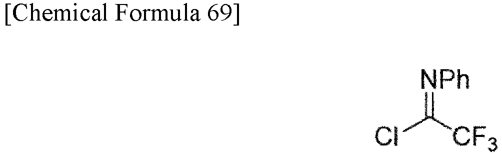
to give the compound represented by formula II-6,
and the step of producing the compound represented by formula II-9 comprises a step of
reacting, in the presence of N-methylimidazole, a compound represented by the following formula II-8: 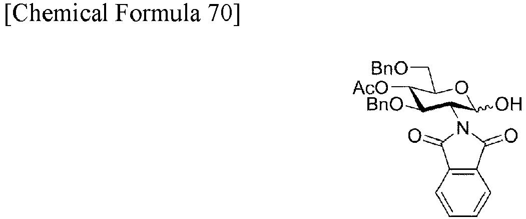
with 2,2,2-trifluoro-N-phenylacetimidoyl chloride (TFPC) represented by the following formula: 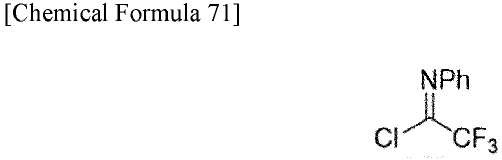
to give the compound represented by formula II-9.

16. The method according to any one of claims 1 to 15, wherein step 3 further comprises a step of
producing a compound represented by the following formula VI-5: 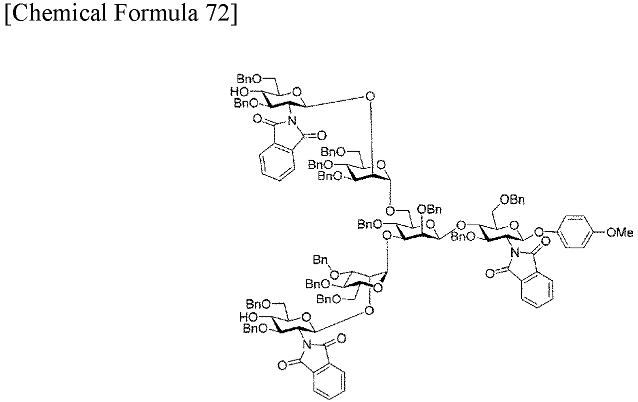
and the step of producing the compound represented by formula VI-5 comprises a step of
reacting, in the presence of methyl trifluoroacetate, a compound represented by the following formula VI-4: 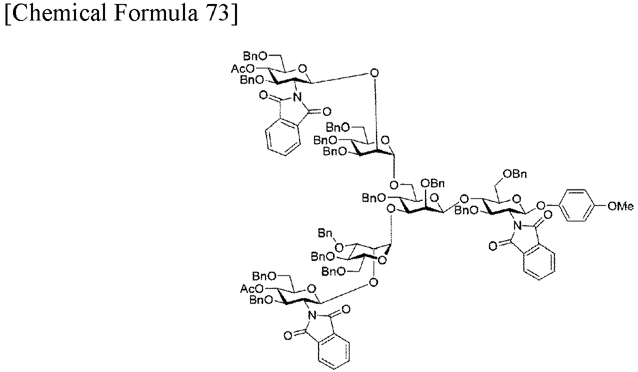
with an alkoxide-based strong base to give the compound represented by formula VI-5.

17. The method according to claim 16, wherein the alkoxide-based strong base is selected from the group consisting of a sodium salt, lithium salt, and potassium salt of C1-C5 alkoxide, and a combination thereof.

18. The method according to any one of claims 1 to 17, wherein step 4 further comprises a step of
producing the compound represented by formula VII-3,
and the step of producing the compound represented by formula VII-3 comprises a step of
bringing a solvent dissolving the compound represented by formula VII-3 into contact with silica gel to perform solid-phase extraction of the compound represented by formula VII-3.

19. The method according to claim 18, wherein the solvent dissolving the compound represented by formula VII-3 is selected from the group consisting of toluene, dichloromethane, chloroform, and a combination thereof.

20. The method according to any one of claims 1 to 19, wherein step 4 comprises a step of
reacting, in the presence of N-methylimidazole, a compound represented by the following formula VIII-3: 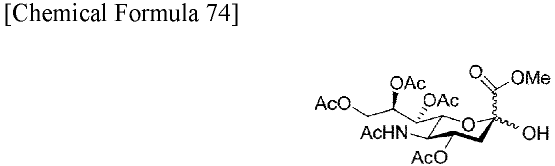
with 2,2,2-trifluoro-N-phenylacetimidoyl chloride (TFPC) represented by the following formula: 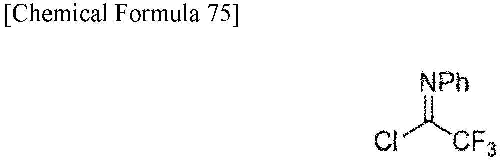
to give a compound represented by the following formula VIII-4:

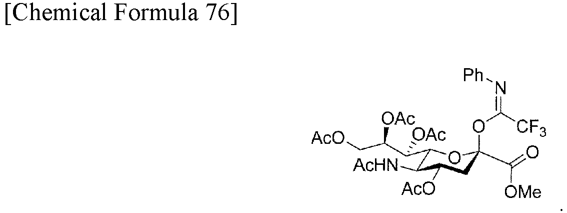

21. The method according to any one of claims 1 to 20, wherein step 4 further comprises a step of
producing a compound represented by the following formula IX-3: 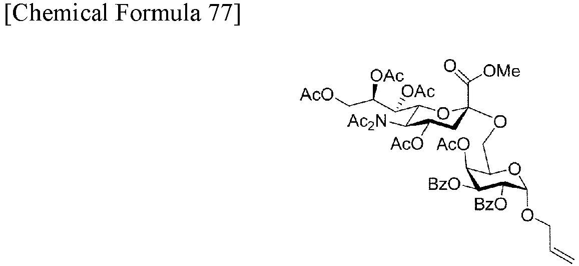
and the step of producing the compound represented by formula IX-3 comprises a step of
bringing a solvent dissolving the compound represented by formula IX-3 into contact with silica gel to perform solid-phase extraction of the compound represented by formula IX-3.

22. The method according to claim 21, wherein the solvent dissolving the compound represented by formula IX-3 is selected from the group consisting of toluene, dichloromethane, chloroform, and a combination thereof.

23. A method for producing a compound represented by the following formula III-2: 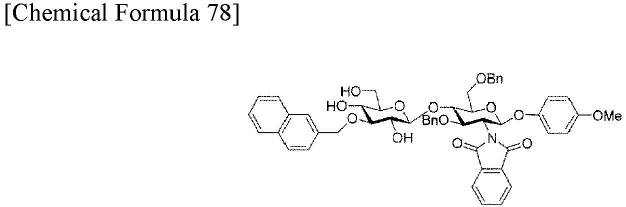 the method comprising a step of
reacting, in the presence of methyl trifluoroacetate, a compound represented by the following formula III-1: 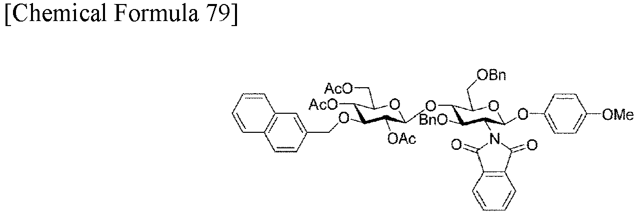
with an alkoxide-based strong base to give the compound represented by formula III-2.

24. The method according to claim 23, wherein the alkoxide-based strong base is selected from the group consisting of a sodium salt, lithium salt, and potassium salt of C1-C5 alkoxide, and a combination thereof.

25. A method for producing a compound represented by formula III-5: 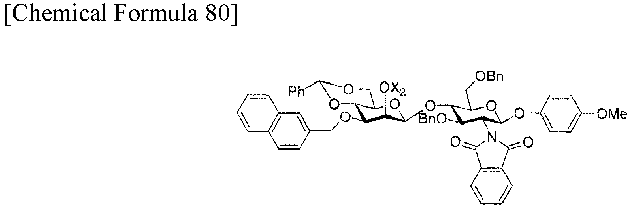
wherein X₂ is an acetyl group
the method comprising a step of
reacting a compound represented by the following formula III-4: 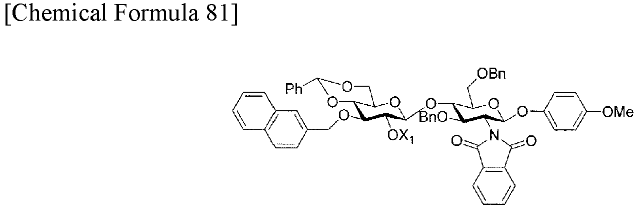
wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group,
with cesium acetate or tetrabutylammonium acetate to give the compound represented by formula III-5.

26. A method for producing a compound represented by formula III-5: 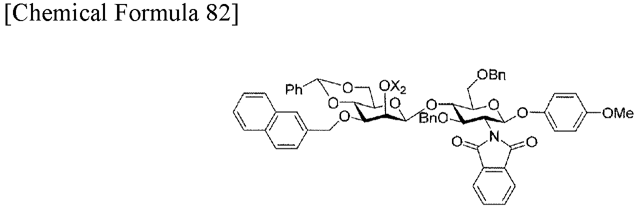
wherein X₂ is a benzoyl group
the method comprising a step of
reacting a compound represented by the following formula III-4: 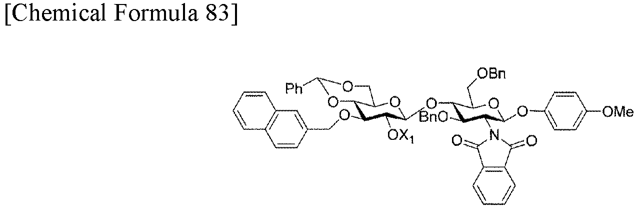
wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group,
with tetrabutylammonium benzoate to give the compound represented by formula III-5.

27. A method for producing a compound represented by the following formula III-8: 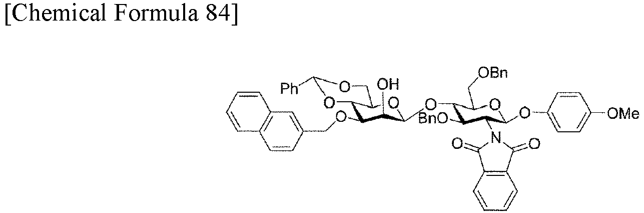 the method comprising a step of
reducing an oxo group attached to the carbon at position 2 of 2-keto-D-glucopyranoside in a compound represented by the following formula III-7: 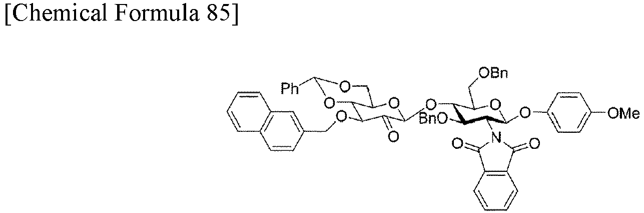
to give the compound represented by formula III-8.

28. The method according to claim 27, wherein the oxo group attached to the carbon at position 2 of 2-keto-D-glucopyranoside in the compound represented by formula III-7 is reduced in the presence of a reducing agent selected from the group consisting of L-selectride; LS-selectride; lithium diisobutyl-tert-butoxyaluminum hydride (LDBBA); a compound represented by the following formula A: 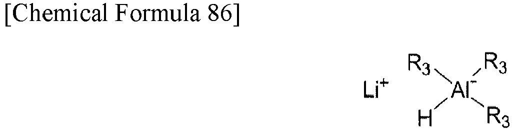
wherein R₃ is a di-tert-butylmethylphenoxide as shown in the following formula: 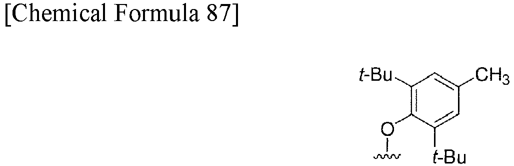
or hydride, provided that at least two R₃ are each di-tert-butylmethylphenoxide; and a combination thereof.

29. The method according to claim 27 or 28, wherein the oxo group attached to the carbon at position 2 of 2-keto-D-glucopyranoside in the compound represented by formula III-7 is reduced in the presence of a reducing agent that is a compound represented by the following formula A: 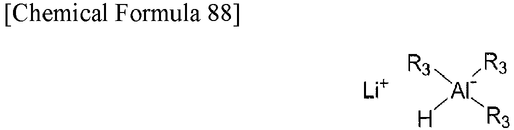
wherein R₃ is a di-tert-butylmethylphenoxide as shown in the following formula: 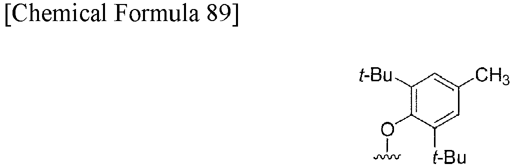
or hydride, provided that at least two R₃ are each di-tert-butylmethylphenoxide.

30. A method for producing a compound represented by the following formula III-9: 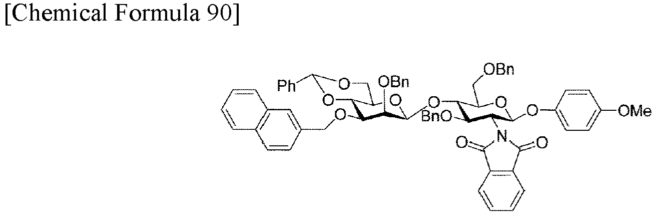 the method comprising a step of
protecting, in the presence of lithium tert-butoxide or lithium tert-amoxide, a hydroxyl group attached to the carbon at position 2 of D-mannopyranoside in a compound represented by the following formula III-8: 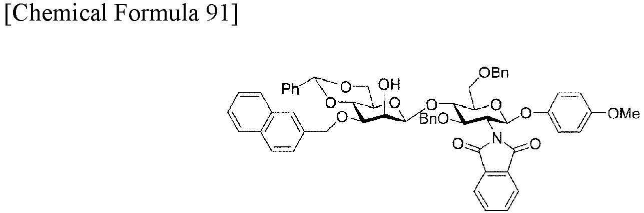
by a benzyl group to give the compound represented by formula III-9.

31. A method for producing a compound represented by the following formula V-5: 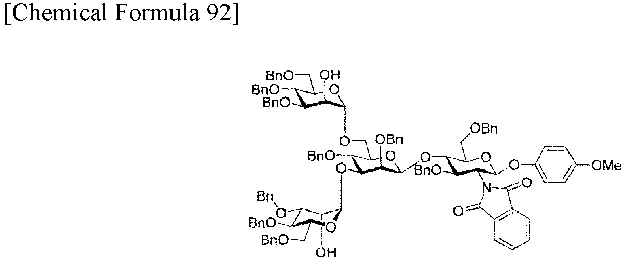 the method comprising a step of
reacting, in the presence of methyl trifluoroacetate, a compound represented by the following formula V-4: 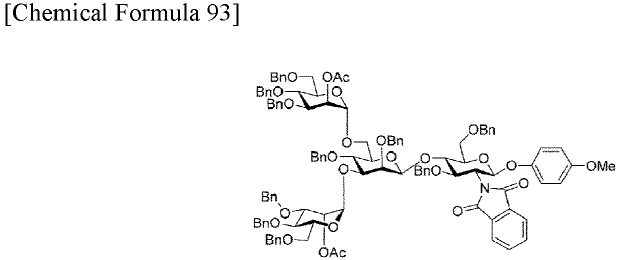
with an alkoxide-based strong base to give the compound represented by formula V-5.

32. The method according to claim 31, wherein the alkoxide-based strong base is selected from the group consisting of a sodium salt, lithium salt, and potassium salt of C1-C5 alkoxide, and a combination thereof.

33. A method for producing a compound represented by the following formula II-5: 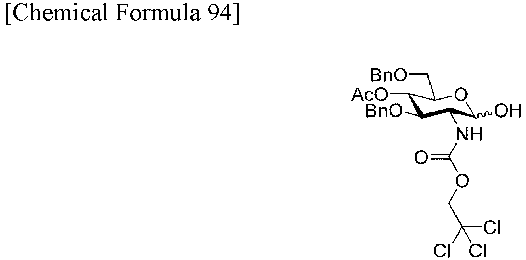
or a compound represented by the following formula II-8: 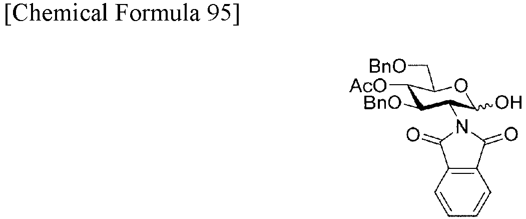
the method comprising a step of
reacting, in fluorous alcohol and water, a compound represented by the following formula II-4: 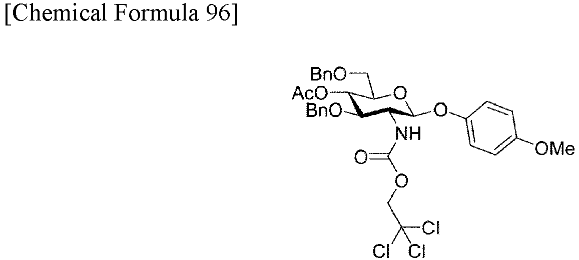
or a compound represented by the following formula II-7: 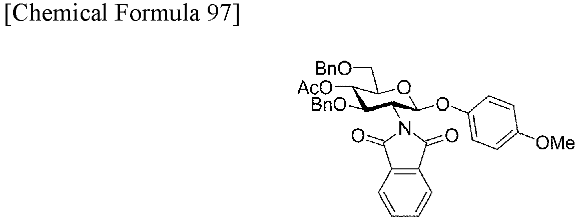
with λ3-iodane to give the compound represented by formula II-5 or the compound represented by formula II-8.

34. The method according to claim 33, wherein the λ3-iodane is selected from the group consisting of [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, [hydroxy(methanesulfonyloxy)iodo]benzene, and a combination thereof.

35. The method according to claim 33 or 34, wherein the fluorous alcohol is selected from the group consisting of hexafluoro 2-propanol (HFIP), 2,2,2-trifluoroethanol (TFE), 2,2,3,3,4,4,5,5-octafluoro-1-pentanol, nonafluoro-tert-butyl alcohol, and a combination thereof.

36. A method for producing a compound represented by the following formula II-6: 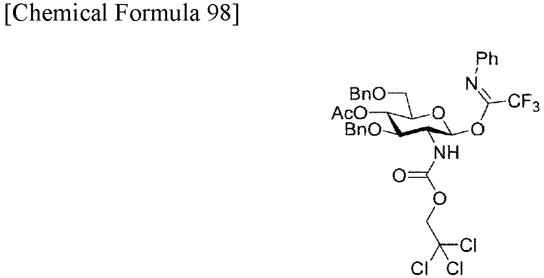
or a compound represented by the following formula II-9: 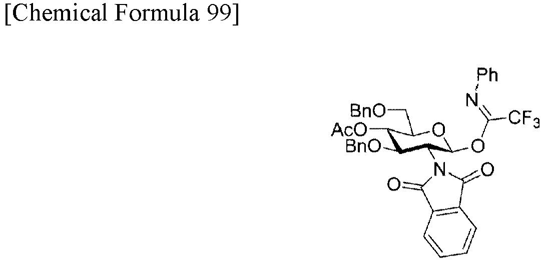
the method comprising a step of
reacting, in the presence of N-methylimidazole, a compound represented by the following formula II-5: 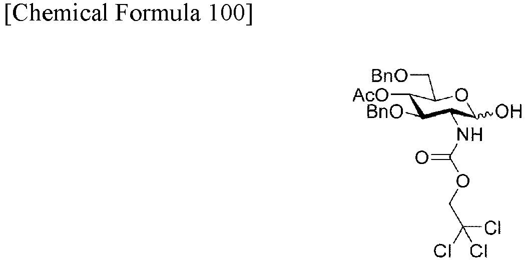
or a compound represented by the following formula II-8: 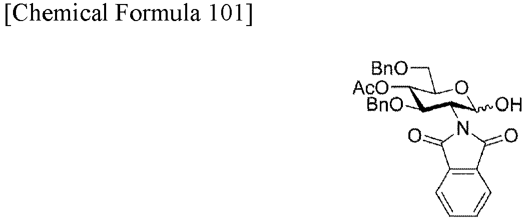
with 2,2,2-trifluoro-N-phenylacetimidoyl chloride (TFPC) represented by the following formula: 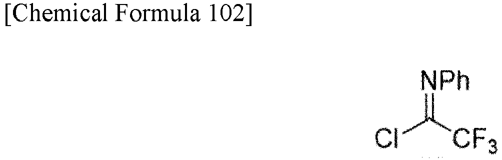
to give the compound represented by formula II-6 or the compound represented by formula II-9.

37. A method for producing a compound represented by the following formula VI-5: 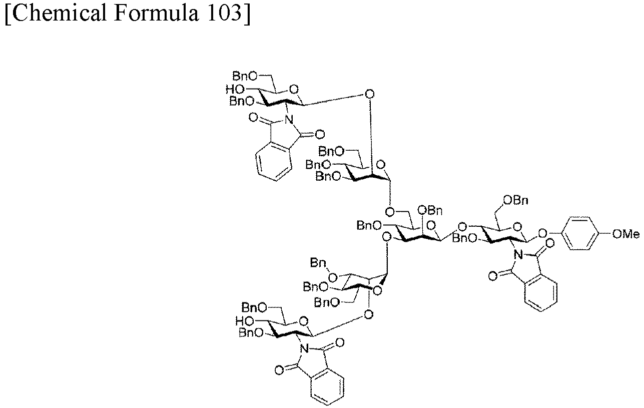 the method comprising a step of
reacting, in the presence of methyl trifluoroacetate, a compound represented by the following formula VI-4: 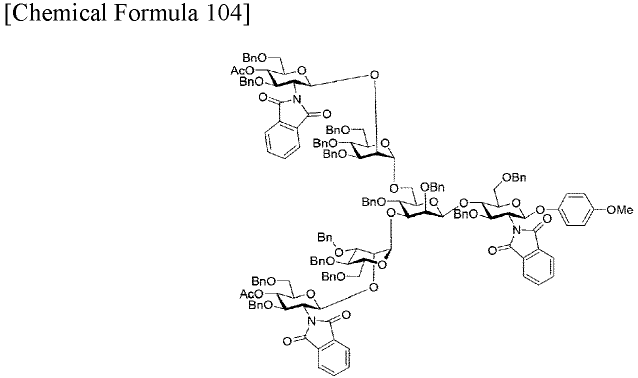
with an alkoxide-based strong base to give the compound represented by formula VI-5.

38. The method according to claim 37, wherein the alkoxide-based strong base is selected from the group consisting of a sodium salt, lithium salt, and potassium salt of C1-C5 alkoxide, and a combination thereof.

39. A method for producing a compound represented by the following formula VII-3 : 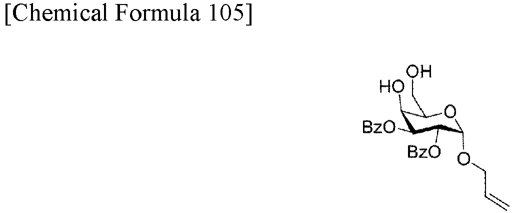 the method comprising a step of
bringing a solvent dissolving the compound represented by formula VII-3 into contact with silica gel to perform solid-phase extraction of the compound represented by formula VII-3.

40. A method for producing a compound represented by the following formula VIII-4: 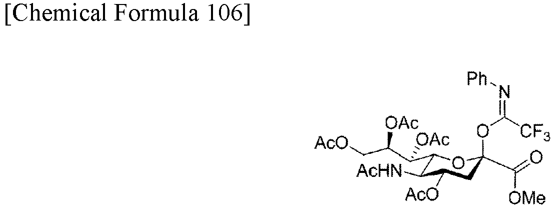 the method comprising a step of
reacting, in the presence of N-methylimidazole, a compound represented by the following formula VIII-3: 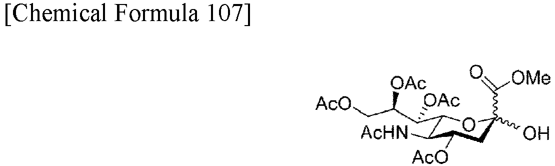
with 2,2,2-trifluoro-N-phenylacetimidoyl chloride (TFPC) represented by the following formula: 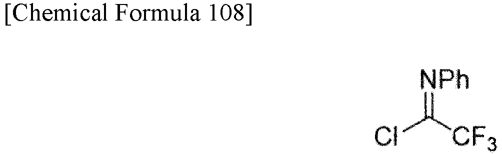
to give the compound represented by formula VIII-4.

41. A method for producing a compound represented by the following formula IX-1: 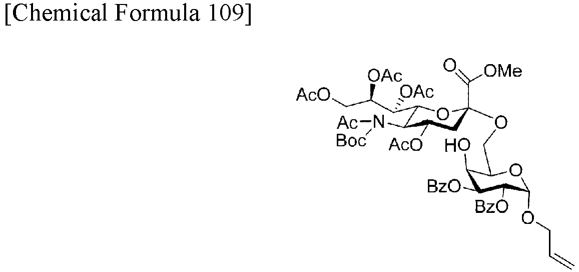 the method comprising a step of
subjecting a compound represented by the following formula VIII-5: 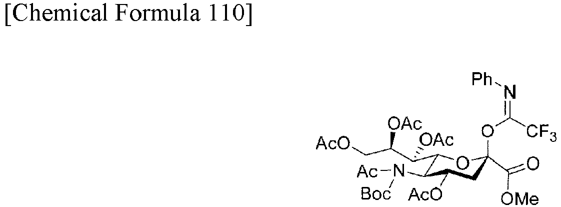
and a compound represented by the following formula VII-3: 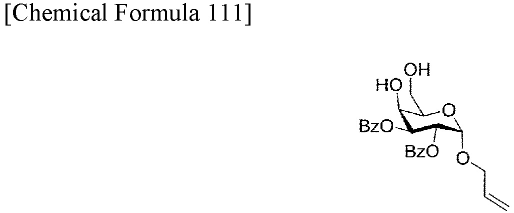
to α-2,6-glycosidic linkage to give the compound represented by formula IX-1.

42. A method for producing a compound represented by the following formula IX-1: 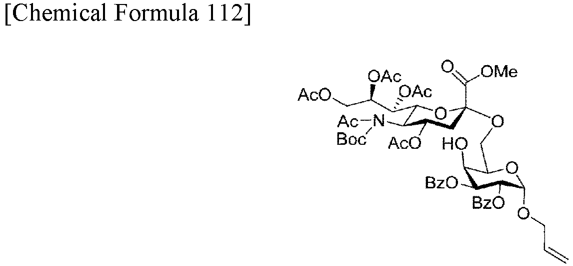 the method comprising a step of
bringing a solvent dissolving the compound represented by formula IX-1 into contact with silica gel to perform solid-phase extraction of the compound represented by formula IX-1.

43. The method according to claim 42, wherein the solvent dissolving the compound represented by formula IX-1 is selected from the group consisting of toluene, dichloromethane, chloroform, and a combination thereof.

44. A method for producing a compound represented by the following formula IX-3: 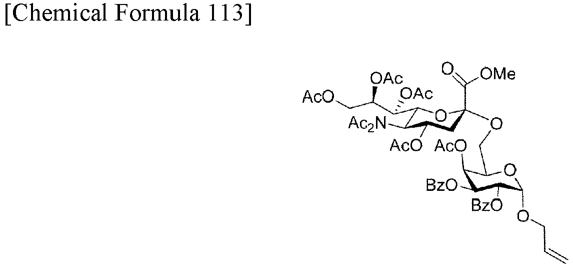 the method comprising a step of
bringing a solvent dissolving the compound represented by formula IX-3 into contact with silica gel to perform solid-phase extraction of the compound represented by formula IX-3.

45. The method according to claim 44, wherein the solvent dissolving the compound represented by formula IX-3 is selected from the group consisting of toluene, dichloromethane, chloroform, and a combination thereof.

46. A method for producing an oligosaccharide represented by the following formula XI: 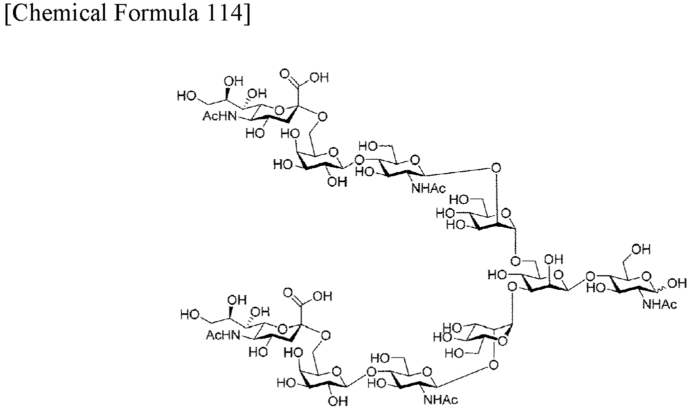 the method comprising a method according to any one of claims 23 to 45.

47. A compound represented by the following formula III-6:

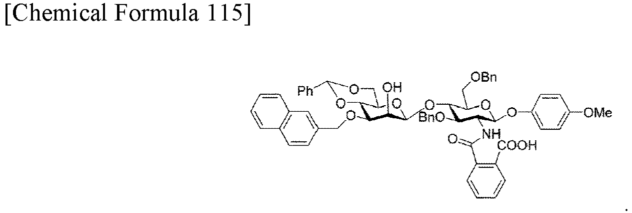

48. A compound represented by the following formula III-10:

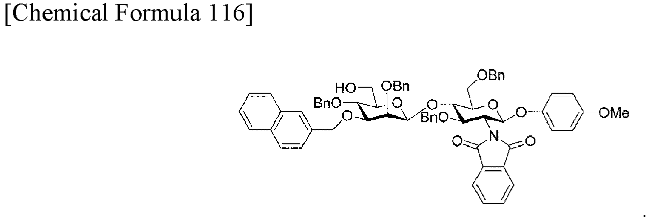

49. A compound represented by the following formula V-1:

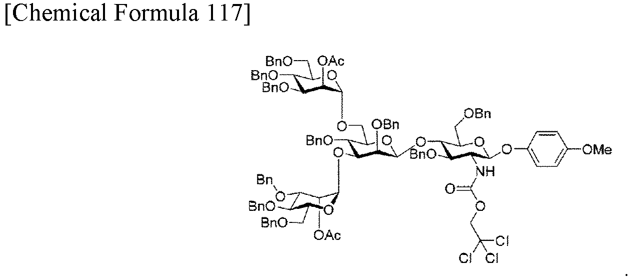

50. A compound represented by the following formula V-2:

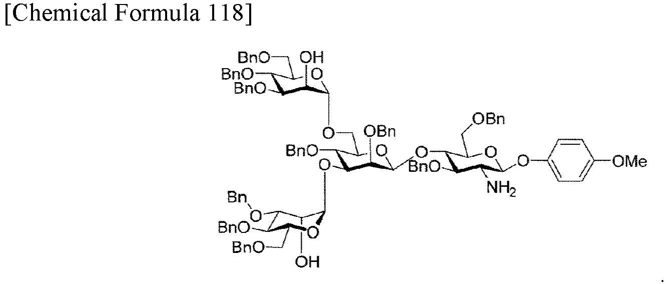

51. A compound represented by the following formula V-3:

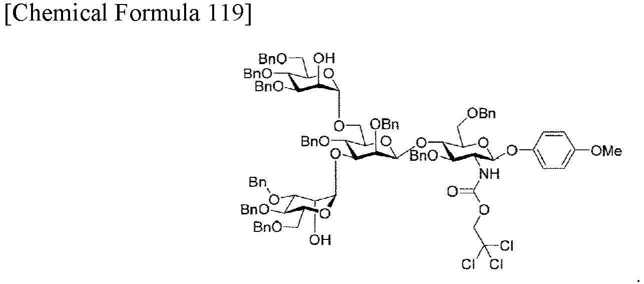

52. A compound represented by the following formula II-6:

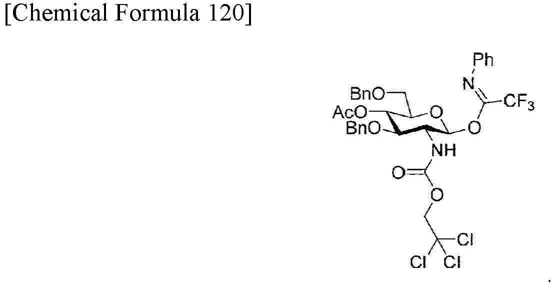

53. A compound represented by the following formula VI-A: 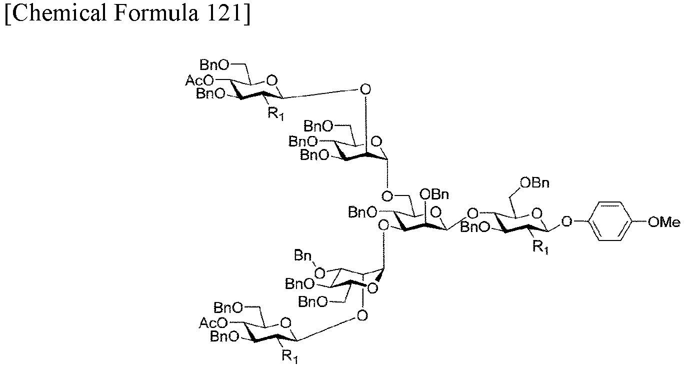 wherein R₁ represents a group selected from the group consisting of

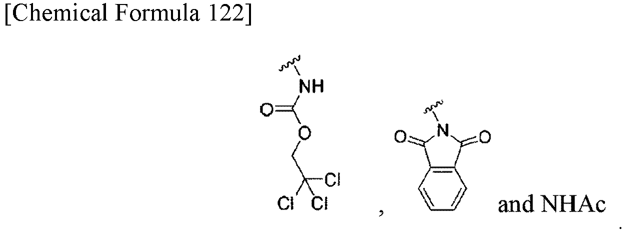

54. A compound represented by the following formula VI-2:

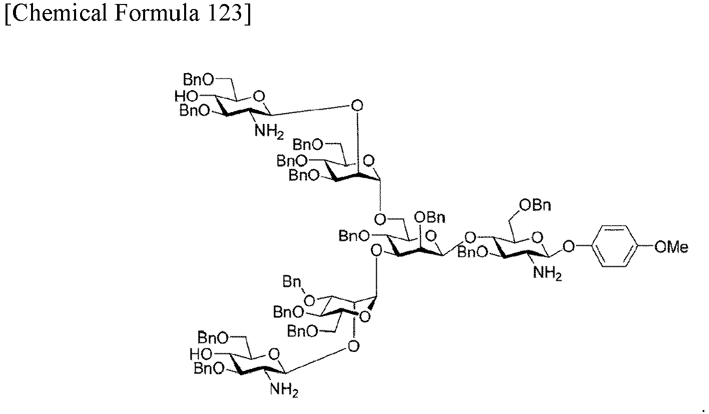

55. A compound represented by the following formula VI-B: 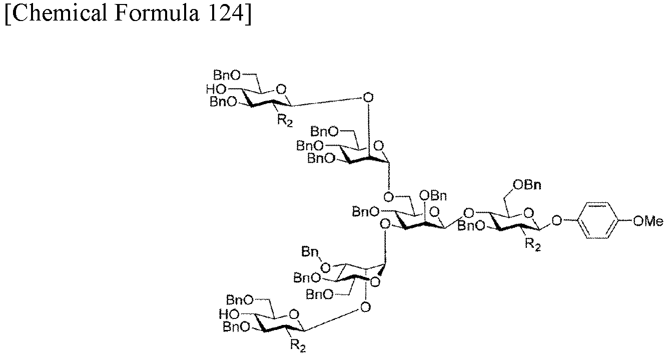 wherein R₂ represents a group selected from the group consisting of

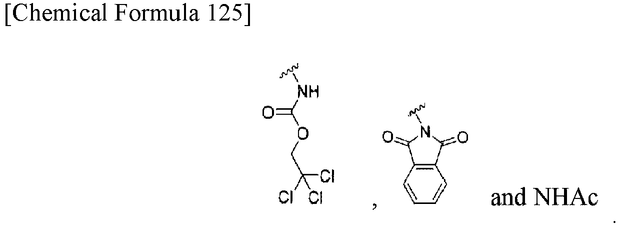

56. A compound represented by the following formula VIII-5:

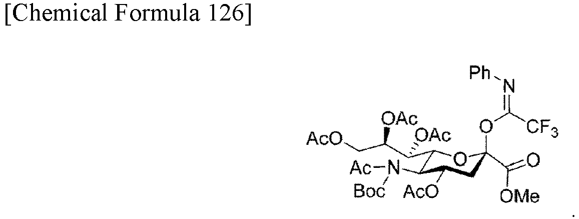

57. A compound represented by the following formula X-1:

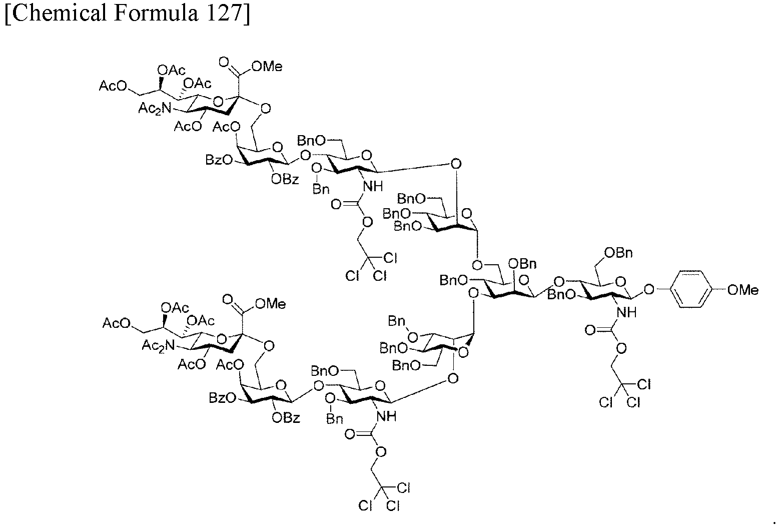

58. A compound represented by the following formula X-2:

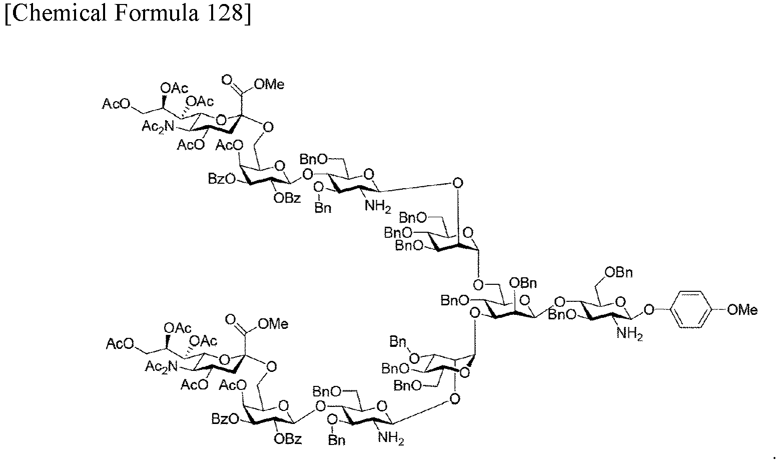

59. A compound represented by the following formula X-3:

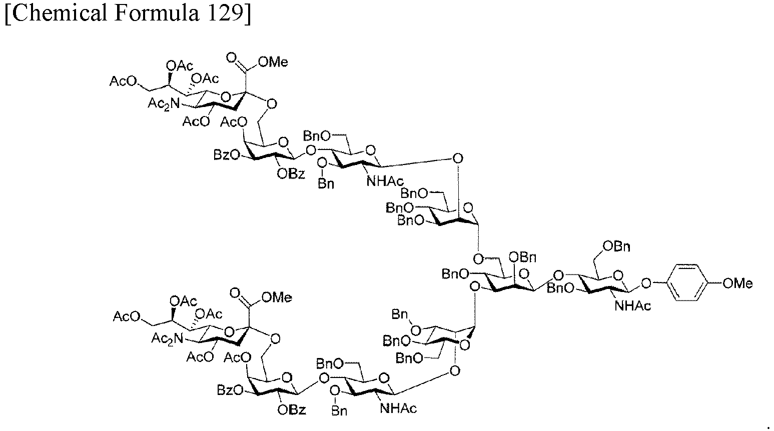

60. A compound represented by the following formula X-4:

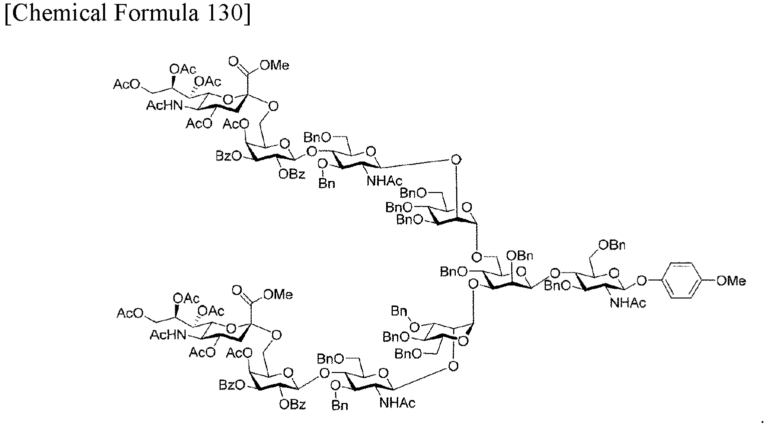

61. A compound represented by the following formula X-5:

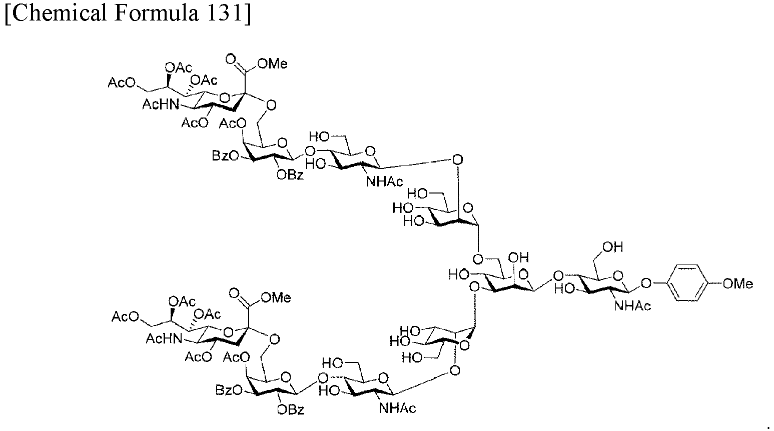

62. A compound represented by the following formula X-6:

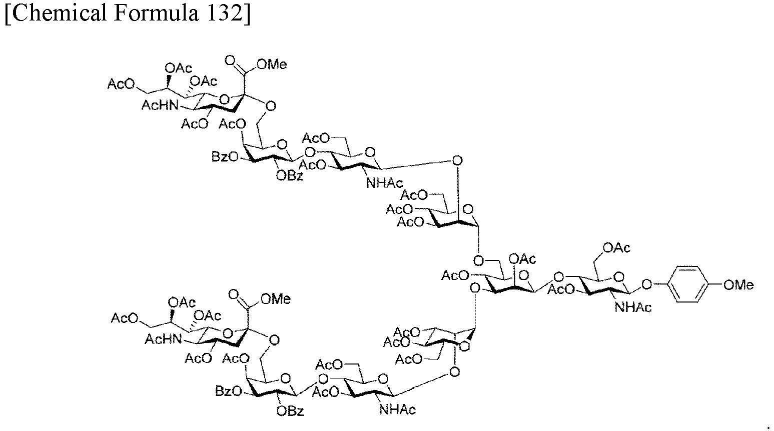

63. A compound represented by the following formula X-7:

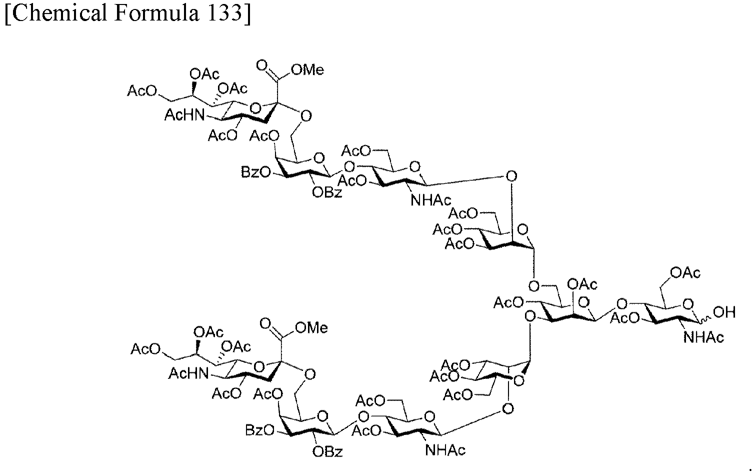

64. A method for producing a glycan-remodeled antibody or a molecule containing an Fc region thereof,
the method comprising a method according to any one of claims 1 to 22 and 46, the method further comprising steps of
obtaining, from the obtained oligosaccharide represented by formula XI, a glycan donor molecule containing N-acetylglucosamine (GlcNAc) with a reducing end activated; and
reacting the glycan donor molecule with an acceptor molecule which is an antibody with core GlcNAc optionally having a fucose as an N297-binding glycan or a molecule containing an Fc region thereof.

65. The method according to claim 64, wherein the GlcNAc with a reducing end activated is oxazolinated GlcNAc.

66. The method according to claim 64 or 65, wherein the glycan donor molecule is a compound (also referred to as "SG(10)-Ox") optionally having a chemically modified non-reducing end and represented by the following formula XII:

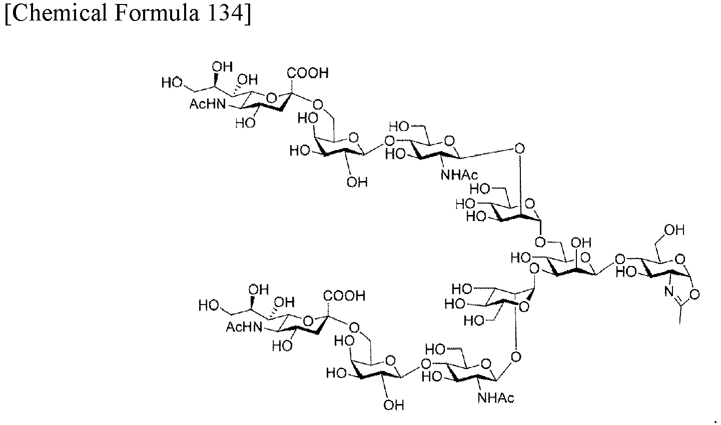

67. The method according to any one of claims 64 to 66, wherein the glycan donor molecule is [N₃-PEG(3)]₂-SG(10)-Ox.

68. The method according to claim 67, further comprising a step of reacting the azide group (N₃-) with a molecule having an alkyne structure.

69. The method according to claim 68, wherein the molecule having an alkyne structure is selected from the group consisting of a chemotherapeutic agent, molecular targeted drug, immune activator, toxin, antimicrobial agent, antiviral agent, diagnostic agent, protein, peptide, amino acid, nucleic acid, antigen, vitamin, and hormone.

70. A method for producing an antibody-drug conjugate, comprising the method according to any one of claims 64 to 69.
